# EUROPEAN PATENT APPLICATION

(11) **EP 4 353 727 A1**
(43) Date of publication of application: **17.04.2024**
(21) Application number: 22201269.2
(22) Date of filing: 13.10.2022
(51) Int. Cl.: C07D 491/04, A61K 31/33, A61P 35/00

(54) **SUBSTITUTED FUROPYRIDINES FOR THERAPEUTIC USE**

(71) Applicant: Masarykova Univerzita, 60177 Brno (CZ)
(72) Inventor: Paruch, Kamil, Tisnov (CZ); Remes, Marek, Otmarov (CZ); Nemec, Vaclav, Brno (CZ); Boeck, Michael Christopher, Brno (CZ); Benovsky, Petr, Sazava (CZ)
(74) Representative: Harber IP s.r.o.

(57) **Abstract**

The invention relates to furopyridine compounds of formula I: 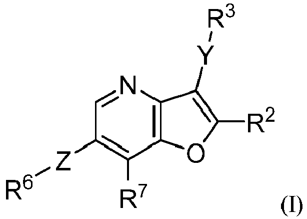 for use in the treatment of FLT3-related, DDR-related and MAP4K-related diseases.

## Description

### Field of Art

The present invention relates to new heterocyclic compounds useful for therapeutic use based on inhibition of FLT3, DDR1 and KHS/MAP4K5 kinases.

### Background Art

Protein kinase-mediated phosphorylation of proteins is central for activation and deactivation of numerous signaling pathways in the cell.

FMS-like tyrosine kinase 3 (FLT3) is a receptor tyrosine kinase that is expressed almost exclusively in the hematopoietic compartment. Its ligand, FLT3 ligand (FL), induces dimerization and activation of its intrinsic tyrosine kinase activity. Activation of FLT3 leads to its autophosphorylation and initiation of several signal transduction cascades. Activation of FLT3 mediates cell survival, cell proliferation, and differentiation of hematopoietic progenitor cells (Physiological Reviews 2019, 99, 1433.). In particular, inhibition of FLT3 has been recognized as an attractive strategy for the treatment of acute myeloid leukemia and numerous FLT3 inhibitors have been clinically profiled (Leukemia 2019, 33, 299.). Overactivity of FLT3 has also been implicated in autoimmune diseases, such as rheumatoid arthritis or autoimmune hepatitis (Hepatology Forum 2021, 2, 112.); and its inhibition has been suggested as a therapeutic approach (Proceedings of the National Academy of Sciences of the United States of America 2005, 102, 16741.). In addition, inhibition of FLT3 could be useful for the treatment of peripheral neuropathic pain (Nature Communications 2018, 9:1042.).

The discoidin domain receptor (DDR) kinase plays critical roles in regulating essential cellular processes such as morphogenesis, differentiation, proliferation, adhesion, migration, invasion, and matrix remodeling. Dysregulation of DDR has been attributed to a variety of human cancer disorders, e.g. non-small-cell lung carcinoma (NSCLC), ovarian cancer, glioblastoma, and breast cancer. Numerous studies have shown that elevated DDR expression levels and/or mutations can be found in a number of cancer cell lines as well as primary tumor tissues, e.g. lung, pancreas, prostate, breast, brain, ovary, and liver (International Journal of Molecular Sciences 2021, 22, 6535. and references therein). DDR1 was found to be a prognostic marker for non-small-cell lung carcinoma (NSCLC) patients. A clinicopathological parameter analysis in NSCLC patients presented a significant connection between DDR1 overexpression and lymph node metastasis (British Journal of Cancer 2007, 96, 808.) Furthermore, overexpression of DDR1 has been linked to collective cell invasion, which is a common pathological feature in many cancer types and likely a critical step in cancer metastasis. Specifically, it has been demonstrated that angiolymphatic invasion can be suppressed via pharmacological inhibition *in vivo* in oral squamous cell carcinoma mouse model, suggesting that DDR1 inhibitors could be used for the treatment of oral cancer (Cancers 2020, 12, 841.).

In addition, DDR dysregulation can be related to some inflammatory and neurodegenerative disorders. Along this line, recent studies indicate that inhibition of DDR has therapeutic potential for the treatment of periodontitis (Journal of Cellular Physiology 2022, 237, 189.) and pulmonary fibrosis (Acta Pharmacologica Sinica 2022, 43, 1769. and Acta Pharmaceutica Sinica B 2022, 12, 1943.). Correspondingly, significant efforts have been invested into the development of DDR inhibitors (Biomolecules 2021, 11, 1671.).

MAP kinases (MAP4Ks) belong to the mammalian Ste20-like family of serine/threonine kinases. MAP4Ks including MAP4K1/HPK1, MAP4K2/GCK, MAP4K3/GLK, MAP4K4/HGK, MAP4K5/KHS, and MAP4K6/MINK play diverse roles in immune cell signaling, immune responses, and inflammation (Advances in Immunology 2016, 129, 277.). MAP4Ks have been directly implicated in numerous diverse disorders, including obesity (Molecular and Cellular Biology 2015, 35, 2356.), insulin resistance (ACSMedicinal Chemistry Letters 2015, 6, 1128. and Journal of Biological Chemistry 2007, 282, 7783.) and obesity-induced hyperinsulinemia (Journal of Biological Chemistry 2016, 291, 16221.), and atherosclerosis (Trends in Endocrinology & Metabolism 2016, 27, 484.).

Aberrant expression/splicing of these kinases is also involved in hepatocellular carcinoma (World Journal of Gastroenterology 2010, 16, 4541.), lung adenocarcinoma (Pathology - Research and Practice 2012, 208, 541.), colorectal cancer (Biochimica et Biophysica Acta -Molecular Cell Research 2018, 1865, 259.), and in transformation and metastatic processes in other human tumor cells (Molecular and Cellular Biology 2003, 23, 2068.).

Inhibition of MAP4Ks can elicit neuroprotective and anti-inflammatory effects, which can be potentially used for the treatment of Alzheimer's disease and other neurodegenerative disorders (Cell Chemical Biology 2019, 26, 1703.).

KHS/MAP4K5 specifically plays an important role in regulating a range of cellular responses; and targeting impaired KHS/MAP4K5 signaling has been suggested as a new therapeutic strategy for pancreatic cancer (PLoS ONE 2016, 11(3): e0152300). Dual inhibition of kinases TAOK1 and KHS/MAP4K5 was found to cause potent inhibition of colorectal and lung cancer cell lines (Journal of Enzyme Inhibition and Medicinal Chemistry 2021, 36, 98.). In addition, the compound AZD4547, targeting MAP4K3, MAP4K5, IRR, RET, and FLT3, exhibits anticancer properties (Molecular Cancer Therapeutics 2015, 14, 2292).

Furopyridine pattern has not been used for inhibition of MAPKs or DDR. 3,5-disubstituted furo[3,2-*b*]pyridines have been reported to be moderately potent inhibitors of kinase FLT3 (WO 2015/165428 A1).

### Summary of the Invention

The present invention relates to compounds of general formula I or pharmaceutically acceptable salts thereof 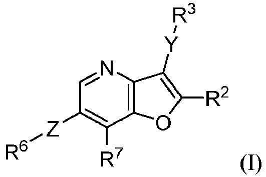 wherein:
R² is selected from H, C1-C4 alkyl and CF₃;
Y is selected from bond, O, S, SO₂, NR⁸ and CR⁸R⁸;
R³ is selected from the group consisting of:
   - C6-C14 aryl,
   - 3-10-membered heteroaryl comprising at least one heteroatom selected from S, O, N; provided that the heteroaryl is not unsubstituted quinoline or unsubstituted isoquinoline;
   wherein each of the listed substituents can optionally be substituted by at least one substituent selected independently from C1-C4 alkyl, C6-C10 aryl, 3-7-membered heteroaryl comprising at least one heteroatom selected from S, O, N, 3-7-membered cycloheteroalkyl comprising at least one heteroatom selected from S, O, N, halogen, OH, HO-C1-C4 alkyl, O(C1-C4 alkyl), O(C3-C7 cycloalkyl), O(C1-C4 halogenalkyl), (C1-C4 alkyl)-O-C1-C4 alkyl, O(C5-C6 aryl or 5-6-membered heteroaryl), SH, S(C1-C4 alkyl), S(C3-C7 cycloalkyl), S(C1-C4 halogenalkyl), S(C5-C6 aryl or 5-6-membered heteroaryl), SO(C1-C4 alkyl), SO₂(C1-C4 alkyl), CF₃, C₂F₅, OCF₃, OC₂F₅, amino (NH₂), NH₂-(C1-C4 alkyl)-, HCO-NH-(C1-C4 alkyl)-, NO₂, CN, N₃, C1-C4 alkylamino, di(C1-C4 alkyl)amino, (C5-C6 aryl or 5-6-membered heteroaryl)amino, di(C5-C6 aryl or heteroaryl)amino, (C1-C4 alkyl)-NH-C1-C4 alkyl, (C1-C4 alkyl)₂-N-C1-C4-alkyl, =O, =S, =N-OH, -(C1-C4 alkylene)=N-OH, =N-O(C1-C4 alkyl), -(C1-C4 alkylene)=N-O(C1-C4 alkyl), -(C1-C4 alkylene)-CHO, -CHO, -COOH, -(C1-C4 alkylene)-COOH, -CONH₂, -(C1-C4 alkylene)-CONH₂, -COO(C1-C4 alkyl), -(C1-C4 alkylene)-COO(C1-C4 alkyl), -CO(C1-C4 alkyl), -(C1-C4 alkylene)-CO(C1-C4 alkyl), -CO(C5-C6 aryl or 5-6-membered heteroaryl), -(C1-C4 alkylene)-CO(C5-C6 aryl or 5-6-membered heteroaryl), (C1-C4 alkyl)-SO₂-, (C1-C4 alkyl)-SO₂-(C1-C4 alkylene)-, (C1-C4 alkyl)-SO-, (C1-C4 alkyl)-SO-(C1-C4 alkylene)-, (C1-C4 alkyl)-SO₂-NH-, (C1-C4 alkyl)-SO₂-NH-(C1-C4 alkylene)-, (C1-C4 alkyl)-SO₂-N(C1-C4 alkyl)-, (C1-C4 alkyl)-SO₂-N(C1-C4 alkyl)-(C1-C4 alkylene)-, (C1-C4 alkyl)-O-CO-, (C1-C4 alkyl)-O-CO-(C1-C4 alkylene)-, (C1-C4 alkyl)-NH-CO-, (C1-C4 alkyl)-NH-CO-(C1-C4 alkylene)-, (C6-C10 aryl)-NH-CO, (C6-C10 aryl)-NH-CO-(C1-C4 alkylene)-, (C1-C4 alkyl)₂N-CO-, (C1-C4 alkyl)₂N-CO-(C1-C4 alkylene)-, NH₂-SO₂-, NH₂-SO₂-(C1-C4-alkylene)-, (C6-C10 aryl)-NH-SO₂-, (C6-C10 aryl)-NH-SO₂-(C1-C4 alkylene)-, (C1-C4 alkyl)-NH-SO₂-, (C1-C4 alkyl)-NH-SO₂-(C1-C4 alkylene)-, (C1-C4 alkyl)₂N-SO₂-, (C1-C4 alkyl)₂N-SO₂-(C1-C4 alkylene)-, (C1-C4 alkyl)-CO-NH-, (C1-C4 alkyl)-CO-NH-(C1-C4 alkylene)-, (C1-C4 alkyl)-CO-N(C1-C4 alkyl)-, (C1-C4 alkyl)-CO-N(C1-C4 alkyl)-(C1-C4 alkylene)-, (C1-C4 alkyl)-OCO-NH-, (C1-C4 alkyl)-OCO-NH-(C1-C4 alkylene)-, (C1-C4 alkyl)-OCO-N(C1-C4 alkyl)-, (C1-C4 alkyl)-OCO-N(C1-C4 alkyl)-(C1-C4 alkylene)-, (C1-C4 alkyl)-CO-NH-CO-, (C1-C4 alkyl)-CO-N(C1-C4 alkyl)-CO-, NH₂-CO-NH-, (C1-C4 alkyl)-NH-CO-NH-, (C1-C4 alkyl)₂N-CO-NH-, NH₂-CO-N(C1-C4 alkyl)-, (C1-C4 alkyl)-NH-CO-N(C1-C4 alkyl)-, (C1-C4 alkyl)₂N-CO-N(C1-C4 alkyl)-, NH₂-S(O)₂-NH-, (C1-C4 alkyl)-NH-S(O)₂-NH-, (C1-C4 alkyl)₂N-S(O)₂-NH-, NH₂-S(O)₂-N(C1-C4 alkyl)-, (C1-C4 alkyl)-NH-S(O)₂-N(C1-C4 alkyl)-, (C1-C4 alkyl)₂N-S(O)₂-N(C1-C4 alkyl)-, (C1-C4 alkyl)₂N-(C1-C4 alkylene)-CO-, (C1-C4 alkyl)₂N-(C1-C4 alkylene)-SO₂-, (C1-C4 alkyl)₂N-(C1-C4 alkylene)-SO₂-NH-, (C1-C4 alkyl)₂N-(C1-C4 alkylene)-NH-SO₂-;
Z is selected from bond, O, S, SO₂, NR⁸ and CR⁸R⁸;
R⁶ is selected from the group consisting of
   - C6-C14 aryl,
   - 3-10-membered heteroaryl comprising at least one heteroatom selected from S, O, N,
   - C3-C8 cycloalkyl, preferably, C4-C7 cycloalkyl,
   - 3-8-membered cycloheteroalkyl comprising 1-2 heteroatom(s) selected from S, O, N,
   - C3-C8 cycloalkenyl,
   - 3-8-membered cycloheteroalkenyl comprising 1-2 heteroatom(s) selected from S, O, N,
   - (C3-C8)cycloalkyl-(C6-C14)aryl,
   - (3-8-membered)cycloheteroalkyl-(C6-C14)aryl, wherein the cycloheteroalkyl comprises 1-2 heteroatom(s) selected from S, O, N,
   - (C3-C8)cycloalkenyl-(C6-C14)aryl,
   - (3-8-membered)cycloheteroalkenyl-(C6-C14)aryl, wherein the cycloheteroalkenyl comprises 1-2 heteroatom(s) selected from S, O, N,
   - (C3-C8)cycloalkyl-(3-10-membered)heteroaryl, wherein the heteroaryl comprises at least one heteroatom selected from S, O, N,
   - (3-8-membered)cycloheteroalkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
   - (C3-C8)cycloalkenyl-(3-10-membered)heteroaryl, wherein the heteroaryl comprises at least one heteroatom selected from S, O, N
   - (3-8-membered)cycloheteroalkenyl-(3-10-membered)heteroaryl, wherein the cycloheteroalkenyl comprises 1-2 heteroatom(s) selected from S, O, N, and wherein the heteroaryl comprises at least one heteroatom selected from S, O, N,
   - (C3-C8)cycloalkyl-(C1-C4)alkyl-(C6-C14)aryl,
   - (3-8-membered)cycloheteroalkyl-(C1-C4)alkyl-(C6-C14)aryl, wherein the cycloheteroalkyl comprises 1-2 heteroatom(s) selected from S, O, N,
   - (C3-C8)cycloalkenyl-(C1-C4)alkyl-(C6-C14)aryl,
   - (3-8-membered)cycloheteroalkenyl-(C1-C4)alkyl-(C6-C14)aryl, wherein the cycloheteroalkenyl comprises 1-2 heteroatom(s) selected from S, O, N,
   - (C3-C8)cycloalkyl-(C1-C4)alkyl-(3-10-membered)heteroaryl, wherein the heteroaryl comprises at least one heteroatom selected from S, O, N,
   - (3-8-membered)cycloheteroalkyl-(C1-C4)alkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
   - (C3-C8)cycloalkenyl-(C1-C4)alkyl-(3-10-membered)heteroaryl, wherein the heteroaryl comprises at least one heteroatom selected from S, O, N
   - (3-8-membered)cycloheteroalkenyl-(C1-C4)alkyl-(3-10-membered)heteroaryl, wherein the cycloheteroalkenyl comprises 1-2 heteroatom(s) selected from S, O, N, and wherein the heteroaryl comprises at least one heteroatom selected from S, O, N,
   - (C3-C8)cycloalkyl-(1-4-membered)heteroalkyl-(C6-C14)aryl,
   - (3-8-membered)cycloheteroalkyl-(1-4-membered)heteroalkyl-(C6-C14)aryl, wherein the cycloheteroalkyl comprises 1-2 heteroatom(s) selected from S, O, N,
   - (C3-C8)cycloalkenyl-(1-4-membered)heteroalkyl-(C6-C14)aryl,
   - (3-8-membered)cycloheteroalkenyl-(1-4-membered)heteroalkyl-(C6-C14)aryl, wherein the cycloheteroalkenyl comprises 1-2 heteroatom(s) selected from S, O, N,
   - (C3-C8)cycloalkyl-(1-4-membered)heteroalkyl-(3-10-membered)heteroaryl, wherein the heteroaryl comprises at least one heteroatom selected from S, O, N,
   - (3-8-membered)cycloheteroalkyl-(1-4-membered)heteroalkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
   - (C3-C8)cycloalkenyl-(1-4-membered)heteroalkyl-(3-10-membered)heteroaryl, wherein the heteroaryl comprises at least one heteroatom selected from S, O, N
   - (3-8-membered)cycloheteroalkenyl-(1-4-membered)heteroalkyl-(3-10-membered)hetero aryl, wherein the cycloheteroalkenyl comprises 1-2 heteroatom(s) selected from S, O, N, and wherein the heteroaryl comprises at least one heteroatom selected from S, O, N,
   - (5-10 membered)heteroalkyl-C6-C14 aryl, wherein the heteroalkyl comprises 1-2 heteroatom(s) selected from S, O, N,
   - (5-10 membered)heteroalkyl-(3-10-membered)heteroaryl wherein the heteroalkyl comprises 1-2 heteroatom(s) selected from S, O, N, and wherein the heteroaryl comprises at least one heteroatom selected from S, O, N,
wherein each of the listed substituents can be unsubstituted or substituted by at least one substituent selected independently from C1-C4 alkyl, C6-C10 aryl, 3-7-membered heteroaryl comprising at least one heteroatom selected from S, O, N, 3-7-membered cycloheteroalkyl comprising at least one heteroatom selected from S, O, N, halogen, OH, HO-C1-C4 alkyl, O(C1-C4 alkyl), O(C3-C7 cycloalkyl), O(C1-C4 halogenalkyl), (C1-C4 alkyl)-O-C1-C4 alkyl, O(C5-C6 aryl or 5-6-membered heteroaryl), SH, S(C1-C4 alkyl), S(C3-C7 cycloalkyl), S(C1-C4 halogenalkyl), S(C5-C6 aryl or 5-6-membered heteroaryl), SO(C1-C4 alkyl), SO₂(C1-C4 alkyl), CF₃, C₂F₅, OCF₃, OC₂F₅, amino (NH₂), NH₂-(C1-C4 alkyl)-, HCO-NH-(C1-C4 alkyl)-, NO₂, CN, N₃, C1-C4 alkylamino, di(C1-C4 alkyl)amino, (C5-C6 aryl or 5-6-membered heteroaryl)amino, di(C5-C6 aryl or heteroaryl)amino, (C1-C4 alkyl)-NH-C1-C4 alkyl, (C1-C4 alkyl)₂-N-C1-C4-alkyl, =O, =S, =N-OH, -(C1-C4 alkylene)=N-OH, =N-O(C1-C4 alkyl), -(C1-C4 alkylene)=N-O(C1-C4 alkyl), -(C1-C4 alkylene)-CHO, -CHO, -COOH, -(C1-C4 alkylene)-COOH, -CONH₂, -(C1-C4 alkylene)-CONH₂, -COO(C1-C4 alkyl), -(C1-C4 alkylene)-COO(C1-C4 alkyl), -CO(C1-C4 alkyl), -(C1-C4 alkylene)-CO(C1-C4 alkyl), -CO(C5-C6 aryl or 5-6-membered heteroaryl), -(C1-C4 alkylene)-CO(C5-C6 aryl or 5-6-membered heteroaryl), (C1-C4 alkyl)-SO₂-, (C1-C4 alkyl)-SO₂-(C1-C4 alkylene)-, (C1-C4 alkyl)-SO-, (C1-C4 alkyl)-SO-(C1-C4 alkylene)-, (C1-C4 alkyl)-SO₂-NH-, (C1-C4 alkyl)-SO₂-NH-(C1-C4 alkylene)-, (C1-C4 alkyl)-SO₂-N(C1-C4 alkyl)-, (C1-C4 alkyl)-SO₂-N(C1-C4 alkyl)-(C1-C4 alkylene)-, (C1-C4 alkyl)-O-CO-, (C1-C4 alkyl)-O-CO-(C1-C4 alkylene)-, (C1-C4 alkyl)-NH-CO-, (C1-C4 alkyl)-NH-CO-(C1-C4 alkylene)-, (C6-C10 aryl)-NH-CO, (C6-C10 aryl)-NH-CO-(C1-C4 alkylene)-, (C1-C4 alkyl)₂N-CO-, (C1-C4 alkyl)₂N-CO-(C1-C4 alkylene)-, NH₂-SO₂-, NH₂-SO₂-(C1-C4-alkylene)-, (C6-C10 aryl)-NH-SO₂-, (C6-C10 aryl)-NH-SO₂-(C1-C4 alkylene)-, (C1-C4 alkyl)-NH-SO₂-, (C1-C4 alkyl)-NH-SO₂-(C1-C4 alkylene)-, (C1-C4 alkyl)₂N-SO₂-, (C1-C4 alkyl)₂N-SO₂-(C1-C4 alkylene)-, (C1-C4 alkyl)-CO-NH-, (C1-C4 alkyl)-CO-NH-(C1-C4 alkylene)-, (C1-C4 alkyl)-CO-N(C1-C4 alkyl)-, (C1-C4 alkyl)-CO-N(C1-C4 alkyl)-(C1-C4 alkylene)-, (C1-C4 alkyl)-OCO-NH-, (C1-C4 alkyl)-OCO-NH-(C1-C4 alkylene)-, (C1-C4 alkyl)-OCO-N(C1-C4 alkyl)-, (C1-C4 alkyl)-OCO-N(C1-C4 alkyl)-(C1-C4 alkylene)-, (C1-C4 alkyl)-CO-NH-CO-, (C1-C4 alkyl)-CO-N(C1-C4 alkyl)-CO-, NH₂-CO-NH-, (C1-C4 alkyl)-NH-CO-NH-, (C1-C4 alkyl)₂N-CO-NH-, NH₂-CO-N(C1-C4 alkyl)-, (C1-C4 alkyl)-NH-CO-N(C1-C4 alkyl)-, (C1-C4 alkyl)₂N-CO-N(C1-C4 alkyl)-, NH₂-S(O)₂-NH-, (C1-C4 alkyl)-NH-S(O)₂-NH-, (C1-C4 alkyl)₂N-S(O)₂-NH-, NH₂-S(O)₂-N(C1-C4 alkyl)-, (C1-C4 alkyl)-NH-S(O)₂-N(C1-C4 alkyl)-, (C1-C4 alkyl)₂N-S(O)₂-N(C1-C4 alkyl)-, (C1-C4 alkyl)₂N-(C1-C4 alkylene)-CO-, (C1-C4 alkyl)₂N-(C1-C4 alkylene)-SO₂-, (C1-C4 alkyl)₂N-(C1-C4 alkylene)-SO₂-NH-, (C1-C4 alkyl)₂N-(C1-C4 alkylene)-NH-SO₂-;
R⁷ is selected from H, C1-C4 alkyl and CF₃;
R⁸ is independently selected from H and C1-C4 alkyl.

The compounds of formula I can be in the form of free bases or in the form of addition salts with pharmaceutically acceptable organic or inorganic acids, such as hydrochloric acid.

Halogens are selected from fluorine, chlorine, bromine and iodine.

Alkyl is a branched or linear saturated hydrocarbyl.

Alkenyl is a branched or linear hydrocarbyl comprising at least one double bond.

Alkylene is a divalent branched or linear, preferably linear, hydrocarbyl residue. A preferred alkylene is methylene.

Cycloalkyl is a saturated hydrocarbyl comprising at least one aliphatic cycle.

Cycloalkenyl is a hydrocarbyl comprising at least one aliphatic cycle and at least one double bond in the cycle.

Aryl is a hydrocarbyl comprising at least one aromatic cycle. Examples of aryl are phenyl, benzyl. Heteroaryl is a heterohydrocarbyl comprising at least one aromatic cycle comprising at least one heteroatom selected from O, S, N.

Heterocyclyl or cycloheteroalkyl is a heterohydrocarbyl comprising at least one aliphatic cycle which contains at least one heteroatom selected from O, S, N in the cycle.

Cycloheteroalkenyl is a heterohydrocarbyl comprising at least one double bond and at least one aliphatic cycle which contains at least one heteroatom selected from O, S, N in the cycle.

Cyclic structures can thus contain one or more cycles, wherein the cycles can be conjugated or connected by a C1-C3 linker.

Preferably R² is selected from H, methyl, ethyl, propyl and isopropyl. More preferably, R² is selected from H and methyl.

Preferably, Y is selected from bond, NR⁸ or CR⁸R⁸. More preferably, Y is selected from bond, NH and CH₂. Even more preferably, Y is a bond.

R³ is preferably selected from phenyl and 5-6 membered heteroaryls containing one or two heteroatoms selected from N, O, S; wherein the phenyl or 5-6 membered heteroaryl is unsubstituted or substituted as described above for R³.

Preferably, Z is selected from bond, NR⁸ or CR⁸R⁸. More preferably, Z is selected from bond, NH and CH₂. Even more preferably, Z is a bond.

R⁶ is preferably selected from the group consisting of
- C6-C14 aryl,
- 3-10-membered heteroaryl comprising at least one heteroatom selected from S, O, N,
- (C3-C8)cycloalkyl-(C6-C14)aryl,
- (3-8-membered)cycloheteroalkyl-(C6-C14)aryl, wherein the cycloheteroalkyl comprises 1-2 heteroatom(s) selected from S, O, N,
- (C3-C8)cycloalkyl-(3-10-membered)heteroaryl, wherein the heteroaryl comprises at least one heteroatom selected from S, O, N,
- (3-8-membered)cycloheteroalkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (C3-C8)cycloalkyl-(C1-C4)alkyl-(C6-C14)aryl,
- (3-8-membered)cycloheteroalkyl-(C1-C4)alkyl-(C6-C14)aryl, wherein the cycloheteroalkyl comprises 1-2 heteroatom(s) selected from S, O, N,
- (C3-C8)cycloalkyl-(C1-C4)alkyl-(3-10-membered)heteroaryl, wherein the heteroaryl comprises at least one heteroatom selected from S, O, N,
- (3-8-membered)cycloheteroalkyl-(C1-C4)alkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (C3-C8)cycloalkyl-(1-4-membered)heteroalkyl-(C6-C14)aryl,
- (3-8-membered)cycloheteroalkyl-(1-4-membered)heteroalkyl-(C6-C14)aryl, wherein the cycloheteroalkyl comprises 1-2 heteroatom(s) selected from S, O, N,
- (C3-C8)cycloalkyl-(1-4-membered)heteroalkyl-(3-10-membered)heteroaryl, wherein the heteroaryl comprises at least one heteroatom selected from S, O, N,
- (3-8-membered)cycloheteroalkyl-(1-4-membered)heteroalkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (5-10 membered)heteroalkyl-C6-C14 aryl, wherein the heteroalkyl comprises 1-2 heteroatom(s) selected from S, O, N,
- (5-10 membered)heteroalkyl-(3-10-membered)heteroaryl wherein the heteroalkyl comprises 1-2 heteroatom(s) selected from S, O, N, and wherein the heteroaryl comprises at least one heteroatom selected from S, O, N,
wherein each of the listed substituents can be unsubstituted or substituted.

More preferably, R⁶ is selected from the group consisting of
- phenyl,
- 5-6-membered heteroaryl comprising at least one heteroatom selected from S, O, N,
- (6-membered)cycloheteroalkyl-phenyl, wherein the cycloheteroalkyl comprises 1-2 heteroatom(s) selected from S, O, N,
- (6-membered)cycloheteroalkyl-(5-6-membered)heteroaryl, wherein the cycloheteroalkyl comprises 1-2 heteroatom(s) selected from S, O, N, and wherein the heteroaryl comprises at least one heteroatom selected from S, O, N,
- (6-membered)cycloheteroalkyl-(C1-C4)alkyl-phenyl, wherein the cycloheteroalkyl comprises 1-2 heteroatom(s) selected from S, O, N,
- (6-membered)cycloheteroalkyl-(C1-C4)alkyl-(5-6-membered)heteroaryl, wherein the cycloheteroalkyl comprises 1-2 heteroatom(s) selected from S, O, N, and wherein the heteroaryl comprises at least one heteroatom selected from S, O, N,
- (6-membered)cycloheteroalkyl-(1-4-membered)heteroalkyl-phenyl, wherein the cycloheteroalkyl comprises 1-2 heteroatom(s) selected from S, O, N,
- (6-membered)cycloheteroalkyl-(1-4-membered)heteroalkyl-(5-6-membered)heteroaryl, wherein the cycloheteroalkyl comprises 1-2 heteroatom(s) selected from S, O, N, and wherein the heteroaryl comprises at least one heteroatom selected from S, O, N,
- (5-10 membered)heteroalkyl-phenyl, wherein the heteroalkyl comprises 1-2 heteroatom(s) selected from S, O, N,
- (5-10 membered)heteroalkyl-(5-6-membered)heteroaryl wherein the heteroalkyl comprises 1-2 heteroatom(s) selected from S, O, N, and wherein the heteroaryl comprises at least one heteroatom selected from S, O, N,
wherein each of the listed substituents can be unsubstituted or substituted.

In some embodiments, R⁶ is selected from the group consisting of
- phenyl,
- 5-6-membered heteroaryl comprising at least one heteroatom selected from S, O, N,
- (6-membered)cycloheteroalkyl-phenyl, wherein the cycloheteroalkyl comprises 1-2 heteroatom(s) selected from O, N and is bound to the phenyl via an N-atom,
- (6-membered)cycloheteroalkyl-(5-6-membered)heteroaryl, wherein the cycloheteroalkyl comprises 1-2 heteroatom(s) selected from O, N and is bound to the heteroaryl via an N-atom, and wherein the heteroaryl comprises at least one heteroatom selected from S, O, N,
- (6-membered)cycloheteroalkyl-(C1-C4)alkyl-phenyl, wherein the cycloheteroalkyl comprises 1-2 heteroatom(s) selected from O, N and is bound to the alkyl via an N-atom,
- (6-membered)cycloheteroalkyl-(C1-C4)alkyl-(5-6-membered)heteroaryl, wherein the cycloheteroalkyl comprises 1-2 heteroatom(s) selected from O, N and is bound to the alkyl via an N-atom, and wherein the heteroaryl comprises at least one heteroatom selected from S, O, N,
- (6-membered)cycloheteroalkyl-(1-4-membered)heteroalkyl-phenyl, wherein the cycloheteroalkyl comprises 1-2 heteroatom(s) selected from O, N and is bound to the heteroalkyl via an N-atom,
- (6-membered)cycloheteroalkyl-(1-4-membered)heteroalkyl-(5-6-membered)heteroaryl, wherein the cycloheteroalkyl comprises 1-2 heteroatom(s) selected from O, N and is bound to the heteroalkyl via an N-atom, and wherein the heteroaryl comprises at least one heteroatom selected from S, O, N,
- (5-10 membered)heteroalkyl-phenyl, wherein the heteroalkyl comprises 1-2 heteroatom(s) selected from O, N and is bound to the phenyl via an N-atom,
- (5-10 membered)heteroalkyl-(5-6-membered)heteroaryl wherein the heteroalkyl comprises 1-2 heteroatom(s) selected from S, O, N, and wherein the heteroaryl comprises at least one heteroatom selected from O, N and is bound to the heteroalkyl via an N-atom,
wherein each of the listed substituents can be unsubstituted or substituted.

In some embodiments, R⁶ is selected from the group consisting of
- phenyl,
- 5-6-membered heteroaryl comprising at least one heteroatom selected from S, O, N,
- (6-membered)cycloheteroalkyl-phenyl, wherein the cycloheteroalkyl comprises 1-2 heteroatom(s) selected from O, N and is bound to the phenyl via an N-atom,
- (6-membered)cycloheteroalkyl-(5-6-membered)heteroaryl, wherein the cycloheteroalkyl comprises 1-2 heteroatom(s) selected from O, N and is bound to the heteroaryl via an N-atom, and wherein the heteroaryl comprises at least one heteroatom selected from S, O, N,
- (6-membered)cycloheteroalkyl-(1-4-membered)heteroalkyl-phenyl, wherein the cycloheteroalkyl comprises 1-2 heteroatom(s) selected from O, N and is bound to the heteroalkyl via an N-atom,
- (6-membered)cycloheteroalkyl-(1-4-membered)heteroalkyl-(5-6-membered)heteroaryl, wherein the cycloheteroalkyl comprises 1-2 heteroatom(s) selected from O, N and is bound to the heteroalkyl via an N-atom, and wherein the heteroaryl comprises at least one heteroatom selected from S, O, N,
- (5-10 membered)heteroalkyl-phenyl, wherein the heteroalkyl comprises 1-2 heteroatom(s) selected from O, N and is bound to the phenyl via an N-atom,
- (5-10 membered)heteroalkyl-(5-6-membered)heteroaryl wherein the heteroalkyl comprises 1-2 heteroatom(s) selected from S, O, N, and wherein the heteroaryl comprises at least one heteroatom selected from O, N and is bound to the heteroalkyl via an N-atom,
wherein each of the listed substituents can be unsubstituted or substituted.

In some embodiments, the heteroaryl moiety in R⁶ is pyridyl which can be unsubstituted or substituted.

In some embodiments, the cycloheteroalkyl moiety in R⁶ is selected from piperidinyl, piperazinyl, morpholinyl, each of which can unsubstituted or substituted.

In some embodiments, the 5-10 membered heteroalkyl moiety in R⁶ comprises 1 or 2 nitrogen atoms.

The substituents listed in R⁶ can be unsubstituted or further substituted by at least one substituent, preferably by one substituent. The at least one substituent is preferably selected independently from C1-C4 alkyl, halogen, OH, HO-C1-C4 alkyl, O(C1-C4 alkyl), O(C5-C6 aryl or heteroaryl), SH, S(C1-C4 alkyl), S(C5-C6 aryl or heteroaryl), CF₃, C₂F₅, OCF₃, OC₂F₅, amino (NH₂), C1-C4 alkylamino, di(C1-C4 alkyl)amino. More preferably, the at least one substituent is selected from C1-C4 alkyl.

Preferably R⁷ is selected from H, methyl, ethyl, propyl and isopropyl. More preferably, R⁷ is selected from H and methyl.

In another aspect, the invention provides the use of the compounds of formula I for use as medicaments.

In particular, the compounds of formula I are suitable for use in the treatment of FLT3-related, DDR-related and MAP4K-related diseases.

More specifically, the compounds of formula I are suitable for use in the treatment of leukemia such as acute myeloid leukemia; autoimmune diseases such as rheumatoid arthritis, autoimmune hepatitis, peripheral neuropathic pain; cancers such as non-small-cell lung carcinoma (NSCLC), ovarian cancer, glioblastoma, breast cancer, lung cancer, lung adenocarcinoma, pancreatic cancer, prostate cancer, brain cancer, colorectal cancer, liver cancer, hepatocellular carcinoma, squamous cell carcinoma; periodontitis; pulmonary fibrosis; obesity; insulin resistance; obesity-induced hyperinsulinemia; atherosclerosis; neurodegenerative disorders such as Alzheimer's disease.

The present invention further comprises a pharmaceutical preparation comprising at least one compound of formula I as defined herein, and at least one pharmaceutically acceptable auxiliary substance selected from pharmaceutically acceptable solvents, fillers and binders.

### Examples

### I. Preparative Examples

### Materials and Methods

All commercially available reagents were used as supplied without further purification. The reaction solvents were purchased anhydrous and were stored under nitrogen. Unless noted otherwise, the reactions were carried out in oven-dried glassware under atmosphere of nitrogen. Column chromatography was carried out using silica gel (pore size 60 Å, 230-400 mesh particle size, 40-63 µm particle size). Purification by preparative thin layer chromatography was performed using plates from Merck (PLC Silica gel 60 F₂₅₄, 1 mm). Reverse phase column chromatography was carried out using C₁₈-reversed phase silica gel (pore size 90 Å, 230-400 mesh particle size, 40-63 µm particle size). NMR spectra were obtained in indicated deuterated solvents; chemical shifts are quoted in parts per million (*δ* referenced to the appropriate deuterated solvent employed. Multiplicities are indicated by s (singlet), d (doublet), t (triplet), q (quartet), p (pentet), quin (quintet), sept (septet), m (multiplet) or (br) broad, or combinations thereof. Coupling constant values are given in Hz.

### General procedure A

### General procedure A for Suzuki cross-coupling of 3-bromo-6-chlorofuro[3,2-b]pyridine at position 3.

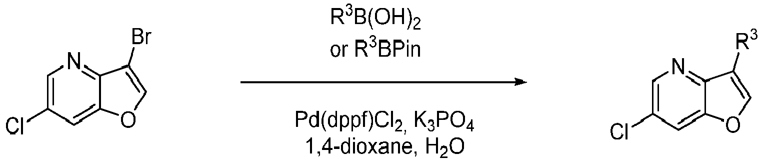

To a degassed solution of 3-bromo-6-chlorofuro[3,2-*b*]pyridine (0.43 mmol), K₃PO₄ (1.29 mmol), and boronic acid or ester (0.56 mmol) in a mixture of 1,4-dioxane/H₂O (4:1; 1.25 mL per 0.1 mmol of 3-bromo-6-chlorofuro[3,2-*b*]pyridine) was added Pd(dppf)Cl₂ (0.013 mmol), and the reaction mixture was stirred at 90 °C; the progress of the reaction was followed by TLC. After consumption of the starting material, the mixture was cooled to 25 °C, diluted with H₂O (10 mL), and extracted with EtOAc (3 × 15 mL). The combined organic extracts were washed with brine (10 mL), dried over MgSO₄ and filtered. The solvent was evaporated *in vacuo* and the residue was purified by flash chromatography

### General procedure B for Suzuki cross-coupling of 6-chloro-3-substituted furo[3,2-b]pyridine at position 6.

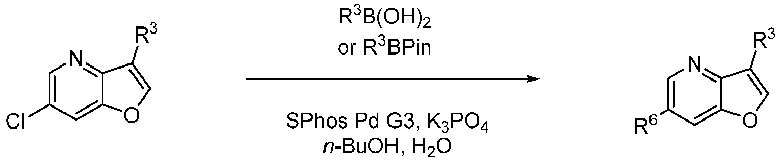

To a degassed solution of 6-chloro-3-substituted furo[3,2-*b*]pyridine (0.229 mmol), K₃PO₄ (0.687 mmol), and boronic acid or ester (0.298 mmol) in a mixture of *n*-BuOH/H₂O (4:1; 1.25 mL per 0.1 mmol of 6-chloro-3-substituted furo[3,2-*b*]pyridine) was added SPhos Pd G3 (0.007 mmol), and the reaction mixture was stirred at 110 °C (the progress of the reaction was followed by TLC). After consumption of the starting material, the mixture was cooled to 25 °C, diluted with H₂O (10 mL), and extracted with EtOAc (3 × 15 mL). The combined organic extracts were washed with brine (10 mL), dried over MgSO₄, and filtered. The solvent was evaporated *in vacuo* and the residue was purified by flash chromatography.

### General procedure C for deprotection of N-Boc-protected compounds

HCl (35% aq., 2 mL, 25.5 mmol) was added to a solution of the respective *N*-Boc-protected compound (0.186 mmol) in MeOH (2 mL) and the reaction mixture was stirred at 50 °C (the progress of the reaction was followed by TLC). After the time indicated for particular reaction, the mixture was cooled to 25 °C. The pH was adjusted to 8 with 2M NaOH (aq., 13 mL) and the resulting solution was extracted with EtOAc (3 × 30 mL). The combined organic extracts were washed with brine (10 mL), dried over MgSO₄, and filtered. The solvent was evaporated *in vacuo* and the residue was purified by flash chromatography.

### General procedure D for deprotection of N-Boc-protected compounds

HCl (35% aq., 2.0 mL) was added to a solution of the respective *N*-Boc-protected compound in MeOH (2.0 mL) and the resulting mixture was stirred at 50°C (the progress of the reaction was followed by TLC). After the time indicated for particular reaction, the mixture was evaporated *in vacuo.* A mixture of DCM/7M NH₃ in MeOH (5.0 mL) and NaHCO₃ (1.0 g, 11.0 mmol) were added; the mixture was stirred at ambient temperature for 1 h and used directly for flash chromatography.

### Preparative Example 1: 5-chloro-2-iodopyridin-3-ol

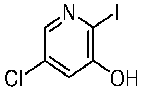

H₂O (80 mL) was added to a mixture of 5-chloropyridin-3-ol (5.12 g, 39.7 mmol), iodine (10.1 g, 39.7 mmol) and Na₂CO₃ (8.83 g, 83.3 mmol), and the resulting mixture was stirred under N₂ at 25 °C for 3.5 hours. The mixture was neutralized with 1 M aqueous solution of HCl (120 mL) and extracted with EtOAc (120 + 70 + 70 mL). The combined organic extracts were washed with brine (80 mL), dried over MgSO₄, filtered, and the solvent was evaporated. The product was obtained as a brown solid (10.1 g; 100 % yield).

¹H NMR (300 MHz, DMSO-*d*₆) *δ* 11.38 (s, 1H), 7.95 (d, *J* = 2.3 Hz, 1H), 7.17 (d, *J* = 2.3 Hz, 1H). ¹³C NMR (126 MHz, DMSO) δ 154.56, 139.48, 130.81, 120.44, 108.53.

FTIR (neat), cm⁻¹: 2843, 2720, 2568, 1744, 1686, 1548, 1411, 1323, 1278, 1241, 1181, 1160, 1113, 1043, 865, 717, 590, 559, 537, 445.

HRMS (APCI): calcd. for C₅H₃ClINO [M+H]⁺ = 255.9021, found [M+H]⁺ = 255.9020

### Preparative Example 2: 6-chloro-2-(trimethylsilyl)furo[3,2-b]pyridine

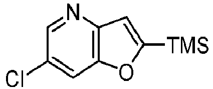

To a degassed solution of 5-chloro-2-iodopyridin-3-ol **(Prep. Example 1;** 5.60 g, 21.9 mmol) in dioxane (30 mL) and TEA (30 mL) were added ethynyltrimethylsilane (4.03 mL, 28.5 mmol), PdCl₂(PPh₃)₂ (461 mg, 0.651 mmol) and CuI (250 mg, 1.31 mmol), and the resulting mixture was stirred at 45 °C for 105 minutes. The solvent was evaporated and the residue was purified by flash chromatography (cyclohexane/EtOAc, gradient from 15:1 to 10:1). The product was obtained as an orange solid (3.59 g, 73 % yield).

¹H NMR (500 MHz, Chloroform-*d*) *δ* 8.52 (dd, *J=* 1.9, 1.4 Hz, 1H), 7.81 - 7.76 (m, 1H), 7.16 - 7.12 (m, 1H), 0.40 (d, *J=* 1.0 Hz, 9H).

¹³C NMR (126 MHz, Chloroform-*d*) *δ* 170.30, 150.58, 146.96, 144.91, 127.25, 118.30, 117.11, -1.92. FTIR (neat), cm⁻¹: 2958, 2918, 2851, 1453, 1381, 1250, 1043, 935, 839, 756, 635, 601.

HRMS (APCI): calcd. for C₁₀H₁₂ClNOSi [M+H]⁺ = 226.0449, found [M+H]⁺ = 226.0458.

### Preparative Example 3: 6-chlorofuro[3,2-b]pyridine

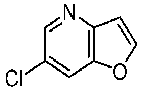

To a solution of 6-chloro-2-(trimethylsilyl)furo[3,2-*b*]pyridine **(Prep. Example 2;** 3.59 g, 15.9 mmol) in methanol (25 mL) was added KF (2.77 mg, 47.7 mmol) and the resulting mixture was stirred at 38 °C for 17 hours. The solvent was evaporated *in vacuo* and the residue was purified by column chromatography (cyclohexane/EtOAc, gradient from 10:1 to 10:3). The product was obtained as a white solid (2.18 g, 89 %).

¹H NMR (500 MHz, Chloroform-*d*) *δ* 8.58 (s, 1H), 7.85 (d, *J=* 2.3 Hz, 1H), 7.83 - 7.78 (m, 1H), 7.03 - 6.96 (m, 1H).

¹³C NMR (126 MHz, Chloroform-*d*) *δ* 149.87, 146.12, 145.39 - 145.20 (m), 118.66, 108.38.

FTIR (neat), cm⁻¹: 3342, 2973, 2926, 1379, 1269, 1087, 1045, 879, 736.

HRMS (APCI): calcd. for C₇H₄ClNO [M+H]⁺ = 154.0054, found = 154.0058.

### Preparative Example 4: 2,3-dibromo-6-chloro-2,3-dihydrofuro[3,2-b]pyridine

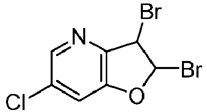

6-chlorofuro[3,2-*b*]pyridine **(Prep. Example 3;** 1.0 g, 6.51 mmol) and DCM (25 mL) were placed into a 100 mL round bottom flask. Bromine (1.0 mL, 19.5 mmol) was added slowly. The mixture was stirred at 25 °C for 3 h. Then, solution of Na₂S₂O₃ (15 mL, 20% aq. with 2 % Na₂CO₃) was added and the resulting mixture was extracted with DCM (3 × 30 mL). The organics were collected, dried over MgSO₄, filtered, and the solvent was evaporated *in vacuo* (while keeping the temperature of bath at 30 °C).

The crude material was purified by flash chromatography (c-hexane/EtOAc, with gradient from 1:0 to 5:1). The product was obtained as a pale beige solid (1.067 g, 52 % yield).

¹H NMR (300 MHz, Chloroform-d) δ 8.37 (d, *J* = 2.0 Hz, 1H), 7.37 (dd, *J=* 1.9, 0.6 Hz, 1H), 6.88 (d, *J=* 1.6 Hz, 1H), 5.68 (d, *J* = 0.6 Hz, 1H).

^{l3}C NMR (75 MHz, CDCl₃) δ 151.18, 146.44, 145.05, 133.78, 120.23, 88.86, 50.61.

### Preparative Example 5: 3-bromo-6-chlorofuro[3,2-b]pyridine

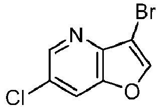

2,3-dibromo-6-chloro-2,3-dihydrofuro[3,2-*b*]pyridine **(Prep. Example 4;** 1.0 g, 3.2 mmol) was dissolved in toluene (15 mL), DBU (1.6 mL, 11.0 mmol) was added and the resulting mixture was stirred at 80 °C for 45 minutes. The solvent was evaporated in *vacuo* and the residue was purified by flash chromatography (c-hexane/EtOAc, gradient from 1:0 to 5:1). The product was obtained as a white solid (514 mg, 69 % yield).

¹H NMR (500 MHz, Chloroform-d) *δ* 8.61 (d, *J=* 2.0 Hz, 1H), 7.89 (s, 1H), 7.82 (d, *J=* 2.0 Hz, 1H). ¹³C NMR (126 MHz, Chloroform-*d*) *δ* 147.40, 147.27, 146.29, 143.52, 128.86, 119.34, 99.71.

FTIR (neat), cm⁻¹: 3094, 3041, 1536, 1457, 1379, 1285, 1074, 995, 910, 875, 772, 603, 496.

HRMS (APCI): calcd. for C₇H₃BrClNO [M+H]⁺ = 231.9159, found = 231.9162.

### Preparative Example 6: 6-chloro-3-(2,6-dimethylpyridin-4-yl)furo[3,2-b]pyridine

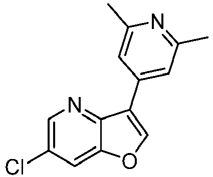

The compound was prepared by the general procedure **A** using 332 mg (1.430 mmol) of 3-bromo-6-chlorofuro[3,2-*b*]pyridine **(Prep. Example 5)** and 400 mg (1.716 mmol) of 2,6-dimethyl-4-(4,4,5,5-tetramethyl-1,3,2-dioxaborolan-2-yl)pyridine; the reaction time was 1 h; flash chromatography (cyclohexane/EtOAc, gradient from 1:0 to 0:1) afforded the compound as a yellow solid (300 mg, 81 % yield).

¹H NMR (500 MHz, Chloroform-d) δ 8.62 (d, *J=* 2.0 Hz, 1H), 8.20 (s, 1H), 7.83 (d, *J=* 2.0 Hz, 1H), 7.62 (s, 2H), 2.60 (s, 6H).

¹³C NMR (126 MHz, CDCl₃) δ 158.56, 148.60, 147.05, 145.65, 143.95, 138.13, 128.12, 120.08, 119.05, 117.94, 77.16, 24.81.

HRMS (APCI): calcd. for C₁₄H₁₁ClN₂O [M+H]⁺ = 259.0633, found = 259.0636.

FTIR (neat), cm⁻¹: 3142, 3019, 2959, 2920, 2850, 1617, 1550, 1383, 1372, 1350, 1277, 1131, 1107, 1081, 910, 896, 880, 846, 806, 782, 597, 561, 546, 525, 456.

### Preparative Example 7: tert-butyl 4-(4-(3-(2,6-dimethylpyridin-4-yl)furo[3,2-b]pyridin-6-yl)phenyl)piperazine-1-carboxylate

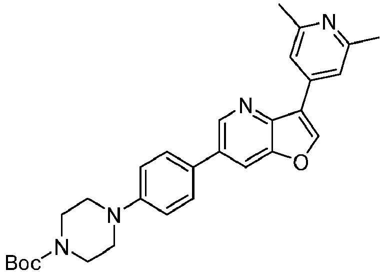

The compound was prepared by the general procedure **B** using 112 mg (0.433 mmol) of 6-chloro-3-(2,6-dimethylpyridin-4-yl)furo[3,2-*b*]pyridine **(Prep. Example 6)** and 202 mg (0.520 mmol) of 4-(4-*tert*-butoxycarbonylpiperazinyl)phenylboronic acid pinacol ester; the reaction time was 1 h; flash chromatography (cyclohexane/EtOAc, gradient from 4:1 to 2:3) afforded the compound as a white solid (122 mg, 58 % yield).

¹H NMR (500 MHz, Chloroform-*d*) δ 8.90 (d, *J* = 1.9 Hz, 1H), 8.22 (s, 1H), 7.93 (d, *J* = 1.9 Hz, 1H), 7.70 (s, 2H), 7.57 (d, *J* = 8.7 Hz, 2H), 7.04 (d, *J* = 8.8 Hz, 2H), 3.66 - 3.54 (m, 4H), 3.23 (t, *J* = 5.2 Hz, 4H), 2.62 (s, 6H), 1.50 (s, 9H).

¹³C NMR (126 MHz, CDCl₃) δ 158.48, 154.87, 151.22, 149.57, 146.43, 145.41, 143.93, 138.86, 133.46, 129.23, 128.36, 120.03, 118.02, 116.85, 116.24, 80.17, 49.04, 43.55, 28.59, 24.85.

HRMS (APCI): calcd. for C₂₉H₃₂N₄O₃ [M+H]⁺ = 485.2547, found = 485.2549.

FTIR (neat), cm⁻¹: 2978, 1678, 1608, 1524, 1430, 1388, 1365, 1239, 1180, 826, 812, 546, 459, 413.

### Preparative Example 8: 3-(2,6-dimethylpyridin-4-yl)-6-(4-(piperazin-1-yl)phenyl)furo[3,2-b]pyridine

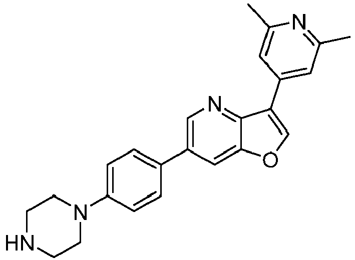

TFA (0.5 mL, 6.535 mmol) was added to a solution of *tert*-butyl 4-(4-(3-(2,6-dimethylpyridin-4-yl)furo[3,2-*b*]pyridin-6-yl)phenyl)piperazine-1-carboxylate **(Prep. Example** 7; 100 mg, 0.206 mmol) in DCM (5 mL) and the reaction mixture was stirred at 23 °C for 2 h. All volatiles were evaporated *in vacuo,* the residue was dissolved in acetonitrile (5 mL), triethylamine (0.15 mL) was added, and the mixture was allowed to stir for 2 min. The product was collected by filtration as a yellow solid (56 mg, 71 % yield).

¹H NMR (500 MHz, Methanol-*d*₄) δ 8.87 (d, *J* = 1.9 Hz, 1H), 8.64 (s, 1H), 8.12 (d, *J=* 1.9 Hz, 1H), 7.88 (s, 2H), 7.65 (d, *J* = 8.8 Hz, 2H), 7.13 (d, *J* = 8.9 Hz, 2H), 3.33 - 3.27 (m, 4H), 3.12 - 3.07 (m, 4H), 2.61 (s, 6H).

¹³C NMR (126 MHz, MeOD) δ 159.05, 152.95, 151.05, 149.48, 145.84, 144.41, 141.31, 134.96, 129.72, 129.03, 120.13, 119.44, 117.61, 117.27, 50.21, 46.32, 23.84.

HRMS (APCI): calcd. for C₂₄H₂₄N₄O [M+H]⁺ = 385.2023; found [M+H]⁺ = 385.2025.

FTIR (neat), cm⁻¹: 1667, 1604, 1523, 1480, 1375, 1229, 1199, 1182, 1120, 1099, 827, 808, 539.

### Preparative Example 9: 6-chloro-3-(pyridin-3-yl)furo[3,2-b]pyridine

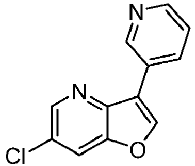

The compound was prepared by the general procedure **A** using 200 mg (0.86 mmol) of 3-bromo-6-chlorofuro[3,2-*b*]pyridine **(Prep. Example 5)** and 137 mg (1.120 mmol) of pyridin-3-ylboronic acid; the reaction time was 1 h; flash chromatography (cyclohexane/EtOAc, gradient from 1:0 to 1:1) afforded the compound as a yellow solid (104 mg, 52 % yield).

¹H NMR (500 MHz, Chloroform-*d*) δ 9.18 (d, *J* = 2.4 Hz, 1H), 8.67 - 8.60 (m, 2H), 8.49 (dt, *J* = 7.9, 2.0 Hz, 1H), 8.19 (s, 1H), 7.86 (d, *J* = 2.0 Hz, 1H), 7.47 - 7.39 (m, 1H).

¹³C NMR (126 MHz, CDCl₃) δ 148.96, 148.52, 147.82, 145.73, 145.69, 144.10, 134.84, 128.24, 126.45, 123.94, 119.10, 119.02.

HRMS (APCI): calcd. for C₁₂H₇ClN₂O [M+H]⁺ = 231.0320; found [M+H]⁺ = 231.0322.

FTIR (neat), cm⁻¹: 3070, 3039, 1610, 1468, 1425, 1388, 1365, 1326, 1281, 1142, 1093, 1077, 1030, 968, 911, 891, 801, 787, 733, 704, 619, 597, 528.

### Preparative Example 10: tert-butyl 4-(4-(3-(pyridn-3-yl)furo[3,2-b]pyridin-6-yl)phenyl)piperazine-1-carboxylate

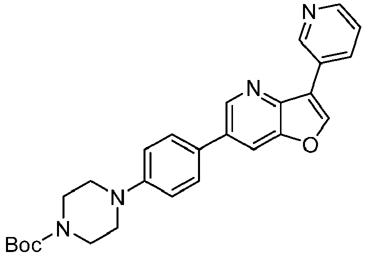

The compound was prepared by the general procedure **B** using 96 mg (0.416 mmol) of 6-chloro-3-(pyridin-3-yl)furo[3,2-*b*]pyridine **(Prep. Example 9)** and 194 mg (0.499 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 1:2) afforded the compound as a yellow solid (171 mg, 90 % yield).

¹H NMR (500 MHz, Chloroform-*d*) δ 9.21 (dd, *J* = 2.3, 0.9 Hz, 1H), 8.88 (d, *J* = 1.9 Hz, 1H), 8.61 (dd, *J* = 4.9, 1.7 Hz, 1H), 8.57 (ddd, *J* = 7.9, 2.2, 1.7 Hz, 1H), 8.19 (s, 1H), 7.95 (d, *J* = 1.9 Hz, 1H), 7.58 (d, *J* = 8.8 Hz, 2H), 7.44 (ddd, *J* = 7.8, 4.8, 0.9 Hz, 1H), 7.05 (d, *J* = 8.9 Hz, 2H), 3.62 (dd, *J* = 6.3, 4.1 Hz, 4H), 3.23 (dd, *J* = 6.3, 4.1 Hz, 4H), 1.50 (s, 9H).

¹³C NMR (126 MHz, CDCl₃) δ 154.88, 151.22, 149.42, 148.61, 147.78, 145.42, 145.04, 144.04, 134.90, 133.56, 129.25, 128.39, 127.13, 123.95, 118.89, 116.86, 116.26, 80.17, 49.05, 43.66, 28.60.

HRMS (APCI): calcd. for C₂₇H₂₈N₄O₃ [M+H]⁺ = 457.2234; found [M+H]⁺ = 457.2237.

FTIR (neat), cm⁻¹ 2977, 2929, 2900, 2857, 2823, 1681, 1608, 1526, 1483, 1462, 1421, 1380, 1362, 1342, 1283, 1239, 1204, 1161, 1128, 1097, 1048, 966, 909, 821, 795, 765, 706, 546, 524.

### Preparative Example 11: 6-(4-(piperazin-1-yl)phenyl)-3-(pyridin-3-yl)furo[3,2-b]pyridine

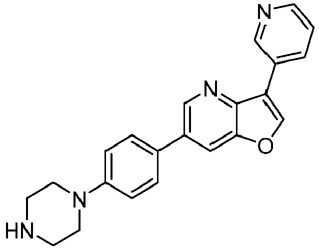

The compound was prepared by the general procedure **C** using 90 mg (0.197 mmol) of *tert*-butyl 4-(4-(3-(pyridin-3-yl)furo[3,2-*b*]pyridin-6-yl)phenyl)piperazine-1-carboxylate **(Prep. Example 10);** the reaction time was 2 h; flash chromatography (DCM/MeOH, gradient from 1:0 to 4:1) afforded the compound as a pale yellow solid (71 mg, 100 % yield).

¹H NMR (300 MHz, DMSO-*d*₆) δ 9.43 (d, *J* = 2.2 Hz, 1H), 8.96 (s, 2H), 8.62 (dd, *J* = 8.0, 2.1 Hz, 1H), 8.58 (dd, *J* = 4.8, 1.7 Hz, 1H), 8.35 (d, *J* = 1.9 Hz, 1H), 7.71 (d, *J* = 8.5 Hz, 2H), 7.54 (dd, *J* = 7.9, 4.8 Hz, 1H), 7.06 (d, *J* = 8.5 Hz, 2H), 3.16 (dd, *J* = 6.4, 3.7 Hz, 4H), 2.89 (t, *J* = 5.0 Hz, 4H). ¹³C NMR (126 MHz, DMSO) δ 151.25, 148.79, 148.45, 147.41, 147.08, 144.45, 142.98, 133.56, 132.57, 127.72, 126.68, 126.57, 123.73, 117.33, 115.68, 115.45, 48.57, 45.23.

HRMS (APCI): calcd. for C₂₂H₂₀N₄O [M+H]⁺ = 357.1710; found [M+H]⁺ = 357.1710.

FTIR (neat), cm⁻¹: 3289, 3033, 2945, 2825, 2748, 1604, 1522, 1481, 1449, 1377, 1333, 1234, 1201, 1144, 1125, 1099, 966, 946, 884, 822, 803, 787, 704, 683, 608, 537.

### Preparative Example 12: 6-chloro-3-(3-methoxyphenyl]furo[3,2-b]pyridine

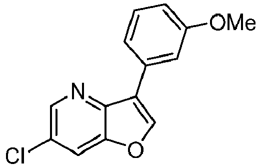

The compound was prepared by the general procedure **A** using 100 mg (0.430 mmol) of 3-bromo-6-chlorofuro[3,2-*b*]pyridine **(Prep. Example 5)** and 85 mg (0.559 mmol) of (3-methoxyphenyl)boronic acid; the reaction time was 2 h; flash chromatography (cyclohexane/EtOAc, gradient from 1:0 to 11:1) afforded the compound as a white solid (68 mg, 61 % yield).

¹H NMR (500 MHz, Chloroform-d) δ 8.62 (d, *J* = 2.0 Hz, 1H), 8.10 (s, 1H), 7.81 (d, *J* = 2.0 Hz, 1H), 7.65 (dd, *J* = 2.6, 1.6 Hz, 1H), 7.58 (ddd, *J* = 7.6, 1.6, 0.9 Hz, 1H), 7.39 (t, *J* = 8.0 Hz, 1H), 6.93 (ddd, *J* = 8.3, 2.6, 0.9 Hz, 1H), 3.89 (s, 3H).

¹³C NMR (126 MHz, CDCl₃) δ 160.14, 148.52, 145.77, 145.33, 144.48, 131.24, 130.03, 127.71, 121.87, 119.61, 118.80, 113.69, 113.07, 55.47.

HRMS (APCI): calcd. for C₁₄H₁₀ClINO₂ [M+H]⁺ = 260.0473; found [M+H]⁺ = 260.0476.

FTIR (neat), cm⁻¹: 3108, 3068, 2964, 2942, 2838, 1611, 1584, 1564, 1480, 1451, 1438, 1387, 1343, 1304, 1287, 1274, 1234, 1209, 1183, 1170, 1128, 1096, 1084, 1071, 1051, 999, 987, 911, 887, 875, 859, 822, 780, 685, 654, 599, 569, 525, 457.

### Preparative Example 13: tert-butyl 4-(4-(3-(3-methoxyphenyl)furo[3,2-b]pyridin-6-yl)phenyl)piperazine-1-carboxylate

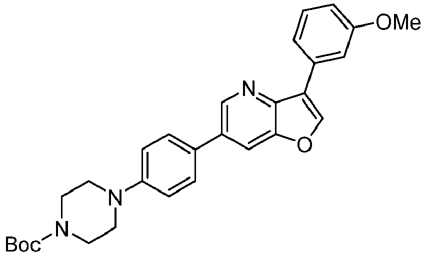

The compound was prepared by the general procedure **B** using 65 mg (0.250 mmol) of 6-chloro-3-(3-methoxyphenyl)furo[3,2-*b*]pyridine **(Prep. Example 12)** and 126 mg (0.325 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 2:1) afforded the compound as a yellow solid (88 mg, 72 % yield).

¹H NMR (500 MHz, Chloroform-d) δ 8.88 (d, *J* = 1.9 Hz, 1H), 8.11 (s, 1H), 7.92 (d, *J* = 2.0 Hz, 1H), 7.70 (dd, *J* = 2.6, 1.5 Hz, 1H), 7.66 (dt, *J* = 7.8, 1.2 Hz, 1H), 7.63 - 7.55 (m, 2H), 7.40 (t, *J* = 7.9 Hz, 1H), 7.05 (d, *J* = 8.6 Hz, 2H), 6.92 (ddd, *J* = 8.3, 2.6, 1.0 Hz, 1H), 3.90 (s, 3H), 3.67 - 3.57 (m, 4H), 3.23 (t, *J* = 5.1 Hz, 4H), 1.50 (s, 9H).

¹³C NMR (126 MHz, CDCl₃) δ 160.15, 154.88, 149.44, 145.17, 145.11, 144.48, 133.02, 131.91, 130.02, 128.38, 121.85, 119.74, 116.96, 116.11, 113.53, 113.01, 80.18, 55.50, 49.21, 43.56, 28.60.

HRMS (APCI): calcd. for C₂₉H₃₁N₃O₄ [M+H]⁺ = 486.2387; found [M+H]⁺ = 486.2391.

FTIR (neat), cm⁻¹ 2974, 2815, 1682, 1604, 1590, 1524, 1480, 1461, 1449, 1412, 1378, 1366, 1336, 1290, 1261, 1251, 1224, 1160, 1134, 1117, 1101, 1042, 996, 908, 887, 860, 826, 813, 791, 773, 693, 544.

### Preparative Example 14: 3-(3-methoxyphenyl)-6-(4-(piperazin-1-yl)phenyl)furo[3,2-b]pyridine

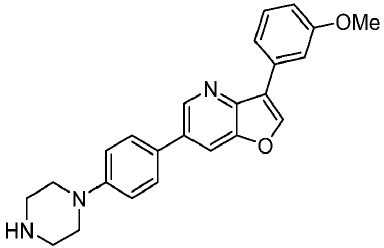

The compound was prepared by the general procedure **C** using 80 mg (0.165 mmol) of *tert*-butyl 4-(4-(3-(3-methoxyphenyl)furo[3,2-*b*]pyridin-6-yl)phenyl)piperazine-1-carboxylate **(Prep. Example 13);** the reaction time was 2 h; flash chromatography (DCM/MeOH, gradient from 1:0 to 6:1) afforded the compound as a white solid (43 mg, 68 % yield).

¹H NMR (500 MHz, DMSO-*d*₆) δ 8.93 (d, *J* = 2.0 Hz, 1H), 8.85 (s, 1H), 8.29 (d, *J* = 1.9 Hz, 1H), 7.88 (dd, *J* = 2.6, 1.5 Hz, 1H), 7.85 (dt, *J* = 7.7, 1.2 Hz, 1H), 7.70 (d, *J* = 8.9 Hz, 2H), 7.41 (t, *J* = 7.9 Hz, 1H), 7.15 - 7.03 (m, 2H), 6.95 (ddd, *J* = 8.3, 2.6, 1.0 Hz, 1H), 3.84 (s, 3H), 3.21 - 3.13 (m, 4H), 2.93 - 2.86 (m, 4H).

¹³C NMR (126 MHz, DMSO) δ 159.48, 151.17, 148.85, 146.85, 144.23, 143.30, 132.24, 131.67, 129.68, 127.70, 126.86, 119.88, 118.88, 115.52, 115.49, 112.94, 112.26, 55.12, 48.51, 45.18.

HRMS (APCI): calcd. for C₂₄H₂₃N₃O₂ [M+H]⁺ = 386.1863; found [M+H]⁺ = 386.1864.

FTIR (neat), cm⁻¹: 2829, 1603, 1590, 1523, 1481, 1450, 1377, 1261, 1242, 1227, 1117, 1100, 1038, 910, 887, 859, 827, 814, 793, 693, 543, 517.

### Preparative Example 15: 6-chloro-3-(2-methoxyphenyl)furo[3,2-b]pyridine

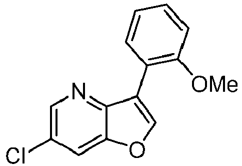

The compound was prepared by the general procedure **A** using 100 mg (0.430 mmol) of 3-bromo-6-chlorofuro[3,2-*b*]pyridine **(Prep. Example 5)** and 85 mg (0.559 mmol) of (2-methoxyphenyl)boronic acid; the reaction time was 2 h; flash chromatography (cyclohexane/EtOAc, gradient from 1:0 to 11:1) afforded the compound as a white solid (110 mg, 98 % yield).

¹H NMR (500 MHz, Chloroform-d) δ 8.82 (dd, *J* = 7.7, 1.8 Hz, 1H), 8.61 (d, *J* = 2.1 Hz, 1H), 8.51 (s, 1H), 7.80 (d, *J* = 2.1 Hz, 1H), 7.34 (ddd, *J* = 8.3, 7.4, 1.8 Hz, 1H), 7.15 (td, *J* = 7.5, 1.2 Hz, 1H), 7.02 (dd, *J* = 8.2, 1.1 Hz, 1H), 3.95 (s, 3H).

¹³C NMR (126 MHz, CDCl₃) δ 156.79, 149.33, 147.53, 145.28, 144.64, 129.92, 128.61, 127.20, 121.10, 119.22, 118.47, 116.55, 110.85, 55.55.

HRMS (APCI): calcd. for C₁₄H₁₀ClNO₂ [M+H]⁺ = 260.0473; found [M+H]⁺ = 260.0475.

FTIR (neat), cm⁻¹: 3178, 3068, 2921, 2836, 1601, 1579, 1495, 1453, 1434, 1385, 1339, 1251, 1185, 1135, 1121, 1092, 1072, 1053, 1021, 962, 940, 920, 908, 867, 809, 774, 744, 733, 698, 645, 593, 582, 534, 474, 464.

### Preparative Example 16: tert-butyl 4-(4-(3-(2-methoxyphenyl)furo[3,2-b]pyridin-6-yl)phenyl)piperazine-1-carboxylate

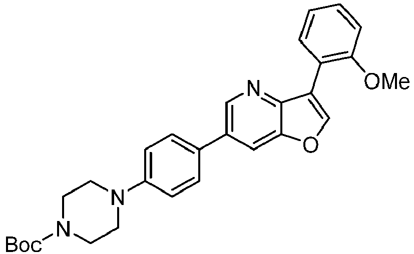

The compound was prepared by the general procedure ***B*** using 100 mg (0.385 mmol) of 6-chloro-3-(2-methoxyphenyl)furo[3,2-*b*]pyridine **(Prep. Example 15)** and 194 mg (0.501 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 4:1) afforded the compound as a white solid (180 mg, 96 % yield).

¹H NMR (500 MHz, Chloroform-d) δ 8.91 (dd, *J* = 7.7, 1.8 Hz, 1H), 8.88 (d, *J* = 1.9 Hz, 1H), 8.53 (s, 1H), 7.92 (d, *J* = 1.9 Hz, 1H), 7.62 - 7.54 (m, 2H), 7.33 (ddd, *J* = 8.2, 7.4, 1.8 Hz, 1H), 7.17 (td, *J* = 7.5, 1.2 Hz, 1H), 7.09 - 7.02 (m, 3H), 3.98 (s, 3H), 3.67 - 3.45 (m, 4H), 3.23 (t, *J* = 5.2 Hz, 4H), 1.50 (s, 9H).

¹³C NMR (126 MHz, CDCl₃) δ 156.84, 154.88, 151.01, 148.77, 148.43, 145.26, 144.46, 132.54, 130.03, 129.76, 128.35, 128.31, 121.18, 119.87, 116.91, 116.52, 115.88, 110.90, 80.13, 55.61, 49.16, 43.71, 28.60.

HRMS (APCI): calcd. for C₂₉H₃₁N₃O₄ [M+H]⁺ = 486.2387; found [M+H]⁺ = 486.2390.

FTIR (neat), cm⁻¹ 2970, 2834, 1678, 1519, 1495, 1463, 1426, 1381, 1363, 1288, 1266, 1249, 1213, 1198, 1161, 1142, 1118, 1094, 1085, 1056, 1042, 1030, 1003, 964, 915, 863, 832, 779, 767, 746, 671, 650, 554, 539, 481.

### Preparative Example 17: 3-(2-methoxyphenyl)-6-(4-(piperazin-1-yl)phenyl)furo[3,2-b]pyridine

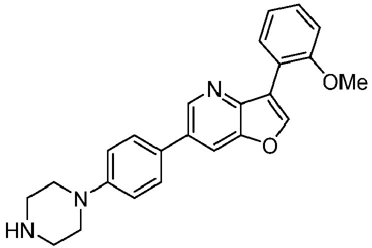

The compound was prepared by the general procedure C using 100 mg (0.206 mmol) of *tert*-butyl 4-(4-(3-(2-methoxyphenyl)furo[3,2-*b*]pyridin-6-yl)phenyl)piperazine-1-carboxylate **(Prep. Example 16);** the reaction time was 2 h; flash chromatography (DCM/MeOH, gradient from 1:0 to 4:1) afforded the compound as a white solid (67 mg, 84 % yield).

¹H NMR (500 MHz, DMSO-*d*₆) δ 9.00 (dd, *J* = 7.7, 1.8 Hz, 1H), 8.93 (d, *J* = 2.0 Hz, 1H), 8.78 (s, 1H), 8.29 (d, *J* = 2.0 Hz, 1H), 7.77 - 7.66 (m, 2H), 7.41 - 7.34 (m, 1H), 7.18 (dd, *J* = 8.4, 1.1 Hz, 1H), 7.13 (td, *J* = 7.5, 1.1 Hz, 1H), 7.07 - 7.03 (m, 2H), 3.96 (s, 3H), 3.14 (dd, *J* = 6.2, 4.0 Hz, 4H), 2.91 - 2.82 (m, 4H).

¹³C NMR (126 MHz, DMSO) δ 156.42, 151.30, 148.73, 147.75, 143.99, 143.77, 132.06, 129.01, 128.36, 127.67, 126.81, 120.34, 119.06, 115.45, 115.39, 115.32, 111.15, 55.46, 48.81, 45.41.

HRMS (APCI): calcd. for C₂₄H₂₃N₃O₂ [M+H]⁺ = 386.1863; found [M+H]⁺ = 386.1866.

FTIR (neat), cm⁻¹: 2932, 2915, 2814, 1603, 1518, 1498, 1466, 1454, 1434, 1419, 1372, 1302, 1247, 1224, 1200, 1189, 1146, 1115, 1090, 1076, 1057, 1021, 963, 919, 882, 829, 809, 780, 764, 722, 697, 650, 545.

### Preparative Example 18: 3-(6-chlorofuro[3,2-b]pyridin-3-yl)benzonitrile

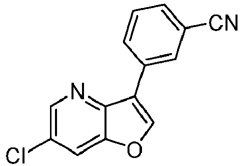

The compound was prepared by the general procedure *A* using 300 mg (1.29 mmol) of 3-bromo-6-chlorofuro[3,2-*b*]pyridine **(Prep. Example 5)** and 247 mg (1.680 mmol) of (3-cyanophenyl)boronic acid; the reaction time was 2 h; flash chromatography (cyclohexane/EtOAc, gradient from 1:0 to 0:1) afforded the compound as a white solid (320 mg, 97 % yield).

¹H NMR (500 MHz, Chloroform-*d*) δ 8.63 (d, *J* = 2.0 Hz, 1H), 8.43 (t, *J* = 1.7 Hz, 1H), 8.28 (dt, *J* = 7.8, 1.5 Hz, 1H), 8.18 (s, 1H), 7.86 (d, *J* = 2.0 Hz, 1H), 7.65 (dt, *J* = 7.7, 1.4 Hz, 1H), 7.58 (t, *J* = 7.8 Hz, 1H).

¹³C NMR (126 MHz, CDCl₃) δ 148.59, 146.13, 145.72, 143.74, 131.52, 131.46, 131.18, 130.66, 129.81, 128.33, 120.07, 119.16, 118.79, 113.36.

HRMS (APCI): calcd. for C₁₄H₇ClN₂O [M+H]⁺ = 255.0320; found [M+H]⁺ = 255.0322.

FTIR (neat), cm⁻¹: 3104, 3069, 2230, 1610, 1586, 1479, 1466, 1384, 1344, 1275, 1131, 1101, 1091, 1072, 1012, 911, 893, 881, 837, 814, 788, 679, 659, 601, 515, 474, 463.

### Preparative Example 19: tert-butyl 4-(4-(3-(3-cyanophenyl)furo[3,2-b]pyridin-6-yl)phenyl)piperazine-1-carboxylate

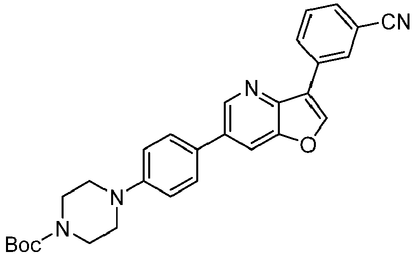

The compound was prepared by the general procedure *B* using 300 mg (1.178 mmol) of 3-(6-chlorofuro[3,2-*b*]pyridin-3-yl)benzonitrile *(Prep. Example 18)* and 595 mg (1.532 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 3:1) afforded the compound as a white solid (368 mg, 65 % yield).

¹H NMR (500 MHz, Chloroform-d) δ 8.89 (d, *J* = 1.9 Hz, 1H), 8.49 (t, *J* = 1.7 Hz, 1H), 8.35 (dt, *J* = 7.8, 1.5 Hz, 1H), 8.17 (s, 1H), 7.94 (d, *J* = 1.9 Hz, 1H), 7.64 (dt, *J* = 7.8, 1.5 Hz, 1H), 7.62 - 7.55 (m, 3H), 7.09 - 7.02 (m, 2H), 3.62 (t, *J* = 5.2 Hz, 4H), 3.24 (t, *J* = 5.2 Hz, 4H), 1.50 (s, 9H).

¹³C NMR (126 MHz, CDCl₃) δ 154.87, 151.20, 149.52, 145.44, 145.43, 143.69, 133.63, 132.21, 131.22, 131.18, 130.66, 129.76, 129.21, 128.39, 120.01, 118.94, 116.88, 116.29, 113.28, 80.18, 49.07, 43.58, 28.59.

HRMS (APCI): calcd. for C₂₉H₂₈N₄O₃ [M+H]⁺ = 481.2234; found [M+H]⁺ = 481.2231.

FTIR (neat), cm⁻¹ 3467,2976, 2930,2820, 2229, 1684, 1604, 1527, 1480, 1411, 1380, 1364, 1237, 1212, 1161, 1119, 1044, 1011, 995, 907, 817, 797, 773, 686, 538, 524, 478.

### Preparative Example 20: 3-(6-(4-(piperazin-1-yl)phenyl)furo[3,2-b]pyridin-3-yl)benzonitrile

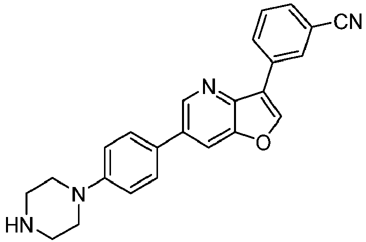

TFA (0.5 mL, 6.535 mmol) was added to a solution of *tert-butyl* 4-(4-(3-(3-cyanophenyl)furo[3,2-*b*]pyridin-6-yl)phenyl)piperazine-1-carboxylate *(Prep. Example 19;* 75 mg, 0.156 mmol) in DCM (5 mL) and the reaction mixture was stirred at 23 °C for 2 h. All volatiles were evaporated *in vacuo,* the residue was dissolved in acetonitrile (5 mL), triethylamine (2.0 mL) was added, all volatiles were evaporated *in vacuo* and the residue was dissolved in a mixture of DCM/EA/MeOH (4:2:1; 10 mL) and extracted with solution of NaHCO₃ (sat. aq.; 3 × 20 mL). The organic layer was dried over MgSO₄, and filtered. The solvent was evaporated *in vacuo* and the residue was purified by flash chromatography (DCM/MeOH; gradient from 1:0 to 4:1). The product was obtained as white solid (43 mg, 72 % yield). ¹H NMR (500 MHz, DMSO-*d*₆) δ 8.98 (s, 1H), 8.97 (d, *J* = 2.0 Hz, 1H), 8.75 (t, *J* = 1.7 Hz, 1H), 8.62 (dt, *J* = 8.0, 1.4 Hz, 1H), 8.33 (d, *J* = 1.9 Hz, 1H), 7.83 (dt, *J* = 7.6, 1.4 Hz, 1H), 7.73 (t, *J* = 7.8 Hz, 1H), 7.71 - 7.67 (m, 2H), 7.11 - 6.99 (m, 2H), 3.15 (dd, *J=* 6.1, 4.0 Hz, 4H), 2.95 - 2.82 (m, 4H).

¹³C NMR (126 MHz, DMSO) δ 151.34, 148.91, 147.74, 144.49, 142.69, 132.63, 131.89, 130.90, 130.85, 129.98, 129.57, 127.71, 126.55, 118.72, 118.12, 115.73, 115.41, 111.82, 48.72, 45.36.

HRMS (APCI): calcd. for C₂₄H₂₀N₄O [M+H]⁺ = 381.1710; found [M+H]⁺ = 381.1714.

FTIR (neat), cm⁻¹: 3587, 3319, 2230, 1663, 1601, 1524, 1481, 1453, 1378, 1351, 1241, 1210, 1146, 1104, 1054, 1011, 887, 852, 797, 749, 726, 686, 666, 637, 621, 526, 477.

### Preparative Example 21: 6-Chloro-3-(pyridin-4-yl)furo[3,2-b]pyridine

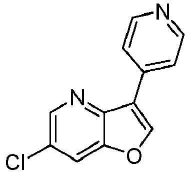

The compound was prepared by the general procedure **A** using 200 mg (0.86 mmol) of 3-bromo-6-chlorofuro[3,2-*b*]pyridine **(Prep. Example 5)** and 137 mg (1.120 mmol) of pyridin-4-ylboronic acid; the reaction time was 1 h; flash chromatography (cyclohexane/EtOAc, gradient from 0 % to 50 % of EtOAc) afforded the compound as a yellow solid (108 mg, 54 % yield).

¹H NMR (500 MHz, Chloroform-*d*) δ 8.71 (d, *J* = 6.2 Hz, 2H), 8.65 (d, *J* = 2.0 Hz, 1H), 8.28 (s, 1H), 8.07 - 7.98 (m, 2H), 7.88 (d, *J=* 2.1 Hz, 1H).

¹³C NMR (126 MHz, CDCl₃) δ 150.18, 148.74, 147.29, 145.91, 143.75, 138.22, 128.43, 121.47, 119.64, 119.25.

HRMS (APCI): calcd. for C₁₂H₇ClN₂O [M+H]⁺ = 231.0320; found [M+H]⁺ = 231.0319.

FTIR (neat), cm⁻¹: 3062, 1610, 1422, 1388, 1284, 1231, 1144, 1090, 995, 979, 914, 880, 824, 729, 655, 618, 600, 518, 427.

### Preparative Example 22: 6-phenyl-3-(pyridin-4-yl)furo[3,2-b]pyridine

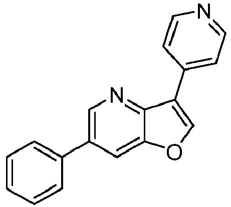

The compound was prepared by the general procedure **B** using 50 mg (0.217 mmol) of 6-chloro-3-(pyridin-4-yl)furo[3,2-*b*]pyridine **(Prep. Example 21)** and 34 mg (0.282 mmol) of phenylboronic acid; the reaction time was 2 h; flash chromatography (cyclohexane/EtOAc, gradient from 1:0 to 4:1) afforded the compound as a white solid (56 mg, 95 % yield).

¹H NMR (500 MHz, Chloroform-*d*) δ 8.93 (d, *J* = 1.9 Hz, 1H), 8.73 (bs, 2H), 8.30 (s, 1H), 8.07 (d, *J*= 5.7 Hz, 2H), 8.00 (d, *J* = 1.8 Hz, 1H), 7.69 - 7.62 (m, 2H), 7.52 (t, *J=* 7.6 Hz, 2H), 7.48 - 7.40 (m, 1H). ¹³C NMR (126 MHz, CDCl₃) δ 150.43, 149.48, 146.87, 145.93, 144.42, 138.43, 137.96, 133.97, 129.38, 128.33, 127.68, 121.47, 119.64, 117.16.

HRMS (APCI): calcd. for C₁₈H₁₂N₂O [M+H]⁺ = 273.1022; found [M+H]⁺ = 273.1020.

FTIR (neat), cm⁻¹ 3043, 1602, 1384, 1367, 1204, 1098, 979, 882, 829, 787, 756, 699, 680, 633, 536, 525, 508.

### Preparative Example 23: 6-(6-morpholinopyridin-3-yl)-3-(pyridin-4-yl)furo[3,2-b]pyridine

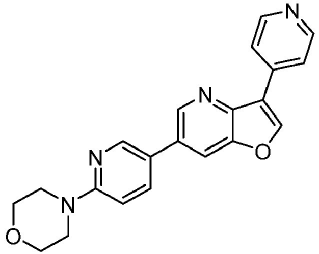

The compound was prepared by the general procedure **B** using 50 mg (0.217 mmol) of 6-chloro-3-(pyridin-4-yl)furo[3,2-*b*]pyridine **(Prep. Example 21)** and 88 mg (0.282 mmol) of 4-(5-(4,4,5,5-tetramethyl-1,3,2-dioxaborolan-2-yl)pyridin-2-yl)morpholine; the reaction time was 2 h; flash chromatography (cyclohexane/EtOAc, gradient from 1:0 to 0:1) afforded the compound as a pale orange solid (35 mg, 45 % yield).

¹H NMR (500 MHz, DMSO-*d*₆) δ 9.09 (s, 1H), 8.99 (s, 1H), 8.69 (d, *J* = 5.3 Hz, 2H), 8.63 (d, *J* = 2.6 Hz, 1H), 8.42 (s, 1H), 8.25 (d, *J=* 5.0 Hz, 2H), 8.05 (dd, *J=* 8.8, 2.7 Hz, 1H), 6.98 (d, *J=* 8.9 Hz, 1H), 3.73 (t, *J* = 4.8 Hz, 4H), 3.54 (t, *J* = 4.8 Hz, 4H).

¹³C NMR (126 MHz, DMSO) δ 158.48, 149.78, 148.74, 148.70, 145.89, 144.18, 142.94, 137.71, 135.96, 130.13, 121.86, 120.56, 117.76, 115.60, 106.78, 65.70, 44.92.

HRMS (APCI): calcd. for C₂₁H₁₈N₄O₂ [M+H]⁺ = 359.1503; found [M+H]⁺ = 359.1504.

FTIR (neat), cm⁻¹ 3040,2978, 2879,2856, 1604, 1509, 1478, 1409, 1384, 1320, 1236, 1206, 1119, 1088, 1055, 942, 822, 802, 722, 671, 638, 524, 475.

### Preparative Example 24: tert-butyl 4-(5-(3-(pyridin-4-yl)furo[3,2-b]pyridin-6-yl)pyridin-2-yl)piperazine-1-carboxylate

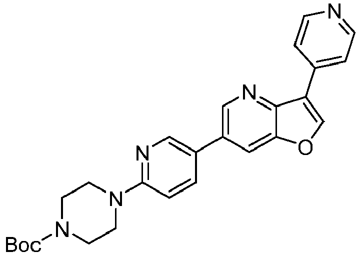

The compound was prepared by the general procedure **B** using 50 mg (0.217 mmol) of 6-chloro-3-(pyridin-4-yl)furo[3,2-*b*]pyridine **(Prep. Example 21)** and 110 mg (0.282 mmol) of 4-(5-(4,4,5,5-tetramethyl-1,3,2-dioxaborolan-2-yl)pyridin-2-yl)-1-Boc-piperazine; the reaction time was 2 h; flash chromatography (cyclohexane/EtOAc, gradient from 1:0 to 0:1) afforded the compound as a pale brown solid (92 mg, 93 % yield).

¹H NMR (500 MHz, Chloroform-*d*) δ 8.86 (d, *J* = 1.9 Hz, 1H), 8.73 (bs, 2H), 8.50 (dd, *J* = 2.6, 0.7 Hz, 1H), 8.29 (s, 1H), 8.06 (d, *J* = 3.6 Hz, 2H), 7.93 *(d, J=* 1.9 Hz, 1H), 7.78 (dd, *J* = 8.7, 2.6 Hz, 1H), 6.77 (dd, *J=* 8.9, 0.7 Hz, 1H), 3.63 (dd, *J* = 6.7, 3.3 Hz, 4H), 3.58 (dd, *J* = 6.6, 3.3 Hz, 4H), 1.50 (s, 9H). ¹³C NMR (126 MHz, CDCl₃) δ 158.92, 154.97, 150.35, 149.58, 146.74, 146.70, 145.10, 144.01, 138.52, 136.57, 131.22, 123.13, 121.51, 119.63, 116.06, 107.27, 80.23, 45.14, 43.44, 28.60.

HRMS (APCI): calcd. for C₂₆H₂₇N₅O₃ [M+H]⁺ = 458.2187; found [M+H]⁺ = 458.2190.

FTIR (neat), cm⁻¹ 2999, 2973, 2844, 1680, 1602, 1509, 1473, 1456, 1402, 1365, 1282, 1267, 1240, 1208, 1165, 1125, 1106, 1088, 1055, 1003, 978, 936, 892, 807, 792, 776, 757, 676, 663, 653, 637, 552, 529, 516.

### Preparative Example 25: 6-(6-(piperazin-1-yl)pyridin-3-yl)-3-(pyridin-4-yl)furo[3,2-b]pyridine

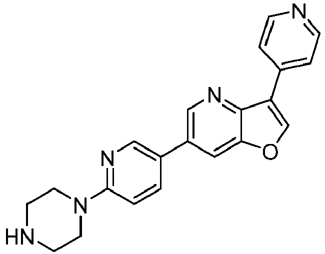

The compound was prepared by the general procedure C using 85 mg (0.186 mmol) of *tert*-butyl 4-(5-(3-(pyridin-4-yl)furo[3,2-*b*]pyridin-6-yl)pyridin-2-yl)piperazine-1-carboxylate **(Prep. Example 24);** the reaction time was 2 h; flash chromatography (DCM/MeOH, gradient from 1:0 to 4:1) afforded the compound as a pale yellow solid (65 mg, 98 % yield).

¹H NMR (500 MHz, DMSO-*d*₆) δ 9.10 (s, 1H), 8.97 (s, 1H), 8.68 (d, *J* = 5.1 Hz, 2H), 8.60 (s, 1H), 8.40 (s, 1H), 8.25 (d, *J* = 5.1 Hz, 2H), 8.00 (d, *J* = 9.1 Hz, 1H), 6.93 (d, *J* = 9.0 Hz, 1H), 3.49 (t, *J* = 4.9 Hz, 4H), 2.79 (t, *J* = 5.0 Hz, 4H).

¹³C NMR (126 MHz, DMSO) δ 158.76, 150.07, 149.03, 148.94, 146.10, 144.28, 142.93, 137.90, 136.03, 130.41, 121.17, 120.68, 117.80, 115.68, 106.81, 45.79, 45.40.

HRMS (APCI): calcd. for C₂₁H₁₉N₅O [M+H]⁺ = 358.1662; found [M+H]⁺ = 358.1663.

FTIR (neat), cm⁻¹: 3267, 2840, 1600, 1510, 1475, 1449, 1409, 1377, 1322, 1247, 1206, 1100, 900, 829, 810, 670, 656, 637, 543, 528, 483.

### Preparative Example 26: tert-butyl 4-(5-(6-chlorofuro[3,2-b]pyridin-3-yl)pyridin-2-yl)piperazine-1-carboxylate

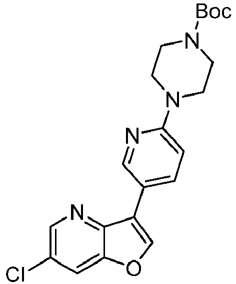

The compound was prepared by the general procedure **A** using 100 mg (0.430 mmol) of 3-bromo-6-chlorofuro[3,2-*b*]pyridine **(Prep. Example 5)** and 218 mg (0.559 mmol) of 4-(5-(4,4,5,5-tetramethyl-1,3,2-dioxaborolan-2-yl)pyridin-2-yl)-1-Boc-piperazine; the reaction time was 2 h; flash chromatography (cyclohexane/EtOAc, gradient from 1:0 to 1:1) afforded the compound as a white solid (105 mg, 59 % yield).

¹H NMR (500 MHz, Chloroform-*d*) δ 8.77 (dd, *J* = 2.4, 0.8 Hz, 1H), 8.58 (d, *J=* 2.0 Hz, 1H), 8.21 (dd, *J*= 8.8, 2.4 Hz, 1H), 8.04 (s, 1H), 7.81 (d, *J* = 2.1 Hz, 1H), 6.75 (dd, *J* = 8.9, 0.8 Hz, 1H), 3.64 - 3.51 (m, 8H), 1.49 (s, 9H).

¹³C NMR (126 MHz, CDCl₃) δ 158.79, 154.98, 148.37, 146.48, 145.25, 144.63, 144.13, 136.53, 127.77, 119.48, 118.79, 115.84, 107.11, 80.13, 45.24, 43.48, 28.60, 27.08.

HRMS (APCI): calcd. for C₂₁H₂₃ClN₄O₃ [M+H]⁺ = 415.1531; found [M+H]⁺ = 415.1530.

FTIR (neat), cm⁻¹: 3085, 2982, 2929, 2891, 2865, 2832, 1691, 1603, 1582, 1487, 1461, 1426, 1384, 1365, 1318, 1279, 1239, 1224, 1185, 1169, 1134, 1082, 1072, 1053, 1026, 1002, 962, 932, 907, 872, 811, 783, 764, 752, 595, 528, 464.

### Preparative Example 27: tert-butyl 4-(5-(6-(4-(4-(tert-butoxycarbonyl)piperazin-1-yl)phenyl)furo[3,2-b]pyridin-3-yl)pyridin-2-yl)piperazine-1-carboxylate

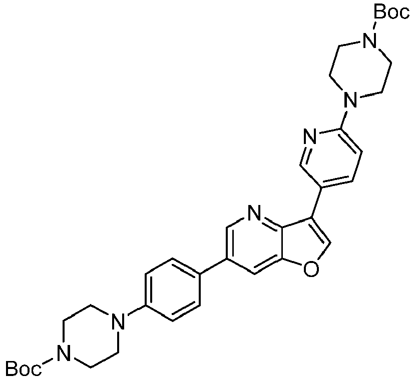

The compound was prepared by the general procedure **B** using 95 mg (0.229 mmol) of *tert*-butyl 4-(5-(6-chlorofuro[3,2-*b*]pyridin-3-yl)pyridin-2-yl)piperazine-1-carboxylate **(Prep. Example 26)** and 116 mg (0.298 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 2:1) afforded the compound as a yellow solid (99 mg, 67 % yield).

¹H NMR (500 MHz, Chloroform-*d*) δ 8.84 (d, *J* = 1.9 Hz, 1H), 8.81 (dd, *J* = 2.4, 0.8 Hz, 1H), 8.30 (dd, *J* = 8.7, 2.3 Hz, 1H), 8.04 (s, 1H), 7.90 (d, *J* = 1.9 Hz, 1H), 7.63 - 7.52 (m, 2H), 7.11 - 7.00 (m, 2H), 6.78 (d, *J* = 8.8 Hz, 1H), 3.65 - 3.55 (m, 12H), 3.23 (t, *J* = 5.2 Hz, 4H), 1.50 (s, 18H).

¹³C NMR (126 MHz, CDCl₃) δ 154.98, 154.88, 151.13, 149.25, 145.07, 144.58, 143.54, 133.13, 129.55, 128.36, 116.88, 116.65, 116.05, 80.15, 49.11, 45.40, 43.66, 28.60.

HRMS (APCI): calcd. for C₃₆H₄₄N₆O₅ [M+H]⁺ = 641.3446; found [M+H]⁺ = 641.3449.

FTIR (neat), cm⁻¹ 2976, 2826, 1689, 1608, 1580, 1524, 1489, 1454, 1408, 1364, 1285, 1235, 1204, 1162, 1121, 1084, 1050, 998, 963, 928, 863, 819, 800, 771, 730, 647, 606, 540, 461.

### Preparative Example 28: 6-(4-(piperazin-1-yl)phenyl)-3-(6-(piperazin-1-yl)pyridin-3-yl)furo[3,2-b]pyridine

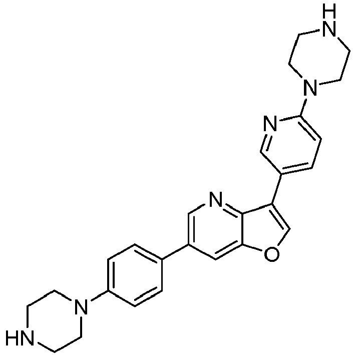

The compound was prepared by the general procedure C using 89 mg (0.139 mmol) of *tert*-butyl 4-(5-(6-(4-(4-(*tert*-butoxycarbonyl)piperazin-1-yl)phenyl)furo[3,2-*b*]pyridin-3-yl)pyridin-2-yl)piperazine-1-carboxylate **(Prep. Example 27);** the reaction time was 2 h; flash chromatography (DCM/MeOH, gradient from 1:0 to 4:1) afforded the compound as a white solid (37 mg, 60 % yield).

¹H NMR (500 MHz, DMSO-*d*₆) δ 8.99 (d, *J=* 2.4 Hz, 1H), 8.90 (d, *J* = 2.0 Hz, 1H), 8.69 (s, 1H), 8.31 (dd, *J* = 8.9, 2.4 Hz, 1H), 8.26 (d, *J* = 1.9 Hz, 1H), 7.68 (d, *J* = 8.8 Hz, 2H), 7.04 (d, *J* = 8.8 Hz, 2H), 6.93 (d, *J* = 8.9 Hz, 1H), 3.53 - 3.43 (m, 4H), 3.13 (dd, *J* = 6.3, 3.7 Hz, 4H), 2.86 (dd, *J* = 6.2, 3.7 Hz, 4H), 2.81 (dd, *J* = 6.1, 4.1 Hz, 4H).

¹³C NMR (126 MHz, DMSO) δ 158.45, 151.32, 148.62, 145.62, 144.69, 144.05, 143.42, 135.43, 132.20, 127.66, 126.77, 117.96, 115.41, 115.37, 115.28, 106.61, 48.89, 45.75, 45.47, 45.35.

HRMS (APCI): calcd. for C₂₆H₂₈N₆O [M+H]⁺ = 441.2397; found [M+H]⁺ = 441.2397

FTIR (neat), cm⁻¹: 3221, 2945, 2830, 1609, 1570, 1525, 1491, 1453, 1410, 1378, 1309, 1244, 1204, 1152, 1124, 1090, 1052, 1021, 942, 886, 817, 786, 746, 659, 533.

### Preparative Example 29: 6-chloro-3-(6-morpholinopyridin-3-yl)furo[3,2-b]pyridine

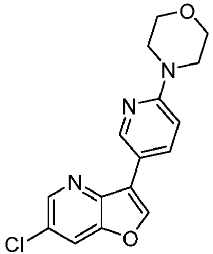

The compound was prepared by the general procedure **A** using 100 mg (0.430 mmol) of 3-bromo-6-chlorofuro[3,2-*b*]pyridine **(Prep. Example 5)** and 162 mg (0.559 mmol) of 1-[5-(4,4,5,5-tetramethyl-1,3,2-dioxaborolan-2-yl)pyridin-2-yl]morpholine; the reaction time was 2 h; flash chromatography (cyclohexane/EtOAc, gradient from 1:0 to 4:1) afforded the compound as a white solid (112 mg, 82 % yield).

¹H NMR (500 MHz, Chloroform-*d*) δ 8.78 (dd, *J* = 2.5, 0.8 Hz, 1H), 8.59 (d, *J=* 2.0 Hz, 1H), 8.24 (dd, *J* = 8.8, 2.4 Hz, 1H), 8.05 (s, 1H), 7.81 (d, *J* = 2.0 Hz, 1H), 6.76 (d, *J* = 8.8 Hz, 1H), 3.92 - 3.79 (m, 4H), 3.70 - 3.49 (m, 4H).

¹³C NMR (126 MHz, CDCl₃) δ 158.89, 148.38, 146.14, 145.28, 144.59, 144.20, 136.69, 127.81, 119.37, 118.81, 116.08, 107.02, 66.86, 45.82.

HRMS (APCI): calcd. for C₁₆H₁₄ClN₃O₂ [M+H]⁺ = 316.0847; found [M+H]⁺ = 316.0850.

FTIR (neat), cm⁻¹: 3084, 3058, 2960, 2871, 2852, 1614, 1601, 1570, 1548, 1493, 1446, 1411, 1386, 1317, 1276, 1245, 1227, 1114, 1073, 1025, 963, 942, 909, 882, 810, 794, 783, 765, 655, 595, 554, 533, 467.

### Preparative Example 30: tert-butyl 4-(4-(3-(6-morpholinopyridin-3-yl)furo[3,2-b]pyridin-6-yl)phenyl)piperazine-1-carboxylate

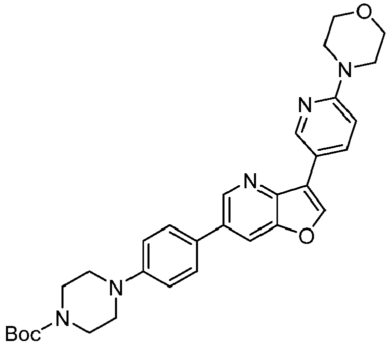

The compound was prepared by the general procedure **B** using 89 mg (0.282 mmol) of 6-chloro-3-(6-morpholinopyridin-3-yl)furo[3,2-*b*]pyridine **(Prep. Example 29)** and 142 mg (0.366 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 2:1) afforded the compound as a yellow solid (106 mg, 69 % yield).

¹H NMR (500 MHz, Chloroform-d) δ 8.85 (d, *J* = 1.9 Hz, 1H), 8.81 (dd, *J* = 2.4, 0.8 Hz, 1H), 8.32 (dd, J= 8.9, 2.4 Hz, 1H), 8.05 (s, 1H), 7.91 (d, *J* = 1.9 Hz, 1H), 7.57 (d, *J* = 8.9 Hz, 2H), 7.04 (d, *J* = 8.8 Hz, 2H), 6.81 - 6.74 (m, 1H), 3.88 - 3.84 (m, 4H), 3.64 - 3.60 (m, 4H), 3.60 - 3.56 (m, 4H), 3.24 - 3.21 (m, 4H), 1.50 (s, 9H).

¹³C NMR (126 MHz, CDCl₃) δ 154.88, 151.13, 149.26, 145.07, 144.60, 143.54, 133.12, 129.56, 128.36, 116.88, 116.84, 116.05, 80.15, 66.90, 49.11, 45.89, 28.60.

HRMS (APCI): calcd. for C₃₁H₃₅N₅O₄ [M+H]⁺ = 542.2762; found [M+H]⁺ = 542.2766.

FTIR (neat), cm⁻¹ 2970, 2853, 1688, 1607, 1579, 1524, 1489, 1451, 1411, 1379, 1365, 1266, 1235, 1203, 1162, 1118, 1088, 1050, 999, 941, 816, 798, 774, 657, 534.

### Preparative Example 31: 3-(6-morpholinopyridin-3-yl)-6-(4-(piperazin-1-yl)phenyl)furo[3,2-b]pyridine

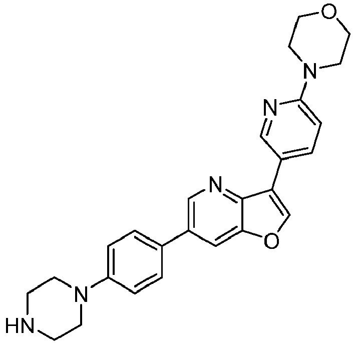

The compound was prepared by the general procedure C using 90 mg (0.166 mmol) of *tert*-butyl 4-(4-(3-(6-morpholinopyridin-3-yl)furo[3,2-*b*]pyridin-6-yl)phenyl)piperazine-1-carboxylate **(Prep. Example 30);** the reaction time was 2 h; flash chromatography (DCM/MeOH, gradient from 1:0 to 4:1) afforded the compound as a white solid (49 mg, 67 % yield).

¹H NMR (500 MHz, DMSO-*d*₆) δ 9.03 (d, *J* = 2.4 Hz, 1H), 8.90 (d, *J* = 1.9 Hz, 1H), 8.73 (s, 1H), 8.36 (dd, *J* = 8.8, 2.4 Hz, 1H), 8.27 (d, *J* = 1.9 Hz, 1H), 7.68 (d, *J* = 8.9 Hz, 2H), 7.04 (d, *J* = 8.9 Hz, 2H), 6.98 (d, *J=* 8.8 Hz, 1H), 3.77 - 3.68 (m, 4H), 3.60 - 3.49 (m, 4H), 3.18 - 3.08 (m, 4H), 2.89 - 2.78 (m, 4H).

¹³C NMR (126 MHz, DMSO) δ 158.30, 151.35, 148.64, 145.58, 144.93, 144.09, 143.36, 135.54, 132.25, 127.66, 126.72, 117.83, 116.08, 115.40, 106.82, 65.90, 48.95, 45.51, 45.07.

HRMS (APCI): calcd. for C₂₆H₂₇N₅O₂ [M+H]⁺ = 442.2238; found [M+H]⁺ = 442.2242.

FTIR (neat), cm⁻¹: 2830, 1607, 1577, 1524, 1487, 1448, 1408, 1376, 1239, 1202, 1114, 1095, 940, 883, 818, 800, 661, 534.

### Preparative Example 32: 6-chloro-3-(1-methyl-1H-pyrazol-4-yl)furo[3,2-b]pyridine

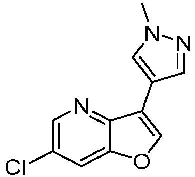

The compound was prepared by the general procedure **A** using 100 mg (0.430 mmol) of 3-bromo-6-chlorofuro[3,2-*b*]pyridine **(Prep. Example 5)** and 116 mg (0.559 mmol) of 1-methylpyrazole-4-boronic acid pinacol ester; the reaction time was 2 h; flash chromatography (cyclohexane/EtOAc, gradient from 1:0 to 2:1) afforded the compound as a white solid (59 mg, 59 % yield).

¹H NMR (500 MHz, Chloroform-*d*) δ 8.55 (d, *J=* 2.1 Hz, 1H), 8.17 (s, 1H), 7.97 (s, 1H), 7.86 (s, 1H), 7.76 (d, *J* = 2.1 Hz, 1H), 3.97 (s, 3H).

¹³C NMR (126 MHz, CDCl₃) δ 148.00, 145.07, 144.44, 143.85, 137.09, 128.77, 127.71, 118.66, 114.69, 110.40,39.18.

HRMS (APCI): calcd. for C₁₁H₈ClN₃O [M+H]⁺ = 234.0429; found [M+H]⁺ = 234.0428.

FTIR (neat), cm⁻¹: 3132, 3088, 2926, 1524, 1459, 1385, 1277, 1197, 1126, 1073, 986, 925, 902, 883, 836, 782, 773, 719, 658, 619, 588, 525, 420.

### Preparative Example 33: tert-butyl 4-(4-(3-(1-methyl-1H-pyrazol-4-yl)furo[3,2-b]pyridin-6-yl)phenyl)piperazine-1-carboxylate

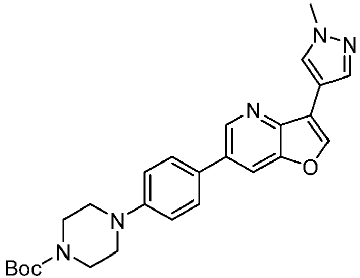

The compound was prepared by the general procedure **B** using 45 mg (0.193 mmol) 6-chloro-3-(1-methyl-1*H*-pyrazol-4-yl)furo[3,2-*b*]pyridine **(Prep. Example 32)** and 97 mg (0.250 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 1:2) afforded the compound as pale yellow solid (73 mg, 82 % yield).

¹H NMR (500 MHz, Chloroform-*d*) δ 8.83 (d, *J* = 1.8 Hz, 1H), 8.25 (s, 1H), 8.00 (s, 1H), 7.89 (dd, *J* = 4.9, 1.3 Hz, 2H), 7.56 (d, *J* = 8.7 Hz, 2H), 7.04 (d, *J* = 8.8 Hz, 2H), 3.99 (s, 3H), 3.76 - 3.53 (m, 4H), 3.22 (t, *J* = 5.2 Hz, 4H), 1.50 (s, 9H).

¹³C NMR (126 MHz, CDCl₃) δ 154.86, 151.07, 148.96, 144.84, 143.31, 137.15, 137.13, 133.15, 129.60, 128.80, 128.78, 128.35, 119.29, 118.50, 116.88, 116.07, 114.56, 110.99, 80.15, 49.11, 43.69, 39.19, 28.59.

HRMS (APCI): calcd. for C₂₆H₂₉N₅O₃ [M+H]⁺ = 460.2343; found [M+H]⁺ = 460.2347.

FTIR (neat), cm⁻¹ 2978, 2930, 1677, 1607, 1526, 1484, 1461, 1420, 1384, 1363, 1340, 1281, 1263, 1237, 1224, 1170, 1130, 1085, 1065, 1047, 997, 982, 928, 909, 869, 844, 823, 793, 765, 731, 714, 690, 662, 648, 631, 607, 594, 549, 526, 502, 454, 426, 412.

### Preparative Example 34: 3-(1-methyl-1H-pyrazol-4-yl)-6-(4-(piperazin-1-yl)phenyl)furo[3,2-b]pyridine

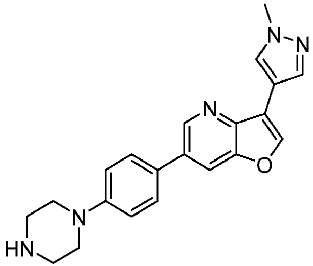

The compound was prepared by the general procedure C using 60 mg (0.131 mmol) of *tert*-butyl 4-(4-(3-(1-methyl-1*H*-pyrazol-4-yl)furo[3,2-*b*]pyridin-6-yl)phenyl)piperazine-1-carboxylate **(Prep. Example 33);** the reaction time was 2 h; flash chromatography (DCM/MeOH, gradient from 1:0 to 4:1) afforded the compound as a beige solid (26 mg, 55 % yield).

¹H NMR (500 MHz, Chloroform-d) δ 8.83 (d, *J=* 1.9 Hz, 1H), 8.24 (s, 1H), 7.99 (s, 1H), 7.93 - 7.85 (m, 2H), 7.55 (d, *J=* 8.7 Hz, 2H), 7.03 (d, *J=* 8.8 Hz, 2H), 3.99 (s, 3H), 3.26 - 3.19 (m, 4H), 3.16 - 3.04 (m, 4H).

¹³C NMR (126 MHz, CDCl₃) δ 151.62, 148.96, 144.91, 144.36, 143.18, 137.14, 133.27, 129.05, 128.75, 128.25, 116.38, 115.94, 114.55, 111.06, 50.02, 46.15, 39.18.

HRMS (APCI): calcd, for C₂₁H₂₁N₅O [M+H]⁺ = 360.1819; found [M+H]⁺ = 360.1820

FTIR (neat), cm⁻¹: 3307, 2937, 2817, 2686, 2467, 1605, 1524, 1482, 1451, 1423, 1381, 1344, 1239, 1202, 1190, 1174, 1146, 1120, 1079, 984, 927, 902, 885, 829, 816, 796, 786, 751, 664, 594, 540, 528.

### Preparative Example 35: 6-chloro-3-(1-methyl-1H-pyrazol-3-yl)furo[3,2-b]pyridine

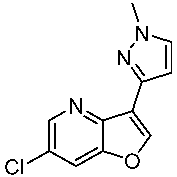

The compound was prepared by the general procedure **A** using 100 mg (0.430 mmol) of 3-bromo-6-chlorofuro[3,2-*b*]pyridine **(Prep. Example 5)** and 116 mg (0.559 mmol) of 1-methyl-3-(4,4,5,5-tetramethyl-1,3,2-dioxaborolan-2-yl)-1*H*-pyrazole; the reaction time was 2 h; flash chromatography (cyclohexane/EtOAc, gradient from 1:0 to 4:1) afforded the compound as a white solid (87 mg, 87 % yield).

¹H NMR (500 MHz, Chloroform-d) δ 8.62 (d, *J* = 2.1 Hz, 1H), 8.25 (s, 1H), 7.81 (d, *J* = 2.1 Hz, 1H), 7.45 (d, *J=* 2.3 Hz, 1H), 6.98 (d, *J=* 2.3 Hz, 1H), 3.99 (s, 3H).

¹³C NMR (126 MHz, CDCl₃) δ 148.17, 145.83, 145.54, 144.29, 141.96, 131.26, 127.62, 118.75, 116.27, 105.58, 39.29.

HRMS (APCI): calcd, for C₁₁H₈ClN₃O [M+H]⁺ = 234.0429; found [M+H]⁺ = 234.0432.

FTIR (neat), cm⁻¹: 3102, 2918, 2850, 1513, 1456, 1370, 1278, 1204, 1148, 1072, 1025, 924, 904, 894, 877, 851, 832, 778, 765, 685, 607, 595, 525, 424.

### Preparative Example 36: tert-butyl 4-(4-(3-(1-methyl-1H-pyrazol-3-yl)furo[3,2-b]pyridin-6-yl)phenyl)piperazine-1-carboxylate

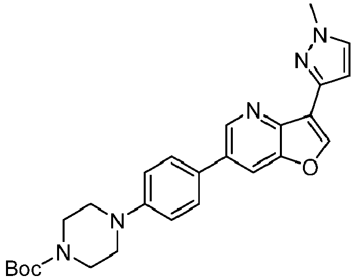

The compound was prepared by the general procedure **B** using 80 mg (0.342 mmol) of 6-chloro-3-(1-methyl-1*H*-pyrazol-3-yl)furo[3,2-*b*]pyridine **(Prep. Example 35)** and 173 mg (0.445 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 2:3) afforded the compound as pale yellow solid (109 mg, 69 % yield).

¹H NMR (500 MHz, Chloroform-*d*) δ 8.88 (d, *J* = 1.9 Hz, 1H), 8.25 (s, 1H), 7.90 (d, *J* = 1.9 Hz, 1H), 7.61 - 7.55 (m, 2H), 7.46 (d, *J* = 2.3 Hz, 1H), 7.09 - 6.99 (m, 3H), 4.00 (s, 3H), 3.75 - 3.49 (m, 4H), 3.22 (t, *J* = 5.2 Hz, 4H), 1.49 (s, 9H).

¹³C NMR (126 MHz, CDCl₃) δ 154.87, 151.04, 149.05, 145.30, 145.12, 144.23, 142.56, 132.93, 131.15, 129.70, 128.35, 116.88, 116.19, 116.04, 105.49, 80.13, 49.13, 43.62, 39.25, 28.58.

HRMS (APCI): calcd, for C₂₆H₂₉N₅O₃ [M+H]⁺ = 460.2343; found [M+H]⁺ = 460.2340.

FTIR (neat), cm⁻¹ 2971, 2821, 1688, 1460, 1426, 1384, 1366, 1288, 1236, 1222, 1202, 1173, 1131, 1090, 1073, 1048, 997, 910, 900, 821, 783, 761, 544.

### Preparative Example 37: 3-(1-methyl-1H-pyrazol-3-yl)-6-(4-(piperazin-1-yl)phenyl)furo[3,2-b]pyridine

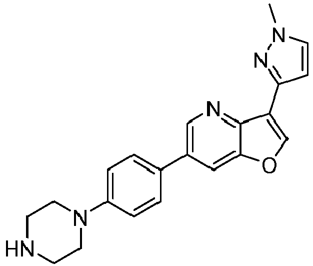

The compound was prepared by the general procedure C using 99 mg (0.215 mmol) of *tert*-butyl 4-(4-(3-(1-methyl-1*H*-pyrazol-3-yl)furo[3,2-*b*]pyridin-6-yl)phenyl)piperazine-1-carboxylate **(Prep. Example 36);** the reaction time was 2 h; flash chromatography (DCM/MeOH, gradient from 1:0 to 4:1) afforded the compound as a pale yellow solid (62 mg, 80 % yield).

¹H NMR (500 MHz, DMSO-*d*₆) δ 9.84 (d, *J* = 1.9 Hz, 1H), 9.52 (s, 1H), 9.20 (d, *J* = 1.9 Hz, 1H), 8.75 (d, *J* = 2.2 Hz, 1H), 8.62 (d, *J* = 8.8 Hz, 2H), 8.14 - 7.86 (m, 3H), 4.13 - 3.96 (m, 4H), 3.91 - 3.65 (m, 4H).

¹³C NMR (126 MHz, DMSO) δ 160.74, 157.81, 154.66, 153.80, 152.62, 150.64, 141.72, 141.01, 137.15, 136.37, 124.91, 124.81, 124.77, 114.15, 58.29, 54.88, 48.03.

HRMS (APCI): calcd, for C₂₁H₂₁N₅O [M+H]⁺ = 360.1819; found [M+H]⁺ = 360.1815

FTIR (neat), cm⁻¹: 3675, 2944, 2927, 2809, 1604, 1511, 1476, 1453, 1369, 1228, 1198, 1123, 1084, 1018, 924, 901, 886, 812, 783, 642, 542, 524, 475.

### Preparative Example 38: 6-chloro-3-(3-nitrophenyl)furo[3,2-b]pyridine

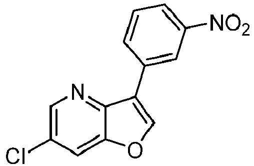

The compound was prepared by the general procedure **A** using 100 mg (0.430 mmol) of 3-bromo-6-chlorofuro[3,2-*b*]pyridine **(Prep. Example 5)** and 93 mg (0.559 mmol) of (3-nitrophenyl)boronic acid; the reaction time was 2 h; flash chromatography (cyclohexane/EtOAc, gradient from 1:0 to 9:1) afforded the compound as a yellow solid (102 mg, 86 % yield).

¹H NMR (500 MHz, Chloroform-d) δ 8.91 (t, *J* = 2.0 Hz, 1H), 8.66 (d, *J* = 2.0 Hz, 1H), 8.48 (dt, *J* = 7.8, 1.4 Hz, 1H), 8.26 (s, 1H), 8.22 (ddd, *J* = 8.2, 2.3, 1.1 Hz, 1H), 7.88 (d, *J* = 2.0 Hz, 1H), 7.66 (t, *J* = 8.0 Hz, 1H).

¹³C NMR (126 MHz, CDCl₃) δ 148.90, 148.64, 146.40, 145.84, 143.75, 133.00, 131.89, 130.02, 128.40, 122.78, 121.87, 120.08, 119.21.

HRMS (APCI): calcd, for C₁₃H₇ClN₂O₃ [M+H]⁺ = 275.0218; found [M+H]⁺ = 275.0217.

FTIR (neat), cm⁻¹: 3113, 1603, 1568, 1519, 1475, 1439, 1385, 1349, 1272, 1144, 1097, 1005, 917, 898, 887, 864, 828, 798, 783, 745, 733, 690, 672, 648, 620, 596, 533, 414.

### Preparative Example 39: tert-butyl 4-(4-(3-(3-nitrophenyl)furo[3,2-b]pyridin-6-yl)phenyl)piperazine-1-carboxylate

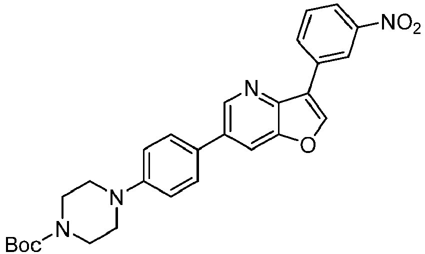

The compound was prepared by the general procedure **B** using 70 mg (0.255 mmol) 6-chloro-3-(3-nitrophenyl)furo[3,2-*b*]pyridine **(Prep. Example 38)** and 129 mg (0.331 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 2:1) afforded the compound as a pale yellow solid (35 mg, 27 % yield),

¹H NMR (500 MHz, Chloroform-*d*) δ 8.96 (t, *J* = 2.0 Hz, 1H), 8.91 (d, *J* = 1.9 Hz, 1H), 8.55 (dt, *J* = 7.8, 1.3 Hz, 1H), 8.25 (s, 1H), 8.21 (ddd, *J=* 8.2, 2.4, 1.0 Hz, 1H), 7.96 *(d, J=* 1.8 Hz, 1H), 7.67 (t, *J* = 8.0 Hz, 1H), 7.59 (d, *J=* 8.8 Hz, 2H), 7.08 (d, *J=* 8.3 Hz, 2H), 3.63 (d, *J=* 5.3 Hz, 4H), 3.25 (t, *J=* 5.1 Hz, 4H), 1.50 (s, 9H).

¹³C NMR (126 MHz, CDCl₃) δ 154.86, 149.55, 148.90, 145.73, 145.52, 143.72, 133.64, 133.05, 132.57, 129.95, 128.43, 122.48, 121.84, 120.00, 117.02, 116.36, 80.23, 49.22, 43.70, 28.60.

HRMS (APCI): calcd, for C₂₈H₂₈N₄O₅ [M+H]⁺ = 501.2132; found [M+H]⁺ = 501.2136.

FTIR (neat), cm⁻¹ 2975, 2929, 1682, 1606, 1572, 1524, 1479, 1461, 1424, 1380, 1365, 1341, 1286, 1230, 1202, 1160, 1126, 1102, 1047, 997, 909, 892, 864, 825, 804, 787, 776, 736, 692, 676, 647, 629, 607, 532, 462, 415.

### Preparative Example 40: 3-(3-nitrophenyl)-6-(4-(piperazin-1-yl)phenyl)furo[3,2-b]pyridine

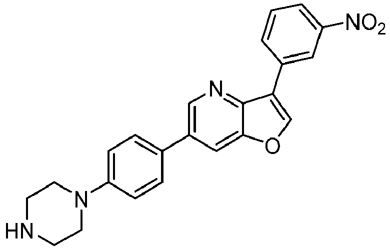

The compound was prepared by the general procedure **C** using 50 mg (0.087 mmol) of *tert*-butyl 4-(4-(3-(3-nitrophenyl)furo[3,2-*b*]pyridin-6-yl)phenyl)piperazine-1-carboxylate **(Prep. Example 39);** the reaction time was 2 h; flash chromatography (DCM/MeOH, gradient from 1:0 to 4:1) afforded the compound as a pale yellow solid (23 mg, 56 % yield).

¹H NMR (500 MHz, DMSO-*d*₆) δ 9.28 (s, 1H), 9.12 (s, 1H), 9.03 (s, 1H), 8.67 (d, *J* = 7.8 Hz, 1H), 8.40 (s, 1H), 8.23 (d, *J* = 8.2 Hz, 1H), 7.80 (dd, *J* = 23.3, 8.1 Hz, 3H), 7.14 *(d, J=* 8.3 Hz, 2H), 3.56 - 3.43 (m, 4H), 3.21 (t, *J* = 5.1 Hz, 4H).

¹³C NMR (126 MHz, DMSO) δ 149.82, 148.91, 148.28, 148.24, 144.68, 142.90, 132.51, 132.41, 132.27, 130.23, 127.94, 127.86, 122.16, 120.72, 118.01, 116.17, 116.04, 45.08, 42.41.

HRMS (APCI): calcd, for C₂₃H₂₀N₄O₃ [M+H]⁺ = 401.1608; found [M+H]⁺ = 401.1613

FTIR (neat), cm⁻¹: 2942, 2840, 2726, 2500, 2476, 1607, 1520, 1475, 1458, 1342, 1252, 1143, 1102, 917, 808, 738, 695, 676, 531.

### Preparative Example 41: 6-chloro-3-(p-tolyl)furo[3,2-b]pyridine

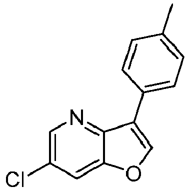

The compound was prepared by the general procedure **A** using 100 mg (0.430 mmol) of 3-bromo-6-chlorofuro[3,2-*b*]pyridine **(Prep. Example 5)** and 76 mg (0.559 mmol) of *p*-tolylboronic acid; the reaction time was 2 h; flash chromatography (cyclohexane) afforded the compound as a white solid (103 mg, 98 % yield).

¹H NMR (500 MHz, Chloroform-*d*) δ 8.61 (d, *J=* 2.1 Hz, 1H), 8.06 (s, 1H), 7.91 (d, *J=* 8.1 Hz, 2H), 7.80 (d, *J* = 2.1 Hz, 1H), 7.30 - 7.27 (m, 2H), 2.41 (s, 3H).

¹³C NMR (126 MHz, CDCl₃) δ 148.46, 145.24, 145.18, 144.63, 137.98, 129.70, 127.57, 127.10, 126.99, 121.93, 118.71,21.45.

HRMS (APCI): calcd, for C₁₄H₁₀ClNO [M+H]⁺ = 244.0524; found [M+H]⁺ = 244.0524

FTIR (neat), cm⁻¹: 3020, 2914, 2857, 1579, 1504, 1464, 1384, 1341, 1286, 1267, 1225, 1136, 1091, 1073, 966, 910, 864, 823, 802, 780, 614, 591, 523, 507.

### Preparative Example 42: tert-butyl 4-(4-(3-(p-tolyl)furo[3,2-b]pyridin-6-yl)phenyl)piperazine-1-carboxylate

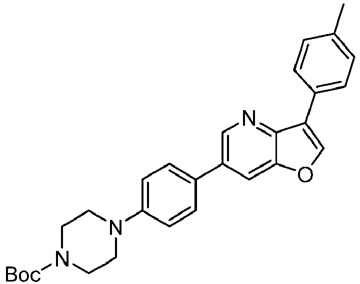

The compound was prepared by the general procedure **B** using 84 mg (0.345 mmol) of 6-chloro-3-(*p-*tolyl)furo[3,2-*b*]pyridine **(Prep. Example 41)** and 174 mg (0.448 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 9:1) afforded the compound as a white solid (23 mg, 14 % yield).

¹H NMR (500 MHz, Chloroform-*d*) δ 8.88 (d, *J* = 1.9 Hz, 1H), 8.09 (s, 1H), 7.97 (d, *J* = 8.2 Hz, 2H), 7.91 (d, *J* = 1.9 Hz, 1H), 7.58 (d, *J* = 8.7 Hz, 2H), 7.30 (d, *J* = 7.9 Hz, 2H), 7.04 (d, *J* = 8.8 Hz, 2H), 3.62 (t, *J* = 5.2 Hz, 4H), 3.23 (t, *J* = 5.2 Hz, 4H), 2.41 (s, 3H), 1.50 (s, 9H).

¹³C NMR (126 MHz, CDCl₃) δ 154.89, 151.09, 149.41, 145.02, 144.66, 144.64, 137.67, 132.93, 129.70, 129.67, 128.35, 127.69, 127.16, 121.91, 116.90, 116.04, 80.15, 49.14, 43.79, 28.60, 21.48.

HRMS (APCI): calcd, for C₂₉H₃₁N₃O₃ [M+H]⁺ = 470.2438; found [M+H]⁺ = 470.2443.

FTIR (neat), cm⁻¹ 2971, 2927, 2860, 2821, 1694, 1606, 1523, 1475, 1423, 1380, 1363, 1251, 1230, 1161, 1122, 1103, 1048, 968, 912, 825, 804, 541, 530, 506.

### Preparative Example 43: 6-(4-(piperazin-1-yl)phenyl)-3-(p-tolyl)furo[3,2-b]pyridine

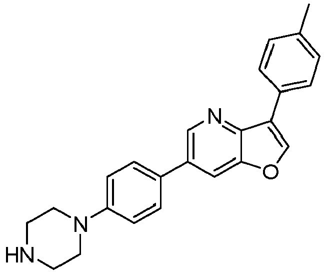

The compound was prepared by the general procedure **C** using 86 mg (0.183 mmol) of *tert*-butyl 4-(4-(3-(*p*-tolyl)furo[3,2-*b*]pyridin-6-yl)phenyl)piperazine-1-carboxylate **(Prep. Example 42);** the reaction time was 2 h; flash chromatography (DCM/MeOH, gradient from 1:0 % to 8:1) afforded the compound as a white solid (55 mg, 81 % yield).

¹H NMR (500 MHz, DMSO-*d*₆) δ 8.92 (d, *J* = 1.9 Hz, 1H), 8.77 (s, 1H), 8.27 (d, *J* = 2.0 Hz, 1H), 8.16 (d, *J* = 8.1 Hz, 2H), 7.69 (d, *J* = 8.8 Hz, 2H), 7.31 (d, *J* = 8.0 Hz, 2H), 7.05 (d, *J* = 8.9 Hz, 2H), 3.21 - 3.09 (m, 4H), 2.91 - 2.79 (m, 4H), 2.36 (s, 3H).

¹³C NMR (126 MHz, DMSO) δ 151.23, 148.79, 146.07, 144.11, 143.41, 136.79, 132.16, 129.18, 127.67, 127.51, 126.85, 126.39, 119.99, 115.46, 115.44, 48.68, 45.30, 39.52, 20.85.

HRMS (APCI): calcd, for C₂₄H₂₃N₃O [M+H]⁺ = 370.1914; found [M+H]⁺ = 370.1914.

FTIR (neat), cm⁻¹: 3254, 3029, 2945, 2918, 2833, 1608, 1523, 1478, 1448, 1380, 1337, 1240, 1215, 1196, 1125, 1091, 966, 897, 815, 799, 652, 524, 508.

### Preparative Example 44: 3-(3-(tert-butylthio)phenyl)-6-chlorofuro[3,2-b]pyridine

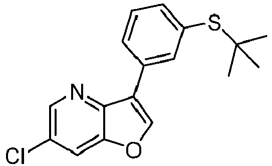

The compound was prepared by the general procedure **A** using 100 mg (0.430 mmol) of 3-bromo-6-chlorofuro[3,2-*b*]pyridine **(Prep. Example 5)** and 117 mg (0.559 mmol) of (3-(*tert-*butylthio)phenyl)boronic acid; the reaction time was 2 h; flash chromatography (cyclohexane) afforded the compound as pale yellow solid (94 mg, 68 % yield).

¹H NMR (500 MHz, Chloroform-*d*) δ 8.62 (d, *J* = 2.0 Hz, 1H), 8.17 - 8.09 (m, 3H), 7.82 (d, *J* = 2.1 Hz, 1H), 7.54 (dt, *J* = 7.7, 1.5 Hz, 1H), 7.45 (t, *J* = 7.6 Hz, 1H), 1.34 (s, 9H).

¹³C NMR (126 MHz, CDCl₃) δ 148.52, 145.72, 145.44, 144.38, 136.95, 135.80, 133.60, 130.35, 129.12, 127.82, 127.64, 121.43, 118.84, 46.23, 31.19.

HRMS (APCI): calcd, for C₁₇H₁₆ClNOS [M+H]⁺ = 318.0714; found [M+H]⁺ = 318.0711.

FTIR (neat), cm⁻¹: 2966, 2921, 2895, 2859, 1468, 1455, 1383, 1362, 1275, 1165, 1137, 1093, 1079, 992, 909, 898, 868, 806, 786, 698, 596, 516.

### Preparative Example 45: tert-butyl 4-(4-(3-(3-(tert-butylthio)phenyl)furo[3,2-b]pyridin-6-yl)phenyl)piperazine-1-carboxylate

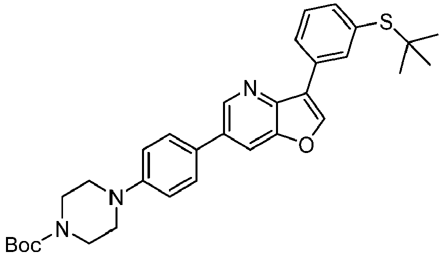

The compound was prepared by the general procedure **B** using 84 mg (0.264 mmol) of 3-(3-(*tert-b*utylthio)phenyl)-6-chlorofuro[3,2-*b*]pyridine **(Prep. Example 44)** and 133 mg (0.344 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 4:1) afforded the compound as a pale yellow solid (130 mg, 90 % yield).

¹H NMR (500 MHz, Chloroform-*d*) δ 8.88 (d, *J* = 1.8 Hz, 1H), 8.22 (dt, *J* = 7.5, 1.5 Hz, 1H), 8.19 - 8.07 (m, 2H), 7.92 (d, *J* = 1.9 Hz, 1H), 7.56 (dd, *J* = 20.2, 8.1 Hz, 3H), 7.47 (t, *J* = 7.7 Hz, 1H), 7.05 (d, *J* = 8.3 Hz, 2H), 3.62 (t, *J* = 5.2 Hz, 4H), 3.23 (t, *J* = 5.2 Hz, 4H), 1.50 (s, 9H), 1.35 (s, 9H).

¹³C NMR (126 MHz, CDCl₃) δ 154.87, 151.07, 149.43, 145.21, 145.09, 144.36, 136.69, 135.80, 133.43, 133.12, 131.03, 129.59, 129.11, 128.36, 127.78, 121.38, 116.91, 116.09, 80.16, 49.14, 46.18, 43.64, 31.21, 28.59.

HRMS (APCI): calcd. for C₃₂H₃₇N₃O₃S [M+H]⁺ = 544.2628; found [M+H]⁺ = 544.2634.

FTIR (neat), cm⁻¹ 2971, 2924, 2899, 2861, 2837, 1697, 1607, 1465, 1422, 1378, 1366, 1341, 1256, 1229, 1205, 1161, 1126, 1100, 1046, 993, 907, 812, 794, 763, 695, 534, 524.

### Preparative Example 46: 3-(3-(tert-butylthio)phenyl)-6-(4-(piperazin-1-yl)phenyl)furo[3,2-b]pyridine

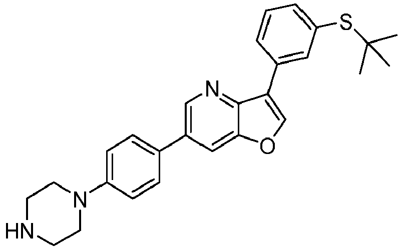

The compound was prepared by the general procedure C using 80 mg (0.147 mmol) of *tert*-butyl 4-(4-(3-(3-(*tert*-butylthio)phenyl)furo[3,2-*b*]pyridin-6-yl)phenyl)piperazine-1-carboxylate **(Prep. Example 45);** the reaction time was 2 h; flash chromatography (DCM/MeOH, gradient from 1:0 to 8:1) afforded the compound as a pale yellow solid (55 mg, 84 % yield).

¹H NMR (500 MHz, DMSO-*d*₆) δ 8.95 (d, *J* = 1.9 Hz, 1H), 8.90 (s, 1H), 8.48 (t, *J* = 1.8 Hz, 1H), 8.31 (d, *J* = 1.9 Hz, 1H), 8.27 (dt, *J* = 7.6, 1.6 Hz, 1H), 7.79 - 7.65 (m, 2H), 7.61 - 7.40 (m, 2H), 7.21 - 6.95 (m, 2H), 3.15 (dd, *J* = 6.1, 4.0 Hz, 4H), 2.98 - 2.76 (m, 4H), 1.31 (s, 9H).

¹³C NMR (126 MHz, DMSO) δ 151.24, 148.87, 146.99, 144.33, 143.14, 135.78, 134.66, 132.60, 132.35, 131.00, 128.99, 127.69, 126.83, 126.74, 119.29, 115.55, 115.45, 48.64, 45.72, 45.29, 30.71.

HRMS (APCI): calcd, for C₂₇H₂₉N₃OS [M+H]⁺ = 444.2104; found [M+H]⁺ = 444.2107.

FTIR (neat), cm⁻¹: 3313, 2973, 2958, 2940, 2924, 2854, 1596, 1526, 1484, 1454, 1382, 1364, 1336, 1241, 1206, 1147, 1126, 1099, 888, 836, 821, 790, 773, 695, 658, 541, 521.

### Preparative Example 47: 4-(3-(pyridin-4-yl)furo[3,2-b]pyridin-6-yl)aniline

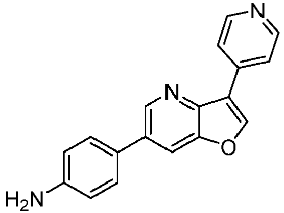

The compound was prepared by the general procedure **B** using 60 mg (0.260 mmol) of 6-chloro-3-(pyridin-4-yl)furo[3,2-*b*]pyridine **(Prep. Example 21)** and 74 mg (0.338 mmol) of 4-(4,4,5,5-tetramethyl-1,3,2-dioxaborolan-2-yl)aniline; the reaction time was 2 h; flash chromatography (cyclohexane/EtOAc, gradient from 1:0 to 0:1) afforded the compound as a pale yellow solid (71 mg, 95 % yield).

¹H NMR (500 MHz, DMSO-*d*₆) δ 9.04 (s, 1H), 8.91 (d, *J* = 1.9 Hz, 1H), 8.69 - 8.66 (m, 2H), 8.28 - 8.23 (m, 3H), 7.56 - 7.48 (m, 2H), 6.81 - 6.65 (m, 2H), 5.40 (bs, 2H).

¹³C NMR (126 MHz, DMSO) δ 150.01, 149.13, 149.06, 148.59, 144.34, 142.18, 138.07, 133.44, 127.82, 123.76, 120.68, 117.78, 115.20, 114.31.

HRMS (APCI): calcd. for C₁₈H₁₃N₃O [M+H]⁺ = 288.1131; found [M+H]⁺ = 288.1128.

FTIR (neat), cm⁻¹ 3435, 3307, 3167, 1642, 1601, 1524, 1477, 1417, 1376, 1360, 1312, 1261, 1202, 1179, 1127, 1104, 882, 827, 808, 780, 732, 670, 660, 652, 634, 531, 521.

### Preparative Example 48: 6-(6-(piperidin-1-yl)pyridin-3-yl)-3-(pyridin-4-yl)furo[3,2-b]pyridine

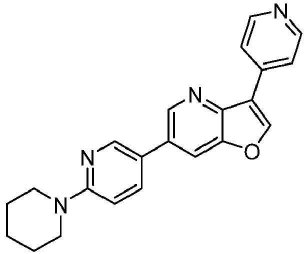

The compound was prepared by the general procedure **B** using 60 mg (0.260 mmol) of 6-chloro-3-(pyridin-4-yl)furo[3,2-*b*]pyridine **(Prep. Example 21)** and 97 mg (0.338 mmol) of 6-(piperidin-1-yl)pyridine-3-boronic acid pinacol ester; the reaction time was 2 h; flash chromatography (cyclohexane/EtOAc, gradient from 1:0 to 0:1) afforded the compound as a pale yellow solid (71 mg, 77 % yield).

¹H NMR (500 MHz, Chloroform-*d*) δ 8.86 (d, *J* = 1.9 Hz, 1H), 8.75 - 8.69 (m, 2H), 8.49 (dd, *J* = 2.6, 0.7 Hz, 1H), 8.27 (s, 1H), 8.10 - 8.02 (m, 2H), 7.92 *(d, J=* 1.9 Hz, 1H), 7.74 (dd, *J* = 8.9, 2.6 Hz, 1H), 6.78 (dd, *J* = 8.9, 0.8 Hz, 1H), 3.64 (dd, *J* = 5.5, 3.3 Hz, 4H), 1.69 (dd, *J* = 7.5, 3.5 Hz, 6H).

¹³C NMR (126 MHz, CDCl₃) δ 159.08, 150.40, 149.65, 146.53, 145.05, 143.78, 138.57, 136.39, 131.51, 121.75, 121.45, 119.63, 115.84, 107.18, 46.46, 25.69, 24.87.

HRMS (APCI): calcd, for C₂₂H₂₀N₄O [M+H]⁺ = 357.1710; found [M+H]⁺ = 357.1709.

FTIR (neat), cm⁻¹ 2934, 2852, 1602, 1510, 1477, 1450, 1408, 1247, 1206, 1127, 809.

### Preparative Example 49: 6-chloro-3-(4-fluorophenyl)furo[3,2-b]pyridine

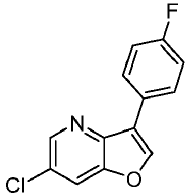

The compound was prepared by the general procedure **A** using 100 mg (0.430 mmol) of 3-bromo-6-chlorofuro[3,2-*b*]pyridine **(Prep. Example 5)** and 78 mg (0.559 mmol) of (4-fluorophenyl)boronic acid; the reaction time was 2 h; flash chromatography (cyclohexane) afforded the compound as a pale yellow solid (70 mg, 66 % yield).

¹H NMR (500 MHz, Chloroform-*d*) δ 8.61 (d, *J* = 2.0 Hz, 1H), 8.08 (s, 1H), 8.02 (dd, *J* = 8.8, 5.3 Hz, 2H), 7.83 (d, *J=* 2.0 Hz, 1H), 7.17 (t, *J=* 8.7 Hz, 2H).

¹³C NMR (126 MHz, Chloroform-*d*) δ 162.74 (d, *J* = 247.5 Hz), 148.53, 145.37, 145.30 (d, *J* = 1.4 Hz), 144.35, 128.99 (d, *J* = 8.0 Hz), 127.88, 126.08 (d, *J* = 3.4 Hz), 121.16, 118.94, 116.06 (d, *J=* 21.6 Hz). HRMS (APCI): calcd, for C₁₃H₇ClFNO [M+H]⁺ = 248.0273; found [M+H]⁺ = 248.0275.

FTIR (neat), cm⁻¹: 3151, 3075, 1568, 1504, 1461, 1386, 1267, 1217, 1163, 1134, 1087, 1071, 967, 912, 871, 833, 798, 783, 713, 611, 585, 521, 506.

### Preparative Example 50: tert-butyl 4-(4-(3-(4-fluorophenyl)furo[3,2-b]pyridin-6-yl)phenyl)piperazine-1-carboxylate

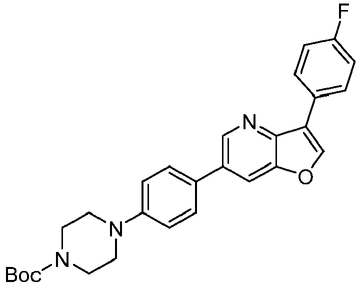

The compound was prepared by the general procedure **B** using 85 mg (0.343 mmol) of 6-chloro-3-(4-fluorophenyl)furo[3,2-*b*]pyridine **(Prep. Example 49)** and 173 mg (0.446 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 2:1) afforded the compound as a white solid (130 mg, 80 % yield).

¹H NMR (500 MHz, Chloroform-d) δ 8.87 (d, *J* = 1.9 Hz, 1H), 8.16 - 8.01 (m, 3H), 7.93 (d, *J* = 1.9 Hz, 1H), 7.58 (d, *J* = 8.8 Hz, 2H), 7.18 (t, *J* = 8.7 Hz, 2H), 7.06 (d, *J* = 8.3 Hz, 2H), 3.66 - 3.55 (m, 4H), 3.23 (t, *J* = 5.2 Hz, 4H), 1.50 (s, 9H).

¹³C NMR (126 MHz, Chloroform-*d*) δ 162,60 (d,J= 247.2 Hz), 154.88, 151.07, 149.42, 145.12, 144.65, 144.31, 133.17, 128.96 (d, *J* = 7.9 Hz), 128.39, 126.74 (d, *J* = 3.4 Hz), 121.08, 116.95, 116.19, 115.99 (d, *J* = 21.6 Hz), 80.19, 49.18, 43.53, 28.61.

HRMS (APCI): calcd, for C₂₈H₂₈FN₃O₃ [M+H]⁺ = 474.2187; found [M+H]⁺ = 474.2187.

FTIR (neat), cm⁻¹ 2975, 2930, 2836, 1686, 1609, 1524, 1504, 1480, 1414, 1380, 1364, 1239, 1203, 1159, 1124, 1104, 1047, 912, 844, 823, 810, 773, 585, 548, 529.

### Preparative Example 51: 3-(4-fluorophenyl)-6-(4-(piperazin-1-yl)phenyl)furo[3,2-b]pyridine

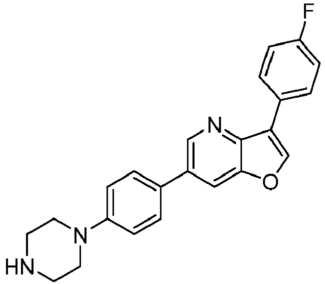

The compound was prepared by the general procedure **C** using 85 mg (0.180 mmol) of *tert*-butyl 4-(4-(3-(4-fluorophenyl)furo[3,2-*b*]pyridin-6-yl)phenyl)piperazine-1-carboxylate **(Prep. Example 50);** the reaction time was 2 h; flash chromatography (DCM/MeOH, gradient from 1:0 to 4:1) afforded the compound as a pale yellow solid (60 mg, 90 % yield).

¹H NMR (500 MHz, DMSO-*d*₆) δ 8.94 (d, *J* = 1.9 Hz, 1H), 8.83 (s, 1H), 8.36 - 8.23 (m, 3H), 7.79 - 7.66 (m, 2H), 7.35 (t, *J* = 8.9 Hz, 2H), 7.21 - 7.05 (m, 2H), 3.32 (dd, *J* = 6.5, 3.8 Hz, 4H), 3.07 (dd, *J* = 6.6, 3.6 Hz, 4H).

¹³C NMR (126 MHz, DMSO) δ 162.49, 160.54, 150.48, 148.77, 146.49, 144.26, 143.24, 132.19, 128.52, 128.45, 127.80, 127.45, 126.90, 126.88, 119.09, 115.85, 115.70, 115.65, 115.48, 46.86, 43.86. HRMS (APCI): calcd, for C₂₃H₂₀FN₃O [M+H]⁺ = 374.1663; found [M+H]⁺ = 374.1663.

FTIR (neat), cm⁻¹: 2931, 2835, 2700, 2612, 2496, 2475, 1605, 1573, 1524, 1504, 1481, 1455, 1378, 1340, 1245, 1217, 1201, 1162, 1123, 1095, 968, 916, 890, 846, 831, 801, 652, 584, 544, 525.

### Preparative Example 52: 3-(6-chlorofuro[3,2-b]pyridin-3-yl)-N,N-dimethylbenzamide

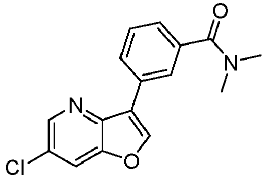

The compound was prepared by the general procedure **A** using 100 mg (0.430 mmol) of 3-bromo-6-chlorofuro[3,2-*b*]pyridine **(Prep. Example 5)** and 108 mg (0.559 mmol) of (3-(dimethylcarbamoyl)phenyl)boronic acid; the reaction time was 2 h; flash chromatography (cyclohexane/EtOAc, gradient from 1:0 to 1:1) afforded the compound as a pale yellow solid (105 mg, 81 % yield).

¹H NMR (500 MHz, Chloroform-d) δ 8.61 (d, *J* = 2.0 Hz, 1H), 8.13 (d, *J* = 8.9 Hz, 2H), 8.09 (t, *J* = 1.7 Hz, 1H), 7.83 (d, *J* = 2.0 Hz, 1H), 7.51 (t, *J* = 7.7 Hz, 1H), 7.41 (dt, *J* = 7.6, 1.4 Hz, 1H), 3.10 (d, *J* = 48.2 Hz, 6H).

¹³C NMR (126 MHz, CDCl₃) δ 171.45, 148.55, 145.93, 145.42, 144.29, 137.12, 130.34, 129.04, 128.32, 127.89, 126.70, 125.93, 121.33, 118.94, 39.82, 35.59.

HRMS (APCI): calcd, for C₁₆H₁₃ClN₂O₂ [M+H]⁺ = 301.0738; found [M+H]⁺ = 301.0741.

FTIR (neat), cm⁻¹: 3091, 3064, 2924, 1618, 1586, 1503, 1467, 1384, 1263, 1198, 1133, 1087, 906, 893, 869, 801, 776, 761, 746, 693, 663, 596, 523.

### Preparative Example 53: tert-butyl 4-(4-(3-(3-(dimethylcarbamoyl)phenyl)furo[3,2-b]pyridin-6-yl)phenyl)piperazine-1-carboxylate

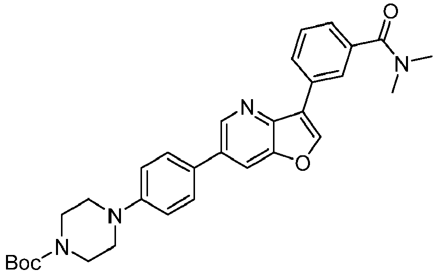

The compound was prepared by the general procedure **B** using 85 mg (0.283 mmol) of 3-(6-chlorofuro[3,2-*b*]pyridin-3-yl)-*N*,*N*-dimethylbenzamide **(Prep. Example 52)** and 143 mg (0.367 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 2:3) afforded the compound as a pale yellow solid (99 mg, 67 % yield).

¹H NMR (500 MHz, Chloroform-d) δ 8.87 (d, *J* = 1.9 Hz, 1H), 8.21 (dt, *J* = 7.8, 1.4 Hz, 1H), 8.14 (s, 1H), 8.13 (t, *J* = 1.7 Hz, 1H), 7.92 (d, *J* = 2.0 Hz, 1H), 7.60 - 7.54 (m, 2H), 7.52 (t, *J* = 7.7 Hz, 1H), 7.40 (dt, *J* = 7.6, 1.4 Hz, 1H), 7.08 - 6.85 (m, 2H), 3.65 - 3.59 (m, 4H), 3.23 (t, *J* = 5.2 Hz, 4H), 3.15 (s, 3H), 3.06 (s, 3H), 1.50 (s, 9H).

¹³C NMR (126 MHz, CDCl₃) δ 171.57, 154.86, 151.08, 149.43, 145.26, 145.16, 144.23, 137.03, 133.17, 130.99, 129.46, 128.99, 128.37, 128.34, 126.40, 125.85, 121.23, 116.87, 116.13, 80.14, 49.09, 43.56, 39.83, 35.56, 28.58.

HRMS (APCI): calcd, for C₃₁H₃₄N₄O₄ [M+H]⁺ = 527.2653; found [M+H]⁺ = 527.2656.

FTIR (neat), cm⁻¹ 3098,2974, 2926,2862, 2827, 1688, 1604, 1526, 1478, 1449, 1426, 1404, 1386, 1363, 1261, 1238, 1162, 1125, 1104, 1077, 1051, 1008, 998, 914, 822, 807, 793, 743, 698, 531.

### Preparative Example 54: N,N-dimethyl-3-(6-(4-(piperazin-1-yl)phenyl)furo[3,2-b]pyridin-3-yl)benzamide

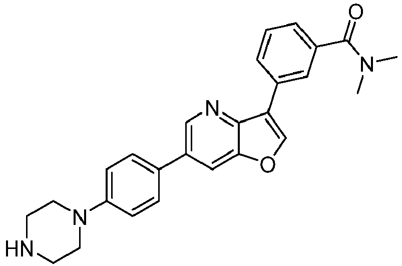

The compound was prepared by the general procedure **C** using 85 mg (0.161 mmol) of *tert*-butyl 4-(4-(3-(3-(dimethylcarbamoyl)phenyl)furo[3,2-*b*]pyridin-6-yl)phenyl)piperazine-1-carboxylate **(Prep. Example 53);** the reaction time was 2 h; flash chromatography (DCM/MeOH, gradient from 1:0 to 4:1) afforded the compound as a pale yellow solid (59 mg, 86 % yield).

¹H NMR (500 MHz, DMSO-*d*₆) δ 8.96 (d, *J* = 1.9 Hz, 1H), 8.92 (s, 1H), 8.37 - 8.27 (m, 3H), 7.76 - 7.68 (m, 2H), 7.56 (t, *J* = 7.7 Hz, 1H), 7.38 (dt, *J* = 7.6, 1.4 Hz, 1H), 7.15 - 7.03 (m, 2H), 3.32 - 3.26 (m, 4H), 3.07 - 2.95 (m, 10H).

¹³C NMR (126 MHz, DMSO) δ 171.90, 169.96, 150.61, 148.85, 147.14, 144.36, 143.24, 137.04, 132.23, 130.57, 128.62, 127.78, 127.33, 127.12, 125.80, 124.80, 119.38, 115.79, 115.70, 47.14, 44.10, 21.01. HRMS (APCI): calcd, for C₂₆H₂₆N₄O₂ [M+H]⁺ = 427.2129; found [M+H]⁺ = 427.2130

FTIR (neat), cm⁻¹: 2931, 2830, 2717, 2477, 1604, 1524, 1380, 1248, 1189, 1146, 1086, 919, 820, 790, 780, 745, 667, 536, 523.

### Preparative Example 55: 6-chloro-3-(furan-3-yl)furo[3,2-b]pyridine

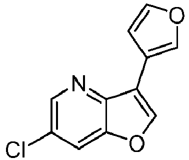

The compound was prepared by the general procedure **A** using 100 mg (0.430 mmol) of 3-bromo-6-chlorofuro[3,2-*b*]pyridine **(Prep. Example 5)** and 72 mg (0.645 mmol) of furan-3-ylboronic acid acid; the reaction time was 2 h; flash chromatography (cyclohexane/EtOAc, gradient from 1:0 to 4:1) afforded the compound as a pale yellow solid (56 mg, 59 % yield).

¹H NMR (500 MHz, Chloroform-d) δ 8.59 (d, *J* = 2.0 Hz, 1H), 8.37 - 8.30 (m, 1H), 7.98 (s, 1H), 7.79 (d, *J* = 2.0 Hz, 1H), 7.53 (t, *J* = 1.8 Hz, 1H), 6.75 (d, *J* = 1.9 Hz, 1H).

¹³C NMR (126 MHz, CDCl₃) δ 148.19, 145.31, 144.66, 144.34, 143.51, 141.18, 127.89, 118.73, 115.08, 114.19, 108.45.

HRMS (APCI): calcd, for C₁₁H₆ClNO₂ [M+H]⁺ = 220.0160; found [M+H]⁺ = 220.0161.

FTIR (neat), cm⁻¹: 3156, 3126, 3066, 1463, 1379, 1274, 1160, 1145, 1085, 1069, 1025, 1004, 918, 881, 870, 779, 728, 651, 602, 586, 521.

### Preparative Example 56: tert-butyl 4-(4-(3-(furan-3-yl)furo[3,2-b]pyridin-6-yl)phenyl)piperazine-1-carboxylate

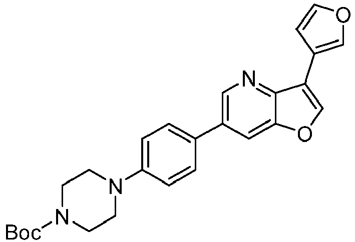

The compound was prepared by the general procedure **B** using 78 mg (0.355 mmol) of 6-chloro-3-(furan-3-yl)furo[3,2-*b*]pyridine **(Prep. Example 55)** and 179 mg (0.462 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 9:1) afforded the compound as a brown solid (116 mg, 73 % yield).

¹H NMR (500 MHz, Chloroform-d) δ 8.84 (d, *J* = 1.9 Hz, 1H), 8.39 (t, *J* = 1.2 Hz, 1H), 7.98 (s, 1H), 7.88 (d, *J* = 1.9 Hz, 1H), 7.59 - 7.51 (m, 3H), 7.09 - 7.00 (m, 2H), 6.78 (dd, *J* = 1.8, 0.8 Hz, 1H), 3.74 - 3.53 (m, 4H), 3.22 (t, *J* = 5.2 Hz, 4H), 1.50 (s, 9H).

¹³C NMR (126 MHz, CDCl₃) δ 154.85, 151.06, 149.05, 145.05, 144.29, 144.00, 143.36, 140.98, 133.20, 129.58, 128.33, 116.86, 115.95, 114.89, 114.71, 108.58, 80.12, 49.09, 43.60, 28.58.

HRMS (APCI): calcd, for C₂₆H₂₇N₃O₄ [M+H]⁺ = 446.2074; found [M+H]⁺ = 446.2073.

FTIR (neat), cm⁻¹ 2975, 2916, 2864, 2836, 1687, 1606, 1522, 1478, 1459, 1421, 1366, 1337, 1265, 1232, 1207, 1157, 1122, 1089, 1045, 1031, 998, 912, 887, 871, 825, 785, 765, 732, 592, 548, 526.

### Preparative Example 57: 3-(furan-3-yl)-6-(4-(piperazin-1-yl)phenyl)furo[3,2-b]pyridine

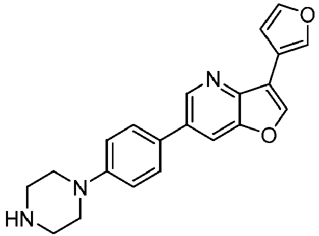

The compound was prepared by the general procedure **C** using 70 mg (0.157 mmol) of *tert*-butyl 4-(4-(3-(furan-3-yl)furo[3,2-*b*]pyridin-6-yl)phenyl)piperazine-1-carboxylate **(Prep. Example 56);** the reaction time was 2 h; flash chromatography (DCM/MeOH, gradient from 1:0 to 4:1) afforded the compound as a brown solid (33 mg, 61 % yield).

¹H NMR (500 MHz, DMSO-*d*₆) δ 8.90 (d, *J* = 1.9 Hz, 1H), 8.63 (s, 1H), 8.51- 8.41 (m, 1H), 8.26 (d, *J* = 1.9 Hz, 1H), 7.82 (t, *J* = 1.7 Hz, 1H), 7.68 (d, *J* = 8.8 Hz, 2H), 7.11 - 7.02 (m, 3H), 5.75 (s, DCM), 3.18 - 3.12 (m, 4H), 2.93 - 2.83 (m, 4H).

¹³C NMR (126 MHz, DMSO) δ 151.34, 148.44, 145.55, 144.28, 143.73, 143.06, 139.96, 132.52, 127.70, 126.79, 115.43, 115.41, 114.70, 113.67, 108.70, 48.90, 45.47.

HRMS (APCI): calcd, for C₂₁H₁₉N₃O₂ [M+H]⁺ = 346.1550; found [M+H]⁺ = 346.1551.

FTIR (neat), cm⁻¹: 3243, 3084, 2949, 2833, 1605, 1522, 1478, 1446, 1377, 1339, 1236, 1204, 1154, 1133, 1078, 1067, 1027, 882, 871, 815, 792, 750, 737, 700, 591, 544, 516.

### Preparative Example 58: 6-(4-(4-methylpiperazin-1-yl)phenyl)-3-(pyridin-4-yl)furo[3,2-b]pyridine

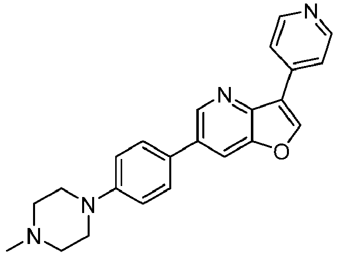

The compound was prepared by the general procedure **B** using 50 mg (0.217 mmol) of 6-chloro-3-(pyridin-4-yl)furo[3,2-*b*]pyridine **(Prep. Example 21)** and 85 mg (0.282 mmol) of 1-methyl-4-(4-(4,4,5,5-tetramethyl-1,3,2-dioxaborolan-2-yl)phenyl)piperazine; the reaction time was 2 h; flash chromatography (DCM/MeOH, gradient from 1:0 to 9:1) afforded the compound as a yellow solid (55 mg, 68 % yield).

¹H NMR (500 MHz, DMSO-*d*₆) δ 9.08 (s, 1H), 8.97 (d, *J* = 1.9 Hz, 1H), 8.69 (d, *J* = 5.2 Hz, 2H), 8.36 (d, *J* = 1.9 Hz, 1H), 8.30 - 8.21 (m, 2H), 7.71 (d, *J* = 8.5 Hz, 2H), 7.08 (d, *J* = 8.5 Hz, 2H), 3.23 (t, *J* = 5.0 Hz, 4H), 4.49 (t, *J* = 5.0 Hz, 4H), 2.25 (s, 3H).

¹³C NMR (126 MHz, DMSO) δ 150.79, 150.08, 149.04, 148.93, 144.63, 142.75, 137.94, 132.70, 127.77, 126.64, 120.68, 117.81, 115.85, 115.49, 54.42, 47.57, 45.68.

HRMS (APCI): calcd, for C₂₃H₂₂N₄O [M+H]⁺ = 371.1866; found [M+H]⁺ = 371.1866.

FTIR (neat), cm⁻¹ 3076, 3055, 2963, 2936, 2844, 2801, 1603, 1524, 1479, 1382, 1293, 1244, 1202, 1161, 1145, 1087, 1008, 978, 921, 815, 789, 672, 638, 526.

### Preparative Example 59: 3-(6-chlorofuro[3,2-b]pyridin-3-yl)-N,N-dimethylbenzenesulfonamide

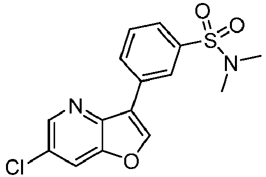

The compound was prepared by the general procedure **A** using 100 mg (0.430 mmol) of 3-bromo-6-chlorofuro[3,2-*b*]pyridine **(Prep. Example 5)** and 174 mg (0.559 mmol) of *N*,*N*-dimethyl-3-(4,4,5,5-tetramethyl-1,3,2-dioxaborolan-2-yl)benzenesulfonamide; the reaction time was 2 h; flash chromatography (cyclohexane/EtOAc, gradient from 1:0 to 4:1) afforded the compound as a white solid (112 mg, 77 % yield).

¹H NMR (500 MHz, Chloroform-d) δ 8.67 - 8.58 (m, 1H), 8.41 (s, 1H), 8.37 (d, *J* = 7.7 Hz, 1H), 8.22 (s, 1H), 7.92 - 7.82 (m, 1H), 7.77 (d, *J* = 7.8 Hz, 1H), 7.66 (t, *J* = 7.8 Hz, 1H), 2.78 (s, 6H).

¹³C NMR (126 MHz, CDCl₃) δ 148.57, 146.24, 145.70, 143.95, 136.61, 131.32, 129.74, 128.19, 127.04, 126.08, 120.53, 119.08, 38.15.

HRMS (APCI): calcd, for C₁₅H₁₃ClN₂O₃S [M+H]⁺ = 337.0408; found [M+H]⁺ = 337.0406.

FTIR (neat), cm⁻¹: 3069, 1387, 1333, 1311, 1269, 1159, 1137, 1096, 986, 958, 917, 873, 835, 801, 784, 715,694,599,578,565,488.

### Preparative Example 60: tert-butyl 4-(4-(3-(3-(N,N-dimethylsulfamoyl)phenyl)furo[3,2-b]pyridin-6-yl)phenyl)piperazine-1-carboxylate

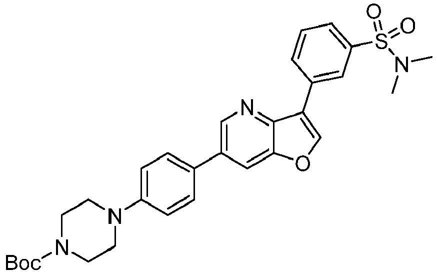

The compound was prepared by the general procedure **B** using 100 mg (0.297 mmol) of 3-(6-chlorofuro[3,2-*b*]pyridin-3-yl)-*N*,*N*-dimethylbenzenesulfonamide **(Prep. Example 59)** and 150 mg (0.386 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 1:1) afforded the compound as a white solid (124 mg, 74 % yield).

¹H NMR (500 MHz, Chloroform-d) δ 8.88 (d, *J* = 1.9 Hz, 1H), 8.47 (dt, *J* = 7.8, 1.4 Hz, 1H), 8.44 (t, *J* = 1.8 Hz, 1H), 8.21 (s, 1H), 7.94 (d, *J* = 1.9 Hz, 1H), 7.76 (dt, *J* = 7.8, 1.5 Hz, 1H), 7.67 (t, *J* = 7.8 Hz, 1H), 7.62 - 7.53 (m, 2H), 7.06 (d, *J* = 8.3 Hz, 2H), 3.62 (t, *J* = 5.2 Hz, 4H), 3.24 (t, *J* = 5.2 Hz, 4H), 2.79 (s, 6H), 1.50 (s, 9H).

¹³C NMR (126 MHz, CDCl₃) δ 154.87, 149.49, 145.56, 145.41, 143.89, 136.45, 133.49, 132.01, 131.44, 129.70, 128.38, 126.76, 126.05, 120.47, 116.92, 116.26, 80.20, 49.12, 43.53, 38.17, 28.58.

HRMS (APCI): calcd, for C₃₀H₃₄N₄O₅S [M+H]⁺ = 563.2323; found [M+H]⁺ = 563.2321.

FTIR (neat), cm⁻¹ 2973, 2926, 2856, 1692, 1606, 1480, 1422, 1383, 1366, 1341, 1234, 1160, 1126, 714, 694, 582.

### Preparative Example 61: N,N-dimethyl-3-(6-(4-(piperazin-1-yl)phenyl)furo[3,2-b]pyridin-3-yl)benzenesulfonamide

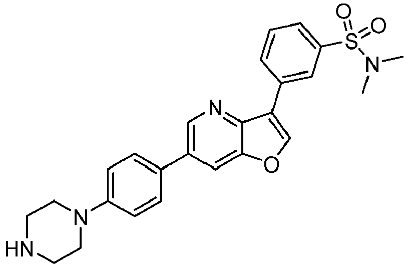

The compound was prepared by the general procedure **C** using 90 mg (0.160 mmol) of *tert*-butyl 4-(4-(3-(3-(*N*,*N*-dimethylsulfamoyl)phenyl)furo[3,2-*b*]pyridin-6-yl)phenyl)piperazine-1-carboxylate **(Prep. Example 60);** the reaction time was 2 h; flash chromatography (DCM/MeOH, gradient from 1:0 to 4:1) afforded the compound as a pale yellow solid (69 mg, 93 % yield).

¹H NMR (500 MHz, DMSO-*d*₆) δ 9.05 (s, 1H), 9.00 (d, *J* = 1.9 Hz, 1H), 8.73 (t, *J* = 1.9 Hz, 1H), 8.54 (dt, *J* = 7.7, 1.6 Hz, 1H), 8.38 (d, *J* = 1.9 Hz, 1H), 7.87 - 7.69 (m, 5H), 7.12 (d, *J* = 8.9 Hz, 2H), 3.40 (dd, *J* = 6.7, 3.7 Hz, 4H), 3.17 (dd, *J* = 6.5, 3.8 Hz, 4H), 2.69 (s, 6H).

¹³C NMR (126 MHz, DMSO) δ 148.90, 147.91, 144.61, 143.05, 135.52, 132.34, 131.69, 130.61, 129.76, 127.88, 126.35, 125.00, 118.60, 116.04, 115.90, 45.84, 43.10, 37.61.

HRMS (APCI): calcd, for C₂₅H₂₆N₄O₃S [M+H]⁺ = 463.1798; found [M+H]⁺ = 463.1794.

FTIR (neat), cm⁻¹: 2927, 2841, 2726, 2500, 2482, 1605, 1336, 1159, 1094, 953, 822, 803, 782, 714, 693, 581, 544, 492.

### Preparative Example 62: 3-(6-chlorofuro[3,2-b]pyridin-3-yl)benzenesulfonamide

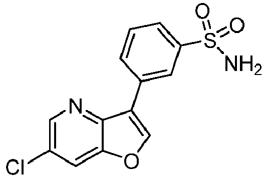

The compound was prepared by the general procedure **A** using 100 mg (0.430 mmol) of 3-bromo-6-chlorofuro[3,2-*b*]pyridine **(Prep. Example 5)** and 158 mg (0.559 mmol) of 3-(4,4,5,5-tetramethyl-1,3,2-dioxaborolan-2-yl)benzenesulfonamide; the reaction time was 2 h; flash chromatography (cyclohexane/EtOAc, gradient from 1:0 to 1:2) afforded the compound as a white solid (110 mg, 83 % yield).

¹H NMR (500 MHz, DMSO-*d*₆) δ 9.01 (s, 1H), 8.73 (d, *J* = 2.1 Hz, 1H), 8.70 (t, *J* = 1.8 Hz, 1H), 8.46 (d, *J* = 2.1 Hz, 1H), 8.35 (dt, *J* = 7.8, 1.4 Hz, 1H), 7.85 (ddd, *J* = 7.8, 1.9, 1.1 Hz, 1H), 7.71 (t, *J* = 7.8 Hz, 1H), 7.42 (s, 2H).

¹³C NMR (126 MHz, DMSO) δ 148.60, 147.98, 144.95, 144.78, 143.47, 130.49, 129.59, 129.46, 126.99, 124.99, 123.47, 119.60, 119.04.

HRMS (APCI): calcd, for C₁₃H₉ClN₂O₃S [M+H]⁺ = 309.0095; found [M+H]⁺ = 309.0093.

FTIR (neat), cm⁻¹: 3319, 3232, 3073, 1385, 1326, 1315, 1273, 1161, 1138, 1101, 1076, 999, 886, 792, 771, 705, 681, 649, 597, 559, 525, 493.

### Preparative Example 63: tert-butyl 4-(4-(3-(3-sulfamoylphenyl)furo[3,2-b]pyridin-6-yl)phenyl)piperazine-1-carboxylate

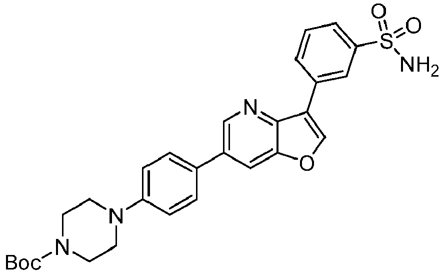

The compound was prepared by the general procedure **B** using 100 mg (0.324 mmol) of 3-(6-chlorofuro[3,2-*b*]pyridin-3-yl)benzenesulfonamide **(Prep. Example 62)** and 164 mg (0.421 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 1:4) afforded the compound as a white solid (102 mg, 59 % yield).

¹H NMR (500 MHz, DMSO-*d*₆) δ 8.97 (d, *J* = 1.9 Hz, 1H), 8.94 (s, 1H), 8.80 (t, *J* = 1.8 Hz, 1H), 8.42 (dt, *J* = 7.7, 1.4 Hz, 1H), 8.35 (d, *J* = 1.9 Hz, 1H), 7.86 - 7.80 (m, 1H), 7.77 - 7.68 (m, 3H), 7.41 (s, 2H), 7.13 - 7.05 (m, 2H), 3.49 (dd, *J* = 6.6, 3.8 Hz, 4H), 3.25 - 3.14 (m, 4H), 1.43 (s, 9H). ¹³C NMR (126 MHz, DMSO) δ 153.82, 150.63, 148.88, 147.43, 144.74, 144.46, 142.98, 132.45, 131.16, 129.48, 129.36, 127.82, 127.32, 124.72, 123.43, 119.00, 116.04, 115.81, 78.96, 47.84, 28.03.

HRMS (APCI): calcd, for C₂₈H₃₀N₄O₅S [M+H]⁺ = 535.2010; found [M+H]⁺ = 535.2006.

FTIR (neat), cm⁻¹ 3274, 2975, 2818, 1699, 1606, 1525, 1486, 1416, 1335, 1263, 1232, 1199, 1159, 1125, 1087, 988, 919, 816, 799, 690, 588, 541, 523, 506.

### Preparative Example 64: 3-(6-(4-(piperazin-1-yl)phenyl)furo[3,2-b]pyridin-3-yl)benzenesulfonamide

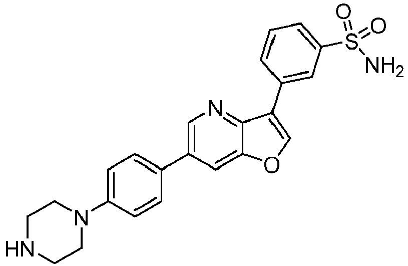

The compound was prepared by the general procedure **C** using 90 mg (0.168 mmol) of *tert*-butyl 4-(4-(3-(3-sulfamoylphenyl)furo[3,2-*b*]pyridin-6-yl)phenyl)piperazine-1-carboxylate **(Prep. Example 63);** the reaction time was 2 h; flash chromatography (DCM/MeOH, gradient from 1:0 to 4:1) afforded the compound as a pale yellow solid (44 mg, 60 % yield).

¹H NMR (500 MHz, DMSO-*d*₆) δ 8.97 (d, *J* = 1.9 Hz, 1H), 8.93 (s, 1H), 8.79 (d, *J* = 1.9 Hz, 1H), 8.42 (d, *J* = 7.7 Hz, 1H), 8.34 (d, *J* = 1.9 Hz, 1H), 7.87 - 7.81 (m, 1H), 7.71 (dt, *J* = 7.9, 3.5 Hz, 3H), 7.41 (s, 2H), 7.06 (d, *J* = 8.5 Hz, 2H), 3.14 (t, *J* = 5.0 Hz, 4H), 2.87 (t, *J* = 5.0 Hz, 4H).

¹³C NMR (126 MHz, DMSO) δ 151.37, 148.90, 147.34, 144.73, 144.40, 142.87, 132.58, 131.18, 129.48, 129.35, 127.73, 126.61, 124.70, 123.42, 119.00, 115.67, 115.42, 48.82, 45.42.

HRMS (APCI): calcd, for C₂₃H₂₂N₄O₃S [M+H]⁺ = 435.1485; found [M+H]⁺ = 435.1489.

FTIR (neat), cm⁻¹: 3325, 3304, 2826, 1603, 1527, 1484, 1380, 1325, 1308, 1240, 1205, 1148, 1118, 1101, 1088, 1004, 922, 890, 878, 832, 815, 794, 686, 659, 591, 548, 533, 523, 513, 474.

### Preparative Example 64: 6-chloro-3-(4-isopropylphenyl)furo[3,2-b]pyridine

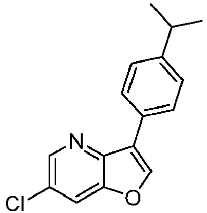

The compound was prepared by the general procedure **A** using 108 mg (0.465 mmol) of 3-bromo-6-chlorofuro[3,2-*b*]pyridine **(Prep. Example 5)** and 99 mg (0.604 mmol) of (4-isopropylphenyl)boronic acid; the reaction time was 2 h; flash chromatography (cyclohexane) afforded the compound as a pale yellow solid (110 mg, 83 % yield).

¹H NMR (500 MHz, Chloroform-d) δ 8.61 (d, *J* = 2.1 Hz, 1H), 8.07 (s, 1H), 7.92 (d, *J* = 8.3 Hz, 2H), 7.81 (d, *J* = 2.0 Hz, 1H), 7.35 (d, *J* = 8.0 Hz, 2H), 2.96 (p, *J* = 6.9 Hz, 1H), 1.29 (d, *J* = 6.9 Hz, 6H). ¹³C NMR (126 MHz, CDCl₃) δ 149.04, 148.50, 145.30, 145.26, 144.71, 127.62, 127.37, 127.34, 127.15, 122.11, 118.78, 34.16, 24.09.

HRMS (APCI): calcd, for C₁₆H₁₄ClNO [M+H]⁺ = 272.0837; found [M+H]⁺ = 272.0839.

FTIR (neat), cm⁻¹: 2955, 2928, 2897, 2866, 1578, 1506, 1461, 1384, 1335, 1281, 1264, 1222, 1136, 1091, 1072, 1052, 1018, 967, 929, 909, 870, 850, 831, 810, 784, 761, 617, 594, 544, 520.

### Preparative Example 66: tert-butyl 4-(4-(3-(4-isopropylphenyl)furo[3,2-b]pyridin-6-yl)phenyl)piperazine-1-carboxylate

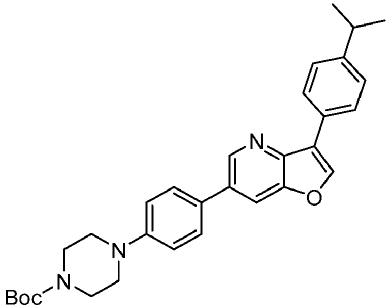

The compound was prepared by the general procedure **B** using 102 mg (0.375 mmol) of 6-chloro-3-(4-isopropylphenyl)furo[3,2-*b*]pyridine **(Prep. Example 65)** and 189 mg (0.488 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 9:1) afforded the compound as a yellowish solid (123 mg, 66 % yield).

¹H NMR (500 MHz, Chloroform-d) δ 8.88 (d, *J* = 1.9 Hz, 1H), 8.08 (s, 1H), 8.04 - 7.95 (m, 2H), 7.91 (d, *J* = 1.9 Hz, 1H), 7.61 - 7.54 (m, 2H), 7.40 - 7.32 (m, 2H), 7.11 - 6.99 (m, 2H), 3.68 - 3.57 (m, 4H), 3.23 (t, *J* = 5.2 Hz, 4H), 2.97 (p, *J* = 6.9 Hz, 1H), 1.50 (s, 9H), 1.30 (d, *J* = 6.9 Hz, 6H).

¹³C NMR (126 MHz, CDCl₃) δ 154.88, 149.38, 148.70, 145.02, 144.69, 144.67, 132.93, 128.37, 128.01, 127.36, 127.11, 122.03, 116.95, 116.09, 80.16, 49.20, 43.59, 34.17, 28.60, 24.12.

HRMS (APCI): calcd, for C₃₁H₃₅N₃O₃ [M+H]⁺ = 498.2751; found [M+H]⁺ = 498.2750.

FTIR (neat), cm⁻¹ 2958, 2930, 2867, 2813, 2362, 2325, 1702, 1681, 1608, 1521, 1477, 1463, 1415, 1376, 1362, 1335, 1282, 1264, 1248, 1229, 1213, 1168, 1134, 1120, 1085, 1058, 1040, 968, 910, 892, 861, 829, 796, 787, 768, 558, 544.

### Preparative Example 67: 3-(4-isopropylphenyl)-6-(4-(piperazin-1-yl)phenyl)furo[3,2-b]pyridine

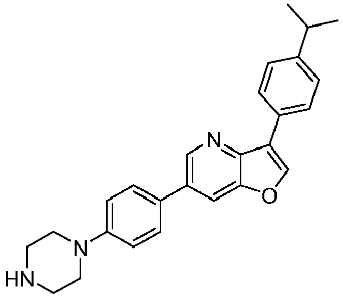

The compound was prepared by the general procedure **C** using 90 mg (0.181 mmol) of *tert*-butyl 4-(4-(3-(4-isopropylphenyl)furo[3,2-*b*]pyridin-6-yl)phenyl)piperazine-1-carboxylate **(Prep. Example 66);** the reaction time was 2 h; flash chromatography (DCM/MeOH, gradient from 1:0 to 9:1) afforded the compound as a yellow solid (63 mg, 88 % yield).

¹H NMR (500 MHz, DMSO-*d*₆) δ 8.92 (d, *J* = 2.0 Hz, 1H), 8.75 (s, 1H), 8.27 (d, *J* = 1.9 Hz, 1H), 8.16 (d, *J* = 8.3 Hz, 2H), 7.69 (d, *J* = 8.8 Hz, 2H), 7.37 (d, *J* = 8.2 Hz, 2H), 7.05 (d, *J* = 8.9 Hz, 2H), 3.22 - 3.10 (m, 4H), 2.94 (p, *J* = 6.9 Hz, 1H), 2.90 - 2.83 (m, 4H), 1.25 (d, *J* = 6.9 Hz, 6H).

¹³C NMR (126 MHz, DMSO) δ 151.32, 148.78, 147.79, 146.08, 144.12, 143.42, 132.19, 127.88, 127.67, 126.78, 126.57, 126.51, 120.09, 115.43, 115.42, 48.87, 45.46, 33.22, 23.78.

HRMS (APCI): calcd, for C₂₆H₂₇N₃O [M+H]⁺ = 398.2227; found [M+H]⁺ = 398.2231.

FTIR (neat), cm⁻¹: 3271, 3028, 2956, 2818, 2753, 1607, 1521, 1476, 1452, 1379, 1335, 1235, 1191, 1151, 1124, 1092, 967, 888, 817, 786, 746, 540.

### Preparative Example 68: 6-chloro-3-(3-(methoxymethyl)phenyl)furo[3,2-b]pyridine

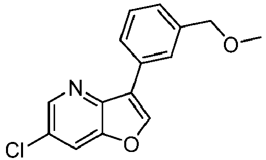

The compound was prepared by the general procedure **A** using 100 mg (0.430 mmol) of 3-bromo-6-chlorofuro[3,2-*b*]pyridine **(Prep. Example 5)** and 93 mg (0.559 mmol) of (3-(methoxymethyl)phenyl)boronic acid; the reaction time was 2 h; flash chromatography (cyclohexane/EtOAc, gradient from 1:0 to 9:1) afforded the compound as a pale yellow solid (102 mg, 87 % yield).

¹H NMR (500 MHz, Chloroform-d) δ 8.62 (d, *J* = 2.1 Hz, 1H), 8.12 (s, 1H), 8.00 (dt, *J* = 7.7, 1.4 Hz, 1H), 7.96 (dt, *J* = 1.8, 0.8 Hz, 1H), 7.82 (d, *J* = 2.1 Hz, 1H), 7.47 (t, *J* = 7.6 Hz, 1H), 7.38 - 7.32 (m, 1H), 4.55 (s, 2H), 3.44 (s, 3H).

¹³C NMR (126 MHz, CDCl₃) δ 148.52, 145.76, 145.32, 144.51, 139.09, 130.12, 129.14, 127.71, 127.45, 126.63, 126.36, 121.89, 118.82, 74.75, 58.37.

HRMS (APCI): calcd, for C₁₅H₁₂ClNO₂ [M+H]⁺ = 274.0629; found [M+H]⁺ = 274.0626.

FTIR (neat), cm⁻¹: 3061, 3013, 2937, 2829, 1469, 1379, 1200, 1129, 1104, 1077, 1016, 964, 908, 893, 870, 786, 691, 595, 524.

### Preparative Example 69: tert-butyl 4-(4-(3-(3-(methoxymethyl)phenyl)furo[3,2-b]pyridin-6-yl)phenyl)piperazine-1-carboxylate

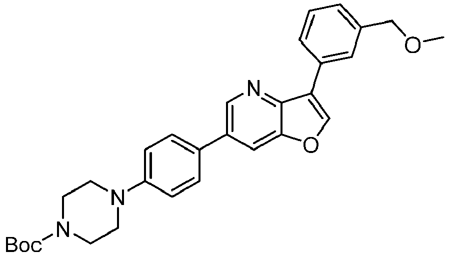

The compound was prepared by the general procedure **B** using 90 mg (0.329 mmol) of 6-chloro-3-(3-(methoxymethyl)phenyl)furo[3,2-*b*]pyridine **(Prep. Example 68)** and 166 mg (0.427 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 3:1) afforded the compound as a yellow solid (112 mg, 68 % yield).

¹H NMR (500 MHz, Chloroform-d) δ 8.88 (d, *J* = 1.9 Hz, 1H), 8.13 (s, 1H), 8.08 (dt, *J* = 7.7, 1.5 Hz, 1H), 8.01 (t, *J* = 1.8 Hz, 1H), 7.92 (dd, *J* = 1.9, 1.0 Hz, 1H), 7.62 - 7.55 (m, 2H), 7.48 (t, *J* = 7.7 Hz, 1H), 7.35 (dt, *J* = 7.8, 1.4 Hz, 1H), 7.04 (d, *J* = 8.4 Hz, 2H), 4.57 (s, 2H), 3.74 - 3.52 (m, 4H), 3.44 (s, 3H), 3.23 (t, *J* = 5.2 Hz, 4H), 1.50 (s, 9H).

¹³C NMR (126 MHz, CDCl₃) δ 154.87, 151.08, 149.42, 145.12, 145.10, 144.49, 138.99, 133.01, 130.79, 129.60, 129.12, 128.35, 127.19, 126.68, 126.38, 121.82, 116.89, 116.07, 80.14, 74.83, 58.34, 49.12, 43.59, 28.59, 27.07.

HRMS (APCI): calcd, for C₃₀H₃₃N₃O₄ [M+H]⁺ = 500.2544; found [M+H]⁺ = 500.2540.

FTIR (neat), cm⁻¹ 2975,2927, 2864,2817, 1682, 1605, 1524, 1480, 1455, 1421, 1375, 1364, 1289, 1248, 1232, 1197, 1161, 1117, 1102, 1045, 1022, 998, 911, 887, 827, 788, 773, 696, 670, 540.

### Preparative Example 70: 3-(3-(methoxymethyl)phenyl)-6-(4-(piperazin-1-yl)phenyl)furo[3,2-b]pyridine

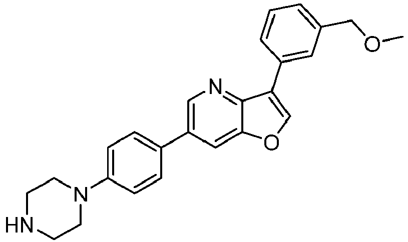

The compound was prepared by the general procedure C using 90 mg (0.180 mmol) of *tert*-butyl 4-(4-(3-(3-(methoxymethyl)phenyl)furo[3,2-*b*]pyridin-6-yl)phenyl)piperazine-1-carboxylate **(Prep. Example 69);** the reaction time was 2 h; flash chromatography (DCM/MeOH, gradient from 1:0 to 4:1) afforded the compound as a yellow solid (44 mg, 61 % yield).

¹H NMR (500 MHz, DMSO-*d*₆) δ 8.95 (d, *J* = 1.9 Hz, 1H), 8.83 (s, 1H), 8.30 (d, *J* = 1.9 Hz, 1H), 8.22 (s, 1H), 8.18 (d, *J* = 7.8 Hz, 1H), 7.76 - 7.63 (m, 2H), 7.48 (t, *J* = 7.7 Hz, 1H), 7.32 (dt, *J* = 7.9, 1.3 Hz, 1H), 7.12 - 7.02 (m, 2H), 4.50 (s, 2H), 3.35 (s, 3H), 3.23 - 3.11 (m, 4H), 2.91 - 2.82 (m, 4H). ¹³C NMR (126 MHz, DMSO) δ 151.09, 148.84, 146.68, 144.24, 143.32, 138.71, 132.23, 130.42, 128.56, 127.71, 126.95, 126.77, 125.67, 125.64, 119.96, 115.53, 73.68, 57.54, 48.33, 45.03, 39.52.

HRMS (APCI): calcd, for C₂₅H₂₅N₃O₂ [M+H]⁺ = 400.2020; found [M+H]⁺ = 400.2023.

FTIR (neat), cm⁻¹: 2943, 2923, 2813, 1605, 1523, 1482, 1450, 1375, 1235, 1180, 1099, 885, 816, 790, 695, 540.

### Preparative Example 71: 6-chloro-3-(3-(trifluoromethyl)phenyl)furo[3,2-b]pyridine

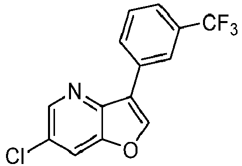

The compound was prepared by the general procedure **A** using 100 mg (0.430 mmol) of 3-bromo-6-chlorofuro[3,2-*b*]pyridine **(Prep. Example 5)** and 106 mg (0.559 mmol) of (3-(trifluoromethyl)phenyl)boronic acid; the reaction time was 2 h; flash chromatography (cyclohexane/EtOAc, gradient from 1:0 to 9:1) afforded the compound as a white solid (125 mg, 98 % yield).

¹H NMR (500 MHz, Chloroform-d) δ 8.63 (d, *J* = 2.0 Hz, 1H), 8.34 - 8.23 (m, 2H), 8.17 (s, 1H), 7.84 (d, *J* = 2.0 Hz, 1H), 7.70 - 7.56 (m, 2H).

¹³C ¹³C NMR (126 MHz, Chloroform-*d*) δ 148.56, 146.03, 145.63, 144.02, 131.46 (q, *J* = 32.3 Hz), 130.93, 130.59 - 130.14 (m), 129.50, 128.11, 127.26, 124.75 (q, *J* = 3.9 Hz), 124.25 (q, *J* = 272.4 Hz), 123.85 (q, *J* = 3.8 Hz), 120.82, 119.01.

HRMS (APCI): calcd, for C₁₄H₇ClF₃NO [M+H]⁺ = 298.0241; found [M+H]⁺ = 298.0244.

FTIR (neat), cm⁻¹: 1387, 1327, 1167, 1128, 1099, 1075, 914, 799, 697.

### Preparative Example 72: tert-butyl 4-(4-(3-(3-(trifluoromethyl)phenyl)furo[3,2-b]pyridin-6-yl)phenyl)piperazine-1-carboxylate

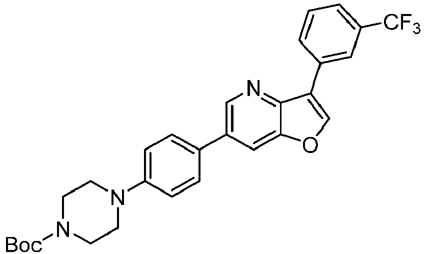

The compound was prepared by the general procedure **B** using 98 mg (0.329 mmol) of 6-chloro-3-(3-(trifluoromethyl)phenyl)furo[3,2-*b*]pyridine **(Prep. Example 71)** and 166 mg (0.427 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 1:2) afforded the compound as a yellow solid (114 mg, 66 % yield).

¹H NMR (500 MHz, Chloroform-*d*) δ 8.90 (d, *J* = 1.9 Hz, 1H), 8.36 (ddd, *J* = 5.0, 3.1, 1.6 Hz, 1H), 8.34 (s, 1H), 8.18 (s, 1H), 7.94 (d, *J* = 1.9 Hz, 1H), 7.61 (dd, *J* = 5.0, 1.3 Hz, 2H), 7.61 - 7.55 (m, 2H), 7.05 (d, *J* = 8.8 Hz, 1H), 3.65 - 3.59 (m, 4H), 3.24 (t, *J* = 5.2 Hz, 4H), 1.50 (s, 9H).

¹³C NMR (126 MHz, Chloroform-*d*) δ 154.88, 151.15, 149.48, 145.37, 144.00, 136.33, 133.40, 131.61, 131.37 (q, *J* = 32.3 Hz), 130.49, 129.46, 129.37, 128.37, 127.42, 125.44 - 123.13 (m), 120.77, 116.89, 116.20, 80.17, 49.09, 43.66, 28.59.

HRMS (APCI): calcd, for C₂₉H₂₈F₃N₃O₃ [M+H]⁺ = 524.2156; found [M+H]⁺ = 524.2161.

FTIR (neat), cm⁻¹ 2978,2930, 2817, 1683, 1607, 1424, 1380, 1365, 1334, 1315, 1251, 1233, 1161, 1122, 1089, 1073, 1047, 1000, 909, 817, 800, 768, 697, 680, 547, 528.

### Preparative Example 73: 6-(4-(piperazin-1-yl)phenyl)-3-(3-(trifluoromethyl)phenyl)furo[3,2-b]pyridine

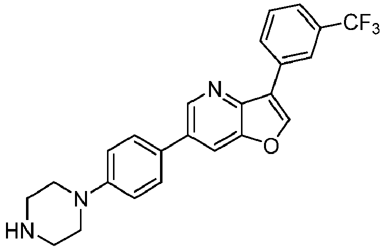

The compound was prepared by the general procedure **C** using 90 mg (0.172 mmol) of *tert*-butyl 4-(4-(3-(3-(trifluoromethyl)phenyl)furo[3,2-*b*]pyridin-6-yl)phenyl)piperazine-1-carboxylate **(Prep. Example 72);** the reaction time was 2 h; flash chromatography (DCM/MeOH, gradient from 1:0 to 4:1) afforded the compound as a yellow solid (68 mg, 93 % yield).

¹H NMR (500 MHz, DMSO-*d*₆) δ 9.02 (s, 1H), 8.98 (d, *J* = 1.9 Hz, 1H), 8.72 (t, *J* = 1.6 Hz, 1H), 8.57 - 8.51 (m, 1H), 8.33 (d, *J* = 1.9 Hz, 1H), 7.82 - 7.67 (m, 4H), 7.06 (d, *J* = 8.9 Hz, 2H), 3.22 - 3.15 (m, 4H), 2.97 - 2.88 (m, 4H).

¹³C NMR (126 MHz, DMSO-*d*₆) δ 151.10, 148.92, 147.68, 144.51, 142.88, 132.51, 131.68, 130.13, 129.75, 129.55 (q, *J* = 31.5 Hz), 127.74, 126.81, 125.32, 123.34 (dq, *J* = 155.7, 3.8 Hz), 123.15, 118.56, 115.72, 115.53, 48.21, 44.94.

HRMS (APCI): calcd, for C₂₄H₂₀F₃N₃O [M+H]⁺ = 424.1631; found [M+H]⁺ = 424.1628.

FTIR (neat), cm⁻¹: 1604, 1523, 1482, 1378, 1334, 1314, 1239, 1198, 1164, 1114, 1089, 1074, 889, 822, 796, 697, 677, 524.

### Preparative Example 74: 5-chloro-2-iodo-6-methylpyridin-3-ol

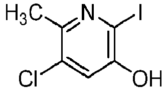

Iodine (884 mg, 3.48 mmol) was added to a mixture of 5-chloro-6-methylpyridin-3-ol (500 mg, 3.48 mmol) and Na₂CO₃ (775 mg, 7.31 mmol) in H₂O (10 mL), and the resulting mixture was stirred under N₂ at 25 °C for 2 hours. The mixture was neutralized with 1M aqueous solution of HCl (11 mL) and extracted with EtOAc (3 × 20 mL). The combined organic extracts were washed with brine (20 mL), dried over MgSO₄, filtered, and the solvent was evaporated *in vacuo.* The product was obtained as brown solid (895 mg, 95 % yield).

¹H NMR (500 MHz, DMSO-*d*₆) δ 11.02 (s, 1H), 7.14 (s, 1H), 2.39 (s, 3H).

¹³C NMR (126 MHz, DMSO) δ 152.74, 146.37, 129.68, 121.24, 107.10, 20.65.

HRMS (APCI): calcd, for C₆H₅ClINO [M+H]⁺ = 269.9177; found [M+H]⁺ = 269.9175

FTIR (neat), cm⁻¹: 2957, 2922, 2851, 1541, 1464, 1400, 1370, 1321, 1265, 1223, 1202, 1089, 1002, 871, 779, 748, 708, 483.

### Preparative Example 75: 6-chloro-5-methyl-2-(trimethylsilyl)furo[3,2-b]pyridine

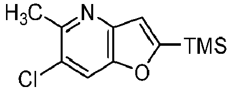

To a degassed solution of 5-chloro-2-iodo-6-methylpyridin-3-ol **(Prep. Example 74,** 800 mg, 2.97 mmol) in dioxane (10 mL) and TEA (4 mL) were added ethynyltrimethylsilane (0.549 mL, 3.86 mmol), PdCl₂(PPh₃)₂ (63 mg, 0.089 mmol) and CuI (34 mg, 0.178 mmol), and the resulting mixture was stirred at 45 °C for 2 h. The solvent was evaporated *in vacuo* and the residue was purified by flash chromatography (cyclohexane/EtOAc, gradient from 1:0 to 9:1). The product was obtained as a beige solid (504 mg, 71 % yield).

¹H NMR (500 MHz, Chloroform-*d*) δ 7.74 (d, *J* = 1.0 Hz, 1H), 7.05 (d, *J* = 1.0 Hz, 1H), 2.70 (s, 3H), 0.36 (s, 9H).

¹³C NMR (126 MHz, CDCl₃) δ 169.91, 151.73, 149.66, 146.23, 126.70, 118.92, 116.75, 23.01, -1.90. HRMS (APCI): calcd. for C₁₁H₁₄ClNOSi [M+H]⁺ = 240.0606, found [M+H]⁺ = 240.0609.

FTIR (neat), cm⁻¹: 3060, 2957, 2897, 1399, 1271, 1249, 1060, 995, 907, 884, 824, 750, 697, 627, 506.

### Preparative Example 76: 6-chloro-5-methylfuro[3,2-b]pyridine

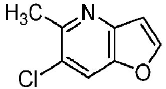

To a solution of 6-chloro-5-methyl-2-(trimethylsilyl)furo[3,2-*b*]pyridine **(Prep. Example 75,** 480 mg, 2.0 mmol) in methanol (10 mL) was added KF (349 mg, 6.0 mmol) and the resulting mixture was stirred at 48 °C for 24 hours. The solvent was evaporated *in vacuo* and the residue was purified by column chromatography (cyclohexane/EtOAc, gradient from 1:0 to 9:1). The product was obtained as a white solid (186 mg, 55 %).

¹H NMR (500 MHz, Chloroform-d) δ 7.78 (d, *J* = 2.3 Hz, 1H), 7.76 (s, 1H), 6.90 (dd, *J* = 2.3, 1.0 Hz, 1H), 2.70 (s, 3H).

¹³C NMR (126 MHz, CDCl₃) δ 152.32, 149.59, 146.56, 145.34, 126.87, 119.23, 107.94, 23.00. HRMS (APCI): calcd. for C₈H₆ClNO [M+H]⁺ = 168.0211, found = 168.0208.

FTIR (neat), cm⁻¹: 3148, 3121, 3066, 2925, 1404, 1373, 1314, 1268, 1124, 1025, 1000, 989, 883, 813, 777, 756, 739, 650, 581, 467.

### Preparative Example 77: 2,3-dibromo-6-chloro-5-methyl-2,3-dihydrofuro[3,2-b]pyridine

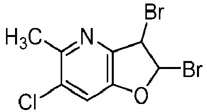

Bromine (0.171 mL, 3.33 mmol) was added slowly at 25 °C to a stirred solution of 6-chloro-5-methylfuro[3,2-*b*]pyridine **(Prep. Example 76,** 186 mg, 1.11 mmol) in DCM (10 mL). The resulting mixture was stirred 3 h. Then, solution of Na₂S₂O₅ (1 g) in water (30 mL) were added and the resulting mixture was extracted with DCM (3 × 30 mL). The combined organic extracts were dried over MgSO₄, filtered, and the solvent was evaporated *in vacuo.* The residue was purified by column chromatography (cyclohexane/EtOAc, gradient from 1:0 to 4:1). The product was obtained as a white solid (258 mg, 71 %).

¹H NMR (500 MHz, Chloroform-d) δ 7.36 (s, 1H), 6.86 (s, 1H), 5.64 (d, *J=* 0.6 Hz, 1H), 2.63 (s, 3H). ¹³C NMR (126 MHz, CDCl₃) δ 152.83, 149.64, 145.42, 132.89, 120.71, 89.12, 51.25, 22.59.

HRMS (APCI): calcd. for C₈H₆Br₂ClNO [M+H]⁺ = 325.8577, found = 325.8573.

FTIR (neat), cm⁻¹: 3129, 3071, 3027, 2987, 1595, 1419, 1292, 1236, 1211, 1174, 1150, 1131, 1018, 983, 946, 879, 781, 770, 711, 652, 566, 548, 469, 417.

### Preparative Example 78: 3-bromo-6-chloro-5-methylfuro[3,2-b]pyridine

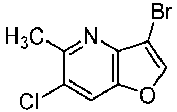

DBU (0.361 mL, 2.42 mmol) was added to the solution of 2,3-dibromo-6-chloro-5-methyl-2,3-dihydrofuro[3,2-*b*]pyridine **(Prep. Example 77,** 240 mg, 0.733 mmol) in toluene (10 mL) and the resulting mixture was stirred at 80 °C for 1 h. The solvent was evaporated and the residue was purified by column chromatography (cyclohexane/EtOAc, gradient from 1:0 to 9:1). The product was obtained as a white solid (158 mg, 87 % yield).

¹H NMR (500 MHz, Chloroform-*d*) δ 7.84 (s, 1H), 7.79 (s, 1H), 2.76 (s, 3H).

¹³C NMR (126 MHz, CDCl₃) δ 153.83, 147.00, 146.21, 142.77, 128.33, 119.73, 99.32, 23.19. HRMS (APCI): calcd. for C₈H₅BrClNO [M+H]⁺ = 245.9316, found = 245.9314.

FTIR (neat), cm⁻¹: 3098, 3037, 1401, 1292, 1265, 1078, 1037, 988, 949, 874, 859, 813, 761, 754, 574, 469.

### Preparative Example 79: 6-chloro-5-methyl-3-(pyridin-4-yl)furo[3,2-b]pyridine

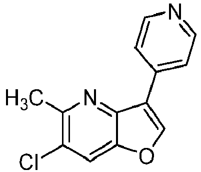

The compound was prepared by the general procedure **A** using 100 mg (0.406 mmol) of 3-bromo-6-chloro-5-methylfuro[3,2-*b*]pyridine **(Prep. Example 78)** and 65 mg (0.527 mmol) of pyridin-4-ylboronic acid; the reaction time was 2 h; flash chromatography (cyclohexane/EtOAc, gradient from 1:0 to 23:77) afforded the compound as a beige solid (23 mg, 23 % yield).

¹H NMR (500 MHz, Chloroform-d) δ 8.82 - 8.59 (m, 2H), 8.20 (s, 1H), 8.07 - 7.97 (m, 2H), 7.80 (s, 1H), 2.76 (s, 3H).

¹³C NMR (126 MHz, CDCl₃) δ 153.19, 150.45, 147.63, 146.80, 143.08, 138.11, 127.64, 121.26, 119.46, 119.25, 23.37.

HRMS (APCI): calcd, for C₁₃H₉ClN₂O [M+H]⁺ = 245.0476; found [M+H]⁺ = 245.0474.

FTIR (neat), cm⁻¹: 3092, 3073, 3028, 2923, 1610, 1578, 1403, 1246, 1223, 1138, 1084, 1020, 992, 886, 811, 768, 755, 700, 684, 655, 600, 520, 469.

### Preparative Example 80: 5-methyl-6-(4-(4-methylpiperazin-1-yl)phenyl)-3-(pyridin-4-yl)furo[3,2-b]pyridine

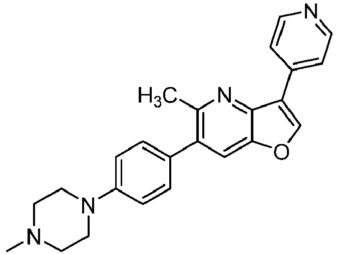

The compound was prepared by the general procedure **B** using 22 mg (0.090 mmol) of 6-chloro-5-methyl-3-(pyridin-4-yl)furo[3,2-*b*]pyridine **(Prep. Example 79)** and 35 mg (0.117 mmol) of 1-methyl-4-(4-(4,4,5,5-tetramethyl-1,3,2-dioxaborolan-2-yl)phenyl)piperazine; the reaction time was 2 h; flash chromatography (DCM/MeOH, gradient from 1:0 % to 17:3) afforded the compound as a white solid (22 mg, 64 % yield).

¹H NMR (500 MHz, DMSO-*d*₆) δ 9.04 (s, 1H), 8.74 - 8.65 (m, 2H), 8.31 - 8.24 (m, 2H), 7.90 (s, 1H), 7.32 (d, *J* = 8.7 Hz, 2H), 7.05 (d, *J* = 8.8 Hz, 2H), 3.22 (t, *J* = 5.1 Hz, 4H), 2.61 (s, 3H). ¹³C NMR (126 MHz, DMSO) δ 152.71, 150.19, 150.05, 148.74, 147.44, 142.38, 138.17, 133.26, 129.92, 129.37, 120.63, 119.59, 117.48, 114.88, 54.52, 47.67, 45.67, 23.93.

HRMS (APCI): calcd, for C₂₄H₂₄N₄O [M+H]⁺ = 385.2023; found [M+H]⁺ = 385.2025.

FTIR (neat), cm⁻¹ 3071, 3054, 2962, 2935, 2793, 1605, 1517, 1450, 1415, 1396, 1373, 1290, 1239, 1216, 1195, 1151, 1141, 1124, 1101, 983, 921, 890, 834, 823, 794, 775, 737, 671, 643, 633, 608, 550, 525.

### Preparative Example 81: 6-(4-(4-methylpiperazin-1-yl)phenyl)-3-(3-nitrophenyl(furo[3,2-b]pyridine

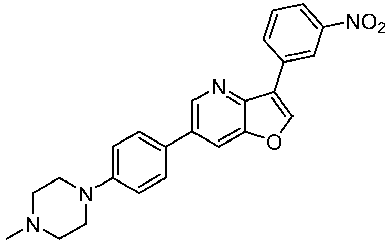

The compound was prepared by the general procedure **B** using 300 mg (1.090 mmol) of 6-chloro-3-(3-nitrophenyl)furo[3,2-*b*]pyridine **(Prep. Example 38)** and 429 mg (1.420 mmol) of 1-methyl-4-(4-(4,4,5,5-tetramethyl-1,3,2-dioxaborolan-2-yl)phenyl)piperazine; the reaction time was 2 h; flash chromatography (DCM/MeOH, gradient from 1:0 to 19:1) afforded the compound as a yellow solid (367 mg, 81 % yield).

¹H NMR (500 MHz, Chloroform-d) δ 8.95 (t, *J* = 2.0 Hz, 1H), 8.91 (d, *J* = 1.9 Hz, 1H), 8.55 (dt, *J* = 7.8, 1.3 Hz, 1H), 8.24 (s, 1H), 8.20 (ddd, *J* = 8.2, 2.3, 1.0 Hz, 1H), 7.95 (d, *J* = 1.9Hz, 1H), 7.66 (t, *J* = 8.0 Hz, 1H), 7.63 - 7.54 (m, 2H), 7.10 - 7.01 (m, 2H), 3.37 - 3.26 (m, 4H), 2.66 - 2.57 (m, 4H), 2.38 (s, 3H).

¹³C NMR (126 MHz, CDCl₃) δ 151.30, 149.58, 148.88, 145.61, 145.52, 143.55, 133.81, 133.04, 132.62, 129.93, 128.51, 128.30, 122.43, 121.81, 119.97, 116.32, 116.22, 55.14, 48.72, 46.27.

HRMS (APCI): calcd, for C₂₄H₂₂N₄O₃ [M+H]⁺ = 415.1765; found [M+H]⁺ = 415.1767.

FTIR (neat), cm⁻¹ 3078,2942, 2843,2794, 1606, 1520, 1482, 1448, 1379, 1340, 1293, 1240, 1203, 1102, 1009, 818, 803, 778, 736, 697, 679, 527.

### Preparative Example 82: 3-(6-(4-(4-methylpiperazin-1-yl)phenyl)furo[3,2-b]pyridin-3-yl)aniline

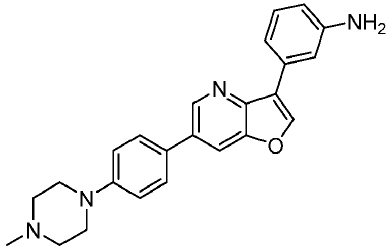

To a degassed heterogenous mixture of Pd/C (10.0 mg) in EtOH (5 mL) was added 6-(4-(4-methylpiperazin-1-yl)phenyl)-3-(3-nitrophenyl)furo[3,2-*b*]pyridine **(Prep. Example 81,** 300 mg, 0.724 mmol), and the resulting mixture was stirred under hydrogen atmosphere at 25 °C for 3 h. Then, the reaction mixture was filtered through syringe filter (Chromafil^{®} Xtra PTFE-20/25 0.25 µm), the solvent was evaporated *in vacuo,* and the residue was purified by flash chromatography (DCM/MeOH, gradient from 1:0 to 19:1). The product was obtained as a pale yellow solid (130 mg, 47 % yield).

¹H NMR (500 MHz, DMSO-*d*₆) δ 8.89 (d, *J* = 1.9 Hz, 1H), 8.63 (s, 1H), 8.26 (d, *J* = 1.9 Hz, 1H), 7.69 (d, *J* = 8.8 Hz, 2H), 7.48 (t, *J* = 2.0 Hz, 1H), 7.29 (dt, *J=* 7.6, 1.3 Hz, 1H), 7.12 (t, *J* = 7.8 Hz, 1H), 7.09 - 7.04 (m, 2H), 6.58 (ddd, *J* = 8.0, 2.4, 1.0 Hz, 1H), 5.14 (s, 2H), 3.26 - 3.17 (m, 5H), 2.47 (t, *J* = 5.1 Hz, 5H), 2.24 (s, 3H).

¹³C NMR (126 MHz, DMSO) δ 150.70, 148.79, 148.75, 146.08, 143.96, 143.55, 132.06, 130.70, 129.03, 127.70, 126.98, 120.87, 115.52, 115.42, 114.42, 113.37, 112.21, 54.46, 47.66, 45.73.

HRMS (APCI): calcd, for C₂₄H₂₄N₄O [M+H]⁺ = 385.2023; found [M+H]⁺ = 385.2026.

FTIR (neat), cm⁻¹: 2937, 2842, 2794, 1604, 1523, 1486, 1448, 1379, 1292, 1235, 1192, 1139, 1098, 1001, 918, 817, 785, 692, 671, 543, 522, 456.

### Preparative Example 83: ethyl (3-(6-(4-(4-methylpiperazin-1-yl)phenyl)furo[3,2-b]pyridin-3-yl)phenyl)carbamate

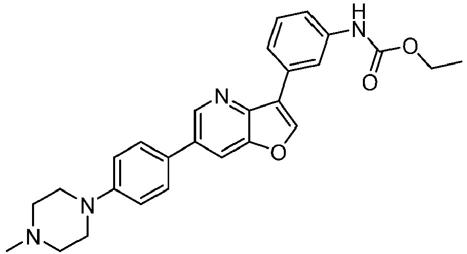

Ethyl chloroformate (0.022 mL, 0.234 mmol) was added to a mixture of 3-(6-(4-(4-methylpiperazin-1-yl)phenyl)furo[3,2-*b*]pyridin-3-yl)aniline **(Prep. Example 82,** 60 mg, 0.156 mmol) and TEA (0.043 mL, 0.312 mmol) in DCM (5 mL) and the resulting mixture was stirred at 25 °C for 2 h. Then, the reaction mixture was diluted with water (3 mL) and extracted with DCM (3 × 5 mL). The combined organic extracts were dried over MgSO₄, filtered, and the solvent was evaporated *in vacuo.* The residue was purified by flash chromatography (DCM/MeOH, gradient from 1:0 to 9:1). The product was obtained as a white solid (52 mg, 73 % yield).

¹H NMR (500 MHz, DMSO-*d*₆) δ 9.68 (s, 1H), 8.91 (d, *J* = 1.9 Hz, 1H), 8.69 (s, 1H), 8.32 (t, *J* = 1.9 Hz, 1H), 8.29 (d, *J* = 1.9 Hz, 1H), 7.81 (dt, *J* = 7.5, 1.5 Hz, 1H), 7.72 - 7.66 (m, 2H), 7.44 (dt, *J* = 8.5, 1.5 Hz, 1H), 7.39 (t, *J* = 7.8 Hz, 1H), 7.10 - 7.04 (m, 2H), 4.16 (q, *J* = 7.1 Hz, 2H), 3.25 - 3.21 (m, 4H), 2.48 (t, *J* = 5.1 Hz, 4H), 2.24 (s, 3H), 1.27 (t, *J* = 7.1 Hz, 3H).

¹³C NMR (126 MHz, DMSO) δ 153.62, 150.72, 148.79, 146.42, 144.17, 143.27, 139.50, 132.27, 130.74, 128.92, 127.72, 126.89, 121.03, 120.36, 117.94, 116.93, 115.51, 60.08, 54.44, 47.63, 45.71, 14.50. HRMS (APCI): calcd, for C₂₇H₂₈N₄O₃ [M+H]⁺ = 457.2234; found [M+H]⁺ = 457.2235.

FTIR (neat), cm⁻¹: 3247, 2956, 2930, 2845, 2794, 2768, 1728, 1606, 1540, 1524, 1479, 1445, 1378, 1292, 1226, 1190, 1161, 1138, 1120, 1102, 1072, 1000, 917, 871, 845, 827, 813, 798, 767, 695, 670, 536, 525.

### Preparative Example 84: N-phenyl-3-(pyridin-4-yl)furo[3,2-b]pyridin-6-amine

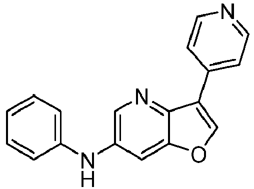

(*S*)-(+)-1-[(*R*)-2-(Diphenylphosphino)ferrocenyl]ethyldi-*t*-butylphosphine (Josiphos SL-J002-2; 2.8 mg, 0.05 mmol) and Pd(OAc)₂ (1.2 mg, 0.05 mmol) were added to a degassed mixture of 6-chloro-3-(pyridin-4-yl)furo[3,2-*b*]pyridine **(Prep. Example 21,** 60 mg, 0.260 mmol), aniline (0.029 mL, 0.312 mmol), and sodium *tert*-butoxide (30 mg, 0.312 mmol) in 1,4-dioxane (5 mL) in a microwave vial. The resulting mixture was irradiated 120 min at 140 °C. Then, the reaction mixture was diluted with water (15 mL), extracted with EtOAc (3 × 5 mL). The combined organic extracts were dried over MgSO₄, filtered, and the solvent was evaporated *in vacuo.* The residue was purified by flash chromatography (DCM/MeOH, gradient from 1:0 to 9:1). The product was obtained as a brown solid (51 mg, 68 % yield).

¹H NMR (500 MHz, DMSO) δ 8.88 (s, 1H), 8.69 - 8.63 (m, 2H), 8.62 (s, 1H), 8.48 (d, *J=* 2.4 Hz, 1H), 8.25 - 8.13 (m, 2H), 7.76 *(d, J=* 2.3 Hz, 1H), 7.30 (dd, *J* = 8.5, 7.3 Hz, 2H), 7.22 - 7.15 (m, 2H), 6.92 (t, *J* = 7.3 Hz, 1H).

¹³C NMR (126 MHz, DMSO) δ 149.95, 149.29, 146.79, 142.51, 138.45, 138.25, 138.07, 136.81, 129.36, 120.73, 120.59, 117.83, 117.05, 104.90.

HRMS (APCI): calcd, for C₁₈H₁₃N₃O [M+H]⁺ = 288.1131; found [M+H]⁺ = 288.1132.

FTIR (neat), cm⁻¹: 3245, 3102, 3028, 2925, 2851, 1593, 1488, 1436, 1415, 1381, 1331, 1282, 1233, 1181, 1099, 874, 837, 804, 751, 706, 671, 621, 607, 576, 560, 522, 499, 471, 445.

### Preparative Example 85: tert-butyl (3-(6-chlorofuro[3.2-b]pyridin-3-yl)benzyl)carbamate

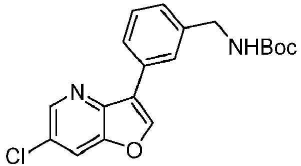

The compound was prepared by the general procedure **A** using 100 mg (0.430 mmol) of 3-bromo-6-chlorofuro[3,2-*b*]pyridine **(Prep. Example 5)** and 140 mg (0.559 mmol) of (3-(((*tert-*butoxycarbonyl)amino)methyl)phenyl)boronic acid; the reaction time was 2 h; flash chromatography (DCM/MeOH, gradient from 1:0 to 44:1) afforded the compound as a pale yellow solid (154 mg, 100 % yield).

¹H NMR (500 MHz, Chloroform-d) δ 8.60 (d, *J* = 2.1 Hz, 1H), 8.10 (s, 1H), 7.95 - 7.90 (m, 2H), 7.82 (d, *J=* 2.1 Hz, 1H), 7.44 (td, *J* = 7.6, 0.8 Hz, 1H), 7.30 (d, *J=* 7.7 Hz, 1H), 4.94 (s, 1H), 4.40 (d, *J=* 5.8 Hz, 2H), 1.48 (s, 9H).

¹³C NMR (126 MHz, CDCl₃) δ 156.09, 148.53, 145.77, 145.30, 144.43, 139.83, 130.29, 129.35, 127.76, 127.33, 126.31, 121.81, 118.88, 79.70, 44.87, 28.58.

HRMS (APCI): calcd, for C₁₉H₁₉ClN₂O₃ [M+H]⁺= 359.1157; found [M+H]⁺= 359.1161.

FTIR (neat), cm⁻¹: 3361, 3082, 2986, 2936, 1687, 1524, 1386, 1363, 1275, 1247, 1165, 1126, 1097, 1087, 1077, 1008, 909, 885, 789, 767, 697, 620, 607, 592.

### Preparative Example 86: tert-butyl 4-(4-(3-(3-(((tert-butoxycarbonyl)amino)methyl)phenyl)furo[3,2-b]pyridin-6-yl)phenyl)piperazine-1-carboxylate

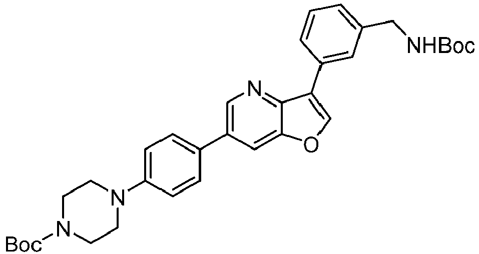

The compound was prepared by the general procedure **B** using 136 mg (0.379 mmol) of *tert-butyl* (3-(6-chlorofuro[3,2-*b*]pyridin-3-yl)benzyl)carbamate **(Prep. Example 85)** and 191 mg (0.493 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 1:1of EtOAc) afforded the compound as a pale yellow solid (144 mg, 65 % yield).

¹H NMR (500 MHz, Chloroform-*d*) δ 8.79 (d, *J* = 1.9 Hz, 1H), 8.03 (s, 1H), 7.93 (dt, *J* = 7.7, 1.5 Hz, 1H), 7.90 (s, 1H), 7.84 (d, *J=* 1.9 Hz, 1H), 7.53 - 7.46 (m, 2H), 7.38 (t, *J=* 7.6 Hz, 1H), 7.22 (d, *J* = 7.7 Hz, 1H), 7.01 - 6.93 (m, 2H), 4.33 (d, *J* = 5.9 Hz, 2H), 3.57 - 3.51 (m, 4H), 3.15 (t, *J* = 5.2 Hz, 4H), 1.42 (s, 9H), 1.40 (s, 9H).

¹³C NMR (126 MHz, CDCl₃) δ 156.09, 154.87, 151.11, 149.41, 145.10, 145.08, 144.40, 139.69, 133.07, 130.97, 129.51, 129.32, 128.34, 127.08, 126.35, 121.73, 116.87, 116.09, 80.14, 49.09, 43.60, 28.59. HRMS (APCI): calcd, for C₃₄H₄₀N₄O₅ [M+H]⁺ = 585.3071; found [M+H]⁺ = 585.3074.

FTIR (neat), cm⁻¹ 3362,2978, 2928,2857, 1713, 1682, 1608, 1521, 1480, 1419, 1381, 1363, 1340, 1290, 1250, 1233, 1164, 1119, 1049, 1013, 999, 919, 879, 813, 791, 773, 696, 660, 531.

### Preparative Example 87: (3-(6-(4-(piperazin-1-yl)phenyl)furo[3,2-b]pyridin-3-yl)phenyl)methanamine

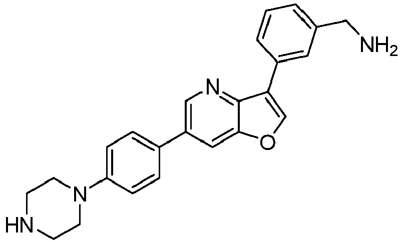

The compound was prepared by the general procedure C using 130 mg (0.222 mmol) of *tert*-butyl 4-(4-(3-(3-(((*tert*-butoxycarbonyl)amino)methyl)phenyl)furo[3,2-*b*]pyridin-6-yl)phenyl)piperazine-1-carboxylate **(Prep. Example 86);** the reaction time was 2 h; flash chromatography (DCM/MeOH, gradient from 1:0 to 4:1) afforded the compound as a pale yellow solid (37 mg, 43 % yield).

¹H NMR (500 MHz, DMSO-*d*₆) δ 8.94 (d, *J* = 1.9 Hz, 1H), 8.78 (s, 1H), 8.29 (d, *J* = 2.0 Hz, 1H), 8.17 (d, *J* = 1.8 Hz, 1H), 8.13 (dt, *J* = 7.7, 1.6 Hz, 1H), 7.74 - 7.66 (m, 2H), 7.44 (t, *J* = 7.6 Hz, 1H), 7.36 (dt, *J* = 7.7, 1.6 Hz, 1H), 7.10 - 6.99 (m, 2H), 3.83 (s, 2H), 3.18 - 3.07 (m, 4H), 2.92 - 2.81 (m, 4H).

¹³C NMR (126 MHz, DMSO) δ 151.35, 148.83, 146.43, 144.19, 143.90, 143.36, 132.25, 130.18, 128.44, 127.68, 126.74, 126.43, 125.35, 124.71, 120.27, 115.47, 115.42, 48.91, 45.51, 45.48.

HRMS (APCI): calcd. for C₂₄H₂₄N₄O [M+H]⁺ = 385.2023; found [M+H]⁺ = 385.2025.

FTIR (neat), cm⁻¹: 3032, 2954, 2922, 2820, 1660, 1604, 1523, 1478, 1450, 1378, 1237, 1182, 1120, 1095, 888, 818, 786, 751, 696, 666, 533.

### Preparative Example 88: 3-(6-chlorofuro[3,2-b]pyridin-3-yl)benzaldehyde

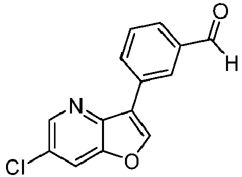

The compound was prepared by the general procedure **A** using 100 mg (0.430 mmol) of 3-bromo-6-chlorofuro[3,2-*b*]pyridine **(Prep. Example 5)** and 84 mg (0.559 mmol) of 3-formylphenylboronic acid; the reaction time was 2 h; flash chromatography (cyclohexane/EtOAc, gradient from 1:0 to 9:1) afforded the compound as a white solid (84 mg, 76 % yield).

¹H NMR (500 MHz, Chloroform-d) δ 10.12 (s, 1H), 8.65 (d, *J=* 2.0 Hz, 1H), 8.54 (t, *J=* 1.7 Hz, 1H), 8.38 (ddd, *J* = 7.7, 1.8, 1.2 Hz, 1H), 8.22 (s, 1H), 7.90 (dt, *J* = 7.6, 1.4 Hz, 1H), 7.87 (d, *J* = 2.0 Hz, 1H), 7.67 (t, *J* = 7.7 Hz, 1H).

¹³C NMR (126 MHz, CDCl₃) δ 192.26, 148.56, 146.07, 145.56, 144.08, 137.09, 132.98, 131.18, 129.75, 129.14, 128.25, 128.08, 120.79, 119.03.

HRMS (APCI): calcd, for C₁₄H₈ClNO₂ [M+H]⁺ = 258.0316; found [M+H]⁺ = 258.0314.

FTIR (neat), cm⁻¹: 3138, 3106, 3068, 2858, 1686, 1602, 1565, 1484, 1461, 1386, 1359, 1270, 1239, 1176, 1126, 1097, 1077, 999, 915, 890, 877, 827, 791, 780, 719, 681, 651, 597, 522, 427, 411.

### Preparative Example 89: 3-(6-(4-(4-methylpiperazin-1-yl)phenyl)furo[3,2-b]pyridin-3-yl)benzaldehyde

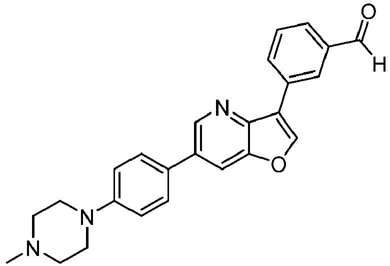

The compound was prepared by the general procedure **B** using 80 mg (0.310 mmol) of 3-(6-chlorofuro[3,2-*b*]pyridin-3-yl)benzaldehyde **(Prep. Example 88)** and 122 mg (0.404 mmol) of 1-methyl-4-(4-(4,4,5,5-tetramethyl-1,3,2-dioxaborolan-2-yl)phenyl)piperazine; the reaction time was 2 h; flash chromatography (DCM/MeOH, gradient from 1:0 to 9:1) afforded the compound as a white solid (109 mg, 88 %yield).

¹H NMR (500 MHz, DMSO-*d*₆) δ 10.11 (s, 1H), 9.02 - 8.91 (m, 2H), 8.84 (t, *J* = 1.7 Hz, 1H), 8.57 (dt, *J* = 7.7, 1.5 Hz, 1H), 8.33 (d, *J* = 1.9 Hz, 1H), 7.92 (dt, *J* = 7.6, 1.4 Hz, 1H), 7.75 (t, *J* = 7.6 Hz, 1H), 7.73 - 7.68 (m, 2H), 7.07 (d, *J=* 8.9 Hz, 2H), 3.25 - 3.21 (m, 4H), 2.49 - 2.43 (m, 4H), 2.24 (s, 3H).

¹³C NMR (126 MHz, DMSO) δ 193.00, 150.76, 148.90, 147.36, 144.42, 143.01, 136.66, 135.66, 132.49, 132.20, 131.47, 129.56, 128.90, 127.73, 126.93, 126.75, 119.00, 115.68, 115.49, 113.69, 82.97, 54.44, 47.61, 45.72, 39.52, 24.63.

HRMS (APCI): calcd, for C₂₅H₂₃N₃O₂ [M+H]⁺ = 398.1863; found [M+H]⁺ = 398.1863.

FTIR (neat), cm⁻¹ 2973, 2940, 2827, 2797, 1699, 1604, 1586, 1524, 1480, 1450, 1376, 1362, 1292, 1238, 1203, 1175, 1140, 1118, 1107, 1008, 921, 887, 821, 799, 790, 688, 671, 655, 538, 521.

### Preparative Example 90: (3-(6-(4-(4-methylpiperazin-1-yl)phenyl)furo[3,2-b]pyridin-3-yl)phenyl)methanol

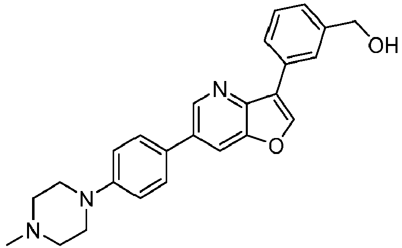

NaBH₄ (14 mg, 0.377 mmol) was added to a solution of 3-(6-(4-(4-methylpiperazin-1-yl)phenyl)furo[3,2-*b*]pyridin-3-yl)benzaldehyde **(Prep. Example 89;** 100 mg, 0.252 mmol) in MeOH (10 mL) at 0 °C and the reaction mixture was stirred at 25 °C for 90 min. The reaction mixture was quenched by addition of 1M HCl (0.4 mL) and the resulting solution was diluted with H₂O (10 mL) and extracted with EtOAc (3 × 15 mL). The combined organic extracts were washed with brine (10 mL), dried over MgSO₄, and filtered. The solvent was evaporated *in vacuo* and the residue was purified by flash chromatography (DCM/MeOH, gradient from 1:0 to 4:1). The product was obtained as a white solid (93 mg, 93 % yield).

¹H NMR (500 MHz, DMSO-*d*₆) δ 8.94 (s, 1H), 8.80 (s, 1H), 8.29 (s, 1H), 8.21 (s, 1H), 8.12 (d, *J=* 7.7 Hz, 1H), 7.70 (d, *J* = 8.2 Hz, 2H), 7.45 (t, *J* = 7.7 Hz, 1H), 7.33 (d, *J* = 7.6 Hz, 1H), 7.07 (d, *J* = 8.3 Hz, 2H), 5.23 (t, *J* = 5.7 Hz, 1H), 4.59 (d, *J* = 5.7 Hz, 2H), 3.26 - 3.16 (m, 4H), 2.48 (t, *J* = 5.1 Hz, 4H), 2.24 (s, 3H).

¹³C NMR (126 MHz, DMSO) δ 150.73, 148.83, 146.50, 144.20, 143.38, 142.92, 132.21, 130.15, 128.37, 127.71, 126.90, 125.75, 124.96, 124.66, 120.21, 115.51, 62.96, 54.45, 47.64, 45.73.

HRMS (APCI): calcd, for C₂₅H₂₅N₃O₂ [M+H]⁺ = 400.2020; found [M+H]⁺ = 400.2016.

FTIR (neat), cm⁻¹: 2946, 2839, 2803, 1603, 1523, 1479, 1448, 1374, 1292, 1234, 1178, 1154, 1138, 1120, 1094, 1044, 1002, 919, 832, 799, 789, 753, 700, 672, 543, 528, 518.

### Preparative Example 91: 3-(6-chlorofuro[3,2-b]pyridin-3-yl)-N,N-diphenylaniline

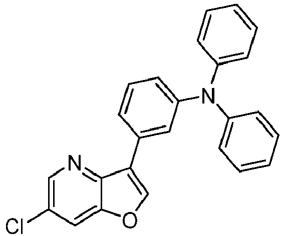

The compound was prepared by the general procedure A using 100 mg (0.430 mmol) of 3-bromo-6-chlorofuro[3,2-*b*]pyridine **(Prep. Example 5)** and 162 mg (0.559 mmol) of (3-(diphenylamino)phenyl)boronic acid; the reaction time was 2 h; flash chromatography (cyclohexane/EtOAc, gradient from 1:0 to 19:1) afforded the compound as a pale yellow solid (121 mg, 71 % yield).

¹H NMR (500 MHz, Chloroform-d) δ 8.56 (d, *J=* 2.0 Hz, 1H), 7.97 (s, 1H), 7.86 - 7.73 (m, 2H), 7.62 (t, *J* = 2.0 Hz, 1H), 7.36 (t, *J* = 7.9 Hz, 1H), 7.29 - 7.20 (m, 6H), 7.17 - 7.12 (m, 4H), 7.11 - 7.06 (m, 2H), 7.05 - 6.98 (m, 3H).

¹³C NMR (126 MHz, CDCl₃) δ 148.51, 148.44, 147.89, 147.88, 145.78, 145.30, 144.46, 130.99, 129.97, 129.56, 129.42, 129.39, 127.66, 124.51, 124.49, 123.85, 123.05, 123.00, 122.93, 122.60, 122.48, 121.95, 121.88, 121.44, 118.76.

HRMS (APCI): calcd, for C₂₅H₁₇ClN₂O [M+H]⁺ = 397.1102; found [M+H]⁺ = 397.1100.

FTIR (neat), cm⁻¹: 3060, 3033, 1583, 1480, 1384, 1294, 1273, 1072, 932, 910, 874, 783, 750, 691, 658, 622, 597, 502.

### Preparative Example 92: tert-butyl 4-(4-(3-(3-(diphenylamino)phenyl)furo[3,2-b]pyridin-6-yl)phepyl)piperazine-1-carboxylate

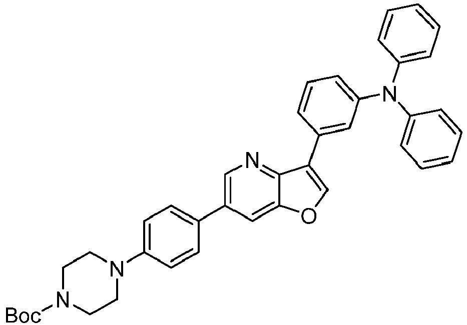

The compound was prepared by the general procedure **B** using 100 mg (0.252 mmol) of 3-(6-chlorofuro[3,2-*b*]pyridin-3-yl)-*N*,*N*-diphenylaniline **(Prep. Example 91)** and 127 mg (0.328 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 4:1) afforded the compound as a pale yellow solid (103 mg, 66 % yield).

¹H NMR (500 MHz, Chloroform-d) δ 8.92 (d, *J=* 1.8 Hz, 1H), 8.07 (s, 1H), 8.00 - 7.94 (m, 2H), 7.74 (t, *J* = 1.9 Hz, 1H), 7.69 - 7.62 (m, 2H), 7.48 (t, *J* = 7.9 Hz, 1H), 7.39 - 7.31 (m, 4H), 7.29 - 7.22 (m, 3H), 7.18 (ddd, *J=* 8.0, 2.3, 1.0 Hz, 1H), 7.16 - 7.08 (m, 4H), 3.72 (t, *J=* 5.2 Hz, 4H), 3.32 (t, *J=* 5.2 Hz, 4H), 1.60 (s, 9H).

¹³C NMR (126 MHz, CDCl₃) δ 154.87, 149.33, 148.42, 147.96, 145.16, 145.09, 144.43, 132.95, 131.68, 129.97, 129.38, 128.35, 124.41, 123.83, 122.92, 122.66, 122.23, 121.81, 116.92, 116.03, 80.16, 49.17, 43.52, 28.60, 27.08.

HRMS (APCI): calcd, for C₄₀H₃₈N₄O₃ [M+H]⁺ = 623.3017; found [M+H]⁺ = 623.3020.

FTIR (neat), cm⁻¹ 2975, 2919, 2851, 2363, 2342, 1695, 1607, 1586, 1524, 1492, 1423, 1267, 1233, 1167, 754, 697.

### Preparative Example 93: N,N-diphenyl-3-(6-(4-(piperazin-1-yl)phenyl)furo[3,2-b]pyridin-3-yl)aniline

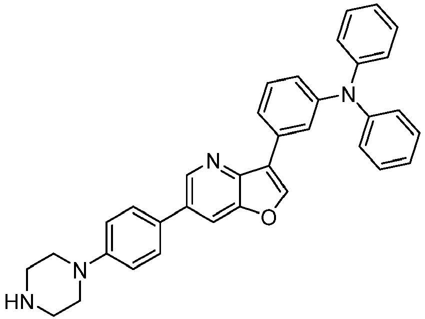

The compound was prepared by the general procedure C using 75 mg (0.120 mmol) of *tert*-butyl 4-(4-(3-(3-(diphenylamino)phenyl)furo[3,2-*b*]pyridin-6-yl)phenyl)piperazine-1-carboxylate **(Prep. Example 92);** the reaction time was 2 h; flash chromatography (DCM/MeOH, gradient from 1:0 to 9:1) afforded the compound as a white solid (54 mg, 86 % yield).

¹H NMR (500 MHz, DMSO-*d*₆) δ 8.84 (d, *J* = 1.9 Hz, 1H), 8.74 (s, 1H), 8.26 (d, *J* = 1.9 Hz, 1H), 8.02 (t, *J* = 2.0 Hz, 1H), 7.91 (dt, *J* = 7.7, 1.3 Hz, 1H), 7.69 - 7.64 (m, 2H), 7.43 (t, *J* = 7.9 Hz, 1H), 7.37 - 7.27 (m, 4H), 7.10 - 7.00 (m, 8H), 6.98 (ddd, *J* = 8.1, 2.3, 1.0 Hz, 1H), 3.15 (dd, *J* = 6.2, 4.0 Hz, 4H), 2.99 - 2.80 (m, 4H).

¹³C NMR (126 MHz, DMSO) δ 171.97, 151.17, 148.78, 147.54, 147.24, 146.77, 144.18, 143.13, 132.24, 131.76, 129.88, 129.45, 127.67, 126.79, 123.71, 123.41, 122.72, 122.56, 121.76, 119.80, 115.46,48.52, 45.17.

HRMS (APCI): calcd, for C₃₅H₃₀N₄O [M+H]⁺ = 523.2492; found [M+H]⁺ = 523.2495.

FTIR (neat), cm⁻¹: 3348, 3036, 2950, 2919, 2847, 1585, 1489, 1453, 1380, 1281, 1261, 1237, 1106, 931, 827, 789, 750, 693, 668, 542, 503.

### Preparative Example 94: 3-(6-(4-(4-methylpiperazin-1-yl)phenyl)furo[3,2-b]pyridin-3-yl)benzyl acetate

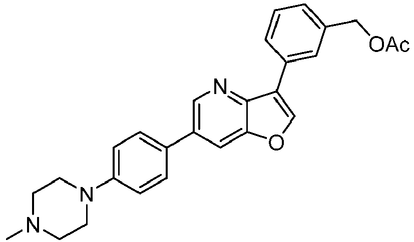

Ac₂O (14 µL, 0.150 mmol) and DMAP (38 mg, 0.313 mmol) were added to a solution of (3-(6-(4-(4-methylpiperazin-1-yl)phenyl)furo[3,2-*b*]pyridin-3-yl)phenyl)methanol **(Prep. Example 90;** 50 mg, 0.125 mmol) in DMF (5 mL) and the reaction mixture was stirred at 60 °C for 60 min. After cooling to ambient temperature, the reaction mixture was diluted with H₂O (10 mL) and extracted with EtOAc (3 × 15 mL). The combined organic extracts were washed with brine (3 × 10 mL), dried over MgSO₄, and filtered. The solvent was evaporated *in vacuo* and the residue was purified by flash chromatography (DCM/MeOH, gradient from 1:0 to 4:1). The product was obtained as a pale yellow solid (42 mg, 76 % yield).

¹H NMR (500 MHz, DMSO-*d*₆) δ 8.95 (d, *J=* 1.9 Hz, 1H), 8.84 (s, 1H), 8.30 (d, *J=* 1.9 Hz, 1H), 8.25 (d, *J* = 1.8 Hz, 1H), 8.23 (dt, *J* = 7.8, 1.4 Hz, 1H), 7.72 - 7.66 (m, 2H), 7.51 (t, J= 7.7 Hz, 1H), 7.37 (dt, *J* = 7.7, 1.5 Hz, 1H), 7.11- 7.01 (m, 2H), 5.16 (s, 2H), 3.26 - 3.21 (m, 4H), 2.25 (s, 3H), 2.11 (s, 3H). ¹³C NMR (126 MHz, DMSO) δ 170.20, 150.71, 148.85, 146.77, 144.28, 143.24, 136.62, 132.29, 130.63, 128.80, 127.72, 127.16, 126.86, 126.16, 126.01, 119.74, 115.57, 115.51, 65.41, 54.41, 47.59, 45.66, 20.70.

HRMS (APCI): calcd, for C₂₇H₂₇N₃O₃ [M+H]⁺ = 442.2125; found [M+H]⁺ = 442.2121.

FTIR (neat), cm⁻¹: 2938, 2845, 2412, 1733, 1607, 1523, 1480, 1457, 1376, 1357, 1243, 1185, 1168, 1107, 1055, 1029, 986, 914, 889, 839, 798, 701, 550, 531.

### Preparative Example 95: (E)-3 -(6-(4-(4-methylpiperazin-1-yl)phenyl)furo[3,2-b]pyridin-3-yl)benzaldehyde oxime

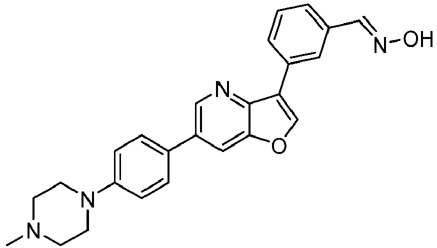

Pyridine (40 µL, 0.503 mmol) and hydroxylamine hydrochloride (26 mg, 0.377 mmol) were added to a solution of 3-(6-(4-(4-methylpiperazin-1-yl)phenyl)furo[3,2-*b*]pyridin-3-yl)benzaldehyde **(Prep. Example 89;** 100 mg, 0.252 mmol) in EtOH (5 mL) at 0 °C and the reaction mixture was stirred at 25 °C for 18 h. The solvent was evaporated *in vacuo* and the residue was diluted with EtOAc (10 mL) and extracted with H₂O (3 × 10 mL). The organic layer was dried over MgSO₄ and filtered. The solvent was evaporated *in vacuo* and the residue was purified by flash chromatography (DCM/MeOH, gradient from 1:0 to 4:1). The product was obtained as a white solid (83 mg, 80 % yield).

¹H NMR (500 MHz, DMSO-*d*₆) δ 11.32 (s, 1H), 10.36 (s, 1H), 8.97 (d, *J=* 1.9 Hz, 1H), 8.87 (s, 1H), 8.56 (t, *J* = 1.8 Hz, 1H), 8.35 (d, *J* = 1.9 Hz, 1H), 8.29 - 8.15 (m, 2H), 7.82 - 7.71 (m, 2H), 7.62 - 7.49 (m, 2H), 7.23 - 7.07 (m, 2H), 3.29 (s, 4H), 2.82 (s, 3H).

¹³C NMR (126 MHz, DMSO) δ 149.29, 148.81, 147.92, 147.04, 144.37, 143.41, 133.51, 132.05, 130.83, 129.05, 128.11, 127.93, 127.30, 125.95, 124.17, 119.67, 116.17, 115.82, 51.98, 45.18, 42.07.

FTIR (neat), cm⁻¹: 3401, 3186, 2966, 2688, 2593, 1605, 1523, 1476, 1457, 1378, 1245, 1185, 1107, 1017, 987, 971, 949, 920, 826, 801, 696, 682, 639, 545, 521.

### Preparative Example 96: N¹,N¹,N²-trimethyl-N²-(4-nitrophenyl)ethane-1,2-diamine

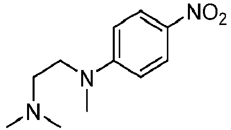

*N*,*N*,*N*-Trimethylethylenediamine (0.561 mL, 4.337 mmol) was added to a mixture of 4-fluoronitrobenzene (612 mg, 4.337 mmol) and K₂CO₃ (1.199 g, 8.675 mmol) in DMSO (20 mL) and the reaction mixture was stirred at 70 °C for 18 h. The reaction mixture was cooled down to 25 °C and treated with brine (25 mL) and EtOAc (30 mL). The organic layer was extracted with brine (5 × 25 mL), dried over MgSO₄, filtered, and evaporated *in vacuo.* The product was obtained as yellow oil (901 mg, 93 % yield).

¹H NMR (500 MHz, Chloroform-d) δ 8.11 *(d, J=* 9.4 Hz, 2H), 6.62 *(d, J=* 9.4 Hz, 2H), 3.57 - 3.53 (m, 2H), 3.10 (s, 3H), 2.61 - 2.43 (m, 2H), 2.30 (s, 6H).

¹³C NMR (126 MHz, CDCl₃) δ 153.54, 137.17, 126.40, 110.33, 56.13, 51.18, 46.01, 39.15.

HRMS (APCI): calcd, for C₁₁H₁₇N₃O₂ [M+H]⁺ = 224.1394; found [M+H]⁺ = 224.1393.

FTIR (neat), cm⁻¹: 2973, 2943, 2821, 2771, 1594, 1519, 1485, 1301, 1255, 1200, 1110, 825, 753.

### Preparative Example 97: N¹-(2-(dimethylamino)ethyl)-N¹-methylbenzene-1,4-diamine

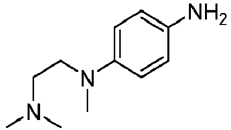

To a degassed mixture of Pd/C (10.0 mg) in EtOH (5 mL) was added *N*¹,*N*¹,*N*²-trimethyl-*N*²-(4-nitrophenyl)ethane-1,2-diamine **(Prep. Example 96,** 900 mg, 4.031 mmol), and the resulting mixture was stirred under hydrogen atmosphere at 25 °C for 18 h. Then, the reaction mixture was filtered through syringe filter (Chromafil^{®} Xtra PTFE-20/25 0.25 µm), the solvent was evaporated *in vacuo.* The product was obtained without further purification as yellowish oil (771 mg, 99 % yield).

¹H NMR (500 MHz, Chloroform-*d*) δ 6.65 (s, 4H), 3.38 - 3.23 (m, 2H), 2.84 (s, 3H), 2.59 - 2.40 (m, 2H), 2.27 (s, 6H).

¹³C NMR (126 MHz, CDCl₃) δ 143.66, 137.72, 116.93, 115.50, 56.37, 52.76, 46.04, 39.72.

FTIR (neat), cm⁻¹: 2946, 2823, 2779, 1673, 1596, 1556, 1515, 1465, 1372, 1303, 1251, 1218, 1156, 1115, 1041, 960, 820.

### Preparative Example 98: N¹-(2-(dimethylamino)ethyl)-N¹-methyl-N⁴-(3-(pyridin-4-yl)furo[3,2-b]pyridin-6-yl)benzene-1,4-diamine

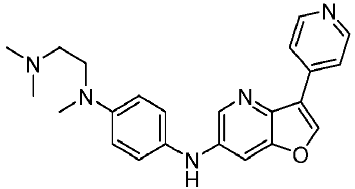

(*S*)-(+)-1-[(*R*)-2-(Diphenylphosphino)ferrocenyl]ethyldi-*t*-butylphosphine (Josiphos SL-J002-2; 2.4 mg, 0.04 mmol) and Pd(OAc)₂ (1.0 mg, 0.04 mmol) were added to a degassed mixture of 6-chloro-3-(pyridin-4-yl)furo[3,2-*b*]pyridine **(Prep. Example 21,** 50 mg, 0.217 mmol), *N*¹-(2-(dimethylamino)ethyl)-*N*¹-methylbenzene-1,4-diamine **(Prep. Example 97,** 50 mg, 0.260 mmol), and sodium *tert*-butoxide (25 mg, 0.260 mmol) in 1,4-dioxane (5 mL) in a microwave vial. The resulting mixture was irradiated 120 min at 140 °C. Then, the reaction mixture was diluted with water (15 mL), extracted with EtOAc (3 × 5 mL). The combined organic extracts were dried over MgSO₄, filtered, and the solvent was evaporated *in vacuo.* The residue was purified by flash chromatography (2% of TEA in DCM/MeOH, gradient from 1:0 to 19:1). The product was obtained as a pale yellow oil (19 mg, 23 % yield).

¹H NMR (500 MHz, Chloroform-*d*) δ 8.71 - 8.61 (m, 2H), 8.30 (d, *J=* 2.4 Hz, 1H), 8.05 (s, 1H), 8.02 - 7.95 (m, 2H), 7.10 (d, *J* = 8.9 Hz, 2H), 6.74 (d, *J* = 8.9 Hz, 2H), 5.65 (s, 1H), 3.51 - 3.41 (m, 2H), 2.97 (s, 3H), 2.59 - 2.48 (m, 2H), 2.32 (s, 6H).

¹³C NMR (126 MHz, CDCl₃) δ 150.44, 150.42, 146.85, 144.12, 141.43, 138.95, 137.02, 136.94, 130.33, 124.36, 121.30, 119.47, 113.53, 102.94, 56.21, 51.68, 46.04, 39.02.

FTIR (neat), cm⁻¹: 3260, 2957, 2937, 2853, 2810, 2785, 2763, 1601, 1575, 1513, 1497, 1419, 1343, 1298, 1284, 1239, 1181, 1158, 1101, 1044, 995, 951, 854, 834, 806, 794, 755, 689, 630, 610, 519, 488.

### Preparative Example 99: 3,6-di(pyridin-4-yl)furo[3,2-b]pyridine

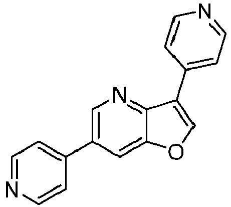

The compound was prepared by the general procedure **B** using 60 mg (0.260 mmol) of 6-chloro-3-(pyridin-4-yl)furo[3,2-*b*]pyridine **(Prep. Example 21)** and 42 mg (0.338 mmol) of 4-pyridinylboronic acid; the reaction time was 2 h; flash chromatography (DCM/MeOH, gradient from 1:0 to 9:1) afforded the compound as a pale yellow solid (41 mg, 58 % yield).

¹H NMR (500 MHz, Chloroform-*d*) δ 8.97 *(d, J=* 1.8 Hz, 1H), 8.75 (dd, *J* = 11.7, 6.2 Hz, 3H), 8.36 (s, 1H), 8.15 - 7.91 (m, 3H), 7.69 - 7.51 (m, 2H).

¹³C NMR (126 MHz, CDCl₃) δ 150.81, 150.54, 149.22, 147.75, 145.99, 145.46, 145.33, 137.97, 130.81, 121.95, 121.40, 119.77, 117.16.

HRMS (APCI): calcd. for C₁₈H₁₂N₂O [M+H]⁺ = 273.1022; found [M+H]⁺ = 273.1020.

FTIR (neat), cm⁻¹ 3088, 3026, 1600, 1385, 1224, 1096, 995, 982, 816, 683, 542, 523.

### Preparative Example 100: 6-Chloro-3-phenylfuro[3,2-b]pyridine

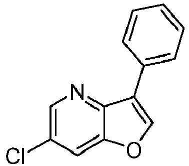

The compound was prepared by the general procedure **A** using 85 mg (0.365 mmol) of 3-bromo-6-chlorofuro[3,2-*b*]pyridine **(Prep. Example 5)** and 53 mg (0.439 mmol) of phenylboronic acid; the reaction time was 2 h; flash chromatography (cyclohexane/EtOAc, gradient from 0 % to 10 % of EtOAc) afforded the compound as a white solid (60 mg, 71 % yield).

¹H NMR (500 MHz, Chloroform-*d*) δ 8.62 (d, *J* = 2.0 Hz, 1H), 8.10 (s, 1H), 8.07 - 7.98 (m, 2H), 7.82 (d, *J=* 2.1 Hz, 1H), 7.48 (dd, *J* = 8.4, 7.0 Hz, 2H), 7.44 - 7.35 (m, 1H).

¹³C NMR (126 MHz, CDCl₃) δ 148.54, 145.60, 145.31, 144.53, 129.97, 129.04, 128.17, 127.71, 127.26, 122.03, 118.83.

HRMS (APCI): calcd. for C₁₃H₈ClNO [M+H]⁺ = 230.0367; found [M+H]⁺ = 230.0370.

FTIR (neat), cm⁻¹: 3063, 1605, 1487, 1443, 1383, 1267, 1222, 1132, 1089, 1070, 965, 906, 880, 817, 777, 751, 691, 654, 617, 594, 520, 503.

### Preparative Example 101: N-(4-(4-methylpiperazin-1-yl)phenyl)-3-phenylfuro[3,2-b]pyridin-6-amine

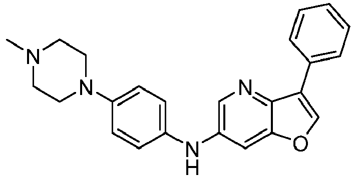

(S)-(+)-1-[(*R*)-2-(Diphenylphosphino)ferrocenyl]ethyldi-*t*-butylphosphine (Josiphos SL-J002-2; 2.4 mg, 0.04 mmol) and Pd(OAc)₂ (1.0 mg, 0.04 mmol) were added to a degassed mixture of 6-chloro-3-phenylfuro[3,2-*b*]pyridine **(Prep. Example 100:,** 50 mg, 0.218 mmol), 4-(4-methylpiperazino)aniline, 50 mg, 0.261 mmol), and sodium *tert*-butoxide (25 mg, 0.261 mmol) in 1,4-dioxane (5 mL) in a microwave vial. The resulting mixture was irradiated 120 min at 140 °C. Then, the reaction mixture was diluted with water (15 mL), extracted with EtOAc (3 × 5 mL). The combined organic extracts were dried over MgSO₄, filtered, and the solvent was evaporated *in vacuo.* The residue was purified by flash chromatography (2 % of TEA in DCM/MeOH, gradient from 1:0 to 9:1). The product was obtained as a pale yellow solid (31 mg, 37 % yield).

¹H NMR (500 MHz, Chloroform-*d*) δ 8.34 (d, *J=* 2.3 Hz, 1H), 8.07 - 8.00 (m, 2H), 7.94 (s, 1H), 7.46 (dd, *J* = 8.4, 7.0 Hz, 2H), 7.40 - 7.30 (m, 2H), 7.13 - 7.06 (m, 2H), 6.99 - 6.90 (m, 2H), 5.67 (s, 1H), 3.20 (t, *J* = 5.0 Hz, 4H), 2.62 (t, *J* = 4.9 Hz, 4H), 2.38 (s, 3H).

¹³C NMR (126 MHz, CDCl₃) δ 149.94, 147.75, 142.95, 139.50, 138.58, 137.44, 134.43, 130.99, 128.90, 127.62, 127.13, 121.94, 121.76, 117.70, 104.34, 67.22, 55.29, 50.85, 49.87, 46.25.

HRMS (APCI): calcd, for C₂₄H₂₄N₄O [M+H]⁺ = 385.2023; found [M+H]⁺ = 385.2024.

FTIR (neat), cm⁻¹: 391, 3056, 2972, 2941, 2929, 2847, 2796, 2756, 1607, 1577, 1564, 1514, 1501, 1464, 1447, 1382, 1339, 1289, 1237, 1189, 1165, 1145, 1107, 1081, 1000, 916, 857, 806, 768, 746, 692, 634, 530, 503.

### Preparative Example 102: 6-chloro-3-(3-phenoxyphenyl)furo[3,2-b]pyridine

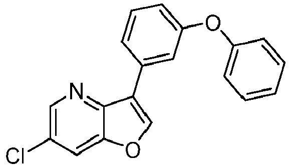

The compound was prepared by the general procedure **A** using 100 mg (0.430 mmol) of 3-bromo-6-chlorofuro[3,2-*b*]pyridine **(Prep. Example 5)** and 120 mg (0.559 mmol) of 3-phenoxybenzeneboronic acid; the reaction time was 3 h; flash chromatography (cyclohexane/EtOAc, gradient from 1:0 to 19:1) afforded the compound as a colorless oil (117 mg, 84 % yield).

¹H NMR (500 MHz, CDCl₃) δ 8.60 (d, J = 2.0 Hz, 1H), 8.09 (s, 1H), 7.84 - 7.80 (m, 2H), 7.72 - 7.69 (m, 1H), 7.44 (t, J = 8.0 Hz, 1H), 7.38 - 7.33 (m, 2H), 7.12 (t, J = 7.4 Hz, 1H), 7.09 - 7.06 (m, 2H), 7.00 (dd, J = 8.1, 2.3 Hz, 1H).

¹³C NMR (126 MHz, CDCl₃) δ 157.81, 157.32, 148.54, 145.88, 145.43, 144.36, 131.75, 130.38, 129.94, 127.83, 123.50, 122.25, 121.52, 119.10, 118.87, 118.48, 117.81.

HRMS (APCI): calcd. for C₁₉H₁₂ClNO₂ [M+H]⁺ = 322.0629; found [M+H]⁺ = 322.0630.

### Preparative Example 103: tert-butyl 4-(4-(3-(3-phenoxyphenyl)furo[3,2-b]pyridin-6-yl)phenyl)piperazine-1-carboxylate

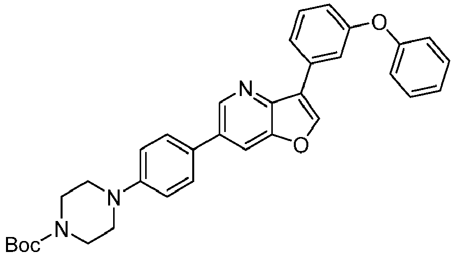

The compound was prepared by the general procedure **B** using 67 mg (0.208 mmol) of 6-chloro-3-(3-phenoxyphenyl)furo[3,2-*b*]pyridine **(Prep. Example 102)** and 105 mg (0.271 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 4:1) afforded the compound as an off-white solid (104 mg, 91 % yield).

¹H NMR (500 MHz, CDCl₃) δ 8.79 (d, J = 1.8 Hz, 1H), 8.01 (s, 1H), 7.83 (dd, J = 4.6, 2.7 Hz, 2H), 7.70 - 7.66 (m, 1H), 7.50 (d, J = 8.7 Hz, 2H), 7.38 (t, J = 7.9 Hz, 1H), 7.28 (t, J = 8.0 Hz, 2H), 7.06 - 6.95 (m, 5H), 6.92 (dd, J = 8.2, 1.7 Hz, 1H), 3.58 - 3.52 (m, 4H), 3.19 - 3.12 (m, 4H), 1.42 (s, 9H).

¹³C NMR (126 MHz, CDCl₃) δ 157.70, 157.46, 154.87, 149.43, 145.25, 145.17, 144.31, 133.10, 132.42, 130.33, 129.90, 128.38, 123.37, 122.39, 121.43, 119.02, 118.28, 117.87, 116.97, 116.12, 80.18, 49.21, 43.56, 28.60.

HRMS (APCI): calcd. for C₃₄H₃₃N₃O₄ [M+H]⁺ = 548.2544; found [M+H]⁺ = 548.2548.

### Preparative Example 104: 3-(3-phenoxyphenyl)-6-(4-(piperazin-1-yl)phenyl)furo[3,2-b]pyridine

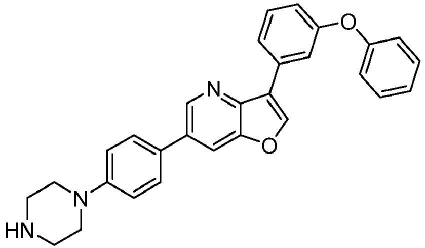

TFA (0.1 mL, 1.31 mmol) was added to a solution of *tert-butyl* 4-(4-(3-(3-phenoxyphenyl)furo[3,2-*b*]pyridin-6-yl)phenyl)piperazine-1-carboxylate **(Prep. Example 103;** 19 mg, 0.035 mmol) in DCM (1.0 mL) and the resulting mixture was stirred at 25 °C; the progress of the reaction was followed by TLC. After consumption of the starting material (1 h), the mixture was alkalized with 2M NaOH (2.0 mL) and extracted with EtOAc (3 × 20 mL). The combined organic phases were dried over MgSO₄ and evaporated *in vacuo.* Flash chromatography (DCM/MeOH, gradient from 9:1 to 7:3) afforded the compound as an off-white solid (9 mg, 54 % yield).

¹H NMR (500 MHz, DMSO) δ 8.91 *(d, J=* 1.9 Hz, 1H), 8.87 (s, 1H), 8.30 (d, *J=* 1.9 Hz, 1H), 8.12 - 8.08 (m, 1H), 8.05 (d, *J=* 7.8 Hz, 1H), 7.68 (d, *J=* 8.8 Hz, 2H), 7.51 (t, *J=* 8.0 Hz, 1H), 7.45 - 7.38 (m, 2H), 7.15 (t, *J* = 7.4 Hz, 1H), 7.09 - 7.03 (m, 4H), 6.98 (dd, *J* = 8.1, 1.9 Hz, 1H), 3.17 - 3.11 (m, 4H), 2.89 - 2.84 (m, 4H).

¹³C NMR (126 MHz, DMSO) δ 156.87, 156.59, 151.33, 148.88, 147.10, 144.28, 143.08, 132.46, 132.36, 130.27, 130.01, 127.68, 126.67, 123.24, 121.79, 119.34, 118.18, 117.83, 117.31, 115.56, 115.42, 48.80, 45.39.

HRMS (APCI): calcd. for C₂₉H₂₅N₃O₂ [M+H]⁺ = 448.2020; found [M+H]⁺ = 448.2017.

### Preparative Example 105: 6-chloro-3-(3-(pyridin-4-yl)phenyl)furo[3,2-b]pyridine

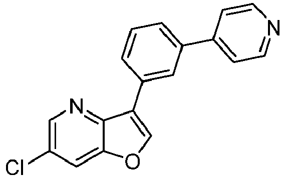

The compound was prepared by the general procedure **A** using 100 mg (0.430 mmol) of 3-bromo-6-chlorofuro[3,2-*b*]pyridine **(Prep. Example 5)** and 111 mg (0.559 mmol) of 3-(pyridin-4-yl)phenylboronic acid; the reaction time was 3 h; flash chromatography (cyclohexane/EtOAc, gradient from 1:0 to 1:1) afforded the compound as a white solid (100 mg, 76 % yield).

¹H NMR (500 MHz, CDCl₃) δ 8.70 (dd, *J* = 4.5, 1.6 Hz, 1H), 8.65 (d, *J* = 2.0 Hz, 1H), 8.37 (t, *J* = 1.6 Hz, 1H), 8.19 (s, 1H), 8.08 (dt, *J* = 7.5, 1.4 Hz, 1H), 7.86 (d, *J* = 2.0 Hz, 1H), 7.65 (dt, *J* = 7.7, 1.5 Hz, 1H), 7.63 - 7.57 (m, 2H).

¹³C NMR (126 MHz, CDCl₃) δ 150.53, 148.61, 148.28, 145.85, 145.56, 144.38, 139.09, 131.03, 129.84, 127.98, 127.77, 126.76, 126.02, 121.92, 121.59, 118.98.

HRMS (APCI): calcd. for C₁₈H₁₁ClN₂O [M+H]⁺ = 307.0633; found [M+H]⁺ = 307.0635.

### Preparative Example 106: tert-butyl 4-(4-(3-(3-(pyridin-4-yl)phenyl)furo[3,2-b]pyridin-6-yl)phenyl)piperazine-1-carboxylate

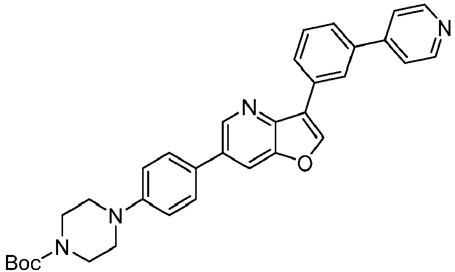

The compound was prepared by the general procedure **B** using 48 mg (0.156 mmol) of 6-chloro-3-(3-(pyridin-4-yl)phenyl)furo[3,2-*b*]pyridine **(Prep. Example 105)** and 79 mg (0.203 mmol) of 4-(4-*tert-*butoxycarbonylpiperazinyl)phenylboronic acid pinacol ester; the reaction time was 2 h; flash chromatography (cyclohexane/EtOAc, gradient from 3:2 to 1:4) afforded the compound as an off-white solid (80 mg, 96 % yield).

¹H NMR (500 MHz, CDCl₃) δ 8.91 (d, *J* = 1.9 Hz, 1H), 8.71 (d, *J* = 5.9 Hz, 2H), 8.47 (s, 1H), 8.20 (s, 1H), 8.15 (dt, *J=* 7.2, 1.6 Hz, 1H), 7.95 (d, *J=* 1.9 Hz, 1H), 7.71 - 7.57 (m, 6H), 7.05 (d, *J=* 8.8 Hz, 2H), 3.65 - 3.59 (m, 4H), 3.27 - 3.21 (m, 4H), 1.50 (s, 9H).

¹³C NMR (126 MHz, CDCl₃) δ 154.89, 151.18, 150.40, 149.50, 148.51, 145.31, 145.17, 144.33, 138.96, 133.27,131.72, 129.79, 129.42, 128.37, 127.85, 126.46, 126.02, 121.99, 121.49, 116.87, 116.16, 80.17, 49.08, 43.78, 28.60.

HRMS (APCI): calcd. for C₃₃H₃₂N₄O₃ [M+H]⁺ = 533.2547; found [M+H]⁺ = 533.2550.

### Preparative Example 107: 6-(4-(piperazin-1-yl)phenyl)-3 -(3-(pyridin-4-yl)phenyl)furo [3,2-b]pyridine

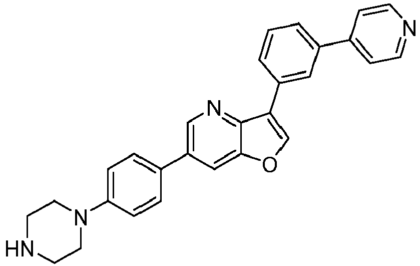

TFA (0.1 mL, 1.31 mmol) was added to a solution of *tert*-butyl 4-(4-(3-(3-(pyridin-4-yl)phenyl)furo[3,2-*b*]pyridin-6-yl)phenyl)piperazine-1-carboxylate **(Prep. Example 106;** 15 mg, 0.029 mmol) in DCM (1.0 mL) and the resulting mixture was stirred at 25 °C; the progress of the reaction was followed by TLC. After consumption of the starting material (1 h), the mixture was alkalized with 2M NaOH (2.0 mL) and extracted with EtOAc (3 × 20 mL). The combined organic phases were dried over MgSO₄ and evaporated *in vacuo.* The compound was isolated as a yellow solid (8 mg, 64 % yield) without further purification.

¹H NMR (500 MHz, DMSO) δ 8.94 - 8.90 (m, 2H), 8.67 - 8,63 (m, 2H), 8.59 (t, *J* = 1.6 Hz, 1H), 8.39 - 8.35 (m, 1H), 8.29 - 8.26 (m, 1H), 7.77 - 7.73 (m, 3H), 7.69 - 7.64 (m, 2H), 7.62 (t, *J=* 7.8 Hz, 1H), 7.06 - 6.89 (m, 1H), 3.14 - 3.06 (m, 4H), 2.87 - 2.79 (m, 4H).

¹³C NMR (126 MHz, DMSO) δ 151.40, 150.26, 148.92, 147.16, 146.94, 144.36, 143.22, 137.70, 132.41, 131.44, 129.60, 127.70, 127.30, 126.65, 125.96, 124.78, 121.32, 119.66, 115.59, 115.41, 48.94, 45.52.. HRMS (APCI): calcd. for C₂₈H₂₄N₄O [M+H]⁺ = 433.2023; found [M+H]⁺ = 433.2021.

### Preparative Example 108: tert-butyl (3-(6-chlorofuro[3,2-b]yridin-3-yl)phenyl)(methyl)carbamate

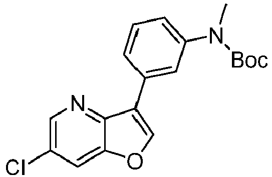

The compound was prepared by the general procedure **A** using 200 mg (0.860 mmol) of 3-bromo-6-chlorofuro[3,2-*b*]pyridine **(Prep. Example 5)** and 282 mg (1.12 mmol) of (3-((*tert-*butoxycarbonyl)(methyl)amino)phenyl)boronic acid; the reaction time was 2 h; flash chromatography (cyclohexane/EtOAc, gradient from 1:0 to 44:1) afforded the compound as a white solid (283 mg, 92 % yield).

¹H NMR (500 MHz, CDCl₃) δ 8.61 (d, *J=* 2.0 Hz, 1H), 8.12 (s, 1H), 7.99 (t, *J=* 1.9 Hz, 1H), 7.83 (d, *J* = 2.0 Hz, 1H), 7.82 - 7.80 (m, 1H), 7.43 (t, *J=* 7.9 Hz, 1H), 7.26 - 7.23 (m, 1H), 3.33 (s, 3H), 1.48 (s, 9H).

¹³C NMR (126 MHz, CDCl₃) δ 154.91, 148.56, 145.78, 145.32, 144.59, 144.39, 130.42, 129.18, 127.81, 125.18, 124.48, 124.07, 121.61, 118.90, 80.60, 37.50, 28.51.

HRMS (APCI): calcd. for C₁₉H₁₉ClN₂O₃ [M+H]⁺= 359.1157; found [M+H]⁺= 359.1161.

### Preparative Example 109: tert-butyl methyl(3-(6-(4-(4-methylpiperazin-1-yl)phenyl)furo[3,2-b]pyridin-3-yl)phenyl)carbamate

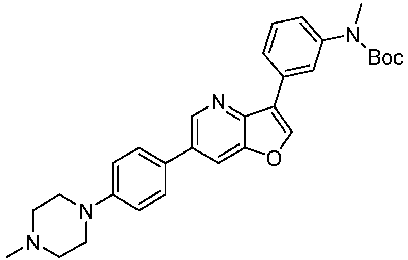

The compound was prepared by the general procedure **B** using 226 mg (0.630 mmol) of *tert-butyl* (3-(6-chlorofuro[3,2-*b*]pyridin-3-yl)phenyl)(methyl)carbamate **(Prep. Example 108)** and 247 mg (0.817 mmol) of 1-methyl-4-(4-(4,4,5,5-tetramethyl-1,3,2-dioxaborolan-2-yl)phenyl)piperazine; the reaction time was 2.5 h; flash chromatography (DCM/MeOH, gradient from 1:0 to 19:1) afforded the compound as an off-white solid (228 mg, 73 % yield).

¹H NMR (500 MHz, CDCl₃) δ 8.87 (d, *J* = 1.9 Hz, 1H), 8.11 (s, 1H), 8.03 (t, *J=* 1.8 Hz, 1H), 7.92 (d, *J* = 1.9 Hz, 1H), 7.91 - 7.87 (m, 1H), 7.59 - 7.55 (m, 2H), 7.44 (t, *J* = 7.9 Hz, 1H), 7.24 (br d, *J=* 8.1 Hz, 1H), 7.07 - 7.03 (m, 2H), 3.34 (s, 3H), 3.33 - 3.28 (m, 4H), 2.65 - 2.58 (m, 4H), 2.38 (s, 3H), 1.48 (s, 9H).

¹³C NMR (126 MHz, CDCl₃) δ 154.97, 151.21, 149.46, 145.14, 145.01, 144.53, 144.29, 133.25, 131.19, 129.12, 128.92, 128.30, 124.97, 124.41, 124.18, 121.54, 116.35, 116.02, 80.52, 55.19, 48.83, 46.32, 37.55, 28.53.

HRMS (APCI): calcd. for C₃₀H₃₄N₄O₃ [M+H]⁺ = 499.2704; found [M+H]⁺ = 499.2706.

### Preparative Example 110: N-methyl-3 -(6-(4-(4-methylpiperazin-1-yl)phenyl)furo[3,2-b]pyridin-3-yl)aniline

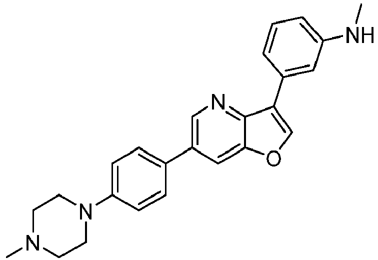

HCl (35% aq., 2.0 mL) was added to a suspension of 140 mg (0.281 mmol) of *tert*-butyl methyl(3-(6-(4-(4-methylpiperazin-1-yl)phenyl)furo[3,2-*b*]pyridin-3-yl)phenyl)carbamate **(Prep. Example 109)** in MeOH (2.0 mL). The reaction mixture was stirred at 50°C for 1 h and then evaporated *in vacuo.* Mixture of DCM/MeOH 95:5 (5.0 mL) and NH₃ (7M in MeOH, 1.0 mL, 7.0 mmol) were added to the mixture and the mixture was stirred for 1 h and then evaporated *in vacuo.* Flash chromatography (DCM/MeOH, gradient from 0 % to 6 % MeOH) afforded the compound as an off-white solid (104 mg, 74 % yield).

¹H NMR (500 MHz, CDCl₃) δ 8.89 (d, *J* = 1.9 Hz, 1H), 8.11 (s, 1H), 7.93 (d, *J* = 1.9 Hz, 1H), 7.62 - 7.57 (m, 2H), 7.46 - 7.42 (m, 1H), 7.39 - 7.36 (m, 1H), 7.32 (t, *J=* 7.8 Hz, 1H), 7.10 - 7.05 (m, 2H), 6.65 (dd, *J=* 7.9, 1.4 Hz, 1H), 3.87 (br s, 1H), 3.35 (br s, 4H), 2.94 (s, 3H), 2.66 (br s, 4H), 2.43 (br s, 3H).

¹³C NMR (126 MHz, DMSO) δ 150.09, 149.60, 148.75, 146.40, 144.10, 143.77, 131.83, 130.75, 129.03, 127.95, 127.84, 120.89, 116.02, 115.58, 114.24, 111.24, 109.79, 52.67, 45.86, 43.10, 29.82.

HRMS (APCI): calcd. for C₂₅H₂₆N₄O [M+H]⁺ = 399.2179; found [M+H]⁺ = 399.2183.

### Preparative Example 111: 6-chloro-3-(3-(trifluoromethoxy)phenyl)furo[3,2-b]pyridine

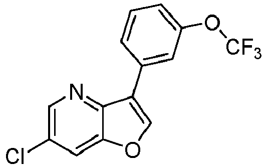

The compound was prepared by the general procedure **A** using 60 mg (0.258 mmol) of 3-bromo-6-chlorofuro[3,2-*b*]pyridine **(Prep. Example 5)** and 70 mg (0.340 mmol) of (3-(trifluoromethoxy)phenyl)boronic acid; the reaction time was 3 h; flash chromatography (cyclohexane/EtOAc, gradient from 1:0 to 93:7) afforded the compound as a white solid (74 mg, 91 % yield).

¹H NMR (500 MHz, CDCl₃) δ 8.64 (d, *J* = 2.0 Hz, 1H), 8.15 (s, 1H), 8.01 - 7.98 (m, 1H), 7.96 (br s, 1H), 7.85 (d, *J* = 2.0 Hz, 1H), 7.50 (t, *J* = 8.0 Hz, 1H), 7.25 - 7.21 (m, 1H).

¹³C NMR (126 MHz, CDCl₃) δ 149.89, 148.59, 146.04, 145.62, 144.05, 132.07, 130.39, 128.09, 125.47, 120.78, 120.72 (q, *J*= 257.4 Hz), 120.36, 119.79, 119.02.

HRMS (APCI): calcd. for C₁₄H₇ClF₃NO₂ [M+H]⁺ = 314.0190; found [M+H]⁺ = 314.0189

### Preparative Example 112: tert-butyl 4-(4-(3-(3-(trifluoromethoxy)phenyl)furo[3,2-b]pyridin-6-yl)phenyl)piperazine-1-carboxylate

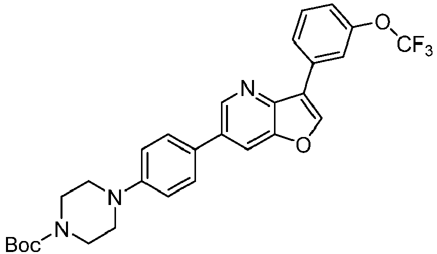

The compound was prepared by the general procedure **B** using 55 mg (0.175 mmol) of 6-chloro-3-(3-(trifluoromethoxy)phenyl)furo[3,2-*b*]pyridine **(Prep. Example 111)** and 88 mg (0.228 mmol) of 4-(4-*tert*-butoxycarbonylpiperazinyl)phenylboronic acid pinacol ester; the reaction time was 1 h; flash chromatography (cyclohexane/EtOAc, gradient from 9:1 to 7:3) afforded the compound as a white solid (81 mg, 86 % yield).

¹H NMR (500 MHz, CDCl₃) δ 8.89 (d, *J* = 1.9 Hz, 1H), 8.15 (s, 1H), 8.09 - 8.04 (m, 1H), 8.01 (s, 1H), 7.93 (d, *J* = 1.9 Hz, 1H), 7.62 - 7.56 (m, 2H), 7.51 (t, *J=* 8.0 Hz, 1H), 7.24 - 7.20 (m, 1H), 7.06 (br d, *J* = 8.6 Hz, 2H), 3.67 - 3.58 (m, 4H), 3.29 - 3.20 (m, 4H), 1.50 (s, 9H).

¹³C NMR (126 MHz, CDCl₃) δ 154.86, 149.88, 149.86, 149.48, 145.39, 145.32, 144.02, 133.33, 132.75, 130.31, 128.40, 125.49, 121.76, 120.70, 120.04, 119.75, 119.72, 116.99, 116.21, 80.20, 49.21, 43.65, 28.59.

### Preparative Example 113: 6-(4-(piperazin-1-yl)phenyl)-3-(3-(trifluoromethoxy)phenyl)furo[3,2-b]pyridine

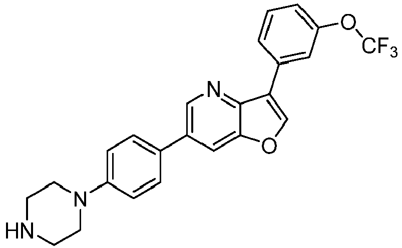

The compound was prepared by the general procedure C using 61 mg (0.113 mmol) of *tert*-butyl 4-(4-(3-(3-(trifluoromethoxy)phenyl)furo[3,2-*b*]pyridin-6-yl)phenyl)piperazine-1-carboxylate **(Prep. Example 112);** the reaction time was 2 h; flash chromatography (DCM/MeOH, gradient from 4:1 to 1:1) afforded the compound as a yellowish solid (44 mg, 89 % yield).

¹H NMR (500 MHz, CDCl₃) δ 8.90 (d, *J* = 1.9 Hz, 1H), 8.14 (s, 1H), 8.09 - 8.04 (m, 1H), 8.01 (br s, 1H), 7.93 (d, *J* = 1.9 Hz, 1H), 7.61 - 7.55 (m, 2H), 7.51 (t, *J* = 8.0 Hz, 1H), 7.24 - 7.20 (m, 1H), 7.07 - 7.03 (m, 2H), 3.30 - 3.21 (m, 4H), 3.14 - 3.03 (m, 4H).

¹³C NMR (126 MHz, CDCl₃) δ 151.70, 149.87, 149.53, 145.36, 145.29, 143.91, 133.51, 132.81, 130.31, 128.85, 128.30, 125.51, 121.77, 120.71, 120.02, 119.76, 116.42, 116.13, 49.94, 46.08.

HRMS (APCI): calcd. for C₂₄H₂₀F₃N₃O₂ [M+H]⁺ = 440.1580; found [M+H]⁺ = 440.1583.

### Preparative Example 114: 6-chloro-3-(3-(2,2,2-trifluoroethoxy)phenyl)furo[3,2-b]pyridine

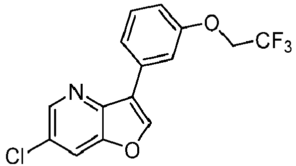

The compound was prepared by the general procedure **A** using 75 mg (0.32 mmol) of 3-bromo-6-chlorofuro[3,2-*b*]pyridine **(Prep. Example 5)** and 92 mg (0.42 mmol) of (3-(2,2,2-trifluoroethoxy)phenyl)boronic acid; the reaction time was 1 h; flash chromatography (cyclohexane/EtOAc, gradient from 1:0 to 9:1) afforded the compound as a yellowish solid (103 mg, 98 % yield).

¹H NMR (500 MHz, CDCl₃) δ 8.63 (d, *J* = 2.0 Hz, 1H), 8.12 (s, 1H), 7.83 (d, *J* = 2.0 Hz, 1H), 7.77 - 7.75 (m, 1H), 7.67 (d, *J* = 7.7 Hz, 1H), 7.42 (t, *J* = 8.0 Hz, 1H), 6.96 (dd, *J* = 8.3, 2.5 Hz, 1H), 4.45 (q, *J* = 8.1 Hz, 2H).

¹³C NMR (126 MHz, CDCl₃) δ 157.96, 148.57, 145.92, 145.46, 144.28, 131.76, 130.32, 127.91, 123.55 (q, J= 278.8 Hz), 121.36, 121.25, 118.94, 114.60, 113.99, 66.11 (q, *J* = 35.9 Hz).

### Preparative Example 115: tert-butyl 4-(4-(3-(3-(2,2,2-trifluoroethoxy)phenyl)furo[3,2-b]pyridin-6-yl)phenyl)piperazine-1-carboxylate

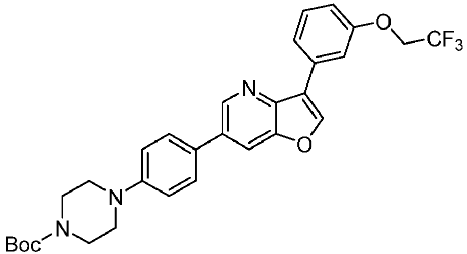

The compound was prepared by the general procedure **B** using 85 mg (0.259 mmol) of 6-chloro-3-(3-(2,2,2-trifluoroethoxy)phenyl)furo[3,2-*b*]pyridine **(Prep. Example 114)** and 131 mg (0.337 mmol) of 4-(4-*tert*-butoxycarbonylpiperazinyl)phenylboronic acid pinacol ester; the reaction time was 2 h; flash chromatography (cyclohexane/Et₂O, gradient from 7:3 to 2:3) afforded the compound as a white solid (98 mg, 69 % yield).

¹H NMR (500 MHz, CDCl₃) δ 8.89 (d, *J=* 1.8 Hz, 1H), 8.13 (s, 1H), 7.93 (d, *J=* 1.8 Hz, 1H), 7.86 - 7.81 (m, 1H), 7.73 (br d, *J=* 7.7 Hz, 1H), 7.60 - 7.56 (m, 2H), 7.43 (t, *J* = 8.0 Hz, 1H), 7.05 (br d, *J* = 8.6 Hz, 2H), 6.96 (dd, *J* = 8.2, 2.4 Hz, 1H), 4.47 (q, *J* = 8.2 Hz, 2H), 3.68 - 3.59 (m, 4H), 3.27 - 3.19 (m, 4H), 1.50 (s, 9H).

¹³C NMR (126 MHz, CDCl₃) δ 157.96, 154.88, 151.08, 149.47, 145.28, 145.20, 144.24, 133.20, 132.44, 130.27, 129.52, 128.38, 123.59 (q, *J* = 278.8 Hz), 121.30, 116.93, 116.18, 114.38, 113.92, 80.18, 66.11 (q, *J=* 35.6 Hz), 49.15, 43.61, 28.60.

HRMS (APCI): calcd. for C₃₀H₃₀F₃N₃O₄ [M+H]⁺ = 554.2261; found [M+H]⁺ = 554.2255.

### Preparative Example 116: 6-(4-(piperazin-1-yl)phenyl)-3-(3-(2,2,2-trifluoroethoxy)phenyl)furo[3,2-b]pyridine

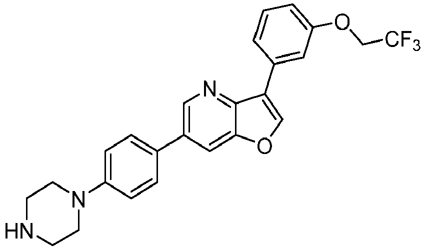

The compound was prepared by the general procedure C using 86 mg (0.155 mmol) of *tert*-butyl 4-(4-(3-(3-(2,2,2-trifluoroethoxy)phenyl)furo[3,2-*b*]pyridin-6-yl)phenyl)piperazine-1-carboxylate **(Prep. Example 115);** the reaction time was 2 h; flash chromatography (MeOH) afforded the compound as a white solid (50 mg, 71 % yield).

¹H NMR (500 MHz, CDCl₃) δ 8.89 (d, *J=* 1.8 Hz, 1H), 8.13 (s, 1H), 7.93 (d, *J=* 1.8 Hz, 1H), 7.85 - 7.80 (m, 1H), 7.73 (br d, *J=* 7.7 Hz, 1H), 7.60 - 7.56 (m, 2H), 7.43 (t, *J=* 8.0 Hz, 1H), 7.07 - 7.03 (m, 2H), 6.96 (dd, *J* = 8.2, 2.4 Hz, 1H), 4.47 (q, *J* = 8.1 Hz, 2H), 3.28 - 3.22 (m, 4H), 3.11 - 3.03 (m, 4H). ¹³C NMR (126 MHz, MeOD) δ 159.30, 153.13, 151.04, 147.73, 145.55, 144.89, 134.84, 133.47, 131.11, 129.82, 129.03, 122.74 (q, *J* = 268.4 Hz), 122.21, 121.31, 117.62, 117.32, 115.34, 114.78, 66.69 (q*, J* = 35.3 Hz), 50.64, 46.52.

HRMS (APCI): calcd. for C₂₅H₂₂F₃N₃O₂ [M+H]⁺ = 454.1737; found [M+H]⁺ = 454.1737.

### Preparative Example 117: 3-(6-chlorofuro[3,2-b]pyridin-3-yl)-N-(p-tolyl)benzamide

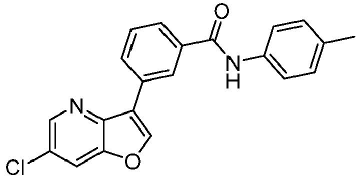

The compound was prepared by the general procedure **A** using 75 mg (0.323 mmol) of 3-bromo-6-chlorofuro[3,2-*b*]pyridine **(Prep. Example 5)** and 107 mg (0.419 mmol) of (3-(*p-*tolylcarbamoyl)phenyl)boronic acid; the reaction time was 2 h; flash chromatography (cyclohexane/EtOAc, gradient from 9:1 to 4:1) afforded the compound as a white solid (70 mg, 61 % yield).

¹H NMR (500 MHz, CDCl₃) δ 8.64 *(d, J=* 2.0 Hz, 1H), 8.57 - 8.54 (m, 1H), 8.26 - 8.22 (m, 1H), 8.21 (s, 1H), 7.93 (br s, 1H), 7.88 - 7.83 (m, 2H), 7.62 - 7.54 (m, 3H), 7.19 (d, *J=* 8.2 Hz, 2H), 2.36 (s, 3H). ¹³C NMR (126 MHz, CDCl₃) δ 165.53, 148.65, 146.23, 145.41, 144.10, 135.99, 135.56, 134.48, 130.72, 130.33, 129.77, 129.53, 128.07, 126.65, 125.80, 121.06, 120.52, 119.21, 21.07.

HRMS (APCI): calcd. for C₂₁H₁₅ClN₂O₂ [M+H]⁺ = 363.0895; found [M+H]⁺ = 363.0893.

### Preparative Example 118: tert-butyl 4-(4-(3-(3-(p-tolylcarbamoyl)phenyl)furo[3,2-b]pyridin-6-yl)phenyl)piperazine-1-carboxylate

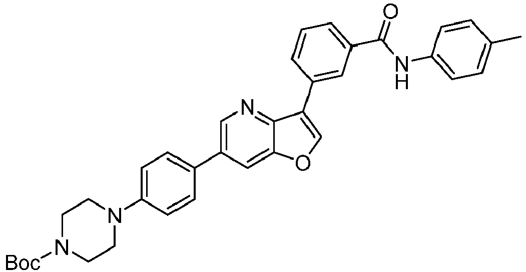

The compound was prepared by the general procedure **B** using 58 mg (0.160 mmol) of 3-(6-chlorofuro[3,2-*b*]pyridin-3-yl)-*N*-(*p*-tolyl)benzamide **(Prep. Example 117)** and 81 mg (0.208 mmol) of 4-(4-*tert*-butoxycarbonylpiperazinyl)phenylboronic acid pinacol ester; the reaction time was 2 h; flash chromatography (cyclohexane/EtOAc 2:3) afforded the compound as a yellowish solid (71 mg, 75 % yield).

¹H NMR (500 MHz, CDCl₃) δ 8.86 (d, *J* = 1.5 Hz, 1H), 8.56 (s, 1H), 8.26 (br d, *J* = 7.7 Hz, 1H), 8.20 - 8.13 (m, 2H), 7.91 (d, *J=* 1.4 Hz, 1H), 7.84 (br d, *J=* 7.7 Hz, 1H), 7.62 - 7.53 (m, 5H), 7.18 (d, *J* = 8.1 Hz, 2H), 7.03 (d, *J* = 8.6 Hz, 2H), 3.67 - 3.57 (m, 4H), 3.27 - 3.17 (m, 4H), 2.35 (s, 3H), 1.50 (s, 9H). ¹³C NMR (126 MHz, CDCl₃) δ 165.77, 154.87, 151.17, 149.49, 145.49, 145.00, 143.96, 135.90, 135.73, 134.28, 133.31, 131.21, 130.27, 129.68, 129.39, 129.18, 128.32, 126.54, 125.68, 120.94, 120.56, 116.82, 116.29, 80.16, 49.02, 43.74, 28.59, 21.05.

HRMS (APCI): calcd. for C₃₆H₃₆N₄O₄ [M+H]⁺ = 589.2809; found [M+H]⁺ = 589.2812.

### Preparative Example 119: 3-(6-(4-(piperazin-1-yl)phenyl)furo[3,2-b]pyridin-3-yl)-N-(p-tolyl)benzamide

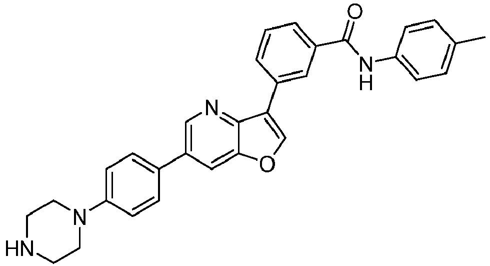

The compound was prepared by the general procedure **D** using 70 mg (0.119 mmol) of *tert*-butyl (4-(3-(3-(*p*-tolylcarbamoyl)phenyl)furo[3,2-*b*]pyridin-6-yl)phenyl)piperazine-1-carboxylate **(Prep. Example 118);** the reaction time was 1 h; flash chromatography (DCM/7M NH₃ in MeOH 97:3) afforded the compound as off-white solid (57 mg, 98 % yield).

¹H NMR (500 MHz, DMSO) δ 10.24 (s, 1H), 8.96 (s, 1H), 8.92 (s, 1H), 8.73 (s, 1H), 8.48 (d, *J=* 7.8 Hz, 1H), 8.32 (s, 1H), 7.91 (d, *J* = 7.8 Hz, 1H), 7.73 - 7.62 (m, 5H), 7.18 (d, *J=* 8.2 Hz, 2H), 7.05 (d, *J* = 8.6 Hz, 2H), 3.17 - 3.10 (m, 4H), 2.92 - 2.81 (m, 4H), 2.29 (s, 3H).

¹³C NMR (126 MHz, CDCl₃) δ 166.09, 151.72, 149.57, 145.49, 144.88, 143.71, 136.14, 136.04, 134.07, 133.52, 130.82, 130.09, 129.60, 129.38, 128.45, 128.20, 127.08, 125.70, 121.08, 120.59, 116.37, 116.31, 49.92, 46.12, 21.03.

HRMS (APCI): calcd. for C₃₁H₂₈N₄O₂ [M+H]⁺ = 489.2285; found [M+H]⁺ = 489.2282.

### Preparative Example 120: N-benzyl-3-(6-chlorofuro[3,2-b]pyridin-3-yl)benzenesulfonamide

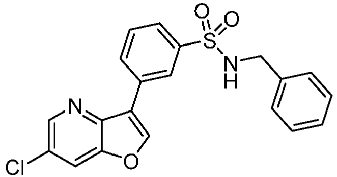

The compound was prepared by the general procedure **A** using 100 mg (0.430 mmol) of 3-bromo-6-chlorofuro[3,2-*b*]pyridine **(Prep. Example 5)** and 163 mg (0.560 mmol) of (3-(*N-*benzylsulfamoyl)phenyl)boronic acid; the reaction time was 2 h; flash chromatography (cyclohexane/EtOAc, gradient from 1:0 to 0:1) afforded the compound as a white solid (86 mg, 50 % yield).

¹H NMR (500 MHz, CDCl₃) δ 8.63 (d, *J* = 2.0 Hz, 1H), 8.51 (t, *J* = 1.7 Hz, 1H), 8.38 - 8.34 (m, 1H), 8.19 (s, 1H), 7.89 - 7.85 (dd, *J* = 4.8, 1.6 Hz, 2H), 7.63 (t, *J* = 7.8 Hz, 1H), 7.29 - 7.21 (m, 5H), 4.78 (t, *J* = 6.0 Hz, 1H), 4.23 (d, *J* = 6.2 Hz, 2H).

¹³C NMR (126 MHz, CDCl₃) δ 148.60, 146.31, 145.68, 143.93, 140.87, 136.29, 131.47, 131.31, 129.89, 128.89, 128.22, 128.17, 128.10, 126.44, 125.51, 120.45, 119.12, 47.61.

### Preparative Example 121: tert-butyl 4-(4-(3-(3-(N-benzylsulfamoyl)phenyl)furo[3,2-b]pyridin-6-yl)phenyl)piperazine-1-carboxylate

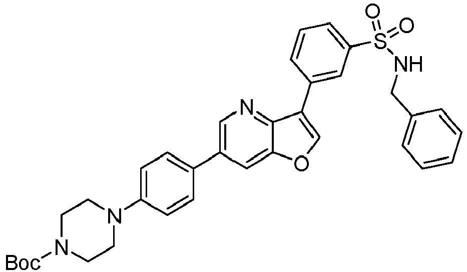

The compound was prepared by the general procedure **B** using 75 mg (0.189 mmol) of *N*-benzyl-3-(6-chlorofuro[3,2-b]pyridin-3-yl)benzenesulfonamide **(Prep. Example 120)** and 95 mg (0.246 mmol) of 4-(4-*tert*-butoxycarbonylpiperazinyl)phenylboronic acid pinacol ester; the reaction time was 2 h; flash chromatography (cyclohexane/EtOAc, gradient from 4:1 to 1:9) afforded the compound as an off-white solid (70 mg, 59 % yield).

¹H NMR (500 MHz, CDCl₃) δ 8.89 *(d, J=* 1.8 Hz, 1H), 8.57 (s, 1H), 8.43 (br d, *J=* 7.6 Hz, 1H), 8.19 (s, 1H), 7.96 (d, *J* = 1.1 Hz, 1H), 7.89 - 7.84 (m, 1H), 7.64 (t, *J* = 7.8 Hz, 1H), 7.61 - 7.55 (m, 2H), 7.31 - 7.20 (m, 5H), 7.06 (d, *J* = 8.6 Hz, 2H), 4.89 (br s, 1H), 4.24 (d, *J* = 6.2 Hz, 2H), 3.77 - 3.52 (m, 4H), 3.32 - 3.16 (m, 4H), 1.50 (s, 9H).

¹³C NMR (126 MHz, CDCl₃) δ 154.89, 151.23, 149.52, 145.63, 145.36, 143.82, 140.76, 136.41, 133.53, 132.14, 131.30, 129.81, 129.19, 128.86, 128.37, 128.11, 126.15, 125.48, 120.37, 116.86, 116.29, 80.19, 49.03, 47.62, 43.78, 28.59.

HRMS (APCI): calcd. for C₃₅H₃₆N₄O₅S [M+H]⁺ = 625.2479; found [M+H]⁺ = 625.2474.

### Preparative Example 122: N-benzyl-3-(6-(4-(piperazin-1-yl)phenyl)furo[3,2-b]pyridin-3-yl)benzenesulfonamide

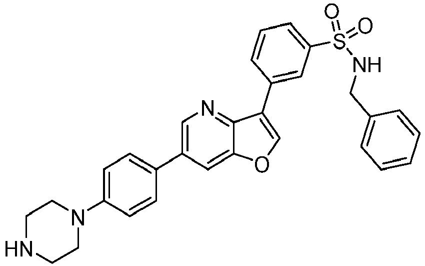

The compound was prepared by the general procedure **D** using 60 mg (0.096 mmol) of *tert*-butyl 4-(4-(3-(3-(*N*-benzylsulfarnoyl)phenyl)furo[3,2-*b*]pyridin-6-yl)phenyl)piperazine-1-carboxylate **(Prep. Example 121)** in MeOH (2.0 mL); the reaction time was 2 h; flash chromatography (DCM/7M NH₃ in MeOH 24:1) afforded the compound as a white solid (17 mg, 34 % yield).

¹H NMR (500 MHz, DMSO) δ 8.99 - 8.92 (m, 2H), 8.76 (s, 1H), 8.46 (d, *J* = 7.8 Hz, 1H), 8.34 (s, 1H), 8.22 (br s, 1H), 7.80 (d, *J* = 7.9 Hz, 1H), 7.70 (t, *J* = 7.7 Hz, 3H), 7.30 - 7.17 (m, 5H), 7.06 (d, *J* = 8.7 Hz, 2H), 4.08 (s, 2H), 3.18 - 3.09 (m, 4H), 2.91 - 2.81 (m, 4H), 2.32 (br s, 1H).

¹³C NMR (126 MHz, CDCl₃) δ 151.83, 149.57, 145.53, 145.41, 143.75, 140.74, 136.39, 133.70, 132.23, 131.35, 129.85, 128.90, 128.63, 128.30, 128.14, 126.15, 125.49, 120.40, 116.38, 116.23, 53.55, 50.07, 47.65, 46.24.

HRMS (APCI): calcd. for C₃₀H₂₈N₄O₃S [M+H]⁺ = 525.1955; found [M+H]⁺ = 525.1958

### Preparative Example 123: N-benzyl-3-(6-chlorofuro[3,2-b]pyridin-3-yl)benzenesulfonamide

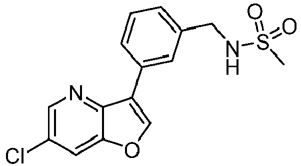

The compound was prepared by the general procedure **A** using 100 mg (0.430 mmol) of 3-bromo-6-chlorofuro[3,2-*b*]pyridine **(Prep. Example 5)** and 128 mg (0.559 mmol) of (3-(methylsulfonamidomethyl)phenyl)boronic acid; the reaction time was 2 h; flash chromatography (cyclohexane/EtOAc, gradient from 9:1 to 3:2) afforded the compound as a white solid (139 mg, 96 % yield).

¹H NMR (500 MHz, CDCl₃) δ 8.61 (d, *J* = 2.0 Hz, 1H), 8.14 (s, 1H), 8.07 (s, 1H), 7.94 (d, *J* = 7.7 Hz, 1H), 7.84 (d, *J* = 2.0 Hz, 1H), 7.47 (t, *J* = 7.7Hz, 1H), 7.36 (d, *J* = 8.0 Hz, 1H), 4.81 (br s, 1H), 4.42 (d, *J=* 6.1 Hz, 2H), 2.94 (s, 3H).

¹³C NMR (126 MHz, CDCl₃) δ 148.58, 145.90, 145.39, 144.29, 137.61, 130.74, 129.64, 127.93, 127.59, 126.84, 126.66, 121.43, 119.00, 47.32, 41.42.

HRMS (APCI): calcd. for C₁₅H₁₃ClN₂O₃S [M+H]⁺ = 337.0408; found [M+H]⁺ = 337.0408.

### Preparative Example 124: tert-butyl 4-(4-(3-(3-(methylsulfonamidomethyl)phenyl)furo[3,2-b]pyndin-6-yl)phenyl)piperazine-1-carboxylate

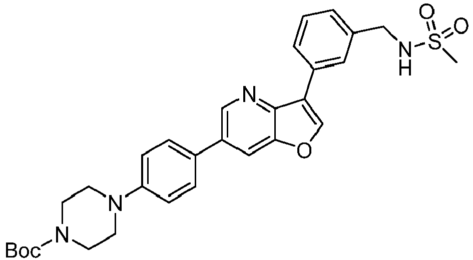

The compound was prepared by the general procedure **B** using 120 mg (0.356 mmol) of *N*-Benzyl-3-(6-chloroiuro[3,2-*b*]pyridin-3-yl)benzenesulfonamide **(Prep. Example 123)** and 180 mg (0.463 mmol) of 4-(4-*tert*-butoxycarbonylpiperazinyl)phenylboronic acid pinacol ester; the reaction time was 2 h; flash chromatography (cyclohexane/EtOAc, gradient from 1:0 to 1:1) afforded the compound as an off-white solid (166 mg, 83 % yield).

¹H NMR (500 MHz, CDCl₃) δ 8.86 (d, *J* = 1.9 Hz, 1H), 8.16 - 8.11 (m, 2H), 7.98 (br d, *J=* 7.7 Hz, 1H), 7.92 (d, *J* = 1.8 Hz, 1H), 7.59 - 7.54 (m, 2H), 7.48 (t, *J* = 7.7 Hz, 1H), 7.35 (br d, *J* = 7.7 Hz, 1H), 7.06 - 7.01 (m, 2H), 4.92 (br s, 1H), 4.43 (d, *J* = 6.1 Hz, 2H), 3.70 - 3.53 (m, 4H), 3.29 - 3.17 (m, 4H), 2.95 (s, 3H), 1.50 (s, 9H).

¹³C NMR (126 MHz, CDCl₃) δ 154.87, 151.15, 149.46, 145.22, 145.05, 144.16, 137.57, 133.23, 131.34, 129.54, 129.28, 128.31, 127.33, 126.75, 126.67, 121.33, 116.83, 116.20, 80.17, 49.03, 47.35, 43.52, 41.34, 28.58.

HRMS (APCI): calcd. for C₃₀H₃₄N₄O₅S [M+H]⁺ = 563.2323; found [M+H]⁺ = 563.2326.

### Preparative Example 125: N-(3-(6-(4-(piperazin-1-yl)phenyl)furo[3,2-b]pyridin-3-yl)benzyl)methanesulfonamide

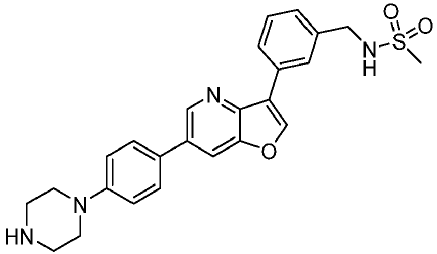

The compound was prepared by the general procedure **C** using 134 mg (0.290 mmol) of *tert*-butyl4-(4-(3-(3-(methylsulfonamidomethyl)phenyl)furo[3,2-*b*]pyridin-6-yl)phenyl)piperazine-1-carboxylate **(Prep. Example 124);** the reaction time was 2 h; flash chromatography (MeOH) afforded the compound as a white solid (90 mg, 67 % yield).

¹H NMR (500 MHz, DMSO) δ 8.94 (d, *J* = 1.9 Hz, 1H), 8.80 (s, 1H), 8.30 (d, *J* = 1.9 Hz, 1H), 8.22 (s, 1H), 8.16 (br d, *J* = 7.8 Hz, 1H), 7.72 - 7.58 (m, 2H), 7.60 (br s, 1H), 7.49 (t, *J* = 7.7 Hz, 1H), 7.38 (d, *J* = 7.7 Hz, 1H), 7.07 - 7.03 (m, 2H), 4.25 (d, *J* = 2.1 Hz, 2H), 3.18 - 3.10 (m, 4H), 2.92 (s, 3H), 2.89 - 2.80 (m, 4H), 2.27 (s, 1H).

¹³C NMR (126 MHz, CDCl₃) δ 151.79, 149.54, 145.19, 145.13, 144.14, 137.47, 133.45, 131.54, 129.65, 128.78, 128.28, 127.32, 126.87, 126.71, 121.36, 116.38, 116.16, 50.11, 47.46, 46.27, 41.50.

HRMS (APCI): calcd. for C₂₅H₂₆N₄O₃S [M+H]⁺ = 463.1798; found [M+H]⁺ = 463.1797.

### Preparative Example 126: tert-butyl (4-(6-chlorofuro[3,2-b]pyridin-3-yl)pyridin-2-yl)carbamate

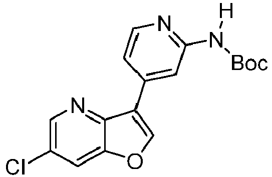

The compound was prepared by the general procedure **A** using 100 mg (0.430 mmol) of 3-bromo-6-chlorofuro[3,2-*b*]pyridine **(Prep. Example 5)** and 179 mg (0.559 mmol) of *tert-butyl* (4-(4,4,5,5-tetramethyl-1,3,2-dioxaborolan-2-yl)pyridin-2-yl)carbamate; the reaction time was 2 h; flash chromatography (cyclohexane/EtOAc 1:4) afforded the compound as a white solid (108 mg, 73 % yield). ¹H NMR (500 MHz, CDCl₃) δ 8.65 (d, *J=* 2.0 Hz, 1H), 8.49 - 8.44 (m, 2H), 8.40 (br d, *J=* 5.2 Hz, 1H), 8.33 (s, 1H), 7.88 (dd, *J* = 5.2, 1.3 Hz, 1H), 7.85 (d, *J* = 2.0 Hz, 1H), 1.57 (s, 9H).

¹³C NMR (126 MHz, CDCl₃) δ 152.93, 152.77, 148.65, 148.51, 147.76, 145.82, 143.90, 140.12, 128.15, 120.06, 119.11, 116.81, 109.36, 81.25, 28.54.

### Preparative Example 127: tert-butyl 4-(4-(3-(2-((tert-butoxycarbonyl)amino)pyridin-4-yl)furo[3,2-b]pyridin-6-yl)phenyl)piperazine-1-carboxylate

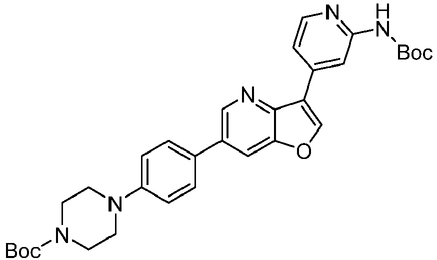

The compound was prepared by the general procedure **B** using 53 mg (0.155 mmol) of *tert*-butyl (4-(6-chlorofuro[3,2-*b*]pyridin-3-yl)pyridin-2-yl)carbamate **(Prep. Example 126)** and 78 mg (0.202 mmol) of 4-(4-*tert*-butoxycarbonylpiperazinyl)phenylboronic acid pinacol ester; the reaction time was 2 h; flash chromatography (cyclohexane/EtOAc 2:3) afforded the compound as white solid (80 mg, 90 % yield).

¹H NMR (500 MHz, CDCl₃) δ 8.91 (d, *J* = 1.9 Hz, 1H), 8.49 (s, 1H), 8.38 (d, *J* = 5.2 Hz, 1H), 8.33 (s, 1H), 7.96 (dd, *J* = 5.2, 1.3 Hz, 1H), 7.94 (d, *J* = 1.9 Hz, 1H), 7.88 (br s, 1H), 7.59 - 7.55 (m, 2H), 7.06 - 7.01 (m, 2H), 3.64 - 3.60 (m, 4H), 3.26 - 3.20 (m, 4H), 1.57 (s, 9H), 1.50 (s, 9H).

¹³C NMR (126 MHz, CDCl₃) δ 154.74, 152.53, 151.05, 149.42, 148.52, 146.91, 145.39, 143.69, 140.54, 133.31, 129.14, 128.23, 119.84, 119.09, 116.71, 116.12, 115.93, 109.10, 81.03, 80.03, 51.02, 48.90, 28.44, 28.37.

### Preparative Example 128: tert-butyl 4-(4-(3-(2-((tert-butoxycarbonyl)amino)pyridin-4-yl)furo[3,2-b]pyridin-6-yl)phenyl)piperazine-1-carboxylate

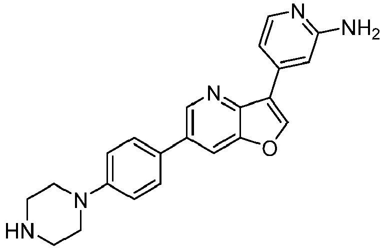

The compound was prepared by the general procedure **D** using 53 mg (0.093 mmol) of *tert*-butyl 4-(4-(3-(2-((*tert*-butoxycarbonyl)amino)pyridin-4-yl)furo[3,2-*b*]pyridin-6-yl)phenyl)piperazine-1-carboxylate **(Prep. Example 127);** the reaction time was 1 h; flash chromatography (DCM/7M NH₃ in MeOH 23:2) afforded the compound as a white solid (47 mg, 98 % yield).

¹H NMR (500 MHz, DMSO) δ 8.91 *(d, J=* 1.9 Hz, 1H), 8.87 (s, 1H), 8.32 (d, *J=* 1.9 Hz, 1H), 8.00 - 7.96 (m, 1H), 7.70 (d, *J=* 8.9 Hz, 2H), 7.48 (s, 1H), 7.16 (dd, *J* = 5.3, 1.4 Hz, 1H), 7.07 (d, *J* = 8.9 Hz, 2H), 6.01 (s, 2H), 3.21 - 3.15 (m, 4H), 2.95 - 2.89 (m, 4H).

¹³C NMR (126 MHz, DMSO) δ 160.25, 150.97, 148.92, 148.21, 148.06, 144.31, 143.07, 138.51, 132.43, 127.80, 127.02, 118.75, 115.75, 115.64, 109.45, 105.12, 47.93, 44.73.

HRMS (APCI): calcd. for C₂₂H₂₁N₅O [M+H]⁺ = 372.1819; found [M+H]⁺ = 372.1818.

### Preparative Example 129: 3-(6-chlorofuro[3,2-b]pyridin-3-yl)-N,N-dimethylaniline

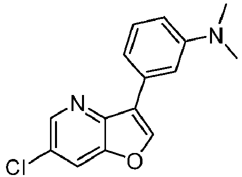

The compound was prepared by the general procedure **A** using 100 mg (0.430 mmol) of 3-bromo-6-chlorofuro[3,2-*b*]pyridine **(Prep. Example 5)** and 92 mg (0.558 mmol) of (3-(dimethylamino)phenyl)boronic acid; the reaction time was 2 h; flash chromatography (cyclohexane/DCM 1:1) afforded the compound as an off-white solid (70 mg, 60 % yield).

¹H NMR (500 MHz, CDCl₃) δ 8.62 (d, *J* = 2.0 Hz, 1H), 8.09 (s, 1H), 7.81 (d, *J=* 2.0 Hz, 1H), 7.47 (s, 1H), 7.38 - 7.28 (m, 2H), 6.77 (d, *J* = 7.5 Hz, 1H), 3.03 (s, 6H).

¹³C NMR (126 MHz, CDCl₃) δ 151.12, 148.49, 145.67, 145.24, 144.79, 130.56, 129.68, 127.49, 122.72, 118.67, 115.68, 112.57, 111.72, 40.81.

HRMS (APCI): calcd. for C₁₅H₁₃ClN₂O [M+H]⁺ = 273.0789; found [M+H]⁺ = 273.0788.

### Preparative Example 130: tert-butyl 4-(4-(3-(3-(dimethylamino)phenyl)furo[3,2-b]pyridin-6-yl)phepyl)piperazine-1-carboxylate

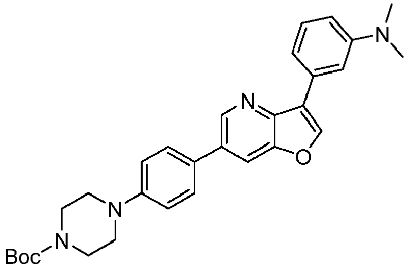

The compound was prepared by the general procedure **B** using 50 mg (0.183 mmol) of 3-(6-chlorofuro[3,2-b]pyn*din-3-yl)-N,N-dimethylaniline **(Prep. Example 129)** and 92 mg (0.238 mmol) of 4-(4-*tert*-butoxycarbonylpiperazinyl)phenylboronic acid pinacol ester; the reaction time was 2 h; flash chromatography (cyclohexane/EtOAc, gradient from 4:1 to 7:3) afforded the compound as white solid (86 mg, 94 % yield).

¹H NMR (500 MHz, CDCl₃) δ 8.79 (d, *J=* 1.9 Hz, 1H), 8.01 (s, 2H), 7.81 (d, *J=* 1.9 Hz, 1H), 7.51 - 7.47 (m, 2H), 7.45 (br s, 1H), 7.33 - 7.24 (m, 2H), 6.97 - 6.92 (m, 2H), 6.71 - 6.67 (m, 1H), 3.55 - 3.49 (m, 4H), 3.16 - 3.10 (m, 4H), 2.95 (s, 6H), 1.42 (s, 9H).

¹³C NMR (126 MHz, CDCl₃) δ 154.86, 151.09, 151.01, 149.35, 145.01, 144.75, 132.77, 131.22, 129.66, 129.62, 128.29, 127.33, 122.62, 119.28, 116.84, 115.91, 112.41, 111.79, 80.11, 49.08, 43.52, 40.87, 28.57.

HRMS (APCI): calcd. for C₃₀H₃₄N₄O₃ [M+H]⁺ = 499.2704; found [M+H]⁺ = 499.2708.

### Preparative Example 131: N,N-dimethyl-3-(6-(4-(piperazin-1-yl)phenyl)furo[3,2-b]pyridin-3-yl)aniline

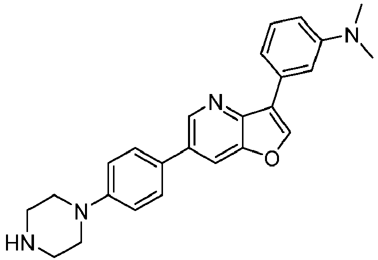

The compound was prepared by the general procedure **D** using 52 mg (0.104 mmol) of *tert*-butyl 4-(4-(3-(2-(dimethylamino)pyridin-4-yl)furo[3,2-*b*]pyridin-6-yl)phenyl)piperazine-1-carboxylate **(Prep. Example 129);** the reaction time was 1 h; flash chromatography (DCM/7M NH₃ in MeOH, 19:1) afforded the compound as a white solid (38 mg, 92 % yield).

¹H NMR (500 MHz, DMSO) δ 8.92 *(d, J=* 1.9 Hz, 1H), 8.78 (s, 1H), 8.26 *(d, J=* 1.9 Hz, 1H), 7.72 - 7.66 (m, 2H), 7.66 - 7.61 (m, 1H), 7.54 (d, *J* = 7.7 Hz, 1H), 7.28 (t, *J* = 7.9 Hz, 1H), 7.07 - 7.02 (m, 2H), 6.74 (dd, *J=* 8.2, 2.4 Hz, 1H), 3.16 - 3.09 (m, 4H), 2.98 (s, 6H), 2.89 - 2.82 (m, 4H). ¹³C NMR (126 MHz, DMSO) δ 151.31, 150.67, 148.83, 146.45, 144.12, 143.56, 132.09, 130.85, 129.07, 127.65, 126.79, 120.79, 115.41, 115.37, 114.83, 111.81, 110.63, 48.90, 45.48, 40.18.

HRMS (APCI): calcd. for C₂₅H₂₆N₄O [M+H]⁺ = 399.2179; found [M+H]⁺ = 399.2182.

### Preparative Example 132: (3-(6-chlorofuro[3.2-b]pyridin-3-yl)phenyl)(phenyl)methanone

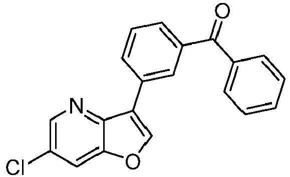

The compound was prepared by the general procedure **A** using 100 mg (0.430 mmol) of 3-bromo-6-chlorofuro[3,2-*b*]pyridine **(Prep. Example 5)** and 179 mg (0.561 mmol) of phenyl(3-(4,4,5,5-tetramethyl-1,3,2-dioxaborolan-2-yl)phenyl)methanone; the reaction time was 2 h; flash chromatography (cyclohexane/EtOAc, 97:3) afforded the compound as an off-white solid (113 mg, 79 % yield).

¹H NMR (500 MHz, CDCl₃) δ 8.59 (d, *J* = 2.0 Hz, 1H), 8.40 - 8.34 (m, 2H), 8.16 (s, 1H), 7.88 - 7.84 (m, 2H), 7.83 (d, *J=* 2.0 Hz, 1H), 7.78 - 7.74 (m, 1H), 7.62 - 7.57 (m, 2H), 7.52 - 7.47 (m, 2H).

¹³C NMR (126 MHz, CDCl₃) δ 196.55, 148.57, 146.04, 145.55, 144.29, 138.39, 137.63, 132.74, 131.27, 130.43, 130.35, 129.74, 129.01, 128.52, 128.50, 127.99, 121.27, 118.98.

HRMS (APCI): calcd. for C₂₀H₁₂ClNO₂ [M+H]⁺ = 334.0629; found [M+H]⁺ = 334.0631.

### Preparative Example 133: (3-(6-(4-(4-methylpiperazin-1-yl)phenyl)furo[3,2-b]pyridin-3-yl)phenyl)(phenyl)methanone

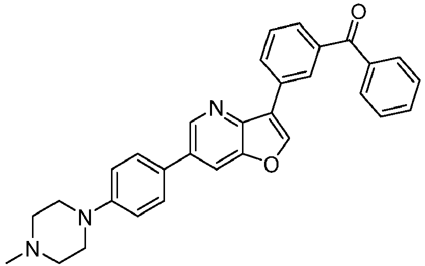

The compound was prepared by the general procedure **B** using 80 mg (0.240 mmol) of (3-(6-chlorofuro[3,2-*b*]pyridin-3-yl)phenyl)(phenyl)methanone **(Prep. Example 132)** and 94 mg (0.311 mmol) of 1-methyl-4-(4-(4,4,5,5-tetramethyl-1,3,2-dioxaborolan-2-yl)phenyl)piperazine; the reaction time was 1 h; flash chromatography (DCM/MeOH, gradient from 1:0 to 9:1) afforded the compound as an off-white solid (109 mg, 96 % yield).

¹H NMR (500 MHz, CDCl₃) δ 8.89 (d, *J* = 1.9 Hz, 1H), 8.51 - 8.47 (m, 1H), 8.45 (t, *J* = 1.5 Hz, 1H), 8.19 (s, 1H), 7.96 (d, *J* = 1.9 Hz, 1H), 7.94 - 7.89 (m, 2H), 7.79 (dt, *J* = 7.7, 1.3 Hz, 1H), 7.67 - 7.57 (m, 4H), 7.56 - 7.50 (m, 2H), 7.11 - 7.04 (m, 2H), 3.42 - 3.33 (m, 4H), 2.72 (br s, 4H), 2.46 (s, 3H).

¹³C NMR (126 MHz, CDCl₃) δ 196.69, 151.24, 149.49, 145.31, 144.13, 138.32, 137.74, 133.45, 132.66, 131.39, 131.14, 130.36, 129.45, 128.98, 128.80, 128.48, 128.30, 121.19, 116.34, 116.12, 55.19, 48.82, 46.32.

HRMS (APCI): calcd. for C₃₁H₂₇N₃O₂ [M+H]⁺ = 474.2176; found [M+H]⁺ = 474.2180.

### Preparative Example 134: tert-butyl (3-(6-chlorofuro[3.2-b]pyridin-3-yl)benzyl)carbamate

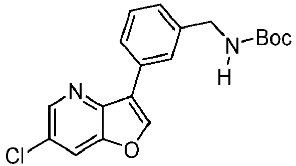

The compound was prepared by the general procedure **A** using 100 mg (0.43 mmol) of 3-bromo-6-chlorofuro[3,2-*b*]pyridine **(Prep. Example 5)** and 141 mg (0.56 mmol) of (3-(((*tert-*butoxycarbonyl)amino)methyl)phenyl)boronic acid; the reaction time was 2 h; flash chromatography (cyclohexane/EtOAc, with gradient from 97:3 to 9:1) afforded the compound as a yellow oil (148 mg, 96 % yield).

¹H NMR (500 MHz, CDCl₃) δ 8.61 (d, *J* = 2.0 Hz, 1H), 8.10 (s, 1H), 7.95 - 7.91 (m, 2H), 7.82 (d, *J*= 2.0 Hz, 1H), 7.44 (t, *J* = 7.8 Hz, 1H), 7.30 (br d, *J* = 7.6 Hz, 1H), 4.93 (br s, 1H), 4.40 (d, *J* = 5.4 Hz, 2H), 1.48 (s, 9H).

¹³C NMR (126 MHz, CDCl₃) δ 156.09, 148.53, 145.76, 145.33, 144.46, 139.83, 130.32, 129.36, 127.77, 127.33, 126.32, 121.83, 118.86, 79.71, 44.88, 28.58.

### Preparative Example 135: tert-butyl (3-(6-(4-(4-methylpiperazin-1-yl)phenpyl)furo[3,2-b]pyridin-3-yl)benzyl)carbamate

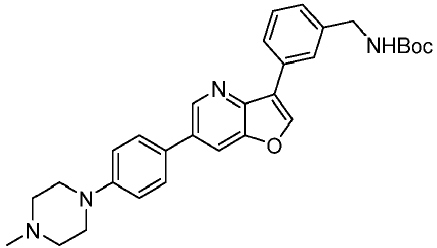

The compound was prepared by the general procedure **B** using 86 mg (0.240 mmol) of *tert-butyl* (3-(6-chlorofuro[3,2-*b*]pyridin-3-yl)benzyl)carbamate **(Prep. Example 134)** and 94 mg (0.311 mmol) of 1-methyl-4-(4-(4,4,5,5-tetramethyl-1,3,2-dioxaborolan-2-yl)phenyl)piperazine; the reaction time was 1 h; flash chromatography (DCM/MeOH, gradient from 1:0 to 9:1) afforded the compound as an off-white solid (113 mg, 94 % yield).

¹H NMR (500 MHz, CDCl₃) δ 8.80 (d, *J=* 1.9 Hz, 1H), 8.03 (s, 1H), 7.97 - 7.87 (m, 2H), 7.85 (d, *J* = 1.9 Hz, 1H), 7.53 - 7.46 (m, 2H), 7.39 (t, *J* = 7.7 Hz, 1H), 7.23 (br d, *J* = 7.6 Hz, 1H), 7.0 - 6.95 (m, 2H), 4.85 (br s, 1H), 4.34 (d, *J=* 5.3 Hz, 2H), 3.28 - 3.19 (m, 4H), 2.59 - 2.51 (m, 4H), 2.31 (s, 3H), 1.41 (s, 9H).

¹³C NMR (126 MHz, CDCl₃) δ 155.94, 151.02, 149.30, 144.97, 144.87, 144.17, 139.52, 133.08, 130.89, 129.19, 128.78, 128.13, 126.93, 126.22, 121.59, 116.20, 115.89, 79.50, 55.01, 48.64, 46.12, 44.82, 28.44.

HRMS (APCI): calcd. for C₃₀H₃₄N₄O₃ [M+H]⁺ = 499.2704; found [M+H]⁺ = 499.2706.

### Preparative Example 136: (3-(6-(4-(4-methylpiperazin-1-yl)phenyl)furo[3,2-b]pyridin-3-yl)phenyl)methanamine

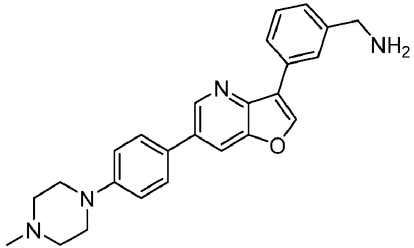

The compound was prepared by the general procedure **D** using 97 mg (0.195 mmol) of *tert*-butyl (3-(6-(4-(4-methylpiperazin-1-yl)phenyl)furo[3,2-*b*]pyridin-3-yl)benzyl)carbamate **(Prep. Example 135);** the reaction time was 1 h; flash chromatography (DCM/MeOH, gradient from 97:3 to 19:1) afforded the compound as an off-white solid (60 mg, 77 % yield).

¹H NMR (500 MHz, CDCl₃) δ 8.79 (d, *J* = 1.9 Hz, 1H), 8.02 (s, 1H), 7.94 (br s, 1H), 7.87 (br d, *J* = 7.7 Hz, 1H), 7.82 (d, *J* = 1.9 Hz, 1H), 7.51 - 7.45 (m, 2H), 7.36 (t, *J* = 7.6 Hz, 1H), 7.22 (br d, *J=* 7.7 Hz, 1H), 7.00 - 6.92 (m, 2H), 3.88 (s, 2H), 3.25 - 3.17 (m, 4H), 2.57 - 2.49 (m, 4H), 2.28 (s, 3H).

¹³C NMR (126 MHz, CDCl₃) δ 151.19, 149.48, 145.13, 145.01, 144.40, 144.04, 133.20, 130.95, 129.25, 128.93, 128.28, 126.68, 126.02, 125.83, 121.93, 116.35, 116.04, 55.19, 48.83, 46.73, 46.32.

HRMS (APCI): calcd. for C₂₅H₂₆N₄O [M+H]⁺ = 399.2179; found [M+H]⁺ = 399.2180.

### Preparative Example 137: N-(3-(6-(4-(4-methylpiperazin-1-yl)phenyl)furo[3,2-b]pyridin-3-yl)benzyl)acetamide

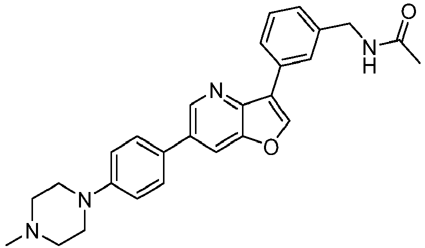

TEA (0.037 mL, 0.263 mmol) and acetic anhydride (0.053 mL, 0.525 mmol) were added to a solution of 42 mg (0.105 mmol) of (3-(6-(4-(4-methylpiperazin-1-yl)phenyl)furo[3,2-*b*]pyridin-3-yl)phenyl)methanamine **(Prep. Example 136)** in DCM (2 mL) , and the mixture was stirred for 3 h at 25 °C. Volatiles were evaporated *in vacuo* and the residue was purified by flash chromatography (cyclohexane/EtOAc, 1:1). The product was obtained as an off-white solid (44 mg, 94 % yield).

¹H NMR (500 MHz, CDCl₃) δ 8.89 (d, *J* = 1.9 Hz, 1H), 8.14 (s, 1H), 8.04 (br s, 1H), 8.00 (br d, *J* = 7.7 Hz, 1H), 7.95 (d, *J* = 1.9 Hz, 1H), 7.62 - 7.57 (m, 2H), 7.49 (t, *J* = 7.7 Hz, 1H), 7.33 (br d, *J* = 7.7 Hz, 1H), 7.09 - 7.05 (m, 2H), 5.88 (br s, 1H), 4.56 (d, *J=* 5.7 Hz, 2H), 3.42 - 3.28 (m, 4H), 2.73 - 2.60 (m, 4H), 2.42 (s, 3H), 2.08 (s, 3H).

¹³C NMR (126 MHz, CDCl₃) δ 170.02, 151.21, 149.49, 145.14, 145.10, 144.26, 139.00, 133.31, 131.21, 129.47, 128.86, 128.29, 127.49, 126.82, 126.54, 121.62, 116.36, 116.11, 55.16, 48.79, 46.28, 44.05, 23.49.

HRMS (APCI): calcd. for C₂₇H₂₈N₄O₂ [M+H]⁺ = 441.2285; found [M+H]⁺ = 441.2288.

### Preparative Example 138: 1-(3-bromophenyl)-N-ethylmethanesulfonamide

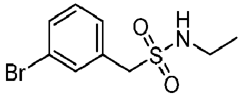

A solution of ethylamine 0.75 mL (1.50 mmol; 2.0M in THF) and 0.21 mL (1.51 mmol) of TEA were added to a solution of 404 mg (1.50 mmol) of (3-bromophenyl)methanesulfonyl chloride in DCM (5.0 mL). The resulting mixture was stirred at 25 °C for 1.5 h after which time the solvent was evaporated *in vacuo.* The residue was purified by flash chromatography (cyclohexane/EtOAc, gradient from 4:1 to 1:1). The product was obtained as a white solid (398 mg, 95 % yield).

¹H NMR (500 MHz, CDCl₃) δ 7.55 (t, *J=* 1.7 Hz, 1H), 7.53 - 7.50 (m, 1H), 7.34 (br d, *J* = 7.7 Hz, 1H), 7.26 (t, *J* = 7.8 Hz, 1H), 4.19 (s, 2H), 4.09 (br s, 1H), 3.10 - 3.02 (m, 2H), 1.16 (t, *J* = 7.2 Hz, 3H).

¹³C NMR (126 MHz, CDCl₃) δ 133.68, 132.03, 131.83, 130.46, 129.42, 122.84, 58.46, 38.98, 16.04. HRMS (APCI): calcd. for C₉H₁₂BrNO₂S [M+NH₄]⁺ = 295.0110; found [M+ NH₄]⁺ = 295.0107.

### Preparative Example 139: N-ethyl-1-(3-(4,4,5,5-tetramethyl-1,3,2-dioxaborolan-2-yl)phepyl)methanesulfonamide

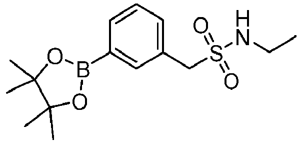

Pd(dppf)Cl₂ (17.4 mg, 0.024 mmol) was added to a degassed suspension of 1-(3-bromophenyl)-*N-*ethylmethanesulfonamide **(Prep. Example 138,** 387 mg, 1.19 mmol), KOAc (467 mg, 4.76 mmol) and (BPin)₂ (423 mg, 1.67 mmol) in 1,4-dioxane (5.1 mL) and the mixture was stirred at 70 °C; the progress of the reaction was followed by TLC. After consumption of the starting material (17 h), the mixture was cooled to 25 °C, filtered and the solvent was evaporated *in vacuo.* The residue was purified by flash chromatography (cyclohexane/EtOAc, gradient from 19:1 to 1:1). The product was obtained as colorless oil (362 mg, 94 % yield).

¹H NMR (500 MHz, CDCl₃) δ 7.87 - 7.79 (m, 2H), 7.58 - 7.51 (m, 1H), 7.45 - 7.39 (m, 1H), 4.27 (s, 2H), 4.01 (t, *J* = 5.5 Hz, 1H), 3.13 - 3.00 (m, 2H), 1.37 (s, 12H), 1.15 (t, *J=* 7.2 Hz, 3H).

¹³C NMR (126 MHz, CDCl₃) δ 137.03, 135.23, 133.50, 130.74, 129.04, 128.41, 84.18, 58.82, 38.95, 25.04, 15.88.

### Preparative Example 140: 1-(3-(6-chlorofuro[3,2-b]pyridin-3-yl)phenyl)-N-ethylmethanesulfonamide

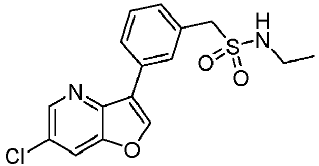

The compound was prepared by the general procedure **A** using 50 mg (0.215 mmol) of 3-bromo-6-chlorofuro[3,2-*b*]pyridine **(Prep. Example 5)** and 91 mg (0.279 mmol) of *N*-ethyl-1-(3-(4,4,5,5-tetramethyl-1,3,2-dioxaborolan-2-yl)phenyl)methanesulfonamide **(Prep. Example 137);** the reaction time was 2 h; flash chromatography (cyclohexane/EtOAc, 1:1) afforded the compound as an off-white solid (70 mg, 91 % yield).

¹H NMR (500 MHz, CDCl₃) δ 8.58 (d, *J* = 2.0 Hz, 1H), 8.15 - 8.09 (m, 2H), 8.01 - 7.96 (m, 1H), 7.82 (d, *J* = 2.0 Hz, 1H), 7.48 (t, *J* = 7.7 Hz, 1H), 7.43 - 7.35 (m, 1H), 4.31 (s, 2H), 3.18 - 3.07 (m, 2H), 1.16 (t, *J* = 7.2 Hz, 3H).

¹³C NMR (126 MHz, CDCl₃) δ 148.42, 145.74, 145.24, 144.15, 130.55, 130.30, 130.24, 129.41, 129.21, 127.80, 127.25, 121.13, 118.85, 58.54, 38.83, 15.87.

HRMS (APCI): calcd. for C₁₆H₁₅ClN₂O₃S [M+H]⁺ = 351.0565; found [M+H]⁺ = 351.0569.

### Preparative Example 141: N-ethyl-1-(3-(6-(4-(4-methylpiperazin-1-yl)phenyl)furo[3,2-b]pyridin-3-yl)phenyl)methanesulfonamide

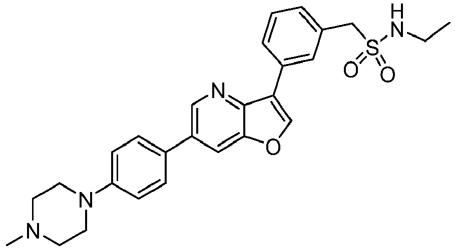

The compound was prepared by the general procedure **B** using 55 mg (0.157 mmol) of 1-(3-(6-chlorofuro[3,2-*b*]pyridin-3-yl)phenyl)-*N*-ethylmethanesulfonamide **(Prep. Example 140)** and 63 mg (0.208 mmol) of 1-methyl-4-(4-(4,4,5,5-tetramethyl-1,3,2-dioxaborolan-2-yl)phenyl)piperazine; the reaction mixture was stirred for 1 h; flash chromatography (DCM/MeOH, with gradient from 1:0 to 9:1) afforded the compound as a white solid (54 mg, 69 % yield).

¹H NMR (500 MHz, DMSO) δ 8.93 *(d, J=* 1.9 Hz, 1H), 8.80 (s, 1H), 8.31 *(d, J=* 1.9 Hz, 1H), 8.28 - 8.25 (m, 1H), 8.25 - 8.20 (m, 1H), 7.72 - 7.68 (m, 2H), 7.51 (t, *J* = 7.7 Hz, 1H), 7.40 - 7.36 (m, 1H), 7.10 - 7.05 (m, 3H), 4.38 (s, 2H), 3.25 - 3.21 (m, 4H), 3.05 - 2.96 (m, 2H), 2.50 - 2.45 (m, 4H), 2.24 (s, 3H), 1.07 (t, *J* = 7.2 Hz, 3H).

¹³C NMR (126 MHz, DMSO) δ 150.74, 148.86, 146.64, 144.24, 143.23, 132.33, 130.93, 130.49, 130.00, 128.82, 128.61, 127.74, 126.86, 126.10, 119.81, 115.59, 115.52, 57.27, 54.44, 47.62, 45.70, 37.59, 15.49.

HRMS (APCI): calcd. for C₂₇H₃₀N₄O₃S [M+H]⁺ = 491.2111; found [M+H]⁺ = 491.2110.

### Preparative Example 142: 1-(3-bromophenyl)-N,N-dimethylmethanesulfonamide

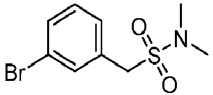

Dimethylamine solution (2.0 M in THF; 0.5 mL, 1.00 mmol) and TEA (0.14 mL, 1.00 mmol) were added to a solution of (3-bromophenyl)methanesulfonyl chloride (270 mg, 1.00 mmol) in DCM (5.0 mL). The resulting mixture was stirred at 25 °C for 2 h and the solvent was then evaporated *in vauco.* The residue was purified by flash chromatography (cyclohexane/EtOAc 1:1). The product was obtained as a white solid (261 mg, 94 % yield).

¹H NMR (500 MHz, CDCl₃) δ 7.54 (t, *J=* 1.7 Hz, 1H), 7.51 - 7.47 (m, 1H), 7.34 (br d, *J=* 7.8 Hz, 1H), 7.24 (t, *J* = 7.8 Hz, 1H), 4.15 (s, 2H), 2.76 (s, 6H).

¹³C NMR (126 MHz, CDCl₃) δ 133.60, 131.88, 131.41, 130.37, 129.40, 122.72, 55.18, 37.88.

### Preparative Example 143: N,N-dimethyl-1-(3-(4.4.5.5-tetramethyl-1,3,2-dioxaborolan-2-yl)phenyl)methanesulfonamide

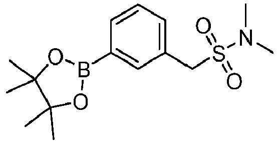

Pd(dppf)Cl₂ (8.9 mg, 0.012 mmol) was added to a degassed suspension of 1-(3-bromophenyl)-*N,N-*dimethylmethanesulfonamide **(Prep. Example 142,** 170 mg, 0.611 mmol), KOAc (239 mg, 2.440 mmol) and (BPin)₂ (217 mg, 0.855 mmol) in 1,4-dioxane (2.6 mL) and the mixture was stirred at 70 °C; the progress of the reaction was followed by TLC. After consumption of the starting material (17 h), the mixture was cooled to 25 °C, filtered and the solvent was evaporated *in vacuo.* The residue was purified by flash chromatography (cyclohexane/EtOAc, 7:3). The product was obtained as colorless oil (115 mg, 58 % yield).

¹H NMR (500 MHz, CDCl₃) δ 7.82 - 7.76 (m, 2H), 7.56 - 7.51 (m, 1H), 7.41 - 7.36 (m, 1H), 4.23 (s, 2H), 2.73 (s, 6H), 1.35 (s, 12H).

¹³C NMR (126 MHz, CDCl₃) δ 137.02, 135.17, 133.51, 129.83 (d, *J* = 233.4), 128.57, 128.31, 84.14, 55.97, 37.93, 25.02.

### Preparative Example 144: 1-(3-(6-chlorofuro[3,2-b]pyridin-3-yl)phenyl)-N,N-dimethylmethanesulfonamide

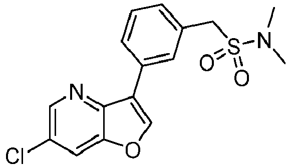

The compound was prepared by the general procedure **A** using 50 mg (0.215 mmol) of 3-bromo-6-chlorofuro[3,2-*b*]pyridine **(Prep. Example 5)** and 91 mg (0.280 mmol) of *N,N-*dimethyl-1-(3-(4,4,5,5-tetramethyl-1,3,2-dioxaborolan-2-yl)phenyl)methanesulfonamide **(Prep. Example 143);** the reaction time was 2 h; flash chromatography (cyclohexane/EtOAc 1:1) afforded the compound as an off-white solid (68 mg, 88 % yield).

¹H NMR (500 MHz, CDCl₃) δ 8.60 (d, *J* = 2.0 Hz, 1H), 8.16 - 8,13 (m, 2H), 8.00 (d, *J* = 7.7 Hz, 1H), 7.83 (d, *J=* 2.0 Hz, 1H), 7.49 (t, *J* = 7.7 Hz, 1H), 7.44 - 7.40 (m, 1H), 4.32 (s, 2H), 2.79 (s, 6H).

¹³C NMR (126 MHz, CDCl₃) δ 148.50, 145.91, 145.32, 144.23, 130.59, 130.32, 129.92, 129.43, 127.84, 127.24, 121.14, 118.92, 56.11, 37.90.

HRMS (APCI): calcd. for C₁₆H₁₅ClN₂O₃S [M+H]⁺ = 351.0565; found [M+H]⁺ = 351.0562.

### Preparative Example 145: N,N-dimethyl-1-(3-(6-(4-(4-methylpiperazin-1-yl)phenyl)furo[3,2-b]pyridin-3-yl)phenyl)methane-sulfonamide

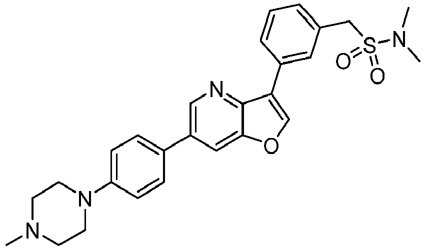

The compound was prepared by the general procedure **B** using 40 mg (0.114 mmol) of 1-(3-(6-chlorofuro[3,2-*b*]pyridin-3-yl)phenyl)-*N,N*-dimethylmethanesulfonamide **(Prep. Example 144)** and 43 mg (0.142 mmol) of 1-methyl-4-(4-(4,4,5,5-tetramethyl-1,3,2-dioxaborolan-2-yl)phenyl)piperazine; the reaction time was 2 h; flash chromatography (MeOH) afforded the compound as a white solid (50 mg, 93 % yield).

¹H NMR (500 MHz, CDCl₃) δ 8.87 (d, *J=* 1.9 Hz, 1H), 8.24 - 8.19 (m, 1H), 8.15 (s, 1H), 8.09 - 8.04 (m, 1H), 7.93 (d, *J* = 1.9 Hz, 1H), 7.60 - 7.55 (m, 2H), 7.50 (t, *J* = 7.7 Hz, 1H), 7.45 - 7.40 (m, 1H), 7.07 - 7.02 (m, 2H), 4.35 (s, 2H), 3.37 - 3.29 (m, 4H), 2.80 (s, 6H), 2.68 - 2.61 (m, 4H), 2.40 (s, 3H). ¹³C NMR (126 MHz, CDCl₃) δ 151.12, 149.50, 145.17, 145.15, 144.17, 136.30, 133.36, 131.38, 130.07, 129.91, 129.50, 129.45, 128.92, 128.30, 127.32, 121.15, 116.42, 116.12, 56.42, 55.08, 48.68, 46.16, 37.98.

HRMS (APCI): calcd. for C₂₇H₃₀N₄O₃S [M+H]⁺ = 491.2111; found [M+H]⁺ = 491.2114.

### Preparative Example 146: 6-chloro-3-(3-(methylthio)phenyl)furo[3,2-b]pyridine

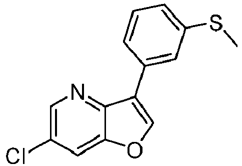

The compound was prepared by the general procedure **A** using 300 mg (1.29 mmol) of 3-bromo-6-chlorofuro[3,2-*b*]pyridine **(Prep. Example 5)** and 282 mg (1.68 mmol) of (3-(methylthio)phenyl)boronic acid; the reaction time was 2 h; flash chromatography (cyclohexane/EtOAc, gradient from 99:1 to 97:3) afforded the compound as an off-white oil (222 mg, 79 % yield).

¹H NMR (500 MHz, CDCl₃) δ 8.62 (d, *J=* 2.0 Hz, 1H), 8.11 (s, 1H), 7.96 (t, *J=* 1.7 Hz, 1H), 7.83 (d, *J* = 2.0 Hz, 1H), 7.82 - 7.78 (m, 1H), 7.40 (t, *J=* 7.8 Hz, 1H), 7.29 - 7.26 (m, 1H), 2.55 (s, 3H).

¹³C NMR (126 MHz, CDCl₃) δ 148.53, 145.82, 145.39, 144.37, 139.43, 130.63, 129.42, 127.82, 126.36, 125.43, 124.01, 121.60, 118.89, 16.10.

HRMS (APCI): calcd. for C₁₄H₁₀ClNOS [M+H]⁺ = 276.0244; found [M+H]⁺ = 276.0244.

### Preparative Example 147: 6-(4-(4-methylpiperazin-1-yl)phenyl)-3-(3-(methylthio)phenyl)furo[3,2-b]pyridine

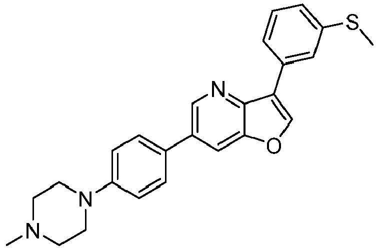

The compound was prepared by the general procedure **B** using 172 mg (0.624 mmol) of 6-chloro-3-(3-(methylthio)phenyl)furo[3,2-*b*]pyridine **(Prep. Example 146)** and 244 mg (0.807 mmol) of 1-methyl-4-(4-(4,4,5,5-tetramethyl-1,3,2-dioxaborolan-2-yl)phenyl)piperazine; the reaction time was 1 h; flash chromatography (DCM/MeOH, gradient from 1:0 to 4:1) afforded the compound as a white solid (213 mg, 83 % yield).

¹H NMR (500 MHz, CDCl₃) δ 8.88 *(d, J=* 1.9 Hz, 1H), 8.11 (s, 1H), 8.01 (t, *J* = 1.7 Hz, 1H), 7.92 (br d, *J=* 1.9 Hz, 1H), 7.90 - 7.87 (m, 1H), 7.60 - 7.55 (m, 2H), 7.41 (t, *J=* 7.8 Hz, 1H), 7.29 - 7.26 (m, 1H), 7.07 - 7.03 (m, 2H), 3.34 - 3.28 (m, 4H), 2.64 - 2.58 (s, 4H), 2.57 (s, 3H), 2.38 (s, 3H). ¹³C NMR (126 MHz, CDCl₃) δ 151.20, 149.45, 145.21, 145.05, 144.27, 139.23, 133.26, 131.39, 129.39, 128.89, 128.28, 126.14, 125.47, 124.13, 121.54, 116.33, 116.01, 55.19, 48.83, 46.32, 16.18.

HRMS (APCI): calcd. for C₂₅H₂₅N₃OS [M+H]⁺ = 416.1791; found [M+H]⁺ = 416.1792.

### Preparative Example 148: 6-chloro-3-(thiophen-3-yl)furo[3,2-b]pyridine

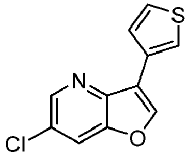

The compound was prepared by the general procedure **A** using 100 mg (0.430 mmol) of 3-bromo-6-chlorofuro[3,2-*b*]pyridine **(Prep. Example 5)** and 72 mg (0.559 mmol) of thiophen-3-ylboronic acid; the reaction time was 2 h; flash chromatography (cyclohexane/EtOAc, gradient from 1:0 to 49:1) afforded the compound as a white solid (87 mg, 86 % yield).

¹H NMR (500 MHz, CDCl₃) δ 8.60 (d, *J* = 2.0 Hz, 1H), 8.24 (dd, *J* = 2.9, 1.1 Hz, 1H), 8.06 (s, 1H), 7.79 (d, *J* = 2.0 Hz, 1H), 7.51 (dd, *J* = 5.0, 1.1 Hz, 1H), 7.43 (dd, *J*= 5.0, 3.0 Hz, 1H).

¹³C NMR (126 MHz, CDCl₃) δ 148.20, 145.31, 145.06, 144.37, 129.74, 127.80, 126.21, 125.76, 123.08, 118.70, 117.98.

HRMS (APCI): calcd. for C₁₁H₆ClNOS [M+H]⁺ = 235.9931; found [M+H]⁺ = 235.9934.

### Preparative Example 149: 6-phenyl-3-(thiophen-3-yl)furo[3,2-b]pyridine

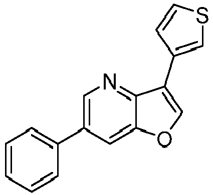

The compound was prepared by the general procedure **B** using 54 mg (0.232 mmol) of 6-chloro-3-(thiophen-3-yl)furo[3,2-*b*]pyridine **(Prep. Example 148)** and 61 mg (0.299 mmol) of 4,4,5,5-tetramethyl-2-phenyl-1,3,2-dioxaborolane; the reaction time was 2 h; flash chromatography (cyclohexane/EtOAc, gradient from 1:0 to 49:1) afforded the compound as a white solid (50 mg, 78 % yield).

¹H NMR (500 MHz, CDCl₃) δ 8.91 (d, *J* = 1.9 Hz, 1H), 8.31 (dd, *J* = 3.0, 1.2 Hz, 1H), 8.12 (s, 1H), 7.96 (d, *J=* 1.9 Hz, 1H), 7.68 - 7.63 (m, 2H), 7.58 (dd, *J=* 5.0, 1.2 Hz, 1H), 7.54 - 7.49 (m, 2H), 7.46 - 7.40 (m, 2H).

¹³C NMR (126 MHz, CDCl₃) δ 149.02, 145.55, 145.11, 144.84, 138.37, 133.51, 130.37, 129.33, 128.12, 127.71, 126.16, 125.95, 122.87, 118.03, 116.83.

HRMS (APCI): calcd. for C₁₇H₁₁NOS [M+H]⁺ = 278.0634; found [M+H]⁺ = 278.0634.

### Preparative Example 150: 2-(3-(6-chlorofuro[3,2-b]pyridin-3-yl)phenyl)-N-ethylacetamide

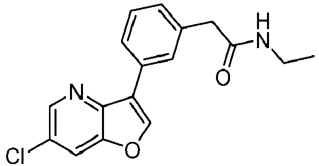

The compound was prepared by the general procedure **A** using 120 mg (0.516 mmol) of 3-bromo-6-chlorofuro[3,2-*b*]pyridine **(Prep. Example 5)** and 195 mg (0.671 mmol) of *N*-ethyl-2-(3-(4,4,5,5-tetramethyl-1,3,2-dioxaborolan-2-yl)phenyl)acetamide; the reaction time was 4 hours. The reaction mixture was filtered through a pad of Celite^{®} 535 and SiO₂ (1:1, 8 g) and the filtrate was evaporated *in vacuo.* The residue was purified by flash chromatography (cyclohexane/EtOAc, gradient from 9:1 to 1:1). The product was obtained as an off-white solid (133 mg, 82 %).

¹H NMR (500 MHz, Chloroform-*d*) δ 8.64 (d, *J* = 2.0 Hz, 1H), 8.15 (s, 1H), 7.93 - 8.01 (m, 2H), 7.87 (d, *J* = 2.0 Hz, 1H), 7.50 (dd, *J* = 7.6, 7.6 Hz, 1H), 7.31 (d, *J* = 7.6 Hz, 1H), 5.47 (s, 1H), 3.68 (s, 2H), 3.25 - 3.34 (m, 2H), 1.11 (t, *J* = 7.2 Hz, 3H)

¹³C NMR (126 MHz, Chloroform-d) δ 148.43, 145.74, 145.19, 144.17, 135.75, 130.57, 129.60, 129.10, 128.22, 127.75, 126.10, 121.46, 118.88, 43.99, 34.62, 14.78.

### Preparative Example 151: tert-butyl 4-(4-(3-(3-(2-(ethylamino)-2-oxoethyl)phenyl)furo[3,2-b]pyridin-6-yl)phenyl)piperazine-1-carboxylate

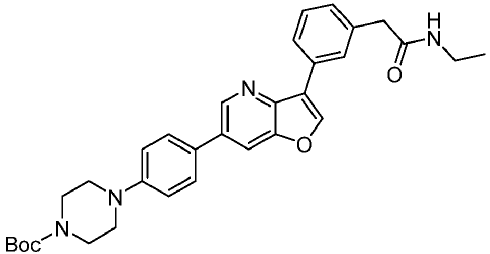

The compound was prepared by the general procedure **B** using 133 mg (0.423 mmol) of 2-(3-(6-chlorofuro[3,2-*b*]pyridin-3-yl)phenyl)-*N*-ethylacetamide **(Prep. Example 150)** and 214 mg (0.550 mmol) of *tert-butyl* 4-(4-(4,4,5,5-tetramethyl-1,3,2-dioxaborolan-2-yl)phenyl)piperazine-1-carboxylate; the reaction time was 2 h. The reaction mixture was filtered through a pad of Celite^{®} 535 and SiO₂ (1:1, 8 g) and the filtrate was evaporated *in vacuo.* The residue was purified by flash chromatography (cyclohexane/EtOAc, gradient from 7:3 to 1:9) and the product was obtained as an off-white solid (200 mg, 87 %).

¹H NMR (500 MHz, Chloroform-d) δ 8.90 *(d, J* = 1.9 Hz, 1H), 8.15 (s, 1H), 8.05 (ddd, *J* = 1.5, 1.5, 7.6 Hz, 1H), 8.01 (dd, *J* = 1.8, 1.8 Hz, 1H), 7.96 (d, *J* = 1.9 Hz, 1H), 7.58-7.62 (m, 2H), 7.51 (dd, J = 7.7, 7.7 Hz, 1H), 7.30 (ddd, *J* = 1.4, 1.4, 7.6 Hz, 1H), 7.05-7.09 (m, 2H), 5.53 (s, 1H), 3.69 (s, 2H), 3.61 - 3.66 (m, 4H), 3.28 - 3.33 (m, 2H), 3.22 - 3.28 (m, 4H), 1.52 (s, 9H), 1.10 (t, *J* = 7.2 Hz, 3H).

¹³C NMR (126 MHz, CDCl₃) δ 170.68, 170.60, 154.77, 151.01, 149.34, 145.07, 144.89, 144.06, 135.69, 133.08, 131.23, 129.58, 129.23, 128.82, 128.31, 126.12, 121.35, 116.73, 116.12, 80.02, 48.94, 44.00, 34.59, 34.45, 28.45, 14.76.

### Preparative Example 152: N-ethyl-2-(3-(6-(4-(piperazin-1-yl)phenyl)furo[3,2-b]pyridin-3-yl)phenyl)acetamide

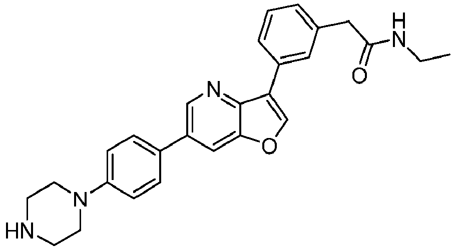

To the heterogeneous mixture of 200 mg (0.370 mmol) of *tert-butyl* 4-(4-(3-(3-(2-(ethylamino)-2-oxoethyl)phenyl)furo[3,2-*b*]pyridin-6-yl)phenyl)piperazine-1-carboxylate **(Prep. Example 151)** in 15.0 mL of MeOH, 6.5 mL of 4M HCl in 1,4-dioxane was added at 0 °C (ice bath) under argon. The mixture was stirred at 0 °C for 90 minutes. Ice bath was removed and the mixture was stirred for additional 4.5 h at 25 °C. The reaction mixture was concentrated *in vacuo,* 500 mg (5.952 mmol) of solid sodium bicarbonate was added to the residue and the mixture was stirred in DCM/MeOH 5:1 (12 mL) for 2 h. The mixture was filtered through a pad of Celite^{®} 535 and SiO₂ (1:1; 8 g) and the pad was washed with 50 mL of the mixture DCM/MeOH 5:1. The filtrate was concentrated *in vacuo* and the residue was purified by flash chromatography (DCM/MeOH, gradient from 1:0 to 4:1) and the product was obtained as an off-white solid (116 mg, 71 %).

¹H NMR (500 MHz, DMSO-*d₆*) δ 8.95 (d, *J* = 2.0 Hz, 1 H), 8.80 (s, 1H), 8.33 (d, *J* = 1.9 Hz, 1H), 8.04 - 8.15 (m, 3H), 7.73-7.77 (m, 2H), 7.43 (dd, *J* = 7.7, 7.7 Hz, 1H), 7.28 (ddd, *J* = 1.4, 1.4, 7.6 Hz, 1H), 7.11 - 7.15 (m, 2H), 3.48 (s, 2H), 3.37 - 3.43 (m, 4H), 3.14 - 3.20 (m, 4H), 3.06 - 3.13 (m, 2H), 1.05 (t, *J* = 7.2 Hz, 3H).

¹³C NMR (126 MHz, Methanol-*d*₄) δ 172.33, 150.38, 149.54, 146.17, 144.06, 143.91, 136.26, 133.05, 130.56, 129.73, 128.61, 128.08, 127.87, 127.71, 125.49, 121.25, 116.78, 116.06, 46.73, 43.74, 42.55, 34.08, 13.36.

HRMS (APCI): calcd. For C₂₇H₂₈N₄O₂ [M+H]⁺ = 441.2285; found [M+H]⁺ = 441.2286

### Preparative Example 153: 2-(3-(6-chlorofuro[3,2-b]pyridin-3-yl)phenyl)acetamide

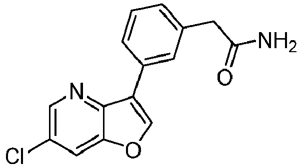

The compound was prepared by the general procedure **A** using 300 mg (0.950 mmol) of 3-bromo-6-chlorofuro[3,2-*b*]pyridine **(Prep. Example 5)** and 323 mg (1.235 mmol) of 2-(3-(4,4,5,5-tetramethyl-1,3,2-dioxaborolan-2-yl)phenyl)acetamide; the reaction time was 6.5 h. The reaction mixture was filtered through a pad of Celite^{®} 535 and SiO₂ (1:1, 8 g) and the filtrate was concentrated *in vacuo.* Flash chromatography (EtOAc) provided the product as an off-white solid (220 mg, 80 %).

¹H NMR (500 MHz, CDCl₃) δ 8.63 (d, *J* = 2.1 Hz, 1H), 8.15 (s, 1H), 7.98 (dd, *J* = 1.7, 8.8 Hz, 2H), 7.85 (d, *J* = 2.0 Hz, 1H), 7.49 (dd, *J* = 7.6, 7.6 Hz, 1H), 7.32 (ddd, *J* = 1.4, 1.4, 7.6 Hz, 1H), 5.57 (bs, 2H), 3.70 (s, 2H).

¹³C NMR (126 MHz, CDCl₃) δ 173.15, 148.41, 145.73, 145.22, 144.14, 135.57, 130.67, 129.62, 128.99, 128.09, 127.73, 126.17, 121.36, 118.84, 43.38.

### Preparative Example 154: 2-(3-(6-(4-(4-methylpiperazin-1-yl)phenyl)furo[3,2-b]pyridin-3-yl)phenyl)acetamide

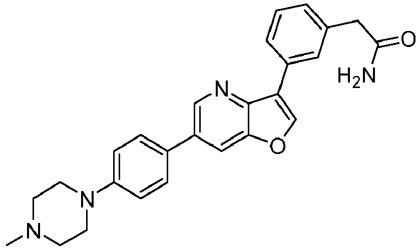

The compound was prepared by the general procedure **B** using 80 mg (0.279 mmol) of 2-(3-(6-chlorofuro[3,2-*b*]pyridin-3-yl)phenyl)acetamide **(Prep. Example 153)** and 110 mg (0.363 mmol) of 1-methyl-4-(4-(4,4,5,5-tetramethyl-1,3,2-dioxaborolan-2-yl)phenyl)piperazine; the reaction time was 3 h. The reaction mixture was hot-filtered through a pad of Celite^{®} 535 and SiO₂ (1:1, 8 g) and the filtrate was concentrated *in vacuo.* Flash chromatography (EtOAc/MeOH, gradient from 1:0 to 7:3) afforded the product as an off-white solid (23 mg, 17 % yield).

¹H NMR (500 MHz, DMSO-*d₆*) δ 8.94 (d, *J* = 1.9 Hz, 1H), 8.79 (s, 1H), 8.31 (d, *J* = 1.9 Hz, 1H), 8.08 - 8.15 (m, 2H), 7.71 (d, *J* = 8.5 Hz, 2H), 7.48 (s, 1H), 7.43 (dd, *J* = 7.6, 7.6 Hz, 1H), 7.29 (d, *J* = 7.6 Hz, 1H), 7.09 (d, *J* = 8.5 Hz, 2H), 6.89 (s, 1H), 3.48 (s, 2H), 3.23 - 3.29 (m, 4H), 2.53 - 2.63 (m, 4H), 2.31 (*s,* 3H).

¹³C NMR (126 MHz, DMSO-*d₆*) δ 172.56, 151.03, 149.34, 147.04, 144.74, 143.90, 137.38, 132.71, 130.77, 128.97, 128.90, 128.27, 127.90, 127.62, 125.15, 120.65, 116.14, 116.08, 54.03, 47.82, 45.73, 42.79.

HRMS (APCI): calcd. For C₂₆H₂₆N₄O₂ [M+H]⁺ = 427.2129; found [M+H]⁺ = 427.2134

### Preparative Example 155: 1-(4-(6-chlorofuro[3,2-b]pyridin-3-yl)pyridin-2-yl)ethan-1-one

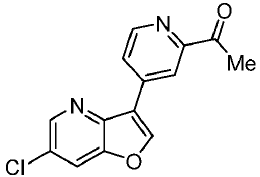

The compound was prepared by the general procedure **A** using 300 mg (1.291 mmol) of 3-bromo-6-chlorofuro[3,2-*b*]pyridine **(Prep. Example 5)** and 415 mg (1.678 mmol) of 1-(4-(4,4,5,5-tetramethyl-1,3,2-dioxaborolan-2-yl)pyridin-2-yl)ethan-1-one; the reaction time was 5 h. The reaction mixture was hot-filtered through a pad of Celite^{®} 535 and SiO₂ (1:1, 8 g) and the filtrate was concentrated *in vacuo.* Flash chromatography (DCM/MeOH, gradient from 99:1 to 49:1) afforded the product as an off-white solid (27 mg, 8 % yield).

¹H NMR (500 MHz, CDCl₃) δ 8.83 (d, *J* = 5.0 Hz, 1H), 8.69 (d, *J* = 2.1 Hz, 1H), 8.57 (d, *J*= 1.7 Hz, 1H), 8.47 (dd, *J* = 1.7, 5.1 Hz, 1H), 8.40 (s, 1H), 7.91 (d, *J* = 2.1 Hz, 1H), 2.81 (s, 3H).

¹³C NMR (126 MHz, CDCl₃) δ 199.99, 153.95, 149.74, 148.58, 147.59, 145.87, 143.45, 139.00, 128.38, 124.71, 119.14, 119.11, 118.74, 25.93.

### Preparative Example 156: 1-(4-(6-(4-(4-methylpiperazin-1-yl)phenyl)furo[3,2-b]pyridin-3-yl)pyridin-2-yl)ethan-1-one

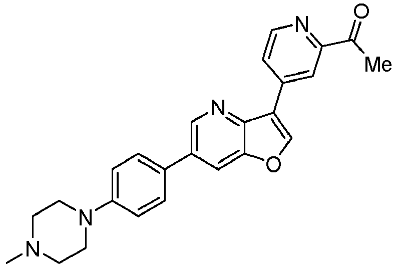

The compound was prepared by the general procedure **B** using 25 mg (0.092 mmol) of 1-(4-(6-chlorofuro[3,2-*b*]pyridin-3-yl)pyridin-2-yl)ethan-1-one **(Prep. Example 155)** and 36 mg (0.119 mmol) of 1-methyl-4-(4-(4,4,5,5-tetramethyl-1,3,2-dioxaborolan-2-yl)phenyl)piperazine; the reaction time was 5 h. The reaction mixture was hot-filtered through a pad of Celite^{®} 535 and SiO₂ (1:1, 8 g) and the filtrate was concentrated *in vacuo.* Flash chromatography (DCM/MeOH, gradient from 1:0 to 19:1) afforded the product as an off-white solid (14 mg, 36 % yield).

¹H NMR (500 MHz, DMSO-*d₆*) δ 9.22 (s, 1H), 9.02 (d, *J*=1.9 Hz, 1H), 8.89 (dd, *J*=0.8, 1.8 Hz, 1H), 8.83 (dd, *J*=0.8, 5.1 Hz, 1H), 8.49 (dd, *J*=1.7, 5.1 Hz, 1H), 8.39 (d, *J*=1.9 Hz, 1H), 7.69 - 7.74 (m, 2H), 7.05-7.11 (m, 2H), 3.21 - 3.26 (m, 4H), 2.70 (s, 3H), 2.46 - 2.50 (m, 4H), 2.24 (s, 3H).

¹³C NMR (126 MHz, DMSO-*d₆*) δ 200.04, 154.24, 151.36, 150.22, 150.15, 149.61, 145.31, 143.10, 139.92, 133.35, 128.31, 127.08, 124.48.118.50, 117.90, 116.42, 116.04, 54.97, 48.12, 46.26, 26.28. HRMS (APCI): calcd. For C₂₅H₂₄N₄O₂ [M+H]⁺ = 413.1972; found [M+H]⁺ = 413.1973

### Preparative Example 157: 3-([1,1'-biphenyl]-3-yl)-6-chlorofuro[3,2-b]pyridine

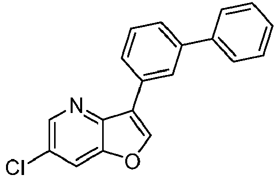

The compound was prepared by the general procedure **A** using 300 mg (1.290 mmol) of 3-bromo-6-chlorofuro[3,2-*b*]pyridine **(Prep. Example 5)** and 332 mg (1.678 mmol) of 3-phenylphenylboronic acid; the reaction time was 3.5 h. The reaction mixture was filtered through a pad of Celite^{®} 535 and SiO₂ (1:1, 8 g) and the filtrate was concentrated *in vacuo.* Flash chromatography (cyclohexane/EtOAc, gradient from 1:0 to 93:7) provided the product as an off-white solid (400 mg, 100 % yield).

¹H NMR (500 MHz, CDCl₃) δ 8.56 (d, *J* = 2.1 Hz, 1H), 8.17 (dd, *J* = 1.8, 1.8 Hz, 1H), 8.09 (s, 1H), 7.95 (ddd, *J* = 1.5, 1.5, 7.5 Hz, 1H), 7.77 (d, *J* = 2.1 Hz, 1H), 7.57 - 7.62 (m, 2H), 7.53 (ddd, *J* = 1.5, 1.5, 7.8 Hz, 1H), 7.48 (dd, *J* = 7.6, 7.6 Hz, 1H), 7.37 - 7.43 (m, 2H), 7.28 - 7.33 (m, 1H).

¹³C NMR (126 MHz, CDCl₃) δ 147.40,144.59, 144.26, 143.39, 140.93, 140.02, 129.30, 128.32, 127.83, 126.62, 126.46, 126.29, 125.89, 125.06, 124.98, 120.88, 117.70.

### Preparative Example 158: 3-([1,1'-biphenyl]-3-yl)-6-(4-(4-methylpiperazin-1-yl)phenyl)furo[3,2-b]pyridine

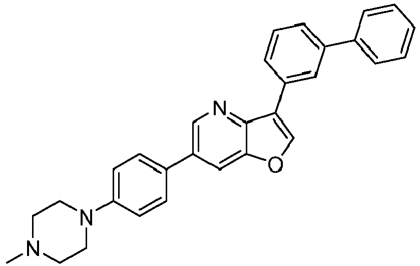

The compound was prepared by the general procedure **B** using 135 mg (0.442 mmol) of 3-([1,1'-biphenyl]-3-yl)-6-chlorofuro[3,2-*b*]pyridine **(Prep. Example 157)** and 174 mg (0.575 mmol) of 1-methyl-4-(4-(4,4,5,5-tetramethyl-1,3,2-dioxaborolan-2-yl)phenyl)piperazine; the reaction time was 2 h. The reaction mixture was hot-filtered through a pad of Celite^{®} 535 and SiO₂ (1:1, 8 g) and the filtrate was concentrated *in vacuo.* Flash chromatography (DCM/MeOH, gradient from 99:1 to 19:1) afforded the product as an off-white solid (55 mg, 28 % yield).

¹H NMR (500 MHz, CDCl₃) δ 8.92 (d, *J* = 1.9 Hz, 1H), 8.31 (dd, *J* = 1.8, 1.8 Hz, 1H), 8.19 (s, 1H), 8.13 (ddd, *J* = 1.6, 1.6, 7.3 Hz, 1H), 7.95 (d, *J* = 1.8 Hz, 1H), 7.69 - 7.75 (m, 2H), 7.57 - 7.65 (m, 4H), 7.47-7.53 (m, 2H), 7.40 (ddd, *J* = 1.3, 7.1, 7.4 Hz, 1H), 7.04 - 7.11 (m, 2H), 3.30 - 3.41 (m, 4H), 2.61 - 2.74 (m, 4H), 2.43 (s, 3H).

¹³C NMR (126 MHz, DMSO-*d₆*) δ 151.17, 149.414, 147.50, 144.85, 143.91, 141.22, 140.67, 132.79, 131.61, 129.80, 129.44, 128.25, 128.09, 127.46, 127.34, 126.45, 126.13, 125.37, 120.49, 116.11, 116.07, 54.86, 48.03, 46.07.

HRMS (APCI): calcd. For C₃₀H₂₇N₃O [M+H]⁺ = 446.2227; found [M+H]⁺ = 446.2230

### Preparative Example 159: 3-([1,1'-biphenyl]-2-yl)-6-chlorofuro[3,2-b]pyridine

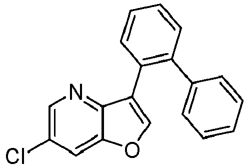

The compound was prepared by the general procedure **A** using 150 mg (0.645 mmol) of 3-bromo-6-chlorofuro[3,2-*b*]pyridine **(Prep. Example 5)** and 166 mg (0.839 mmol) of 2-phenylphenylboronic acid; the mixture was stirred for 3.5 h. The reaction mixture was filtered through a pad of Celite^{®} 535 and SiO₂ (1:1, 8 g) and the filtrate was concentrated *in vacuo.* Flash chromatography (cyclohexane/EtOAc, gradient from 1:0 to 93:7) provided the product as a white solid (190 mg, 97 % yield).

¹H NMR (500 MHz, CDCl₃) δ 8.39 (d, *J* = 2.0 Hz, 1H), 7.89 (ddd, *J* = 7.6, 1.0, 1.0 Hz, 1H), 7.53 (d, *J* = 2.1 Hz, 1H), 7.37 - 7.30 (m, 1H), 7.29 - 7.26 (m, 2H), 7.13 - 7.10 (m, 5H), 6.98 (s, 1H).

¹³C NMR (126 MHz, CDCl₃) δ 147.83, 147.43, 145.16, 145.11, 141.74, 141.65, 130.60, 129.38, 128.27, 128.11, 127.74, 127.68, 127.46, 127.32, 127.14, 120.88, 118.51.

### Preparative Example 160: tert-butyl 4-(4-(3-([1,1'-biphenyl]-2-yl)furo[3,2-b]pyridin-6-yl)phenyl)piperazine-1-carboxylate

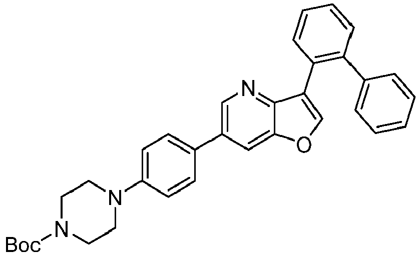

The compound was prepared by the general procedure **B** using 197 mg (0.644 mmol) of 3-([1,1'-biphenyl]-2-yl)-6-chlorofuro[3,2-*b*]pyridine **(Prep. Example 159)** and 325 mg (0.837 mmol) of *tert-*butyl 4-(4-(4,4,5,5-tetramethyl-1,3,2-dioxaborolan-2-yl)phenyl)piperazine-1-carboxylate. The reaction time was 2.5 h. The reaction mixture was filtered through a pad of Celite^{®} 535 and SiO₂ (1:1, 8 g) and the filtrate was concentrated. Column chromatography (cyclohexane/EtOAc, gradient from 9:1 to 4:1) provided the product as a pale yellow solid (240 mg, 70 % yield).

NMR (500 MHz, CDCl₃) δ 8.85 (d, *J* = 1.9 Hz, 1H), 8.14 (ddd, *J* = 1.0, 1.0, 7.8 Hz, 1H), 7.87 (d, *J* = 1.9 Hz, 1H), 7.56 - 7.61 (m, 2H), 7.54 (ddd, *J* = 3.5, 5.4, 7.7 Hz, 1H), 7.44 - 7.48 (m, 2H), 7.29 - 7.34 (m, 5H), 7.14 (s, 1H), 7.08 (d, *J* = 8.3 Hz, 2H), 3.60 - 3.70 (m, 4H), 3.20 - 3.30 (m, 4H), 1.52 (s, 9H). ¹³C NMR (126 MHz, CDCl₃) δ 154.71, 148.31, 147.30, 144.83, 141.87, 141.52, 132.67, 130.58, 130.53, 129.44, 128.20, 127.80, 127.74, 127.00, 120.55, 116.83, 80.03, 60.36, 49.09, 28.44.

### Preparative Example 161: 3-([1,1'-biphenyl]-2-yl)-6-(4-(piperazin-1-yl)phenyl)furo[3,2-b]pyridine

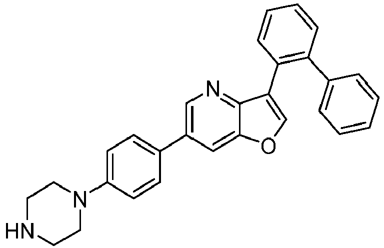

The compound was prepared by the general procedure C using 190 mg (0.357 mmol) of *tert*-butyl 4-(4-(3-([1,1'-biphenyl]-2-yl)furo[3,2-*b*]pyridin-6-yl)phenyl)piperazine-1-carboxylate **(Prep. Example 160);** the reaction time was 2 h; flash chromatography (DCM/MeOH, gradient from 1:0 to 1:1) afforded the compound as a pale yellow solid (134 mg, 87 % yield).

¹H NMR (500 MHz, CD₃OD) δ 8.65 (d, *J* = 1.9 Hz, 1H), 8.07 (d, *J* = 1.9 Hz, 1H), 7.79 - 7.84 (m, 1H), 7.59 - 7.64 (m, 2H), 7.46 - 7.54 (m, 4H), 7.20 - 7.29 (m, 5H), 7.10 - 7.15 (m, 2H), 3.29 - 3.32 (m, 4H), 3.10 - 3.14 (m, 4H).

¹³C NMR (126 MHz, CD₃OD) δ 151.24, 148.64, 147.48, 144.50, 143.68, 142.27, 141.58, 133.19, 130.79, 130.02, 128.97, 128.77, 127.97, 127.92, 127.64, 127.12, 126.62, 121.24, 116.38, 116.00, 48.36, 44.63.

HRMS (APCI): calcd. For C₂₉H₂₅N₃O [M+H]⁺ = 432.2070; found [M+H]⁺ = 432.2071

### Preparative Example 162: 6-chloro-3-(3-(cyclobutylthio)phenyl)furo[3,2-b]pyridine

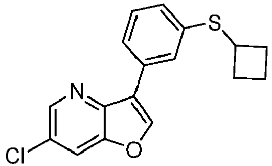

The compound was prepared by the general procedure **A** using 150 mg (0.645 mmol) of 3-bromo-6-chlorofuro[3,2-*b*]pyridine **(Prep. Example 5)** and 175 mg (0.839 mmol) of (3-(cyclobutylthio)phenyl)boronic acid; the reaction time was 2.5 h. The reaction mixture was hot-filtered through a pad of Celite^{®} 535 and SiO₂ (1:1, 8 g) and the filtrate was concentrated *in vacuo.* Flash chromatography (cyclohexane/EtOAc, gradient from 1:0 to 19:1) provided the product as yellowish viscous oil (202 mg, 99 % yield).

¹H NMR (500 MHz, CDCl₃) δ 8.54 (d, *J=* 2.0 Hz, 1H), 8.03 (s, 1H), 7.88 (dd, *J* = 1.7, 1.7 Hz, 1H), 7.72 - 7.79 (m, 2H), 7.23 - 7.33 (m, 1H), 7.12 - 7.20 (m, 1H), 3.82 - 3.95 (m, 1H), 2.37 - 2.51 (m, 2H), 1.87 - 2.13 (m, 4H).

¹³C NMR (126 MHz, CDCl₃) δ 148.37, 145.59, 145.24, 144.25, 137.91, 130.46, 129.21, 128.30, 127.64, 127.30, 124.31, 121.41, 118.69, 40.26, 30.69, 18.81.

HRMS (APCI): calcd. For C₁₇H₁₄ClNOS [M+H]⁺ = 316.0557; found [M+H]⁺ = 316.0557

### Preparative Example 163: tert-butyl 4-(4-(3-(3-(cyclobutylthio)phenyl)furo[3,2-b]pyridin-6-yl)phenyl)piperazine-1-carboxylate

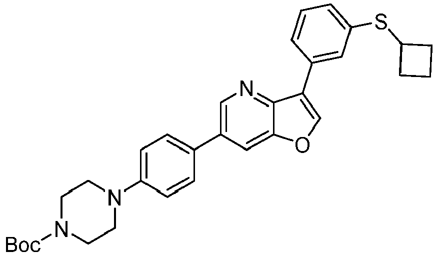

The compound was prepared by the general procedure **B** using 204 mg (0.646 mmol) of 6-chloro-3-(3-(cyclobutylthio)phenyl)furo[3,2-*b*]pyridine **(Prep. Example 162)** and 326 mg (0.840 mmol) of *tert-*butyl 4-(4-(4,4,5,5-tetramethyl-1,3,2-dioxaborolan-2-yl)phenyl)piperazine-1-carboxylate. The reaction time was 3 h. The reaction mixture was filtered through a pad of Celite^{®} 535 and SiO₂ (1:1, 8 g) and the filtrate was concentrated *in vacuo.* Flash chromatography (cyclohexane/EtOAc, gradient from 9:10 to 83:17) afforded the product as a yellow solid (230 mg, 66 % yield).

¹H NMR (500 MHz, CDCl₃) δ 8.79 (d, *J* = 2.0 Hz, 1H), 8.02 (s, 1H), 7.93 (dd, *J* = 1.8, 1.8 Hz, 1H), 7.81 - 7.85 (m, 2H), 7.49 (d, *J* = 8.9 Hz, 2H), 7.30 (dd, *J* = 7.7, 7.7 Hz, 1H), 7.16 (d, *J* = 6-6 Hz, 1H), 6.96 (d, *J* = 8.9 Hz, 2H), 3.91 (p, *J* = 7.8 Hz, 1H), 3.50 - 3.57 (m, 4H), 3.11 - 3.18 (m, 4H), 2.41 - 2.49 (m, 2H), 1.87 - 2.13 (m, 4H), 1.42 (s, 9H).

¹³C NMR (126 MHz, CDCl₃) δ 154.72, 150.95, 149.33, 145.01, 144.94, 144.23, 137.70, 132.92, 131.15, 129.40, 129.19, 128.20, 128.13, 127.38, 124.45, 121.33, 116.73, 115.89, 80.00, 48.97, 43.43, 40.43, 30.82, 28.475, 18.80.

### Preparative Example 164: 3-(3-(cyclobutylthio)phenyl)-6-(4-(piperazin-1-yl)phenyl)furo[3,2-b]pyridine

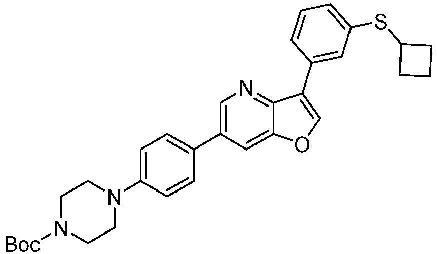

The compound was prepared by the general procedure C using 190 mg (0.351 mmol) of *tert*-butyl 4-(4-(3-(3-(cyclobutylthio)phenyl)furo[3,2-*b*]pyridin-6-yl)phenyl)piperazine-1-carboxylate **(Prep. Example 163);** the reaction time was 2 h; flash chromatography (cyclohexane/EtOAc, gradient from 1:0 to 0:1) afforded the compound as a pale yellow solid (70 mg, 45 % yield).

¹H NMR (500 MHz, CD₃OD) δ 8.86 (d, *J* = 1.9 Hz, 1H), 8.44 (s, 1H), 8.14 (d, *J* = 1.9 Hz, 1H), 8.09 (dd, *J* = 1.8, 1.8 Hz, 1H), 7.85 (ddd, *J* = 1.4, 1.4, 7.7 Hz, 1H), 7.66 - 7.70 (m, 2H), 7.40 (dd, *J* = 7.7, 7.5 Hz, 1H), 7.27 (ddd, *J* = 1.1, 1.9, 7.9 Hz, 1H), 7.13 - 7.18 (m, 2H), 4.07 (p, *J* = 7.8 Hz, 1H), 3.33 - 3.36 (m, 4H), 3.13 - 3.18 (m, 4H), 2.52 - 2.62 (m, 2H), 2.03 - 2.18 (m, 4H).

¹³C NMR (126 MHz, CD₃OD) δ 151.55, 149.37, 147.48, 144.80, 143.72, 137.59, 132.78, 131.72, 129.77, 128.22, 127.45, 127.41, 126.36, 124.12, 119.96, 116.06, 49.05, 48.65, 45.36, 30.56, 18.81. HRMS (APCI): calcd. For C₂₇H₂₇N₃OS [M+H]⁺ = 442.1948; found [M+H]⁺ = 442.1951

### Preparative Example 165: methyl 3-(6-chlorofuro[3,2-b]pyridin-3-yl)benzoate

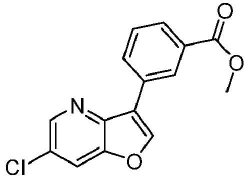

The compound was prepared by the general procedure **A** using 120 mg (0.516 mmol) of 3-bromo-6-chlorofuro[3,2-*b*]pyridine **(Prep. Example 5)** and 121 mg (0.671 mmol) of 3-(methoxycarbonyl)phenylboronic acid; the reaction time was 4 h. The reaction mixture was filtered through a pad of Celite^{®} 535 and SiO₂ (1:1, 8 g) and the filtrate was concentrated *in vacuo.* Flash chromatography (cyclohexane/EtOAc, gradient from 1:0 to 19:1) provided the product as an off-white solid (82 mg, 55 % yield).

¹H NMR (500 MHz, CDCl₃) δ 8.57 (d, *J* = 2.0 Hz, 1H), 8.53 (dd, *J* = 1.8, 1.8 Hz, 1H), 8.25 (ddd, *J* = 1.4, 1.4, 7.8 Hz, 1H), 8.12 (s, 1H), 7.97 (ddd, *J* = 1.4, 1.4, 7.8 Hz, 1H), 7.77 (d, *J* = 2.1 Hz, 1H), 7.50 (dd, *J* = 7.8, 7.8 Hz, 1H), 3.89 (s, 3H).

¹³C NMR (126 MHz, DMSO-d6) δ 166.61, 149.04, 148.50, 145.46, 144.12, 131.66, 130.92, 130.77, 129.76, 128.95, 127.67, 127.42, 120.06, 119.70, 52.79.

### Preparative Example 166: tert-butyl 4-(4-(3-(3-(butoxycarbonyl)phenyl)furo[3,2-b]pyridin-6-yl)phenyl)piperazine-1-carboxylate

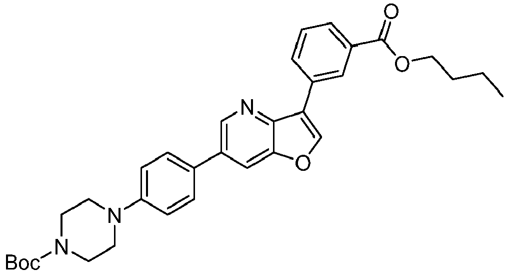

The compound was prepared by the general procedure **B** using 80 mg (0.278 mmol) of methyl 3-(6-chlorofuro[3,2-*b*]pyridin-3-yl)benzoate **(Prep. Example 165)** and 140 mg (0.361 mmol) of *tert*-butyl 4-(4-(4,4,5,5-tetramethyl-1,3,2-dioxaborolan-2-yl)phenyl)piperazine-1-carboxylate. The reaction time was 4 h. The reaction mixture was filtered through a pad of Celite^{®} 535 and SiO₂ (1:1, 8 g) and the filtrate was concentrated *in vacuo.* Flash chromatography (cyclohexane/EtOAc, gradient from 11:0 to 0:1, followed by DCM/MeOH, gradient from 4:1 to 7:3) afforded the product as a pale beige solid (56 mg, 37 %).

¹H NMR (500 MHz, CDCl₃) δ 8.92 (d, *J* = 1.9 Hz, 1H), 8.66 (dd, *J* = 1.7, 1.7 Hz, 1H), 8.47 (ddd, *J* = 1.5, 1.5, 7.8 Hz, 1H), 8.22 (s, 1H), 8.06 (ddd, *J* = 1.5, 1.5, 7.8 Hz, 1H), 7.97 (d, *J* = 1.9 Hz, 1H), 7.58-7.64 (m, 3H), 7.08 - 7.15 (m, 2H), 4.40 (t, *J* = 6.6 Hz, 2H), 3.61 - 3.71 (m, 4H), 3.22 - 3.31 (m, 4H), 1.77 - 1.87 (m, 2H), 1.52 (s, 9H), 1.49 - 1.61 (m, 2H), 1.03 (t, *J* = 7.4 Hz, 3H).

¹³C NMR (126 MHz, CDCl₃) δ 166.61, 154.71, 149.31, 145.24, 145.08, 144.10, 133.05, 131.62, 131.13, 130.87, 128.98, 128.68, 128.27, 127.93, 121.02, 116.90, 116.09, 80.06, 64.99, 49.14, 43.4, 30.83, 28.45, 19.30, 13.79

### Preparative Example 167: butyl 3-(6-(4-(piperazin-1-yl)phenyl)furo[3,2-b]pyridin-3-yl)benzoate

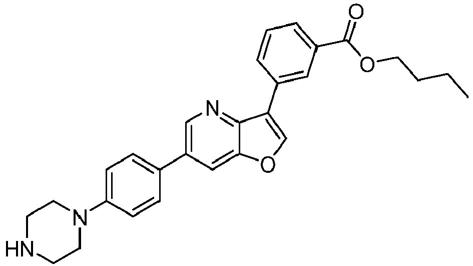

To the heterogeneous mixture of 23 mg (0.041 mmol) of *tert*-butyl 4-(4-(3-(3-(butoxycarbonyl)phenyl)furo[3,2-*b*]pyridin-6-yl)phenyl)piperazine-1-carboxylate **(Prep. Example 166)** in 5.0 mL of anhydrous 1,4-dioxane, 0.73 mL of 4M HCl in 1,4-dioxane was added at 25 °C. The mixture was stirred at 25 °C for 5 h. The progress of the reaction was monitored by TLC. The reaction mixture was concentrated *in vacuo,* 500 mg (5.952 mmol) of solid sodium bicarbonate was added to the residue and the mixture was stirred in DCM/MeOH 5:1 (12 mL) for 2.5 h. The mixture was filtered through a pad of Celite^{®} 535 and SiO₂ (1:1; 8 g) and the pad was washed with 50 mL of the mixture DCM/MeOH 5:1. The filtrate was concentrated *in vacuo* and the residue was purified by flash chromatography (DCM/MeOH, gradient from 9:1 to 4:1) and the product was obtained as a yellowish solid (12 mg, 65 %).

¹H NMR (500 MHz, CDCl₃) δ 8.91 (d, *J* = 2.0 Hz, 1H), 8.66 (dd, *J* = 1.8, 1.8 Hz, 1H), 8.47 (ddd, *J* = 7.8, 1.5, 1.5 Hz, 1H), 8.22 (s, 1H), 8.06 (ddd, *J* = 7.8, 1.5, 1.5 Hz, 1H), 7.96 (d, *J* = 1.9 Hz, 1H), 7.65 - 7.57 (m, 3H), 7.12 - 7.05 (m, 2H), 4.40 (t, *J* = 6.6 Hz, 2H), 3.50 - 3.45 (m, 4H), 3.34 - 3.28 (m, 4H), 1.87 - 1.77 (m, 2H), 1.60 - 1.49 (m, 2H), 1.03 (t, *J* = 7.4 Hz, 3H).

¹³C NMR (126 MHz, CDCl₃) δ 166.61, 150.23, 149.26, 145.31, 145.14, 144.32, 132.86, 131.62, 131.15, 130.87, 130.45, 128.98, 128.72, 128.38, 127.93, 121.04, 117.25, 116.03, 65.00, 47.63, 44.16, 30.83, 19.30, 13.78.

### Preparative Example 168: 6-chloro-3-(3-fluorophenyl)furo[3,2-b]pyridine

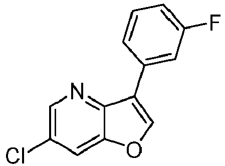

The compound was prepared by the general procedure **A** using 100 mg (0.430 mmol) of 3-bromo-6-chlorofuro[3,2-*b*]pyridine **(Prep. Example 5)** and 78 mg (0.559 mmol) of (3-fluorophenyl)boronic acid; the reaction time was 2 h; flash chromatography (cyclohexane) afforded the compound as a white solid (98 mg, 92 % yield).

¹H NMR (500 MHz, Methanol-*d*₄) δ 8.61 (d, *J* = 2.1 Hz, 1H), 8.53 (s, 1H), 8.08 (d, *J* = 2.0 Hz, 1H), 7.96 (ddd, *J* = 10.5, 2.7, 1.6 Hz, 1H), 7.89 (ddd, *J* = 7.8, 1.6, 0.9 Hz, 1H), 7.46 (td, *J* = 8.0, 6.1 Hz, 1H), 7.09 (tdd, *J* = 8.5, 2.7, 0.9 Hz, 1H).

¹³C NMR (126 MHz, MeOD) δ 165.45, 163.51, 150.01, 148.74, 146.12, 145.27, 133.65, 133.58, 131.53, 131.46, 129.75, 128.99, 128.22, 123.78, 123.75, 121.53, 121.51, 120.23, 115.60, 115.42, 114.94, 114.75.

HRMS (APCI): calcd, for C₁₃H₇ClFNO [M+H]⁺ = 248.0273; found [M+H]⁺ = 248.0274.

FTIR (neat), cm⁻¹: 3106, 3085, 3073, 1617, 1589, 1488, 1436, 1386, 1352, 1266, 1232, 1185, 1128, 1100, 1076, 998, 919, 878, 851, 815, 779, 686, 600, 514, 453, 438.

### Preparative Example 169: tert-butyl 4-(4-(3-(3-fluorophenyl)furo[3,2-b]pyridin-6-yl)phenyl)piperazine-1-carboxylate

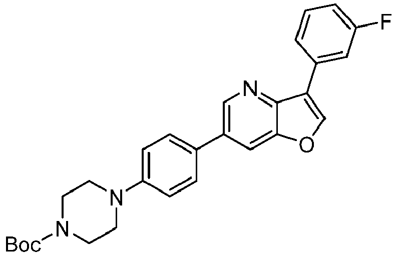

The compound was prepared by the general procedure **B** using 80 mg (0.323 mmol) of 6-chloro-3-(3-fluorophenyl)furo[3,2-*b*]pyridine **(Prep. Example 168)** and 163 mg (0.420 mmol) of 4-(4-*tert-*butoxycarbonylpiperazinyl)phenylboronic acid pinacol ester; the reaction time was 2 h; flash chromatography (cyclohexane/EtOAc, gradient from 1:0 to 7:3) afforded the compound as a pale yellow solid (98 mg, 64 % yield).

¹H NMR (500 MHz, Chloroform-d) δ 8.88 (d, *J* = 1.9 Hz, 1H), 8.12 (s, 1H), 7.93 - 7.84 (m, 3H), 7.59 - 7.54 (m, 2H), 7.44 (td, *J* = 7.9, 6.0 Hz, 1H), 7.09 - 7.00 (m, 3H), 3.68 - 3.57 (m, 4H), 3.28 - 3.19 (m, 4H), 1.50 (s, 10H).

¹³C NMR (126 MHz, CDCl₃) δ 164.26, 162.31, 154.86, 151.12, 149.44, 145.28, 145.21, 144.08, 133.25, 132.82, 132.75, 130.47, 130.40, 129.37, 128.33, 122.75, 122.72, 120.92, 120.89, 116.85, 116.12, 114.75, 114.58, 114.25, 114.07, 80.14, 77.16, 49.06, 43.42, 28.58.

HRMS (APCI): calcd, for C₂₈H₂₈FN₃O₃ [M+H]⁺ = 474.2187; found [M+H]⁺ = 474.2187.

FTIR (neat), cm⁻¹ 2977, 2921, 2825, 1689, 1607, 1524, 1480, 1448, 1425, 1401, 1379, 1365, 1259, 1233, 1215, 1160, 1131, 1115, 1088, 1046, 999, 904, 876, 845, 817, 786, 689, 539, 519.

### Preparative Example 170: 3-(3-fluorophenyl)-6-(4-(piperazin-1-yl)phenyl)furo[3,2-b]pyridine

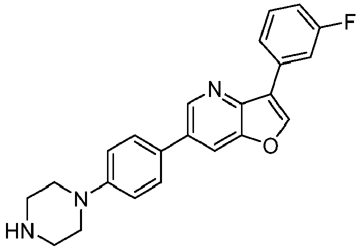

The compound was prepared by the general procedure C using 90 mg (0.190 mmol) of *tert*-butyl 4-(4-(3-(3-fluorophenyl)furo[3,2-*b*]pyridin-6-yl)phenyl)piperazine-1-carboxylate **(Prep. Example 169);** the reaction time was 2 h; flash chromatography (DCM/MeOH, gradient from 1:0 to 4:1) afforded the compound as a pale yellow solid (59 mg, 83 % yield).

¹H NMR (300 MHz, Chloroform-*d*) δ 8.95 (d, *J* = 1.9 Hz, 1H), 8.93 (s, 1H), 8.33 (d, *J* = 1.9 Hz, 1H), 8.22 - 8.06 (m, 2H), 7.74 - 7.67 (m, 2H), 7.61 - 7.48 (m, 1H), 7.20 (td, *J* = 8.6, 2.7 Hz, 1H), 7.09 - 7.03 (m, 2H), 3.25 - 3.14 (m, 4H), 2.94 (dd, *J* = 6.6, 3.5 Hz, 4H).

¹³C NMR (126 MHz, DMSO) δ 163.33, 161.40, 151.22, 148.89, 147.38, 144.37, 142.94, 132.83, 132.76, 132.45, 130.67, 130.60, 127.71, 126.73, 122.45, 122.43, 118.88, 118.86, 115.66, 115.47, 114.26, 114.10, 113.08, 112.89, 48.52, 45.19, 39.52.

HRMS (APCI): calcd, for C₂₃H₂₀FN₃O [M+H]⁺ = 374.1663; found [M+H]⁺ = 374.1664.

FTIR (neat), cm⁻¹: 2943, 2846, 2822, 1604, 1587, 1570, 1523, 1481, 1449, 1380, 1335, 1260, 1242, 1216, 1146, 1116, 1097, 911, 891, 847, 821, 788, 689, 664, 544, 516.

### Preparative Example 171: 6-(4-(2-(piperidin-1-yl)ethoxy)phenyl)-3-(pyridin-4-yl)furo[3,2-b]pyridine

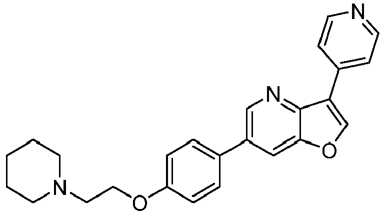

The compound was prepared by the general procedure **B** using 140 mg (0.607 mmol) of 6-chloro-3-(pyridin-4-yl)furo[3,2-*b*]pyridine **(Prep. Example 21)** and 261 mg (0.789 mmol) of 1-(2-(4-(4,4,5,5-tetramethyl-1,3,2-dioxaborolan-2-yl)phenoxy)ethyl)piperidine; the reaction time was 75 min; flash chromatography (column: 6 g of C₁₈-coated silica gel; eluent: H₂O/7M NH₃ in MeOH, gradient from 2:1 to 1:49) followed by preparative TLC (DCM/7M NH₃ in MeOH, 16:1) afforded the compound as a white solid (133 mg, 60 % yield).

¹H NMR (500 MHz, Chloroform-d) *δ* 8.89 (d, *J* = 1.9 Hz, 1H), 8.77 - 8.68 (m, 2H), 8.27 (s, 1H), 8.10 - 8.03 (m, 2H), 7.95 (d, *J* = 1.8 Hz, 1H), 7.62 - 7.54 (m, 2H), 7.10 - 7.03 (m, 2H), 4.20 (t, *J* = 6.0 Hz, 2H), 2.85 (t, *J* = 6.0 Hz, 2H), 2.68 - 2.49 (m, 4H), 1.65 (p, *J* = 5.7 Hz, 4H), 1.55 - 1.42 (m, 2H).

¹³C NMR (126 MHz, Chloroform-d) *δ* 159.21, 150.53, 149.56, 146.57, 145.65, 143.88, 138.41, 133.66, 130.43, 128.73, 121.41, 119.63, 116.63, 115.56, 66.24, 57.98, 55.21, 25.97, 24.25.

FTIR (neat), cm⁻¹: 3040, 2932, 2783, 1603, 1520, 1479, 1262, 1245, 1204, 1127, 1097, 1033, 826, 732, 670, 537, 518.

### Preparative Example 172: 6-(4-(4-methylpiperazin-1-yl)phenyl)-3-phenylfuro[3,2-b]pyridine

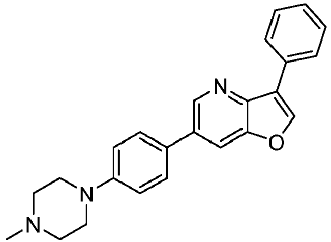

The compound was prepared by the general procedure **B** using 100 mg (0.435 mmol) of 6-chloro-3-phenylfuro[3,2-*b*]pyridine **(Prep. Example 100)** and 158 mg (0.523 mmol) of 1-methyl-4-(4-(4,4,5,5-tetramethyl-1,3,2-dioxaborolan-2-yl)phenyl)piperazine; the reaction time was 2 h; flash chromatography (DCM/MeOH, gradient from 1:0 to 9:1) afforded the compound as a white solid (153 mg, 95 % yield).

¹H NMR (500 MHz, Chloroform-d) δ 8.89 (d, *J* = 1.9 Hz, 1H), 8.11 (s, 1H), 8.11 - 8.06 (m, 2H), 7.92 (d, *J* = 1.8 Hz, 1H), 7.60 - 7.53 (m, 2H), 7.49 (dd, *J* = 8.4, 7.1 Hz, 2H), 7.38 (d, *J* = 7.4 Hz, 1H), 7.09 - 7.02 (m, 2H), 3.34 (t, *J* = 5.0 Hz, 4H), 2.66 (bs, 4H), 2.41 (s, 3H).

¹³C NMR (126 MHz, CDCl₃) δ 151.11, 149.47, 145.12, 144.89, 144.42, 133.16, 130.70, 129.01, 128.29, 127.86, 127.28, 121.96, 116.39, 116.03, 55.22, 55.13, 53.54, 49.13, 48.76, 46.22.

HRMS (APCI): calcd, for C₂₄H₂₃N₃O [M+H]⁺ = 370.1914; found [M+H]⁺ = 370.1911.

FTIR (neat), cm⁻¹: 2940, 2843, 2800, 1604, 1525, 1478, 1445, 1381, 1293, 1245, 1201, 1159, 1140, 1122, 1098, 967, 918, 820, 789, 779, 752, 691, 671, 530, 504.

### Preparative Example 173: N-methyl-N-(3-(6-(4-(4-methylpiperazin-1-yl)phenyl)furo[3,2-b]pyridin-3-yl)phenyl)acetamide

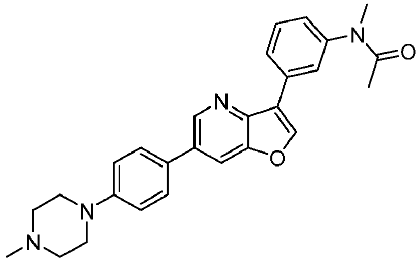

TEA (0.052 mL, 0.370 mmol) and acetic anhydride (0.074 mL, 0.740 mmol) were added to a solution of 59 mg (0.148 mmol) of *N*-methyl-3-(6-(4-(4-methylpiperazin-1-yl)phenyl)furo[3,2-*b*]pyridin-3-yl)aniline **(Prep. Example 110)** in DCM (2 mL) and the mixture was stirred for 3 h at 25 °C. Volatiles were evaporated *in vacuo* and the residue was purified by flash chromatography (DCM/MeOH, with gradient from 1:0 to 4:1). The product was obtained as an off-white solid (65 mg, 100 % yield).

¹H NMR (500 MHz, CDCl₃) δ 8.89 (d*, J* = 1.9 Hz, 1H), 8.15 (s, 1H), 8.09 - 8.02 (m, 2H), 7.93 (d, *J* = 1.9 Hz, 1H), 7.60 - 7.55 (m, 2H), 7.53 (t, *J* = 7.8 Hz, 1H), 7.19 (bd, *J* = 7.7 Hz, 1H), 7.09 - 7.02 (m, 2H), 3.35 (s, 3H), 3.33 - 3.28 (m, 4H), 2.65 - 2.58 (m, 4H), 2.38 (s, 3H), 1.98 (s, 3H).

¹³C NMR (126 MHz, CDCl₃) δ 170.82, 151.27, 149.54, 145.33, 145.12, 143.97, 133.52, 132.59, 130.32, 130.28, 128.72, 128.30, 126.27, 126.25, 125.97, 120.86, 116.35, 116.11, 55.18, 48.80, 46.31, 37.39, 22.75.

HRMS (APCI): calcd. for C₂₇H₂₈N₄O₂ [M+H]⁺ = 441.2285; found [M+H]⁺ = 441.2286.

### Preparative Example 174: 6-chloro-3-(3-(methylsulfonyl)phenyl)furo[3,2-b]pyridine

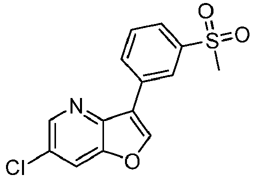

The compound was prepared by the general procedure **A** using 100 mg (0.430 mmol) of 3-bromo-6-chlorofuro[3,2-*b*]pyridine **(Prep. Example 5)** and 103 mg (0.515 mmol) of (3-(methylsulfonyl)phenyl)boronic acid; the reaction mixture was stirred for 2 h; flash chromatography (cyclohexane/EtOAc, gradient from 1:0 to 3:1) afforded the compound as a white solid (96 mg, 73 % yield).

¹H NMR (500 MHz, CDCl₃) δ 8.64 (d, *J* = 2.0 Hz, 1H), 8.58 (t, *J* = 1.7 Hz, 1H), 8.48 - 8.43 (m, 1H), 8.24 (s, 1H), 7.96 - 7.92 (m, 1H), 7.87 (d, *J* = 2.0 Hz, 1H), 7.70 (t, *J* = 7.8 Hz, 1H), 3.13 (s, 3H). ¹³C NMR (126 MHz, CDCl₃) δ 148.59, 146.38, 145.76, 143.84, 141.49, 132.30, 131.78, 130.14, 128.29, 126.63, 125.74, 120.30, 119.15, 44.67.

HRMS (APCI): calcd. for C₁₄H₁₀ClNO₃S [M+H]⁺ = 308.0143; found [M+H]⁺ = 308.0147.

### Preparative Example 175: 6-(4-(4-methylpiperazin-1-yl)phenyl)-3-(3-(methylsulfonyl)phenyl)furo[3,2-b]pyridine

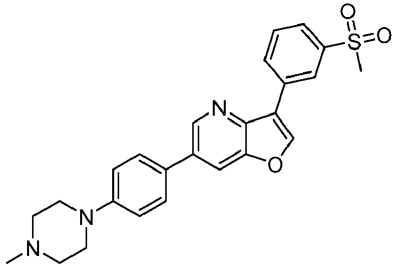

The compound was prepared by the general procedure **B** using 59 mg (0.192 mmol) of 6-chloro-3-(3-(methylsulfonyl)phenyl)furo[3,2-*b*]pyridine **(Prep. Example 174)** and 79 mg (0.261 mmol) of 1-methyl-4-(4-(4,4,5,5-tetramethyl-1,3,2-dioxaborolan-2-yl)phenyl)piperazine; the reaction mixture was stirred for 2 h; flash chromatography (DCM/MeOH, gradient from 1:0 to 93:7) afforded the compound as a white solid (54 mg, 63 % yield).

1H NMR (500 MHz, CDCl₃) δ 8.88 (d, J = 1.9 Hz, 1H), 8.62 (t, J = 1.7 Hz, 1H), 8.55 - 8.51 (m, 1H), 8.22 (s, 1H), 7.96 - 7.89 (m, 2H), 7.69 (t, J = 7.8 Hz, 1H), 7.59 - 7.54 (m, 2H), 7.07 - 7.01 (m, 2H), 3.35 - 3.27 (m, 4H), 3.13 (s, 3H), 2.65 - 2.58 (m, 4H), 2.37 (s, 3H).

¹³C NMR (126 MHz, CDCl₃) δ 151.26, 149.51, 145.59, 145.42, 143.61, 141.34, 133.68, 132.49, 132.31, 130.04, 128.51, 128.26, 126.25, 125.64, 120.17, 116.29, 116.15, 55.13, 48.72, 46.28, 44.66.

HRMS (APCI): calcd. for C₂₅H₂₅N₃O₃S [M+H]⁺ = 448.1689; found [M+H]⁺ = 448.1692.

### Preparative Example 176: 4,4,5,5-tetramethyl-2-(3-(perfluoroethyl)phenyl)-1,3,2-dioxaborolane

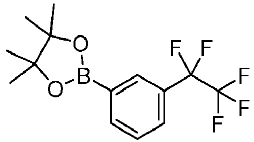

Pd(dppf)Cl₂ (39.9 mg, 0.055 mmol) was added to the degassed solution of 1-bromo-3-(perfluoroethyl)benzene (300 mg, 1.092 mmol), bis(pinacolato) diboron (306 mg, 1.200 mmol) and potassium acetate (321 mg, 3.276 mmol) in 6.0 mL of anhydrous 1,4-dioxane and the mixture was heated to 85 °C for 6.5 h. The mixture was cooled to 25 °C and stirred for additional 86 h. The reaction was monitored by TLC. The mixture was filtered through a pad of Celite^{®}535 and the filtrate was washed with brine (3 × 25 mL). Combined aqueous layers were extracted with 25 mL of EtOAc, and combined organic layers were dried over anhydrous MgSO₄, filtered, and concentrated in vacuo. The residue was purified by flash chromatography (cyclohexane/EtOAc, gradient from 1:0 to 9:1). The product was obtained as colorless liquid (271 mg, 67 % yield).

¹H NMR (500 MHz, CDCl₃) δ 7.97 - 7.94 (m, 1H), 7.94 - 7.89 (m, 1H), 7.62 - 7.58 (m, 1H), 7.45 - 7.40 (m, 1H), 1.29 (s, 12 H).

¹³C NMR (126 MHz, CDCl₃) δ 138.17, 132.53, 132.48, 132.43, 129.04, 128.98, 128.93, 128.20, 128.00, 84.27, 24.86 did not report perfluoroethyl carbons that are in 110-120 ppm region

### Preparative Example 177: 6-chloro-3-(3-(perfluoroethyl)phenyl)furo[3,2-b]pyridine

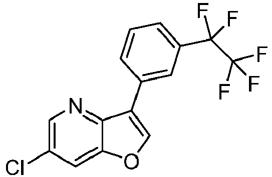

The compound was prepared by the general procedure **A** using 120 mg (0.516 mmol) of 3-bromo-6-chlorofuro[3,2-*b*]pyridine **(Prep. Example 5)** and 216 mg (0.671 mmol) of 4,4,5,5-tetramethyl-2-(3-(perfluoroethyl)phenyl)-1,3,2-dioxaborolane **(Prep. Example 176);** the reaction time was 2.5 h. The reaction mixture was hot-filtered through a pad of Celite^{®} 535 and SiO₂ (1:1, 8 g) and the filtrate was concentrated *in vacuo.* Flash chromatography (cyclohexane/EtOAc, gradient from 1:0 to 16:1) provided the product as a white solid (175 mg, 98 % yield).

¹H NMR (500 MHz, CDCl₃) δ 8.57 (d, *J* = 2.1 Hz, 1H), 8.25 (d, *J* = 7.2 Hz, 1H), 8.17 (s, 1H), 8.11 (s, 1H), 7.78 (d, *J* = 2.0 Hz, 1H), 7.58 - 7.51 (m, 2H).

¹³C NMR (126 MHz, CDCl₃) δ 148.42, 145.52, 143.89, 130.53, 129.66, 129.47, 129.35, 129.28, 125.86, 125.81, 125.77, 124.92, 124.87, 124.82, 120.67, 120.59, 120.27, 118.87, 118.00, 117.69, 115.73, 115.54, 115.23, 113.52, 113.21, 111.50, 111.20.

HRMS (APCI): calcd. For C₁₅H₇ClF₅NO [M+H]⁺ = 348.0209; found [M+H]⁺ = 348.0208

### Preparative Example 178: 6-(4-(4-methylpiperazin-1-yl)phenyl)-3-(3-(perfluoroethyl)phenyl)furo[3,2-b]pyridine

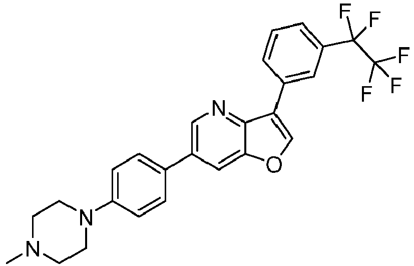

The compound was prepared by the general procedure **B** using 100 mg (0.288 mmol) of 6-chloro-3-(3-(perfluoroethyl)phenyl)furo[3,2-*b*]pyridine **(Prep. Example 177)** and 113 mg (0.374 mmol) of 1-methyl-4-(4-(4,4,5,5-tetramethyl-1,3,2-dioxaborolan-2-yl)phenyl)piperazine; the reaction time was 1.5 h; the reaction mixture was hot-filtered through a pad of Celite^{®} 535 and SiO₂ (1:1, 8 g), and the filtrate was concentrated *in vacuo.* Flash chromatography (DCM/MeOH, gradient from 1:0 to 19:1) afforded the product as an off-white solid (75 mg, 53 % yield).

¹H NMR (500 MHz, CDCl₃) δ 8.92 (d, *J* = 2.0 Hz, 1H), 8.44 (d, *J* = 7.6 Hz, 1H), 8.30 (s, 1H), 8.20 (s, 1H), 7.96 (d, *J* = 1.8 Hz, 1H), 7.66 (dd, *J* = 7.5, 7.5 Hz, 1H), 7.64 - 7.57 (m, 3H), 7.08 (d, *J* = 8.9 Hz, 2H), 3.36 - 3.31 (m, 4H), 2.66 - 2.61 (m, *J* = 5.1 Hz, 4H), 2.40 (s, 3H).

¹³C NMR (126 MHz, CDCl₃) δ 151.11, 149.37, 145.27, 145.13, 143.74, 133.41, 131.55, 130.59, 129.55, 129.36, 129.30, 129.17, 128.55, 128.21, 125.48, 124.83, 120.62, 116.17, 115.97, 55.03, 48.64, 46.16. HRMS (APCI): calcd. For C₂₆H₂₂F₅N₃O [M+H]⁺ = 488.1756; found [M+H]⁺ = 488.1753

### Preparative Example 179: 6-benzyl-3-phenylfuro[3,2-b]pyridine

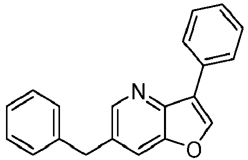

The compound was prepared by the general procedure **B** using 41 mg (0.178 mmol) of 6-chloro-3-phenylfuro[3,2-*b*]pyridine **(Prep. Example 100)** and 50 mg (0.231 mmol) of 2-benzyl-4,4,5,5-tetramethyl-1,3,2-dioxaborolane; the reaction time was 2 h; flash chromatography (cyclohexane/toluene, gradient from 1:0 to 1:1) afforded the compound as a white solid (5.3 mg, 10 % yield).

¹H NMR (500 MHz, CDCl₃) δ 8.58 (d, *J* = 1.7 Hz, 1H), 8.08 - 8.02 (m, 3H), 7.57 (d, *J* = 1.7 Hz, 1H), 7.50 - 7.44 (m, 2H), 7.38 - 7.29 (m, 3H), 7.25 - 7.21 (m, 3H), 4.15 (s, 2H).

¹³C NMR (126 MHz, CDCl₃) δ 149.23, 147.09, 144.67, 144.49, 140.25, 132.98, 130.69, 129.04, 128.99, 128.91, 127.84, 127.26, 126.71, 121.87, 118.82, 39.32.

HRMS (APCI): calcd. for C₂₀H₁₅NO [M+H]⁺ = 286.1226; found [M+H]⁺ = 286.1227.

### Preparative Example 180: 6-chloro-3-(3-(methylsulfinyl)phenyl)furo[3,2-b]pyridine

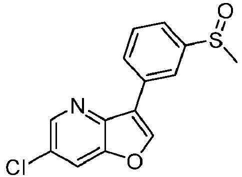

The compound was prepared by the general procedure **A** using 100 mg (0.430 mmol) of 3-bromo-6-chlorofuro[3,2-*b*]pyridine **(Prep. Example 5)** and 110 mg (0.559 mmol) of (3-(methylsulfinyl)phenyl)boronic acid; the reaction time was 2 h; flash chromatography (cyclohexane/EtOAc, gradient from 1:0 to 1:1) afforded the compound as a white solid (113 mg, 90 % yield).

¹H NMR (500 MHz, CDCl₃) δ 8.60 (d, *J* = 2.0 Hz, 1H), 8.31 (br s, 1H), 8.24 (dt, *J* = 6.8, 1.8 Hz, 1H), 8.21 (s, 1H), 7.83 (d, *J* = 2.0 Hz, 1H), 7.66 - 7.59 (m, 2H), 2.80 (s, 3H).

¹³C NMR (126 MHz, CDCl₃) δ 148.56, 146.81, 146.27, 145.60, 145.57, 144.04, 131.57, 130.02, 129.67, 128.09, 122.93, 121.95, 120.76, 119.04, 44.23.

HRMS (APCI): calcd. for C₁₄H₁₀ClNO₂S [M+H]⁺ = 292.0194; found [M+H]⁺ = 292.0192.

### Preparative Example 181: 6-(4-(4-methylpiperazin-1-yl)phenyl)-3-(3-(methylsulfinyl)phenyl)furo[3,2-b]pyridine

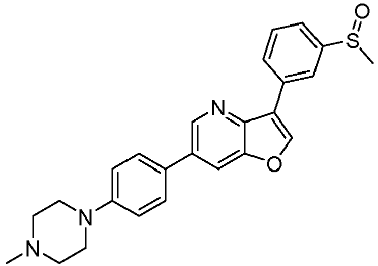

The compound was prepared by the general procedure **B** using 109 mg (0.340 mmol) of 6-chloro-3-(3-(methylsulfinyl)phenyl)furo[3,2-*b*]pyridine **(Prep. Example 180)** and 134 mg (0.422 mmol) of 1-methyl-4-(4-(4,4,5,5-tetramethyl-1,3,2-dioxaborolan-2-yl)phenyl)piperazine; the reaction time was 2 h; flash chromatography (DCM/MeOH, gradient from 1:0 to 93:7) afforded the compound as an off-white solid (93 mg, 63 % yield).

¹H NMR (500 MHz, CDCl₃) δ 8.89 (d, *J* = 1.8 Hz, 1H), 8.39 - 8.32 (m, 2H), 8.22 (s, 1H), 7.94 (d, *J* = 1.9 Hz, 1H), 7.69 - 7.61 (m, 2H), 7.59 - 7.55 (m, 2H), 7.08 - 7.03 (m, 2H), 3.35 - 3.27 (m, 4H), 2.81 (s, 3H), 2.65 - 2.57 (m, 4H), 2.38 (s, 3H).

¹³C NMR (126 MHz, CDCl₃) δ 151.28, 149.54, 146.63, 145.52, 145.34, 143.88, 133.58, 132.30, 130.02, 129.75, 128.68, 128.30, 122.61, 121.98, 120.71, 116.34, 116.18, 55.17, 48.79, 46.31, 44.22.

HRMS (APCI): calcd. for C₂₅H₂₅N₃O₂S [M+H]⁺ = 432.1740; found [M+H]⁺ = 432.1743.

### Preparative Example 182: 1-(3-(6-chlorofuro[3,2-b]pyridin-3-yl)phenyl)urea

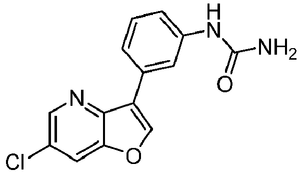

The compound was prepared by the general procedure **A** using 191 mg (0.822 mmol) of 3-bromo-6-chlorofuro[3,2-*b*]pyridine **(Prep. Example 5)** and 280 mg (1.069 mmol) of 1-(3-(4,4,5,5-tetramethyl-1,3,2-dioxaborolan-2-yl)phenyl)urea; the reaction time was 3 h. The reaction mixture was hot-filtered through a pad of Celite^{®} 535 and SiO₂ (1:1, 8 g) and the filtrate was concentrated *in vacuo.* Flash chromatography (DCM/MeOH, gradient from 1:0 to 10:1) afforded the product as a pale beige solid (42 mg, 18 % yield).

¹H NMR (500 MHz, DMSO-*d₆*) δ 8.81 (s, 1H), 8.68 (d, *J* = 2.0 Hz, 1H), 8.65 (s, 1H), 8.42 (d, *J* = 2.0 Hz, 1H), 8.08 (dd, *J* = 1.9, 1.9 Hz, 1H), 7.64 - 7.58 (m, 2H), 7.34 (dd, *J* = 7.9, 7.9 Hz, 1H), 5.86 (s, 2H). ¹³C NMR (126 MHz, DMSO-*d₆*) δ 156.44, 148.45, 148.22, 145.15, 144.40, 141.38, 130.44, 129.50, 127.21, 120.84, 120.09, 119.90, 117.96, 116.74.

HRMS (APCI): calcd. For C₁₄H₁₀ClN₃O₂ [M+H]⁺ = 288.0534; found [M+H]⁺ = 288.0537

### Preparative Example 183: 1-(3-(6-phepylfuro[3,2-b]pyridin-3-yl)phenyl)urea

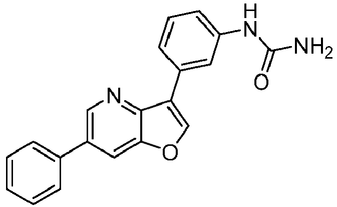

The compound was prepared by the general procedure **B** using 60 mg (0.209 mmol) of 1-(3-(6-chlorofuro[3,2-*b*]pyridin-3-yl)phenyl)urea **(Prep. Example 182)** and 33 mg (0.271 mmol) of phenylboronic acid. The reaction time was 24 h. The reaction mixture was hot-filtered through a pad of Celite^{®} 535 and SiO₂ (1:1, 8 g) and the filtrate was concentrated *in vacuo.* Flash chromatography (DCM/MeOH, gradient from 1:0 to 19:1) afforded the product as a pale brown solid (36 mg, 53 % yield). ¹H NMR (500 MHz, DMSO-*d₆*) δ 8.96 (d, *J* = 1.8 Hz, 1H), 8.80 (s, 1H), 8.67 (s, 1H), 8.41 (d, *J* = 1.8 Hz, 1H), 8.16 (dd, *J* = 2.0, 2.0 Hz, 1H), 7.85 (d, *J* = 8.4 Hz, 2H), 7.69 (d, *J* = 9.2 Hz, 1H), 7.63 (d, *J* = 9.5 Hz, 1H), 7.55 (dd, *J* = 7.6, 7.6 Hz, 2H), 7.49 - 7.42 (m, 1H), 7.35 (dd, *J* = 7.9, 7.9 Hz, 1H), 5.87 (s, 2H).

¹³C NMR (126 MHz, DMSO-*d₆*) δ 156.47, 149.15, 147.61, 145.19, 144.85, 141.36, 137.70, 132.75, 130.98, 129.63, 129.47, 128.50, 127.83, 120.82, 120.08, 117.80, 117.35, 116.74.

HRMS (APCI): calcd. For C₂₀H₁₅N₃O₂ [M+H]⁺ = 330.1237; found [M+H]⁺ = 330.1239

### Preparative Example 184: 5-chloro-3-(cinnamyloxy)-2-iodopyridine

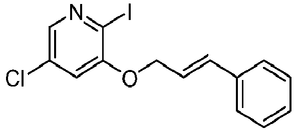

Cinnamyl bromide (463 mg, 2.349 mmol) was added to a mixture of 5-chloro-2-iodopyridin-3-ol **(Prep. Example 1;** 500 mg, 1.957 mmol) and K₂CO₃ (649 mg, 4.698 mmol) in acetone (16 mL). The resulting reaction mixture was stirred under reflux for 24 hours. After cooling to ambient temperature, water (30 mL) was added and the mixture was extracted with EtOAc (3 × 40 mL). The organic parts were washed with brine (50 mL), dried over Na₂SO₄, filtered, and the solvent was evaporated in *vacuo.* Flash chromatography (cyclohexane/EtOAc, gradient from 1:0 to 20:1) afforded the product as pale yellow oil (712 mg, 98 % yield).

¹H NMR (500 MHz, Chloroform-d) δ 8.03 (d, *J* = 2.1 Hz, 1H), 7.47 - 7.40 (m, 2H), 7.39 - 7.33 (m, 3H), 7.33 - 7.28 (m, 1H), 7.03 (d, *J* = 2.1 Hz, 1H), 6.82 (d, *J* = 16.0 Hz, 1H), 6.38 (dt, *J* = 15.9, 5.6 Hz, 1H), 4.79 (dd, *J* = 5.6, 1.6 Hz, 2H).

¹³C NMR (126 MHz, CDCl₃) δ 154.83, 141.48, 136.08, 134.45, 132.21, 128.89, 128.87, 128.51, 126.89, 122.27, 118.94, 109.30, 70.41.

HRMS (APCI): calcd. For C₁₄H₁₁ClINO [M+H]⁺ = 371.9647; found [M+H]⁺ = 371.9647.

### Preparative Example 185: 3-benzyl-6-chlorofuro[3,2-b]pyridine

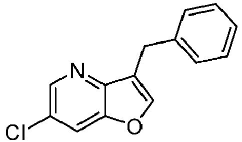

Pd(OAc)₂ (5.4 mg, 0.024 mmol) was added to a degassed mixture of 5-chloro-3-(cinnamyloxy)-2-iodopyridine (Prep. Example 184; 150 mg, 0.404 mmol), K₂CO₃ (140 mg, 1.009 mmol), HCOONa (28 mg, 0.404 mmol), and tetrabutylammonium chloride (123 g, 0.444 mmol) in *N*,*N*-dimethylformamide (3 mL) and the reaction mixture was stirred at 80 °C for 3 hours. The reaction mixture was diluted with EtOAc (30 mL) and extracted with brine (6 × 30 mL). The organic layer was dried over Na₂SO₄, filtered, and the solvent was evaporated in *vacuo.* Flash chromatography (cyclohexane) afforded the product as a white solid (38 mg, 39 % yield).

¹H NMR (500 MHz, Chloroform-d) δ 8.14 (d, *J* = 2.0 Hz, 1H), 7.43 (t, *J* = 7.7 Hz, 2H), 7.35 (t, *J* = 3.2 Hz, 1H), 7.29 (dd, *J* = 9.9, 8.3 Hz, 3H), 7.16 (d, *J* = 2.0 Hz, 1H), 5.58 (d, *J* = 3.2 Hz, 2H). ¹³C NMR (126 MHz, CDCl₃) δ 156.96, 146.50, 141.73, 136.18, 132.55, 131.74, 129.11, 128.92, 128.89, 128.78, 128.01, 120.90, 117.21, 75.60.

HRMS (APCI): calcd. For C₁₄H₁₀ClNO [M+H]⁺ = 244.0524; found [M+H]⁺ = 244.0525.

### Preparative Example 186: 3-benzyl-6-(4-(4-methylpiperazin-1-yl)phenyl)furo[3,2-b]pyridine

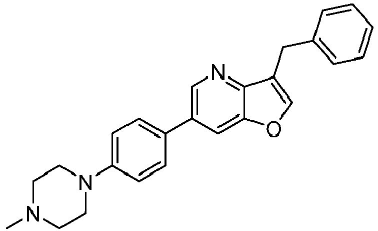

The compound was prepared by the general procedure **B** using 30 mg (0.123 mmol) 3-benzyl-6-chlorofuro[3,2-*b*]pyridine **(Prep. Example 185)** and 45 mg (0.148 mmol) of 1-methyl-4-(4-(4,4,5,5-tetramethyl-1,3,2-dioxaborolan-2-yl)phenyl)piperazine; the reaction time was 3 h; flash chromatography (DCM/MeOH, gradient from 1:0 to 10:1) afforded the compound as a pale yellow solid (16 mg, 34 % yield).

¹H NMR (500 MHz, Chloroform-d) δ 8.42 (d, *J* = 1.8 Hz, 1H), 7.55 - 7.49 (m, 2H), 7.43 (dd, *J* = 8.3, 7.1 Hz, 2H), 7.36 (t, *J* = 3.2 Hz, 1H), 7.34 - 7.28 (m, 4H), 7.05 - 6.97 (m, 2H), 5.58 (d, *J* = 3.2 Hz, 2H), 3.33 (t, *J* = 5.2 Hz, 4H), 2.64 (s, 4H), 2.40 (s, 3H).

¹³C NMR (126 MHz, CDCl₃) δ 157.24, 151.23, 145.84, 141.62, 137.47, 136.69, 133.92, 129.04, 128.79, 128.05, 127.56, 119.44, 116.25, 114.31, 75.08, 55.05, 48.60, 46.14.

HRMS (APCI): calcd. For C₂₅H₂₅N₃O [M+H]⁺ = 384.2070; found [M+H]⁺ = 384.2067.

### Preparative Example 187: 6-chloro-7-methyl-3-phenylfuro[3,2-b]pyridine

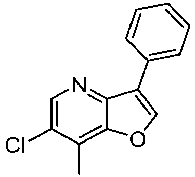

To a cold (-78 °C) solution of 6-Chloro-3-phenylfuro[3,2-*b*]pyridine (Prep. Example 100; 100 mg, 0.435 mmol) in THF (6 mL) was added dropwise *n*-BuLi (2.5 M solution in hexane, 0.226 mL, 0.566 mmol) under argon and the resulting mixture was stirred at -78 °C for 1 hour. A solution of iodomethane (0.054 mL, 0.871 mmol) in THF (2 mL) was added dropwise at -78 °C and the reaction mixture was allowed to warm to 25 °C and stirred for 1 hour at 25 °C. The mixture was quenched with brine (1 mL) and the solvent was evaporated. The residue was purified by flash chromatography (cyclohexane/toleuene, gradient from 1:0 to 10:1) to yield the product as a pale yellow solid (24 mg, 23 %).

¹H NMR (500 MHz, Chloroform-d) δ 8.58 (s, 1H), 8.09 (s, 1H), 8.07 - 7.98 (m, 2H), 7.51 - 7.45 (m, 2H), 7.40 - 7.32 (m, 1H), 2.60 (s, 3H).

¹³C NMR (126 MHz, CDCl₃) δ 148.58, 145.56, 145.01, 143.65, 130.30, 129.03, 128.96, 128.37, 128.04, 127.29, 127.26, 122.34, 77.16, 12.39.

HRMS (APCI): calcd. For C₁₄H₁₀ClNO [M+H]⁺ = 244.0524; found [M+H]⁺ = 244.0527.

### Preparative Example 188: 7-methyl-6-(4-(4-methylpiperazin-1-yl)phenyl)-3-phenylfuro[3,2-b]pyridine

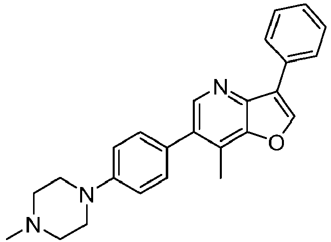

The compound was prepared by the general procedure **B** using 24 mg (0.098 mmol) of 6-chloro-7-methyl-3-phenylfuro[3,2-*b*]pyridine **(Prep. Example 187)** and 36 mg (0.118 mmol) of 1-methyl-4-(4-(4,4,5,5-tetramethyl-1,3,2-dioxaborolan-2-yl)phenyl)piperazine; the reaction time was 3 h; flash chromatography (DCM/MeOH, gradient from 1:0 to 9:1) afforded the compound as a white solid (34 mg, 90 % yield).

¹H NMR (500 MHz, Chloroform-d) δ 8.54 (s, 1H), 8.11 (s, 1H), 8.11 - 8.05 (m, 3H), 7.52 - 7.45 (m, 3H), 7.39 - 7.32 (m, 1H), 7.34 - 7.27 (m, 2H), 7.05 - 7.01 (m, 3H), 3.37 - 3.29 (m, 5H), 2.65 (dd, *J* = 6.2, 3.9 Hz, 6H), 2.52 (s, 3H), 2.40 (s, 3H).

¹³C NMR (126 MHz, CDCl₃) δ 150.58, 149.12, 147.38, 144.39, 143.72, 133.90, 130.96, 130.79, 129.01, 128.99, 128.55, 128.30, 127.90, 127.73, 127.29, 122.24, 116.41, 115.77, 55.21, 48.79, 46.20, 12.68. HRMS (APCI): calcd. For C₂₅H₂₅N₃O [M+H]⁺ = 384.2070; found [M+H]⁺ = 384.2068.

### II. Biological assays

The compounds were profiled in the HotSpot radiometric assays (Reaction Biology).

Briefly,
1. Substrate indicated in Table 1 was dissolved in freshly prepared reaction buffer (20 mM HEPES (pH 7.5), 10 mM MgCl₂, 1 mM EGTA, 0.01% Brij35, 0.02 mg/ml BSA, 0.1 mM Na₃VO₄, 2 mM DTT, 1% DMSO) at the concentration indicated in Table 1.
2. Corresponding kinase (concentration indicated in Table 1) was added and the mixture was gently mixed.
3. Tested compound dissolved in DMSO was added into the kinase reaction mixture.
4. ³³P-ATP (specific activity 0.01 µCi/µL final) was added into the reaction mixture to initiate the reaction.
5. The kinase reaction was incubated for 120 minutes at room temperature.
6. Reaction mixture was spotted onto P81 ion exchange paper (Whatman # 3698-915).
7. The filters were extensively washed with 0.75% phosphoric acid.
7. The radioactive phosphorylated substrate remaining on the filter paper was measured.

**Table 1**

| kinase | kinase concentration in the assay (nM) | substrate | substrate concentration in the assay (µM) |
|---|---|---|---|
| DDR1 | 200 | IRS1tide | 20 |
| FLT3 | 4 | ABLtide | 20 |
| KHS/MAP4K5 | 1.5 | MBP | 20 |

### Data Analysis:

Kinase activity data were expressed as the percent remaining kinase activity in tested samples compared to the vehicle (DMSO) reactions. IC₅₀ values and curve fits were obtained using Prism4 Software (GraphPad).

Table 2 summarizes the inhibitory activities of indicated compounds tested in the *in vitro* kinase assays described above.
A: 20 nM < IC₅₀ < 100 nM
B: 100 nM < IC₅₀ < 500 nM
C: 500 nM < IC₅₀ < 3000 nM

**Table 2**

| **compound** | **kinase** | | |
|---|---|---|---|
| | DDR1 | FLT3 | KHS/MAP4K5 |
| Preparative Example 8: 3-(2,6-dimethylpyridin-4-yl)-6-(4-(piperazin-1-yl)phenyl)furo[3,2-*b*]pyridine | B | B | |
| Preparative Example 11: 6-(4-(piperazin-1-yl)phenyl)-3-(pyridin-3-yl)furo[3,2-*b*]pyridine | B | A | C |
| Preparative Example 14: 3-(3-methoxyphenyl)-6-(4-(piperazin-1-yl)phenyl)furo[3,2-*b*]pyridine | B | A | C |
| Preparative Example 17: 3-(2-methoxyphenyl)-6-(4-(piperazin-1-yl)phenyl)furo[3,2-*b*]pyridine | B | B | C |
| Preparative Example 20: 3-(6-(4-(piperazin-1-yl)phenyl)furo[3,2-*b*]pyridin-3-yl)benzonitrile | B | A | C |
| Preparative Example 22: 6-phenyl-3-(pyridin-4-yl)furo[3,2-*b*]pyridine | | B | |
| Preparative Example 23: 6-(6-morpholinopyridin-3-yl)-3-(pyridin-4-yl)furo[3,2-*b*]pyridine | | A | |
| Preparative Example 25: 6-(6-(piperazin-1-yl)pyridin-3-yl)-3-(pyridin-4-yl)furo[3,2-*b*]pyridine | C | A | |
| Preparative Example 28: 6-(4-(piperazin-1-yl)phenyl)-3-(6-(piperazin-1-y1)pyridin-3-yl)furo[3,2-*b*]pyridine | | | |
| Preparative Example 34: 3-(1-methyl-1*H*-pyrazol-4-yl)-6-(4-(piperazin-1-yl)phenyl)furo[3,2-*b*]pyridine | A | B | B |
| Preparative Example 37: 3-(1-methyl-1*H*-pyrazol-3-yl)-6-(4-(piperazin-1-yl)phenyl)furo[3,2-*b*]pyridine | B | B | |
| Preparative Example 40: 3-(3-nitrophenyl)-6-(4-(piperazin-1-yl)phenyl)furo[3,2-*b*]pyridine | A | B | C |
| Preparative Example 43: 6-(4-(piperazin-1-yl)phenyl)-3-(*p-*tolyl)furo[3,2-*b*]pyridine | B | B | C |
| Preparative Example 46: 3-(3-(*tert*-butylthio)phenyl)-6-(4-(piperazin-1-yl)phenyl)furo[3,2-*b*]pyridine | C | C | |
| Preparative Example 47: 4-(3-(pyridin-4-yl)furo[3,2-*b*]pyridin-6-yl)aniline | | A | |
| Preparative Example 48: 6-(6-(piperidin-1-yl)pyridin-3-yl)-3-(pyridin-4-yl)furo[3,2-*b*]pyridine | | B | |
| Preparative Example 51: 3-(4-fluorophenyl)-6-(4-(piperazin-1-yl)phenyl)furo[3,2-*b*]pyridine | A | A | C |
| Preparative Example 54: *N*,*N*-dimethyl-3-(6-(4-(piperazin-1-yl)phenyl)furo[3,2-*b*]pyridin-3-yl)benzamide | B | B | C |
| Preparative Example 57: 3-(furan-3-yl)-6-(4-(piperazin-1-yl)phenyl)furo[3,2-*b*]pyridine | B | A | C |
| Preparative Example 58: 6-(4-(4-methylpiperazin-1-yl)phenyl)-3-(pyridin-4-yl)furo[3,2-*b*]pyridine | B | A | |
| Preparative Example 61: *N*,*N*-dimethyl-3-(6-(4-(piperazin-1-yl)phenyl)furo[3,2-*b*]pyridin-3-yl)benzenesulfonamide | B | A | C |
| Preparative Example 64: 3-(6-(4-(piperazin-1-yl)phenyl)furo[3,2-*b*]pyridin-3-yl)benzenesulfonamide | B | A | C |
| Preparative Example 67: 3-(4-isopropylphenyl)-6-(4-(piperazin-1-yl)phenyl)furo[3,2-*b*]pyridine | | C | |
| Preparative Example 70: 3-(3-(methoxymethyl)phenyl)-6-(4-(piperazin-1-yl)phenyl)furo[3,2-*b*]pyridine | B | A | C |
| Preparative Example 73: 6-(4-(piperazin-1-yl)phenyl)-3-(3-(trifluoromethyl)phenyl)furo[3,2-*b*]pyridine | C | C | |
| Preparative Example 80: 5-methyl-6-(4-(4-methylpiperazin-1-yl)phenyl)-3-(pyridin-4-yl)furo[3,2-*b*]pyridine | | B | |
| Preparative Example 83: ethyl (3-(6-(4-(4-methylpiperazin-1-yl)phenyl)furo[3,2-*b*]pyridin-3-yl)phenyl)carbamate | C | A | |
| Preparative Example 84: *N*-phenyl-3-(pyridin-4-yl)furo[3,2-*b*]pyridin-6-amine | | B | |
| Preparative Example 87: (3-(6-(4-(piperazin-1-yl)phenyl)furo[3,2-*b*]pyridin-3-yl)phenyl)methanamine | B | A | B |
| Preparative Example 90: (3-(6-(4-(4-methylpiperazin-1-yl)phenyl)furo[3,2-*b*]pyridin-3-yl)phenyl)methanol | B | A | B |
| Preparative Example 93: *N*,*N*-diphenyl-3-(6-(4-(piperazin-1-yl)phenyl)furo[3,2-*b*]pyridin-3-yl)aniline | | C | |
| Preparative Example 94: 3-(6-(4-(4-methylpiperazin-1-yl)phenyl)furo [3,2-*b*]pyridin-3-yl)benzyl acetate | B | A | C |
| Preparative Example 98: *N*¹-(2-(dimethylamino)ethyl)-*N*¹-methyl-*N*⁴-(3-(pyridin-4-yl)furo[3,2-*b*]pyridin-6-yl)benzene-1,4-diamine | C | B | |
| Preparative Example 99: 3,6-di(pyridin-4-yl)furo[3,2-*b*]pyridine | | C | |
| Preparative Example 101: *N*-(4-(4-methylpiperazin-1-yl)phenyl)-3-phenylfuro[3,2-*b*]pyridin-6-amine | B | B | |
| Preparative Example 104: 3-(3-phenoxyphenyl)-6-(4-(piperazin-1-yl)phenyl)furo[3,2-*b*]pyridine | B | B | C |
| Preparative Example 107: 6-(4-(piperazin-1-yl)phenyl)-3-(3-(pyridin-4-yl)phenyl)furo [3,2-*b*]pyridine | B | B | |
| Preparative Example 110: *N*-methyl-3-(6-(4-(4-methylpiperazin-1-yl)phenyl)furo[3,2-*b*]pyridin-3-yl)aniline | B | A | C |
| Preparative Example 113: 6-(4-(piperazin-1-yl)phenyl)-3-(3-(trifluoromethoxy)phenyl)furo[3,2-*b*]pyridine | C | C | |
| Preparative Example 116: 6-(4-(piperazin-1-yl)phenyl)-3-(3-(2,2,2-trifluoroethoxy)phenyl)furo[3,2-*b*]pyridine | C | B | |
| Preparative Example 119: 3-(6-(4-(piperazin-1-yl)phenyl)furo[3,2-*b*]pyridin-3-yl)-*N*-(*p*-tolyl)benzamide | C | C | |
| Preparative Example 122: *N*-benzyl-3-(6-(4-(piperazin-1-yl)phenyl)furo[3,2-*b*]pyridin-3-yl)benzenesulfonamide | C | B | C |
| Preparative Example 125: *N*-(3-(6-(4-(piperazin-1-yl)phenyl)furo[3,2-*b*]pyridin-3-yl)benzyl)methanesulfonamide | B | A | C |
| Preparative Example 130: *N,N*-dimethyl-3-(6-(4-(piperazin-1-yl)phenyl)furo[3,2-*b*]pyridin-3-yl)aniline | B | B | |
| Preparative Example 132: (3-(6-(4-(4-methylpiperazin-1-yl)phenyl)furo[3,2-*b*]pyridin-3-yl)phenyl)(phenyl)methanone | B | B | |
| Preparative Example 135: *tert*-butyl (3-(6-(4-(4-methylpiperazin-1-yl)phenyl)furo[3,2-*b*]pyridin-3-yl)benzyl)carbamate | B | B | |
| Preparative Example 137: *N*-(3-(6-(4-(4-methylpiperazin-1-yl)phenyl)furo[3,2-*b*]pyridin-3-yl)benzyl)acetamide | B | A | |
| Preparative Example 141: *N*-ethyl-1-(3-(6-(4-(4-methylpiperazin-1-yl)phenyl)furo[3,2-*b*]pyridin-3-yl)phenyl)methanesulfonamide | C | C | |
| Preparative Example 145: *N,N*-dimethyl-1-(3-(6-(4-(4-methylpiperazin-1-yl)phenyl)furo[3,2-*b*]pyridin-3-yl)phenyl)methane-sulfonamide | C | B | |
| Preparative Example 147: 6-(4-(4-methylpiperazin-1-yl)phenyl)-3-(3-(methylthio)phenyl)furo[3,2-*b*]pyridine | B | B | |
| Preparative Example 149: 6-phenyl-3-(thiophen-3-yl)furo[3,2-*b*]pyridine | | C | |
| Preparative Example 152: *N*-ethyl-2-(3-(6-(4-(piperazin-1-yl)phenyl)furo[3,2-*b*]pyridin-3-yl)phenyl)acetamide | A | A | |
| Preparative Example 154: 2-(3-(6-(4-(4-methylpiperazin-1-yl)phenyl)furo[3,2-*b*]pyridin-3-yl)phenyl)acetamide | B | A | C |
| Preparative Example 156: 1-(4-(6-(4-(4-methylpiperazin-1-yl)phenyl)furo[3,2-*b*]pyridin-3-yl)pyridin-2-yl)ethan-1-one | C | A | |
| Preparative Example 158: 3-([1,1'-biphenyl]-3-yl)-6-(4-(4-methylpiperazin-1-yl)phenyl)furo[3,2-*b*]pyridine | B | C | |
| Preparative Example 161: 3-([1,1'-biphenyl]-2-yl)-6-(4-(piperazin-1-yl)phenyl)furo[3,2-*b*]pyridine | B | C | |
| Preparative Example 164: 3-(2-(cyclobutylthio)phenyl)-6-(4-(piperazin-1-yl)phenyl)furo[3,2-*b*]pyridine | B | B | C |
| Preparative Example 167: butyl 3-(6-(4-(piperazin-1-yl)phenyl)furo[3,2-*b*]pyridin-3-yl)benzoate | B | A | C |
| Preparative Example 170: 3-(3-fluorophenyl)-6-(4-(piperazin-1-yl)phenyl)furo[3,2-*b*]pyridine | B | A | C |
| Preparative Example 171: 6-(4-(2-(piperidin-1-yl)ethoxy)phenyl)-3-(pyridin-4-yl)furo[3,2-*b*]pyridine | B | A | C |
| Preparative Example 172: 6-(4-(4-methylpiperazin-1-yl)phenyl)-3-phenylfuro[3,2-*b*]pyridine | B | A | C |
| Preparative Example 173: *N*-methyl-*N*-(3-(6-(4-(4-methylpiperazin-1-yl)phenyl)furo[3,2-*b*]pyridin-3-yl)phenyl)acetamide | C | C | |
| Preparative Example 175: 6-(4-(4-methylpiperazin-1-yl)phenyl)-3-(3-(methylsulfonyl)phenyl)furo[3,2-*b*]pyridine | B | B | |
| Preparative Example 178: 6-(4-(4-methylpiperazin-1-yl)phenyl)-3-(3-(perfluoroethyl)phenyl)furo[3,2-*b*]pyridine | | | |
| Preparative Example 179: 6-benzyl-3-phenylfuro[3,2-*b*]pyridine | | | |
| Preparative Example 181: 6-(4-(4-methylpiperazin-1-yl)phenyl)-3-(3-(methylsulfinyl)phenyl)furo[3,2-*b*]pyridine | B | A | C |
| Preparative Example 183: 1-(3-(6-phenylfuro[3,2-*b*]pyridin-3-yl)phenyl)urea | | A | |
| Preparative Example 186: 3-benzyl-6-(4-(4-methylpiperazin-1-yl)phenyl)furo[3,2-*b*]pyridine | C | B | |
| Preparative Example 188: 7-methyl-6-(4-(4-methylpiperazin-1-yl)phenyl)-3-phenylfuro[3,2-*b*]pyridine | B | B | |

## Claims

1. Compound of general formula I 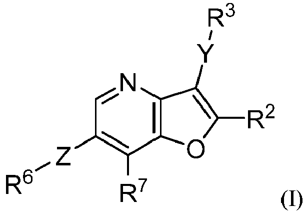 wherein:
R² is selected from H, C1-C4 alkyl and CF₃;
Y is selected from bond, O, S, SO₂, NR⁸ and CR⁸R⁸;
R³ is selected from the group consisting of:
- C6-C14 aryl,
- 3-10-membered heteroaryl comprising at least one heteroatom selected from S, O, N; provided that the heteroaryl is not unsubstituted quinoline or unsubstituted isoquinoline;
wherein each of the listed substituents can optionally be substituted by at least one substituent selected independently from C1-C4 alkyl, C6-C10 aryl, 3-7-membered heteroaryl comprising at least one heteroatom selected from S, O, N, 3-7-membered cycloheteroalkyl comprising at least one heteroatom selected from S, O, N, halogen, OH, HO-C1-C4 alkyl, O(C1-C4 alkyl), O(C3-C7 cycloalkyl), O(C1-C4 halogenalkyl), (C1-C4 alkyl)-O-C1-C4 alkyl, O(C5-C6 aryl or 5-6-membered heteroaryl), SH, S(C1-C4 alkyl), S(C3-C7 cycloalkyl), S(C1-C4 halogenalkyl), S(C5-C6 aryl or 5-6-membered heteroaryl), SO(C1-C4 alkyl), SO₂(C1-C4 alkyl), CF₃, C₂F₅, OCF₃, OC₂F₅, amino (NH₂), NH₂-(C1-C4 alkyl)-, HCO-NH-(C1-C4 alkyl)-, NO₂, CN, N₃, C1-C4 alkylamino, di(C1-C4 alkyl)amino, (C5-C6 aryl or 5-6-membered heteroaryl)amino, di(C5-C6 aryl or heteroaryl)amino, (C1-C4 alkyl)-NH-C1-C4 alkyl, (C1-C4 alkyl)₂-N-C1-C4-alkyl, =O, =S, =N-OH, -(C1-C4 alkylene)=N-OH, =N-O(C1-C4 alkyl), -(C1-C4 alkylene)=N-O(C1-C4 alkyl), -(C1-C4 alkylene)-CHO, -CHO, -COOH, -(C1-C4 alkylene)-COOH, -CONH₂, -(C1-C4 alkylene)-CONH₂, -COO(C1-C4 alkyl), -(C1-C4 alkylene)-COO(C1-C4 alkyl), -CO(C1-C4 alkyl), -(C1-C4 alkylene)-CO(C1-C4 alkyl), -CO(C5-C6 aryl or 5-6-membered heteroaryl), -(C1-C4 alkylene)-CO(C5-C6 aryl or 5-6-membered heteroaryl), (C1-C4 alkyl)-SO₂-, (C1-C4 alkyl)-SO₂-(C1-C4 alkylene)-, (C1-C4 alkyl)-SO-, (C1-C4 alkyl)-SO-(C1-C4 alkylene)-, (C1-C4 alkyl)-SO₂-NH-, (C1-C4 alkyl)-SO₂-NH-(C1-C4 alkylene)-, (C1-C4 alkyl)-SO₂-N(C1-C4 alkyl)-, (C1-C4 alkyl)-SO₂-N(C1-C4 alkyl)-(C1-C4 alkylene)-, (C1-C4 alkyl)-O-CO-, (C1-C4 alkyl)-O-CO-(C1-C4 alkylene)-, (C1-C4 alkyl)-NH-CO-, (C1-C4 alkyl)-NH-CO-(C1-C4 alkylene)-, (C6-C10 aryl)-NH-CO, (C6-C10 aryl)-NH-CO-(C1-C4 alkylene)-, (C1-C4 alkyl)₂N-CO-, (C1-C4 alkyl)₂N-CO-(C1-C4 alkylene)-, NH₂-SO₂-, NH₂-SO₂-(C1-C4-alkylene)-, (C6-C10 aryl)-NH-SO₂-, (C6-C10 aryl)-NH-SO₂-(C1-C4 alkylene)-, (C1-C4 alkyl)-NH-SO₂-, (C1-C4 alkyl)-NH-SO₂-(C1-C4 alkylene)-, (C1-C4 alkyl)₂N-SO₂-, (C1-C4 alkyl)₂N-SO₂-(C1-C4 alkylene)-, (C1-C4 alkyl)-CO-NH-, (C1-C4 alkyl)-CO-NH-(C1-C4 alkylene)-, (C1-C4 alkyl)-CO-N(C1-C4 alkyl)-, (C1-C4 alkyl)-CO-N(C1-C4 alkyl)-(C1-C4 alkylene)-, (C1-C4 alkyl)-OCO-NH-, (C1-C4 alkyl)-OCO-NH-(C1-C4 alkylene)-, (C1-C4 alkyl)-OCO-N(C1-C4 alkyl)-, (C1-C4 alkyl)-OCO-N(C1-C4 alkyl)-(C1-C4 alkylene)-, (C1-C4 alkyl)-CO-NH-CO-, (C1-C4 alkyl)-CO-N(C1-C4 alkyl)-CO-, NH₂-CO-NH-, (C1-C4 alkyl)-NH-CO-NH-, (C1-C4 alkyl)₂N-CO-NH-, NH₂-CO-N(C1-C4 alkyl)-, (C1-C4 alkyl)-NH-CO-N(C1-C4 alkyl)-, (C1-C4 alkyl)₂N-CO-N(C1-C4 alkyl)-, NH₂-S(O)₂-NH-, (C1-C4 alkyl)-NH-S(O)₂-NH-, (C1-C4 alkyl)₂N-S(O)₂-NH-, NH₂-S(O)₂-N(C1-C4 alkyl)-, (C1-C4 alkyl)-NH-S(O)₂-N(C1-C4 alkyl)-, (C1-C4 alkyl)₂N-S(O)₂-N(C1-C4 alkyl)-, (C1-C4 alkyl)₂N-(C1-C4 alkylene)-CO-, (C1-C4 alkyl)₂N-(C1-C4 alkylene)-SO₂-, (C1-C4 alkyl)₂N-(C1-C4 alkylene)-SO₂-NH-, (C1-C4 alkyl)₂N-(C1-C4 alkylene)-NH-SO₂-;
Z is selected from bond, O, S, SO₂, NR⁸ and CR⁸R⁸;
R⁶ is selected from the group consisting of
- C6-C14 aryl,
- 3-10-membered heteroaryl comprising at least one heteroatom selected from S, O, N,
- C3-C8 cycloalkyl, preferably, C4-C7 cycloalkyl,
- 3-8-membered cycloheteroalkyl comprising 1-2 heteroatom(s) selected from S, O, N,
- C3-C8 cycloalkenyl,
- 3-8-membered cycloheteroalkenyl comprising 1-2 heteroatom(s) selected from S, O, N,
- (C3-C8)cycloalkyl-(C6-C14)aryl,
- (3-8-membered)cycloheteroalkyl-(C6-C14)aryl, wherein the cycloheteroalkyl comprises 1-2 heteroatom(s) selected from S, O, N,
- (C3-C8)cycloalkenyl-(C6-C14)aryl,
- (3-8-membered)cycloheteroalkenyl-(C6-C14)aryl, wherein the cycloheteroalkenyl comprises 1-2 heteroatom(s) selected from S, O, N,
- (C3-C8)cycloalkyl-(3-10-membered)heteroaryl, wherein the heteroaryl comprises at least one heteroatom selected from S, O, N,
- (3-8-membered)cycloheteroalkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (C3-C8)cycloalkenyl-(3-10-membered)heteroaryl, wherein the heteroaryl comprises at least one heteroatom selected from S, O, N
- (3-8-membered)cycloheteroalkenyl-(3-10-membered)heteroaryl, wherein the cycloheteroalkenyl comprises 1-2 heteroatom(s) selected from S, O, N, and wherein the heteroaryl comprises at least one heteroatom selected from S, O, N,
- (C3-C8)cycloalkyl-(C1-C4)alkyl-(C6-C14)aryl,
- (3-8-membered)cycloheteroalkyl-(C1-C4)alkyl-(C6-C14)aryl, wherein the cycloheteroalkyl comprises 1-2 heteroatom(s) selected from S, O, N,
- (C3-C8)cycloalkenyl-(C1-C4)alkyl-(C6-C14)aryl,
- (3-8-membered)cycloheteroalkenyl-(C1-C4)alkyl-(C6-C14)aryl, wherein the cycloheteroalkenyl comprises 1-2 heteroatom(s) selected from S, O, N,
- (C3-C8)cycloalkyl-(C1-C4)alkyl-(3-10-membered)heteroaryl, wherein the heteroaryl comprises at least one heteroatom selected from S, O, N,
- (3-8-membered)cycloheteroalkyl-(C1-C4)alkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (C3-C8)cycloalkenyl-(C1-C4)alkyl-(3-10-membered)heteroaryl, wherein the heteroaryl comprises at least one heteroatom selected from S, O, N
- (3-8-membered)cycloheteroalkenyl-(C1-C4)alkyl-(3-10-membered)heteroaryl, wherein the cycloheteroalkenyl comprises 1-2 heteroatom(s) selected from S, O, N, and wherein the heteroaryl comprises at least one heteroatom selected from S, O, N,
- (C3-C8)cycloalkyl-(1-4-membered)heteroalkyl-(C6-C14)aryl,
- (3-8-membered)cycloheteroalkyl-(1-4-membered)heteroalkyl-(C6-C14)aryl, wherein the cycloheteroalkyl comprises 1-2 heteroatom(s) selected from S, O, N,
- (C3-C8)cycloalkenyl-(1-4-membered)heteroalkyl-(C6-C14)aryl,
- (3-8-membered)cycloheteroalkenyl-(1-4-membered)heteroalkyl-(C6-C14)aryl, wherein the cycloheteroalkenyl comprises 1-2 heteroatom(s) selected from S, O, N,
- (C3-C8)cycloalkyl-(1-4-membered)heteroalkyl-(3-10-membered)heteroaryl, wherein the heteroaryl comprises at least one heteroatom selected from S, O, N,
- (3-8-membered)cycloheteroalkyl-(1-4-membered)heteroalkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (C3-C8)cycloalkenyl-(1-4-membered)heteroalkyl-(3-10-membered)heteroaryl, wherein the heteroaryl comprises at least one heteroatom selected from S, O, N
- (3-8-membered)cycloheteroalkenyl-(1-4-membered)heteroalkyl-(3-10-membered)hetero aryl, wherein the cycloheteroalkenyl comprises 1-2 heteroatom(s) selected from S, O, N, and wherein the heteroaryl comprises at least one heteroatom selected from S, O, N,
- (5-10 membered)heteroalkyl-C6-C14 aryl, wherein the heteroalkyl comprises 1-2 heteroatom(s) selected from S, O, N,
- (5-10 membered)heteroalkyl-(3-10-membered)heteroaryl wherein the heteroalkyl comprises 1-2 heteroatom(s) selected from S, O, N, and wherein the heteroaryl comprises at least one heteroatom selected from S, O, N,
wherein each of the listed substituents can be unsubstituted or substituted by at least one substituent selected independently from C1-C4 alkyl, C6-C10 aryl, 3-7-membered heteroaryl comprising at least one heteroatom selected from S, O, N, 3-7-membered cycloheteroalkyl comprising at least one heteroatom selected from S, O, N, halogen, OH, HO-C1-C4 alkyl, O(C1-C4 alkyl), O(C3-C7 cycloalkyl), O(C1-C4 halogenalkyl), (C1-C4 alkyl)-O-C1-C4 alkyl, O(C5-C6 aryl or 5-6-membered heteroaryl), SH, S(C1-C4 alkyl), S(C3-C7 cycloalkyl), S(C1-C4 halogenalkyl), S(C5-C6 aryl or 5-6-membered heteroaryl), SO(C1-C4 alkyl), SO₂(C1-C4 alkyl), CF₃, C₂F₅, OCF₃, OC₂F₅, amino (NH₂), NH₂-(C1-C4 alkyl)-, HCO-NH-(C1-C4 alkyl)-, NO₂, CN, N₃, C1-C4 alkylamino, di(C1-C4 alkyl)amino, (C5-C6 aryl or 5-6-membered heteroaryl)amino, di(C5-C6 aryl or heteroaryl)amino, (C1-C4 alkyl)-NH-C1-C4 alkyl, (C1-C4 alkyl)₂-N-C1-C4-alkyl, =O, =S, =N-OH, -(C1-C4 alkylene)=N-OH, =N-O(C1-C4 alkyl), -(C1-C4 alkylene)=N-O(C1-C4 alkyl), -(C1-C4 alkylene)-CHO, -CHO, -COOH, -(C1-C4 alkylene)-COOH, -CONH₂, -(C1-C4 alkylene)-CONH₂, -COO(C1-C4 alkyl), -(C1-C4 alkylene)-COO(C1-C4 alkyl), -CO(C1-C4 alkyl), -(C1-C4 alkylene)-CO(C1-C4 alkyl), -CO(C5-C6 aryl or 5-6-membered heteroaryl), -(C1-C4 alkylene)-CO(C5-C6 aryl or 5-6-membered heteroaryl), (C1-C4 alkyl)-SO₂-, (C1-C4 alkyl)-SO₂-(C1-C4 alkylene)-, (C1-C4 alkyl)-SO-, (C1-C4 alkyl)-SO-(C1-C4 alkylene)-, (C1-C4 alkyl)-SO₂-NH-, (C1-C4 alkyl)-SO₂-NH-(C1-C4 alkylene)-, (C1-C4 alkyl)-SO₂-N(C1-C4 alkyl)-, (C1-C4 alkyl)-SO₂-N(C1-C4 alkyl)-(C1-C4 alkylene)-, (C1-C4 alkyl)-O-CO-, (C1-C4 alkyl)-O-CO-(C1-C4 alkylene)-, (C1-C4 alkyl)-NH-CO-, (C1-C4 alkyl)-NH-CO-(C1-C4 alkylene)-, (C6-C10 aryl)-NH-CO, (C6-C10 aryl)-NH-CO-(C1-C4 alkylene)-, (C1-C4 alkyl)₂N-CO-, (C1-C4 alkyl)₂N-CO-(C1-C4 alkylene)-, NH₂-SO₂-, NH₂-SO₂-(C1-C4-alkylene)-, (C6-C10 aryl)-NH-SO₂-, (C6-C10 aryl)-NH-SO₂-(C1-C4 alkylene)-, (C1-C4 alkyl)-NH-SO₂-, (C1-C4 alkyl)-NH-SO₂-(C1-C4 alkylene)-, (C1-C4 alkyl)₂N-SO₂-, (C1-C4 alkyl)₂N-SO₂-(C1-C4 alkylene)-, (C1-C4 alkyl)-CO-NH-, (C1-C4 alkyl)-CO-NH-(C1-C4 alkylene)-, (C1-C4 alkyl)-CO-N(C1-C4 alkyl)-, (C1-C4 alkyl)-CO-N(C1-C4 alkyl)-(C1-C4 alkylene)-, (C1-C4 alkyl)-OCO-NH-, (C1-C4 alkyl)-OCO-NH-(C1-C4 alkylene)-, (C1-C4 alkyl)-OCO-N(C1-C4 alkyl)-, (C1-C4 alkyl)-OCO-N(C1-C4 alkyl)-(C1-C4 alkylene)-, (C1-C4 alkyl)-CO-NH-CO-, (C1-C4 alkyl)-CO-N(C1-C4 alkyl)-CO-, NH₂-CO-NH-, (C1-C4 alkyl)-NH-CO-NH-, (C1-C4 alkyl)₂N-CO-NH-, NH₂-CO-N(C1-C4 alkyl)-, (C1-C4 alkyl)-NH-CO-N(C1-C4 alkyl)-, (C1-C4 alkyl)₂N-CO-N(C1-C4 alkyl)-, NH₂-S(O)₂-NH-, (C1-C4 alkyl)-NH-S(O)₂-NH-, (C1-C4 alkyl)₂N-S(O)₂-NH-, NH₂-S(O)₂-N(C1-C4 alkyl)-, (C1-C4 alkyl)-NH-S(O)₂-N(C1-C4 alkyl)-, (C1-C4 alkyl)₂N-S(O)₂-N(C1-C4 alkyl)-, (C1-C4 alkyl)₂N-(C1-C4 alkylene)-CO-, (C1-C4 alkyl)₂N-(C1-C4 alkylene)-SO₂-, (C1-C4 alkyl)₂N-(C1-C4 alkylene)-SO₂-NH-, (C1-C4 alkyl)₂N-(C1-C4 alkylene)-NH-SO₂-;
R⁷ is selected from H, C1-C4 alkyl and CF₃;
R⁸ is independently selected from H and C1-C4 alkyl.

2. Compound of general formula I according to claim 1, wherein
R² is selected from H, methyl, ethyl, propyl and isopropyl; and/or
R⁷ is selected from H, methyl, ethyl, propyl and isopropyl.

3. Compound of general formula I according to claim 1 or 2, wherein
Y is selected from bond, NR⁸ or CR⁸R⁸; and/or
Z is selected from bond, NR⁸ or CR⁸R⁸;
more preferably Y is bond and/or Z is bond.

4. Compound of general formula I according to any one of claims 1 to 3, wherein R³ is selected from phenyl and 5-6 membered heteroaryls containing one or two heteroatoms selected from N, O, S; wherein the phenyl or 5-6 membered heteroaryl is unsubstituted or substituted.

5. Compound of general formula I according to any one of claims 1 to 4, wherein R⁶ is selected from the group consisting of
- C6-C14 aryl,
- 3-10-membered heteroaryl comprising at least one heteroatom selected from S, O, N,
- (C3-C8)cycloalkyl-(C6-C14)aryl,
- (3-8-membered)cycloheteroalkyl-(C6-C14)aryl, wherein the cycloheteroalkyl comprises 1-2 heteroatom(s) selected from S, O, N,
- (C3-C8)cycloalkyl-(3-10-membered)heteroaryl, wherein the heteroaryl comprises at least one heteroatom selected from S, O, N,
- (3-8-membered)cycloheteroalkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (C3-C8)cycloalkyl-(C1-C4)alkyl-(C6-C14)aryl,
- (3-8-membered)cycloheteroalkyl-(C1-C4)alkyl-(C6-C14)aryl, wherein the cycloheteroalkyl comprises 1-2 heteroatom(s) selected from S, O, N,
- (C3-C8)cycloalkyl-(C1-C4)alkyl-(3-10-membered)heteroaryl, wherein the heteroaryl comprises at least one heteroatom selected from S, O, N,
- (3-8-membered)cycloheteroalkyl-(C1-C4)alkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (C3-C8)cycloalkyl-(1-4-membered)heteroalkyl-(C6-C14)aryl,
- (3-8-membered)cycloheteroalkyl-(1-4-membered)heteroalkyl-(C6-C14)aryl, wherein the cycloheteroalkyl comprises 1-2 heteroatom(s) selected from S, O, N,
- (C3-C8)cycloalkyl-(1-4-membered)heteroalkyl-(3-10-membered)heteroaryl, wherein the heteroaryl comprises at least one heteroatom selected from S, O, N,
- (3-8-membered)cycloheteroalkyl-(1-4-membered)heteroalkyl-(3-10-membered)heteroaryl, wherein the cycloheteroalkyl comprises 1-2 heteroatom(s) selected from S, O, N, and wherein the heteroaryl comprises at least one heteroatom selected from S, O, N,
- (5-10 membered)heteroalkyl-C6-C14 aryl, wherein the heteroalkyl comprises 1-2 heteroatom(s) selected from S, O, N,
- (5-10 membered)heteroalkyl-(3-10-membered)heteroaryl wherein the heteroalkyl comprises 1-2 heteroatom(s) selected from S, O, N, and wherein the heteroaryl comprises at least one heteroatom selected from S, O, N,
wherein each of the listed substituents can be unsubstituted or substituted.

6. Compound according to any one of claims 1 to 5, wherein R⁶ is selected from the group consisting of
- phenyl,
- 5-6-membered heteroaryl comprising at least one heteroatom selected from S, O, N,
- (6-membered)cycloheteroalkyl-phenyl, wherein the cycloheteroalkyl comprises 1-2 heteroatom(s) selected from O, N and is bound to the phenyl via an N-atom,
- (6-membered)cycloheteroalkyl-(5-6-membered)heteroaryl, wherein the cycloheteroalkyl comprises 1-2 heteroatom(s) selected from O, N and is bound to the heteroaryl via an N-atom, and wherein the heteroaryl comprises at least one heteroatom selected from S, O, N,
- (6-membered)cycloheteroalkyl-(1-4-membered)heteroalkyl-phenyl, wherein the cycloheteroalkyl comprises 1-2 heteroatom(s) selected from O, N and is bound to the heteroalkyl via an N-atom,
- (6-membered)cycloheteroalkyl-(1-4-membered)heteroalkyl-(5-6-membered)heteroaryl, wherein the cycloheteroalkyl comprises 1-2 heteroatom(s) selected from O, N and is bound to the heteroalkyl via an N-atom, and wherein the heteroaryl comprises at least one heteroatom selected from S, O, N,
- (5-10 membered)heteroalkyl-phenyl, wherein the heteroalkyl comprises 1-2 heteroatom(s) selected from O, N and is bound to the phenyl via an N-atom,
- (5-10 membered)heteroalkyl-(5-6-membered)heteroaryl wherein the heteroalkyl comprises 1-2 heteroatom(s) selected from S, O, N, and wherein the heteroaryl comprises at least one heteroatom selected from O, N and is bound to the heteroalkyl via an N-atom,
wherein each of the listed substituents can be unsubstituted or substituted.

7. Compound of general formula I according to any one of claims 1 to 6, wherein the substituents listed in R⁶ are unsubstituted or further substituted by at least one substituent, preferably by one substituent, wherein the at least one substituent is selected independently from C1-C4 alkyl, halogen, OH, HO-C1-C4 alkyl, O(C1-C4 alkyl), O(C5-C6 aryl or heteroaryl), SH, S(C1-C4 alkyl), S(C5-C6 aryl or heteroaryl), CF₃, C₂F₅, OCF₃, OC₂F₅, amino (NH₂), C1-C4 alkylamino, di(C1-C4 alkyl)amino. More preferably, the at least one substituent is selected from C1-C4 alkyl.

8. Compound of general formula I according to claim 1, which is selected from the group consisting of the following compounds:

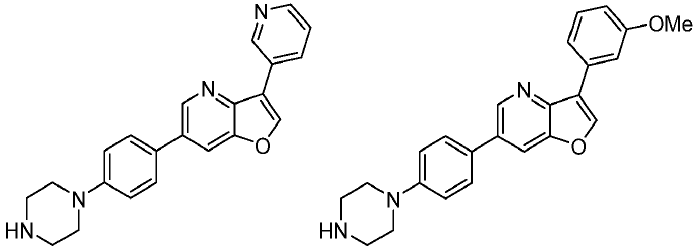

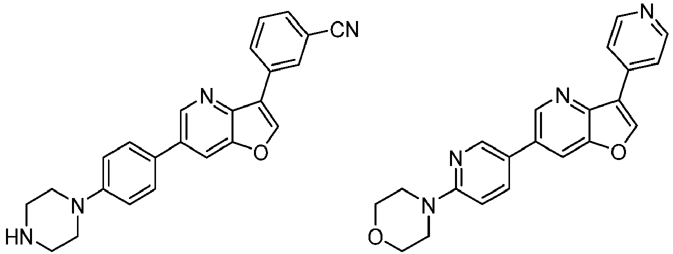

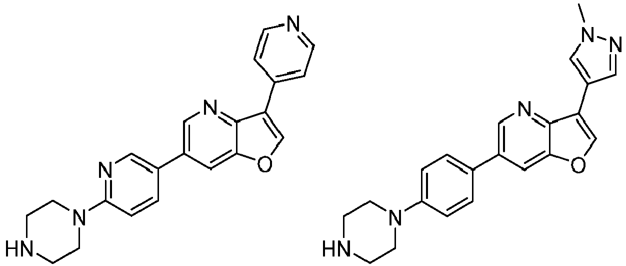

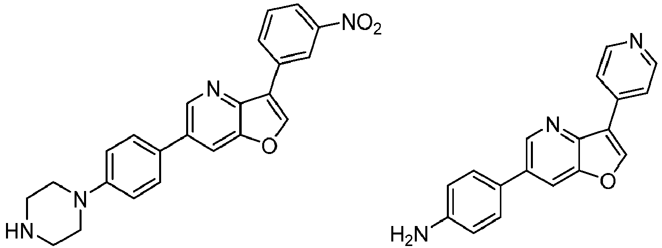

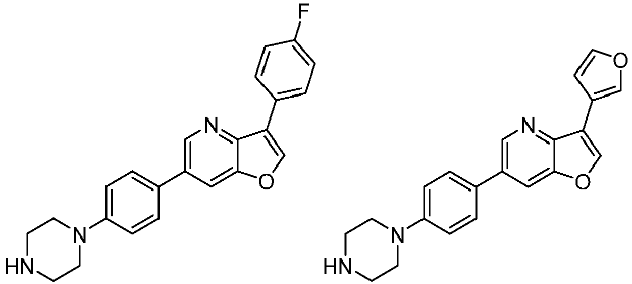

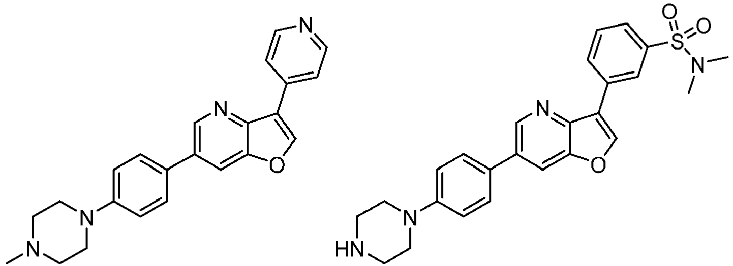

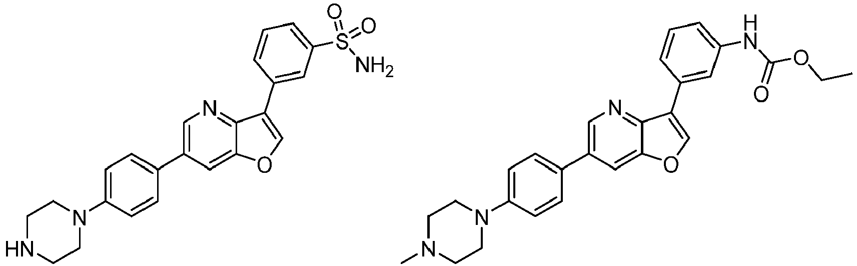

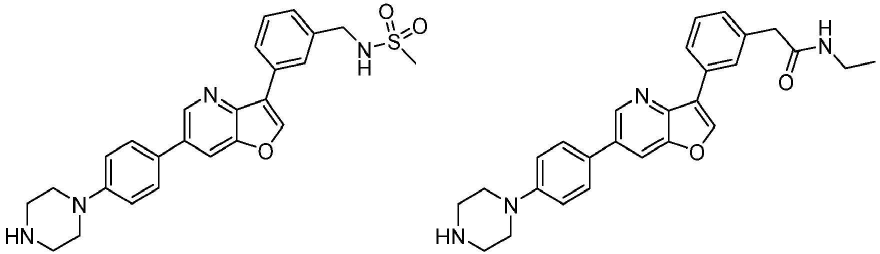

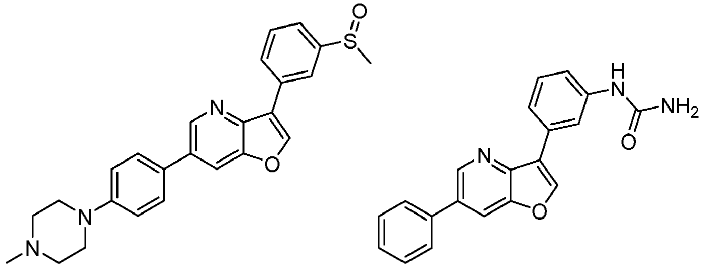

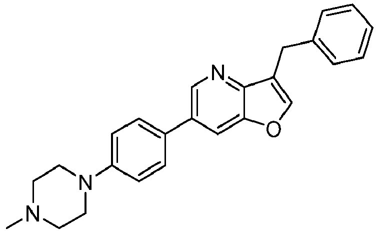

9. Compound of general formula I according to any one of claims 1 to 8 for use as a medicament.

10. Compound of general formula I according to any one of claims 1 to 8 for use in the treatment of FLT3-related, DDR-related and MAP4K-related diseases.

11. Compound of general formula I for use according to claim 9 or 10, wherein the compound is for use in the treatment of cancer, inflammatory disorders, autoimmune disorders, neurodegenerative disorders or metabolic disorders.

12. Compound of general formula I for use according to claim 9 or 10, wherein the compound is for use in the treatment of leukemia, rheumatoid arthritis, autoimmune hepatitis, peripheral neuropathic pain, non-small-cell lung carcinoma (NSCLC), ovarian cancer, glioblastoma, breast cancer, lung cancer, lung adenocarcinoma, pancreatic cancer, prostate cancer, brain cancer, colorectal cancer, liver cancer, hepatocellular carcinoma, squamous cell carcinoma, periodontitis, pulmonary fibrosis, obesity, insulin resistance, obesity-induced hyperinsulinemia, atherosclerosis or Alzheimer's disease.

13. A pharmaceutical preparation comprising at least one compound of formula I according to any one of claims 1 to 8, and at least one pharmaceutically acceptable auxiliary substance selected from pharmaceutically acceptable solvents, fillers and binders.
